# EUROPEAN PATENT SPECIFICATION

(11) **EP 1 237 888 B1**
(45) Date of publication and mention of the grant of the patent: **13.09.2006**
(21) Application number: 00984282.4
(22) Date of filing: 13.12.2000
(51) Int. Cl.: C07D 413/12, C07D 413/14, C07D 403/14, C07D 487/04, C07D 261/16, C07D 401/12, C07D 417/12, A61K 31/42, A61P 9/12

(54) **BIPHENYL SULFONAMIDES AS DUAL ANGIOTENSIN ENDOTHELIN RECEPTOR ANTAGONISTS**
BIPHENYL - SULFONAMIDE ALS DUALE ANGIOTENSIN - ENDOTHELIN - REZEPTOR - ANTAGONISTEN
BIPHENYL SULFONAMIDES UTILISES COMME DOUBLES ANTAGONISTES DES RECEPTEURS DE L'ANGIOTENSINE ET DE L'ENDOTHELINE

(30) Priority: 15.12.1999 US 464037; 11.01.2000 US 481197; 25.02.2000 US 513779; 26.06.2000 US 604322; 22.08.2000 US 643640
(43) Date of publication of application: 11.09.2002
(73) Proprietor: Bristol-Myers Squibb Company, Princeton, NJ 08543-4000 (US)
(72) Inventor: MURUGESAN, Natesan, Princeton Junction, NJ 08550 (US); TELLEW, John, E., Pennington, NJ 08534 (US); MACOR, John, E., Guilford, CT 06437 (US); GU, Zhengxiang, Princeton, NJ 08540 (US)
(74) Representative: Vossius & Partner
(86) International application number: PCT/US2000/033730
(87) International publication number: WO 2001/044239

(56) References cited:
- WO-A-98/04260
- WO-A-98/33780
- GB-A- 2 264 710
- US-A- 5 260 328
- US-A- 5 399 578
- US-A- 5 514 696
- US-A- 5 554 625
- US-A- 5 612 359
- US-A- 5 760 038
- US-A- 5 780 473
- US-A- 5 846 990

## Description

The present invention relates to biphenyl sulfonamide compounds which are combined angiotensin and endothelin receptor antagonists, to the use of such compounds for the preparation of pharmaceutical compositions for treatment of conditions such as hypertension and other diseases, and to pharmaceutical compositions containing such compounds.

The present invention provides biphenyl sulfonamide compounds of the following formula I, enantiomers (including atropisomers), diastereomers, salts and metabolites thereof: 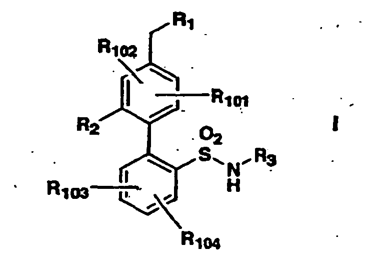 wherein:
R₁ is 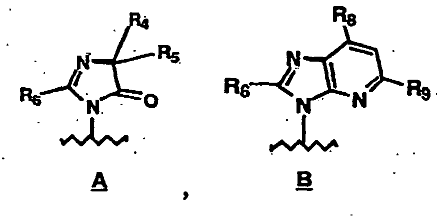 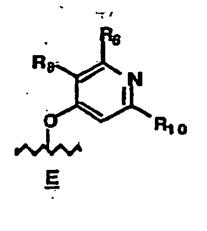 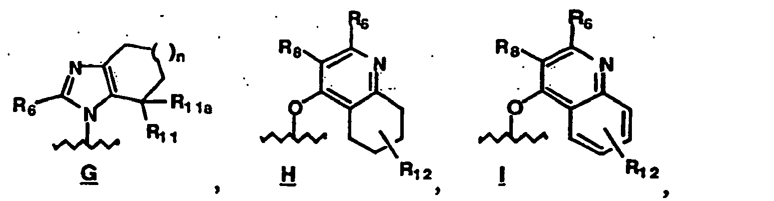 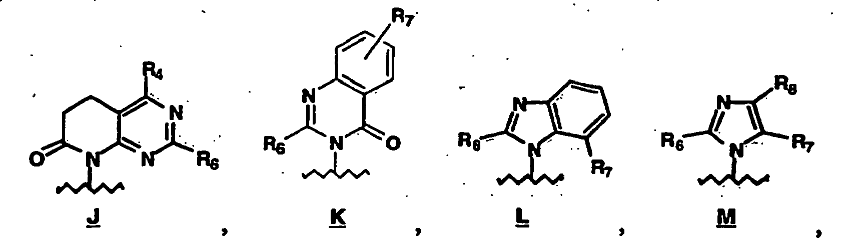 or 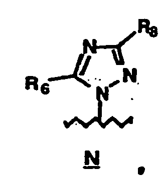
R₂ is hydrogen, halogen, -CHO, alkyl, haloalkyl, (cycloalkyl)alkyl, alkenyl, alkynyl, alkoxyalkyl, haloalkoxyalkyl, alkoxy, aryloxy alkoxyalkoxy, cyano, hydroxy, hydroxyalkyl, nitro, -CH(OR₁₃)(OR₁₄), -(CH₂)_{w}Y; with the proviso that when R₁ is B, R₂ is not hydrogen, halogen, alkyl, haloalkyl, alkoxy, hydroxyalkyl, nitro, -(CH₂)_{w}NR₁₉R₂₀ or -NHSO₂R₂₂;
R₃ is heteroaryl;
R₄ and R₅ are each independently alkyl, hydroxyalkyl, cycloalkyl, hydroxy substituted cycloalkyl, alkoxyalkyl, or hydroxy substituted alkoxyalkyl, or R₄ and R₅ together form a cyclobutyl, cyclopentyl, cyclohexyl, tetrahydrofuranyl or tetrahydropyranyl ring which may be optionally substituted with one or more hydroxy groups;
R₆ is alkyl, hydroxyalkyl, haloalkyl, hydroxy substituted haloalkyl, cycloalkyl, hydroxy substituted cycloalkyl, (cycloalkyl)alkyl, hydroxy substituted (cycloalkyl)(alkyl), aralkyl, alkoxy, hydroxy substituted alkoxy, alkoxyalkyl, hydroxy substituted alkoxyalkyl, or -NR₁₆R₁₇;
R₇ is -(C₂)_{w}-CO₂R₁₅, -(CH₂)_{w}-(C=O)NR₁₆R₁₇, -(CH₂)_{w}-NR₁₅(C=O)NR₁₆R₁₇, -(CH₂)_{w}-CH₂OH, -(CH₂)_{w}-(C=O)R₁₅, tetrazolyl, oxadiazolyl or triazolyl wherein said tetrazolyl, oxadiazolyl or triazolyl may optionally be substituted with hydrogen, alkyl, hydroxy or halogen;
R₈, R₉, R₉ₐ, R₁₀ and R₁₂ are each independently hydrogen, halogen, alkyl, hydroxyalkyl, cycloalkyl, (cycloalkyl)alkyl, aryl, heteroaryl, arylalkyl, alkylthioalkyl, alkoxy or alkoxyalkyl, or R₉ and R₉ₐ together with the carbon atom to which they are bonded form a cycloalkyl ring;
R₁₁ and R₁₁ₐ are each independently hydrogen, alkoxy, or together form a carbonyl;
R₁₃ and R₁₄ are alkyl or together form a five to six-membered ring;
R₁₅, R₁₆ and R₁₇ are independently hydrogen, alkyl, hydroxyalkyl, cycloalkyl, (cycloalkyl)alkyl, alkoxyalkyl, aralkyl, heterocycloalkyl, aryl, heteroaryl or (CH₂)_{w}Q, or R₁₆ and R₁₇ may together form a four to six-membered heterocyclic ring;
n is 1 or 2;
w is 0, 1, or 2;
Y is heteroaryl, -COOH, -COOR₁₈, -CONR₁₉R₂₀, -NR₁₉R₂₀, -NR₁₉-OR₂₀,-NR₂₁(C=O)R₂₂, -NR₂₁(C=O)NR₁₉R₂₀, -N(R₁₉)-(alk)-NR₂₁(C=O)R₂₂, -NR₂₁(C=O)OR₁₈, -NR₂₁SO₂R₂₂, -SO₂R₂₂, Q, R or S;
Q is 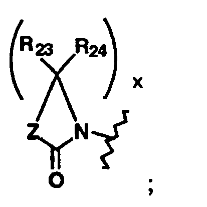
R is 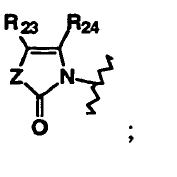
S is 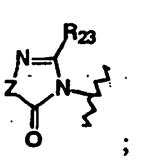
R₁₈, R₁₉, R₂₀, R₂₁ and R₂₂ are each independently hydrogen, alkyl, haloalkyl, alkoxyalkyl, cycloalkyl, alkenyl, alkynyl, aryl, aralkyl, heteroaryl, or R₁₉ and R₂₀ may together form a four to seven-membered heterocyclic ring;
R₂₃ and R₂₄ are each independently hydrogen, alkyl or cycloalkyl, or may together form a three to seven membered cycloalkyl ring;
Z is oxygen, 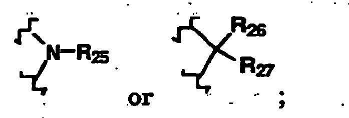
x is 2, 3 or 4;
R₂₅, R₂₆ and R₂₇ are each independently hydrogen, alkyl or cycloalkyl, or R₂₆ and R₂₇ may together form a three to seven-membered cycloalkyl ring;
R₁₀₁, R₁₀₂, R₁₀₃, and R₁₀₄ are each independently hydrogen, halogen, -CHO, alkyl, haloalkyl, (cycloalkyl)alkyl, alkenyl, alkynyl, alkoxyalkyl, haloalkoxyalkyl, alkoxy, alkoxyalkoxy, cyano, hydroxy, hydroxyalkyl, nitro, -CH(OR₁₃)(OR₁₄), or -(CH₂)_{w}Y; provided that at least one of R₁₀₁, R₁₀₂, R₁₀₃ and R₁₀₄ is other than hydrogen
wherein said rings; aryl alone or as part of another group; or heteroaryl alone or as part of another group may each optionally be substituted by one or more hydrogen, halogen, cyano, alkyl, hydroxyalkyl, alkoxy, nitro or trifluoromethyl groups.

The compounds of the formula I and salts thereof may be used as combined endothelin and angiotensin receptor antagonists.

Compounds of the formula I and salts thereof wherein one or more, and especially all, of R₁, R₂, R₃, R₄, R₅, R₆, R₇, R₈, R₉, R₁₀, R₁₁, R₁₁ₐ, R₁₂, R₁₅, R₁₆, R₁₇, R₁₈, R₁₉, R₂₀, R₂₁, R₂₂, R₂₃, R₂₄, R₂₅, R₂₆, R₂₇, R₁₀₁, R₁₀₂, R₁₀₃, R₁₀₄, n, w, Y, Q, Z, and x are selected from the following definitions, are preferred compounds of the present invention:
R₁ is 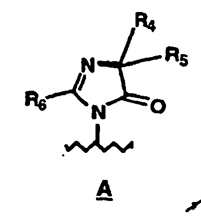 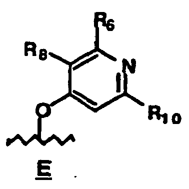 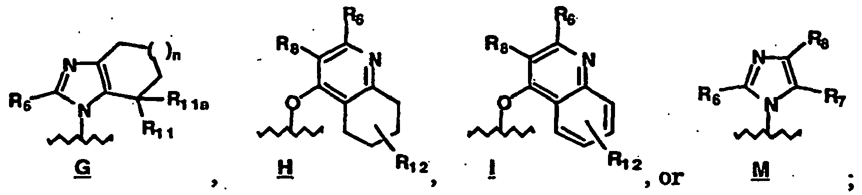
R₂ is alkyl, haloalkyl, (cycloalkyl)alkyl, alkoxyalkyl, haloalkoxyalkyl, alkoxy, alkoxyalkoxy, hydroxyalkyl, or -(CH₂)_{w}Y, or when R₁ is D, R₂ is hydrogen, alkyl, haloalkyl, (cycloalkyl)alkyl, alkoxyalkyl, haloalkoxyalkyl, alkoxy, alkoxyalkoxy, hydroxyalkyl, or (CH₂)_{w}Y;
R₃ is isoxazolyl, pyridizinyl, pyrazinyl or pyrimidinyl, each optionally substituted with one to three of the following substituents: hydrogen, halogen, cyano, alkyl, alkoxy, trifluoromethyl or nitro;
R₄ and R₅ are each independently alkyl, cycloalkyl, or R₄ and R₅ together form a cyclobutyl, cyclopentyl or cyclohexyl ring;
R₆ is alkyl, haloalkyl, cycloalkyl or alkoxy;
R₇ is -CO₂R₁₅, -(C=O)NR₁₆R₁₇ or -CH₂OH;
R₈, R₉, R₁₀ and R₁₂ are each independently hydrogen, halogen, alkyl, cycloalkyl, alkoxy or alkoxyalkyl;
R₁₁ and R₁₁ₐ are each independently hydrogen, alkoxy, or together form a carbonyl;
R₁₅, R₁₆ and R₁₇ are independently hydrogen, alkyl or cycloalkyl or R₁₆ and R₁₇ may together form a four to six-membered heterocyclic ring;
n is 1 or 2;
w is 0, 1, or 2;
Y is -COOR₁₈, -NR₂₁(C=O)R₂₂, -NR₂₁,(C=O)NR₁₉R₂₀, -NR₂₁(C=O)OR₁₈, -NR₂₁SO₂R₂₂, -SO₂R₂₂ or Q ;
Q is 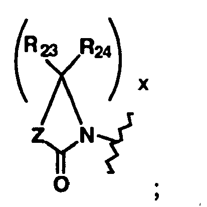
R₁₈, R₁₉, R₂₀, R₂₁ and R₂₂ are each independently hydrogen, alkyl, cycloalkyl, or R₁₉ and R₂₀ may together form a four to seven-membered heterocyclic ring;
R₂₃ and R₂₄ are each independently hydrogen, alkyl or cycloalkyl, or may together form a three to seven membered cycloalkyl ring;
Z is oxygen, 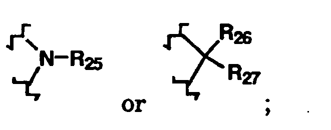
x is 2, 3 or 4;
R₂₆, R₂₆ and R₂₇ are each independently hydrogen, alkyl or cycloalkyl, or R₂₆ and R₂₇ may together form a three to seven-membered cycloalkyl ring;
R₁₀₁, R₁₀₂, R₁₀₃, and R₁₀₄ are each independently hydrogen, halogen, alkoxy or alkyl.

Compounds of the formula I and salts thereof wherein one or more, and especially all, of R₁, R₂, R₃, R₄, R₅, R₆, R₇, R₈, R₉, R₁₀, R₁₁, R₁₁ₐ, R₁₅, R₁₆, R₁₇, R₁₈, R₁₉, R₂₀, R₂₁, R₂₂, R₂₃, R₂₄, R₂₅, R₂₆, R₂₇, R₁₀₁, R₁₀₂, R₁₀₃, R₁₀₄, n, w, Y, Q, Z, and x are selected from the following definitions, are more preferred compounds of the present invention:
R₁ is 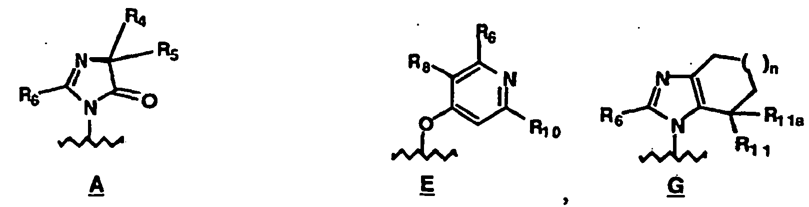 or 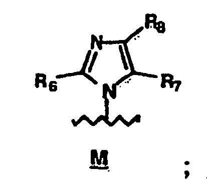
R₂ is alkyl, haloalkyl, (cycloalkyl)alkyl, alkoxyalkyl, haloalkoxyalkyl, alkoxy, hydroxyalkyl, or -(CH₂)_{w}Y; or when R₁ is D, R₂ is hydrogen, alkyl, haloalkyl, (cycloalkyl)alkyl, alkoxyalkyl, haloalkoxyalkyl, alkoxy, alkoxyalkoxy, hydroxyalkyl, or -(CH₂)_{w}Y;
R₈ is isoxazolyl, optionally substituted with one or two of the following substituents: hydrogen, halogen, cyano, alkyl, alkoxy, trifluoromethyl or nitro;
R₄ and R₅ are each independently alkyl, cycloalkyl, or R₄ and R₅ together form a cyclobutyl, cyclopentyl or cyclohexyl ring;
R₆ is alkyl, haloalkyl, cycloalkyl or alkoxy;
R₇ is -CO₂R₁₅ or -(C=O)NR₁₆R₁₇;
R₈, R₉ and R₁₀ are each independently hydrogen, halogen, alkyl, cycloalkyl alkoxy or alkoxyalkyl;
R₁₁ and R₁₁ₐ together form a carbonyl;
R₁₅, R₁₆ and R₁₇ are independently hydrogen, alkyl, or cycloalkyl or R₁₆ and R₁₇ may together form a four to six-membered heterocyclic ring;
n is 2;
w is 0, 1, or 2;
Y is -NR₂₁C=O)R₂₂, -NR₂₁(C=O)NR₁₉R₂₀, -NR₂₁(C=O)OR₁₈,
   -NR₂₁SO₂R₂₂, -SO₂R₂₂ or Q;
Q is 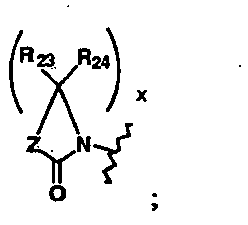
R₁₈, R₁₉, R₂₀, R₂₁ and R₂₂ are each independently hydrogen, alkyl, cycloalkyl, or R₁₉ and R₂₀ may together form a four to seven-membered heterocyclic ring;
R₂₃ and R₂₄ are each independently hydrogen, alkyl or cycloalkyl, or may together form a three to seven membered cycloalkyl ring;
Z is oxygen, 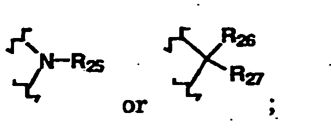
x is 2, 3 or 4;
R₂₅, R₂₆ and R₂₇ are each independently hydrogen, alkyl or cycloalkyl, or R₂₆ and R₂₇ may together form a three to seven-membered cycloalkyl ring;
R₁₀₁, R₁₀₂, R₁₀₃, and R₁₀₄ are each independently hydrogen, halogen, or alkyl provided that at least are of R₁₀₁, R₁₀₂, R₁₀₃ and R₁₀₄ is other than hydrogen

Compounds of the formula I and salts thereof wherein one or more, and especially all, of R₁, R₂, R₃, R₄, R₅, R₆, R₈, R₁₀, R₁₁, R₁₁ₐ, R₁₈, R₁₉, R₂₀, R₂₁, R₂₂, R₂₃, R₂₄, R₂₅, R₂₆, R₂₇, R₁₀₁, R₁₀₂, R₁₀₃, R₁₀₄, w, Y, Q, Z, and x are selected from the following definitions, are most preferred compounds of the present invention:
R₁ is 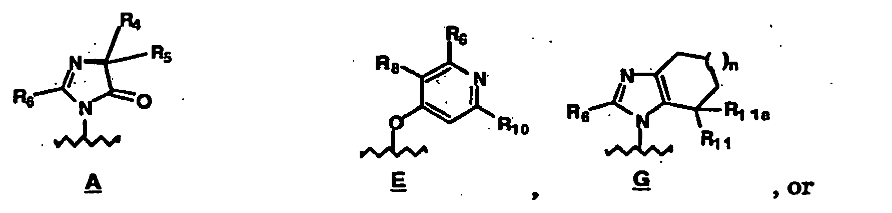 or 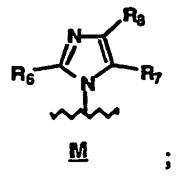
R₂ is alkyl, haloalkyl, (cycloalkyl)alkyl, alkoxyalkyl, haloalkoxyalkyl, alkoxy, alkoxyalkoxy, hydroxyalkyl, or -(CH₂)_{w} Y;
R₃ is isoxazol-5-yl or isoxazol-3-yl independently substituted with two of following substituents: alkyl or halogen;
R₄ and R₅ are each independently alkyl, cycloalkyl, or R₄ and R₅ together form a cyclobutyl, cyclopentyl or cyclohexyl ring;
R₆ is alkyl, halo alkyl, cycloalkyl or alkoxy;
R₇ is -CO₂R₁₅ or -(C=O)NR₁₆R₁₇ ;
R₈, R₉, and R₁₀ are independently H, alkyl, cycloalkyl, alkoxy or alkoxyalkyl;
n is 2;
w is 0,1, or 2;
Y is -NR₂₁(C=O)R₂₂, -NR₂₁(C=O)NR₁₉R₂₀, -NR₂₁(C=O)OR₁₈, -NR₂₁SO₂R₂₂ or Q;
Q is 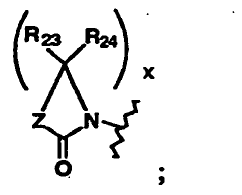
R₁₈, R₁₉, R₂₀, R₂₁ and R₂₂ are each independently hydrogen, alkyl, cycloalkyl, or R₁₉ and R₂₀ may together form a four to seven-membered heterocyclic ring;
R₂₃ and R₂₄ are each independently hydrogen, alkyl or cycloalkyl, or may together form a three to seven membered cycloalkyl ring;
Z is 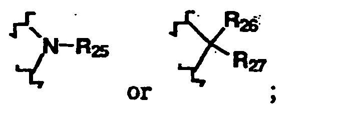
x is 2;
R₂₅, R₂₈ and R₂₇ are each independently hydrogen, alkyl or cycloalkyl, or R₂₆ and R₂₇ may together form a three to seven-membered cycloalkyl ring;
R₁₀₁, R₁₀₂, R₁₀₃, and R₁₀₄ are each independently hydrogen, halogen, or alkyl provided that at least one of R₁₀₁, R₁₀₂, R₁₀₃ and R₁₀₄ is other than hydrogen. Especially preferred are compounds where
R₁ is selected from A, or E;
R₂ is selected from alkyl, alkoxyalkyl, and haloalkoxyalkyl,
R₃ is isoxazol-3-yl independently substituted with two of following substituents: alkyl or halogen;
R₄ and R₅ together form a cyclobutyl, cyclopentyl or cyclohexyl ring;
R₆ is alkyl;
R₇ is -(C=O)NR₁₆R₁₇;
R₈, R₉, and R₁₀ are independently alkyl or alkoxy; and
R₁₀₁, R₁₀₂, R₁₀₃, and R₁₀₄ are each independently hydrogen, halogen, or alkyl provided that at least one of R₁₀₁, R₁₀₂, R₁₀₃ and R₁₀₄ is other than hydrogen

The following are definitions of terms used in this specification. The initial definition provided for a group or term herein applies to that group or term throughout the present specification, individually or as part of another group, unless otherwise indicated.

The terms "alk" or "alkyl" refer to straight or branched chain hydrocarbon groups having 1 to 12 carbon atoms, preferably 1 to 8 carbon atoms. Lower alkyl groups, that is, alkyl groups of 1 to 4 carbon atoms, are most preferred.

The term "alkenyl" refers to straight or branched chain hydrocarbon groups having 2 to 12 carbon atoms, preferably 2 to 4 carbon atoms, and at least one double carbon to carbon bond, such as ethenyl.

The term "alkynyl" refers to straight or branched chain hydrocarbon groups having 2 to 12 carbon atoms, preferably 2 to 4 carbon atoms, and at least one triple carbon to carbon bond, such as ethynyl.

The term "alkoxy" refers to an alkyl group bonded through an oxygen (-O-).

The term "aryloxy" refers to an aryl group bonded through an oxygen (-O-).

The term "thioalkyl" refers to an alkyl group bonded through a sulfer (-S-).

The term "carbonyl" refers to the group -(C=O)-.

The terms "ar" or "aryl" refer to phenyl, naphthyl and biphenyl. Phenyl is a preferred aryl group. Aryl groups may be optionally substituted with one or more (such as one to three) of the following substituents: hydrogen, halogen, cyano, alkyl, alkoxy, nitro or trifluoromethyl groups.

The term "cycloalkyl" refers to fully saturated cyclic hydrocarbon groups having 3 to 8 ring carbon atoms.

The terms "halogen" and "halo" refer to fluorine, chlorine, bromine and iodine. Haloalkyl refers to an alkyl chain substituted with from one to three halogens.

The term "heteroaryl" refers to furyl, thienyl, pyrrolyl, pyridyl, pyrimidyl, pyrazinyl, pyridazinyl, triazinyl, oxazolyl, isoxazolyl, thiazolyl, isothiazolyl, oxadiazolyl, thiadiazolyl, imidazolyl, triazolyl, and tetrazolyl, each of which may optionally be substituted where appropriate by one or more (such as one to three) of the following: hydrogen, halogen, cyano, alkyl, hydroxyalkyl, alkoxy, nitro or trifluoromethyl.

The terms "heterocyclic" or "heterocyclo" refer to optionally substituted, non-aromatic cyclic groups, for example, 4 to 7 membered monocyclic, 7 to 11 membered bicyclic, or 10 to 15 membered tricyclic ring systems, which have at least one heteroatom in at least one carbon atom-containing ring. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system.

Exemplary monocyclic heterocyclic groups include azetidinyl, pyrrolidinyl, oxetanyl, imidazolinyl , oxazolidinyl, isoxazolinyl, thiazolidinyl, isothiazolidinyl, tetrahydrofuranyl, piperidinyl, piperazinyl, 2-oxopiperazinyl, 2-oxopiperidinyl, 2-oxopyrrolodinyl, 2-oxoazepinyl, azepinyl, 4-piperidonyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, and the like.

The term "ring" encompasses homocyclic (i.e., as used herein, all the ring atoms are carbon) or "heterocyclic" (i.e., as used herein, the ring atoms include carbon and one to four heteroatoms selected from N, O and /or S, also referred to as heterocyclo), where, as used herein, each of which (homocyclic or heterocyclic) may be saturated or partially or completely unsaturated (such as heteroaryl), and each of which (homocyclic or heterocyclic) may optionally be substituted by one or more (such as one to three) hydrogen, halogen, cyano, alkyl, alkoxy, nitro or trifluoromethyl groups.

Throughout the specification, groups and substituents thereof may be chosen to provide stable moieties and compounds.

The compounds of formula I form salts which are also within the scope of this invention. Reference to a compound of the formula I herein is understood to include reference to salts thereof, unless otherwise indicated. The term "salt(s)", as employed herein, denotes acidic and/or basic salts formed with inorganic and/or organic acids and bases. In addition, when a compound of formula I contains both a basic moiety and an acidic moiety, zwitterions ("inner salts") may be formed and are included within the term "salt(s)" as used herein. Pharmaceutically acceptable (i.e., non-toxic, physiologically acceptable) salts are preferred, although other salts are also useful, e.g., in isolation or purification steps which may be employed during preparation. Salts of the compounds of the formula I may be formed, for example, by reacting a compound I with an amount of acid or base, such as an equivalent amount, in a medium such as one in which the salt precipitates or in an aqueous medium followed by lyophilization.

The compounds of formula I which contain a basic moiety may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates, lactates, maleates (formed with maleic acid), methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

The compounds of formula I which contain an acidic moiety may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, hydrabamines (formed with N,N-bis(dehydroabietyl)ethylenediamine), N-methyl-D-glucamines, N-methyl-D-glucamides, t-butyl amines, and salts with amino acids such as arginine, lysine and the like. Basic nitrogen-containing groups may be quaternized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

Prodrugs and solvates of the compounds of the invention are also contemplated herein. The term "prodrug", as employed herein, denotes a compound which, upon administration to a subject, undergoes chemical conversion by metabolic or chemical processes to yield a compound of the formula I, or a salt and/or solvate thereof. Solvates of the compounds of formula I are preferably hydrates. Any tautomers which may exist are also contemplated herein as part of the present invention.

All stereoisomers of the present compounds, such as those which may exist due to asymmetric carbons on the R substituents, including enantiomeric forms (which may exist even in the absence of asymmetric carbons, e.g., atropisomers) and diastereomeric forms, are contemplated within the scope of this invention. Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the IUPAC 1974 Recommendations.

The present invention can be applied to the extensive prior art in the field of angiotensin antagonists to obtain additional novel compounds possessing potent antagonist activity at both endothelin and angiotensin receptors. In particular, a large number of groups (known to be useful within the field of angiotensin receptor antagonists) can be substituted at the R₁ position of Formula I without departing from the scope of the present invention. The table below outlines examples of additional suitable R₁ groups.

| **Patent No.** | **Description of R**_{**1**} |
|---|---|
| JP 09291078 | Substituted imidazoles |
| JP 09301956 | Substituted imidazoles |
| JP 09323991 | carboxy methylidene cyclohepta imidazoles |
| WO 97/40040 | Pyrimidin-4-ones |
| US 5674879 | tetrahydro-imidazopyridines |
| WO 97/30036 | N-acylaminoacid derivatives |
| JP 09110691 | Imidazotetrahydropyridines |
| JP 08208640 | Benzimidazoles |
| JP 08165292 | purine derivatives |
| JP 08143552 | Cyclohepta imidazoles |
| JP 08113572 | Imidazopyridines |
| WO 96/10559 | Ureas |
| EP 708103 | Imidazolones |
| WO 96/08476 | Pyrimidinones |
| JP 08041053 | Pyrimidines |
| JP 08034780 | Cyclohept:imidazoles |
| WO 96/05195 | naptho-fused lactams |
| WO 96/04273 | Pyrazoles |
| EP 696583 | Benzimidazoles |
| JP 07316055 | Pyrimidines |
| WO 95/34564 | Pyridylimidazoles |
| JP 07309871 | Hydrazotriazoles |
| WO 95/32198 | Pyridylimidazoles |
| AU 95/16257 | Imidazopyridines |
| WO 95/24902 | Imidazoles |
| DE 4407488 | Pyridones |
| WO 95/22543 | Imidazoles |
| WO 95/21838 | Pyridylimidazoles |
| JP 07157485 | fused pyrimidinones |
| US 5411980 | N-arylsubstituted-1,2,4-triazolinones |
| WO 95/16677 | pyrimidine/pyrimidinones |
| WO 95/16675 | Benzazepin-3-yl-ureas |
| WO 95/16692 | Benzazepin-3-yl-ureas |
| US 5426105 | Dihydroimidazopyridines |
| US 5424450 | Imidazolinones |
| DE 4341453 | 4-oxo-imidazo-pyridines |
| JP 07112975 | Amino-azoles |
| DE 4339868 | 4-oxo-imidazo-pyridazines |
| WO 95/12598 | benzazepinyl urea macrocycles |
| EP 648763 | Imidazoles |
| WO 95/09632 | benzazepin-3-yl-ureas |
| EP 647627 | pyridinones |
| JP 07048357 | aminoacids |
| WO 95/03290 | benzofused lactams |
| DE 4342724 | 2-oxo-1,2-dihydro-pyridines |
| WO 93/17023 | pyrazolopyrimidines |
| US 5385894 | 6-aminoquinazolines |
| JP 07002776 | N-arylmethlpyridines |
| JP 06340668 | pyrazolotriazoles |
| US 5380719 | quinoxalines and its N-oxides |
| GB 2280438 | carboxymethylidene cycloheptimidazoles |
| WO/95/00517 | imidazoles |
| US 5378704 | benzo- and pyrido-1,2,4-thiadiazines |
| JP 06287182 | alkylglycines |
| EP 623610 | pyridine and pyridones |
| US 5358947 | pyrazolotriazinones |
| WO 94/22838 | pyrazoles |
| EP 621276 | 2,3,6-substituted quinazolinone derivatives |
| WO 94/21629 | 1-phenyl-imidazol-2-ones |
| JP 06211814 | thioureas |
| EP 618207 | 5,8-dihydro-6H-pyrido-pyrimidin-7-ones |
| DE 4305279 | triazolopyrimidines |
| WO 94/17069 | pyrazoles |
| WO 94/17067 | pyrimidones |
| US 5338736 | 2,3,6-substituted quinazolinone derivatives |
| JP 06184086 | thioureas |
| DE 4300912 | 1-benzyl-1,2,3,4-tetrahydroquinazolin-2-ones or related analogs |
| US 5330987 | pyridopyrimidinones |
| EP 607077 | 4-pyrimidinones |
| WO 94/13675 | pyrazolotriazoles |
| US 5326776 | 5-membered heterocyclic rings comprising 1-4 N or 2 N and 1 O and comprising 0-2 double bonds |
| EP 602521 | imidazopyridines |
| WO 94/11379 | pyridooxazinones |
| WO 94/11012 | dipeptide analogs |
| EP 591891 | benzimidazoles |
| US 5315013 | pyrazoles |
| JP 06100541 | pyrazolinones |
| WO 94/04516 | 5-membered ring heterocyles containing 1-4 heteroatoms |
| JP 06087833 | vinyl imidazoles |
| EP 594022 | 2-pyridones |
| EP 594019 | 2-pyridones |
| JP 06073008 | alkoxy-pyridines |
| JP 06072985 | ureas and thioureas |
| DE 4233590 | benzimidazoles |
| JP 06065236 | dihydrobenzimidazolones |
| JP 06056826 | quinoxalines |
| EP 589665 | 4,5,6,7-tetrahydro-1H-imidazo[4,5-c]pyridine-6-carboxamides |
| JP 06041122 | quinoxalines |
| DE 4230464 | imidazolylbenzimidazoles |
| US 5296480 | 2-alkyl-5,6,7,8-tetrahydro-pyrido (4,3-d)-pyrimidine-4-ones |
| US 5294617 | quinazolinones |
| US 5294611 | quinazolinones |
| US 5292734 | quinazolinones |
| JP 06032782 | pyrazoles |
| US 5290780 | quinazolinones |
| JP 06025250 | thieno[3,4-d]imidazoles |
| JP 06025229 | imidazoles |
| WO 94/03453 | pyrazolyl[4,3-c]pyridines |
| WO 94/03449 | imidazoles |
| WO 94/03435 | imidazole-5-carboxylic acids |
| US 5288720 | quinazolinones |
| JP 06016661 | imidazoles |
| US 5284853 | quinazolinones |
| US 5284852 | quinazolinones |
| JP 06009638 | pyrazolopyrimidines |
| WO 94/02467 | imidazoles |
| US 5281604 | quinazolinones |
| US 5281603 | quinazolinones |
| US 5281602 | pyridopyrimidines |
| DE 4224133 | benzimidazoles |
| US 5276048 | imidazoles |
| GB 2268743 | pyridines/pyrazines/pyrimidines |
| JP 05310696 | pyridines |
| EP 574846 | imidazopyridines |
| DE 4237656 | benzimidazoles |
| FR 2690442 | pyrazolopyrimidines |
| GB 2267089 | cycloheptimidazoles |
| EP 573218 | imidazoles |
| WO 93/23391 | indoles, 7-azaindoles |
| US 5260325 | N-linked amides or thioamides |
| DE 4212748 | benzimidazoles |
| EP 566020 | benzimidazoles |
| DE 4212250 | benzimidazoles |
| US 5250548 | aminopyridines |
| GB 2265900 | pyrazoles |
| EP 562936 | imidazolines |
| WO 93/18035 | aza-phthalimides |
| WO 93/17682 | bicyclic heterocycles |
| WO 93/17681 | 5-membered ring heterocycles |
| EP 561252 | 2-oxoquinolines |
| JP 05201994 | pyridazinones |
| GB 2264709 | imidazopyridines |
| JP 05194418 | 2-aminopyrimidines |
| WO 93/16049 | 4-oxo-dihydropyridines |
| JP 05178836 | pyridines |
| EP 556080 | pyrazolo[1,5-a]pyridines or imidazo[1.5-a]pyridines |
| US 5231094 | triazolopyrimidines |
| EP 554107 | 1,2,4-triazole |
| EP 554098 | imidazo[4,5b]pyridines |
| WO 93/14086 | 1-isoquinolones |
| EP 552765 | benzimidazoles |
| JP 05155884 | benzopyrazines or pyridopyrazines |
| WO 93/13077 | hydantoins connected to imidazoles |
| US 5225408 | oxadiazinone |
| JP 05140152 | quinazolindione |
| EP 550313 | pyrazolone and pyrimidinones |
| US 5219856 | imidazoles |
| EP 547514 | imidazopyridines |
| US 5218125 | imidazoles |
| GB 2262096 | 4-aminopyrimidines |
| US 5214153 | imidazoles |
| US 5212195 | indoles, azaindoles |
| EP 543263 | benzimidazoles |
| DE 4221583 | pyridones |
| US 5208234 | imidazolephosphonic acids |
| WO 93/08193 | imidazo-tetrahydropyridazines |
| WO 93/08171 | pyrimidocycloalkanes |
| WO 93/08169 | aminopyrimidines |
| EP 539086 | pyrimidinolactams |
| EP 537937 | pyrazinopyrimidinones |
| DE 4132632 | imidazolylpropenoic acids |
| EP 535465 | imidazolylpropenoic acids |
| EP 535463 | imidazolylpropenoic acids |
| EP 535420 | imidazoles |
| EP 534706 | quinazolinone or pyridopyrimidinones |
| WO 93/05044 | pyrazolotriazines |
| W0 93/05025 | pyrazoles |
| WO 93/04059 | imidazoles |
| WO 93/04045 | imidazolinones |
| JP 05032661 | imidazolinones |
| EP 532410 | imidazolinones and larger ring analogs |
| EP 531876 | imidazoindolizines |
| EP 531874 | 4,5,6,7-tetrahydroimidazo[4,5-c]pyridine-4-carboxylic acids |
| EP 530702 | 1,2-dihydro-2-oxopyridines |
| WO 93/03040 | thienopyrimidin-4-ones |
| WO 93/03033 | imidazo[4,5-d]pyridazines |
| WO 93/03018 | pyrimidines |
| US 5187168 | quinazoline derivatives |
| JP 05017480 | thienoimidazoles |
| US 5185340 | oxypyrimidines |
| US 5182288 | lactams |
| FR 2677016 | acyl amino acid derivatives |
| WO 93/00341 | imidazoles |
| WO 92/22533 | 4-aminoquinolines |
| US 5124335 | fused pyrroles |
| EP 490820 | acylamino derivatives |
| EP 490587 | pyrazolo-pyrimidine and imidazopyridazines |
| EP 495626 | oxypyridines, oxyquinolines, and imidazoles |
| EP 497150 | 3-quinazolin-4-ones |
| EP 497121 | imidazoles |
| EP 498721 | 1,4-dihydroquinolin-4-ones |
| US 5132216 | imidazopyridines |
| EP 499416 | oxypyridines |
| EP 499415 | aminopyridines |
| EP 499414 | Fused oxypyridines |
| EP 500409 | 4-pyrimidones |
| EP 500297 | 2-pyridones and 2-pyrimidones |
| EP 502725 | fused pyrimidones |
| EP 502575 | 1-(2H)-isoquinolinones |
| EP 502314 | benzimidazoles |
| EP 505098 | imidazoles |
| EP 503785 | imidazoles |
| JP 04230683 | 7-aza and 4- azabenzimidazoles (i.e., imidazopyridines) |
| EP 507594 | quinolines |
| JP 04235988 | imidazopyridazinediones |
| EP 505893 | imidazopyridines |
| FR 2672891 | pyrazolones |
| US 5145699 | pyridopyrimidines |
| EP 515265 | pyrimidine derivatives |
| EP 511791 | pyrrolopyridines |
| JP 04257564 | benzimidazoles, imidazopyridines, imidazopyrazines |
| WO 92/16524 | benzo-fused lactams |
| EP 516392 | naphyridones and pyrido[c,b]pyrrolidones |
| WO 92/19211 | imidazobenzoquinones |
| JP 04295478 | imidazo[4,5-b]pridines |
| EP 5212768 | triazolo pyrimidines |
| WO 92/21666 | thiazole derivatives |
| EP 518033 | imidazo[4,5-c]pyridine-4-carboxylates and other heterocycles |
| US 5087702 | 3H-imidazo-[4,5-b] pyridines |
| US 5087634 | N-substituted imidazol-2-ones |
| WO 92/00977 | imidazoles |
| EP 475206 | various 6-membered heterocycles |
| WO 92/02508 | oxyquinolines |
| WO 92/04343 | tetrahydrobenzazoles |
| WO 92/04335 | 1H-1,2,4-triazoles |
| EP 475898 | Azacyclic compounds including imidazolinones |
| EP 481448 | dihydropyrimidines |
| QO 92/05161 | 1,3,5-trisubstituted 1,2,4-triazoles |
| WO 92/07852 | xanthine derivatives |
| JP 04120072 | pyrimidine derivatives |
| EP 487252 | quinolines and 1,5-naphthyridine derivatives |
| EP 483683 | thienoimidazoles |
| EP 470543 | fused imidazoles |
| EP 468470 | fused imidazoles |
| EP 467207 | purines |
| WO 91/19715 | imidazo[4,5-d]pyridazines |
| WO 91/19697 | pyridines |
| EP 465368 | imidazoles |
| EP 465323 | pyrimidines |
| WO 91/18888 | triazolones |
| EP 461039 | benzimidazoles |
| US 5066586 | pyridoimidazoles |
| EP 459136 | benzimidazoles |
| WO 91/17148 | triazoles |
| EP 456510 | pyridoimidazoles |
| EP 456442 | quinolines |
| WO 91/15479 | imidazoles, oxazoles, thiazoles |
| WO 91/15209 | pyrimidines |
| EP 453210 | pyridines |
| WO 91/14679 | imidazolinones or pyrimidinones |
| US 5053329 | imidazopyridines |
| US 5049565 | conjugated imidazopyridines |
| EP 449699 | pyrazoles |
| EP 446062 | pyrazoles |
| EP 445811 | pyridin-4-ones and pyrimidin-4-ones |
| EP 443983 | amides, sulfonamides, carbamates |
| EP 443568 | thienopyridin-4-ones, thienopyrimidin-2,4-diones |
| EP 442473 | pyrimidin-2,4-diones |
| EP 435827 | pyrimidinones |
| EP 434038 | fused imidazoles |
| EP 432737 | cycloheptimidazolones |
| WO 91/07404 | azaquinolines |
| EP 430300 | xanthines |
| EP 426021 | fused imidazoles |
| EP 425921 | benzimidazoles |
| EP 424317 | pyrimidines |
| EP 420237 | fused imidazoles |
| EP 419048 | pyrimidinones |
| EP 415886 | fused imidazoles |
| EP 412848 | oxyquinolines |
| EP 412594 | triazolinones |
| EP 411766 | quinazolinones |
| EP 411507 | pyrazole-3-carboxylates |
| WO 91/00281 | imidazoles |
| WO 91/00277 | imidazoles |
| EP 409332 | triazoles |
| EP 407342 | pyrimidinones |
| EP 407102 | fused imidazoles |
| EP 401030 | fused imidazoles |
| EP 400974 | fused imidazoles |
| EP 400835 | benzimidazoles |
| EP 399732 | benzimidazoles |
| EP 399731 | fused imidazoles |
| EP 392317 | benzimidazoles |
| EP 890719 | imidazoles |
| EP 323841 | pyrroles, pyrazoles, and triazoles |
| EP 291969 | biphenyltetrazoles |
| EP 253310 | imidazoles |
| WO 95/28419 | indazoles |
| EP 638572 | fused imidazoles |
| DE 4320432 | aminopyridyl, imidazoles, fused imidazoles |
| JP 06279437 | imidazoles |
| EP 624583 | pyridones |
| EP 623611 | pyridines or 2-pyridones |
| US 5348955 | diacyl piperazines |
| WO 94/07486 | benzo-fused lactams |
| JP 06128256 | imidazopyridines |
| US 5298517 | imidazoles |
| US 5286729 | quinazolinones |
| DE 4203872 | imidazo[1,2-a]pyridines |
| GB 2263637 | fused imidazoles |
| US 5177097 | imidazolones |
| EP 519831 | imidazoles and pyrimidines |
| US 5153347 | phosphonates or phosphinates |
| DE 4034728 | thienoimidazoles |
| DE 4032522 | thienoimidazoles |
| EP 461040 | fused imidazoles |
| DE 4006693 | benzimidazoles |
| US 4916129 | imidazoles |
| US 4880804 | benzimidazoles |
| WO 97/15556 | 3-spiroindolin-2-ones |
| US 5266583 | imidazoles |
| EP 573218 | imidazoles |
| US 5264447 | imidazoles |
| EP 569794 | benzopyridones or pyridopyridones |
| US 5091390 | fused imidazoles |
| US 5256658 | morpholines, piperidines, piperazines, thiomorpholines |
| EP 546358 | benzimidazoles |

### Methods of Preparation

The compounds of the present invention may be prepared by methods such as those illustrated in the following Schemes I to XIII**.** Solvents, temperatures, pressures, and other reaction conditions may be selected by one of ordinary skill in the art. Starting materials are commercially available or readily prepared by one of ordinary skill in the art.

The following are the definitions of symbols used throughout Schemes I to XIII:
- AA: hydrogen, halogen (chloro, bromo, iodo) or -OSO₂CF₃;
- BB: suitable nitrogen protecting group, exemplified by methoxymethyl- [MOM], benzyloxymethyl- [BOM], 2-(trimethylsilyl)ethoxymethyl- [SEM], methoxyethoxymethyl-[MEM], or *t*-butyl groups;
- DD: Sₙ2 or Sₙ1 leaving group exemplified by halogen (Cl, Br, I) and sulfonates (-OSO₂-aryl (e.g., -OSO₂PH or -OSO₂PhCH₃), or -OSO₂-alkyl (e.g., -OSO₂CH₃, or -OSO₂CF₃));
- EE: halogen (chloro, bromo, iodo) or -OSO₂CF₃;
- GG: boronate ester or boronic acid, or trialkylstannane;
- HH: metal atom such as tin, zinc, magnesium or lithium as part of an organometallic compound used as an intermediate for transition metal mediated aryl-aryl coupling reactions;
- JJ: -CN, -CHO, or -CO₂R₂₀ wherein R₂₀ is hydrogen or C₁ to C₃ alkyl.

Exemplary conditions for forming and removing suitable nitrogen protecting groups may be found in T. W. Greene and P. G. M. Wuts, Protecting Groups in Organic Synthesis, John Wiley & Sons, Inc, New York, 1991, pp. 309-405. One skilled in the art can also recognize that the heteroaryl sulfonamide-NH in compounds of the invention will also have carboxylic acid character, and accordingly, methods used to protect carboxylic acids may be applicable to protecting the nitrogen NH of the sulfonamides in the invention, including intermediates to compounds of formula I. Exemplary conditions for forming and removing suitable carboxylic acid protecting groups may be found in T. W. Greene and P. G. M. Wuts, Protecting Groups in Organic Synthesis, John Wiley & Sons, Inc, New York, 1991, pp. 175-276.

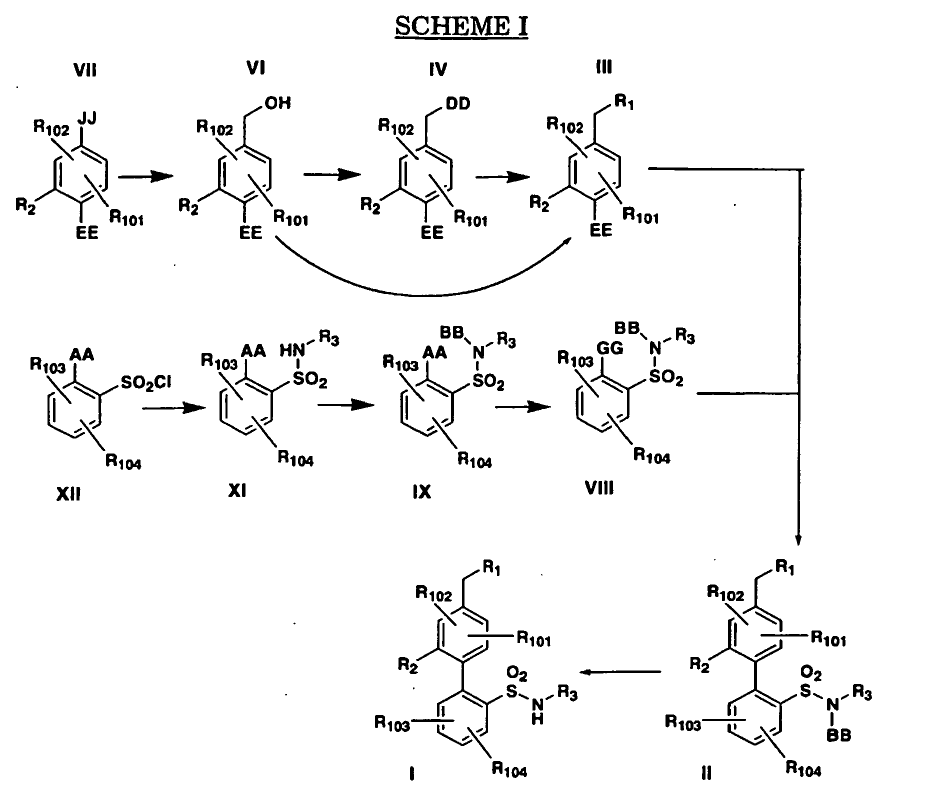

Compounds of formula I may be prepared from the deprotection of a compound of formula II wherein BB is a suitable nitrogen protecting group. Exemplary conditions for deprotection, and nitrogen protecting groups, may be found in T. W. Greene and P. G. M. Wuts, Protecting Groups in Organic Synthesis, John Wiley & Sons, Inc, New York, 1991, pp. 309-405. Preferred nitrogen protecting groups are the methoxymethyl (MOM), methoxyethoxymethyl (MEM), and 2-(trimethylsilyl)ethoxymethyl (SEM) groups.

Compounds of formula II may be prepared from a palladium catalyzed coupling of a compound of formula III with a compound of formula VIII, in the presence of a suitable base in an inert solvent. Exemplary palladium catalysts include tetrakis(triphenylphosphine) palladium(0), palladium(II) chloride or palladium(II) acetate. The preferred palladium catalyst is tetrakis(triphenylphosphine) palladium(0). Exemplary bases include tertiary amines, such as, but not limited to, triethylamine, or aqueous potassium, sodium, or cesium carbonate. The preferred base is aqueous sodium carbonate. Exemplary solvents include tetrahydrofuran, 1,4-dioxane, acetonitrile, toluene, benzene, or straight chain alcohols, or a combination thereof. The preferred solvent is a mixture of toluene and ethanol. Exemplary reaction temperatures are between about 25°C to 125°C, preferably between about 65°C and 110°C.

Compounds of formula III may be prepared from a compound of formula IV via displacement of the leaving group (DD) by the conjugate base of a compound R₁-H, wherein R₁ is as previously defined, using a base in an inert solvent. Exemplary bases include sodium carbonate, potassium carbonate, cesium carbonate, sodium hydride, potassium hydride, or alkyl lithiums. The preferred base is sodium hydride. Exemplary inert solvents include ethers (tetrahydrofuran, 1,4-dioxane, diethyl ether), or N,N-dimethylformamide. The preferred solvent is N,N-dimethylformamide. Exemplary reaction temperatures are between about 0°C to 154°C, preferably between about 65°C and 110°C.

Compounds of formula III may also be prepared via a Mitsunobu reaction between a compound of formula VI and the conjugate acid R₁-H, preferably using a phosphine and oxidizing agent, in an inert solvent. Exemplary phosphines include trialkylphosphines, triarylphosphines and polymer supported triarylphosphines. The preferred phosphine is triphenylphosphine. Exemplary oxidizing reagents include diethyl azodicarboxylate, diisopropyl azodicarboxylate, or carbon tetrabromide. The preferred oxidizing reagent is diethyl azodicarboxylate. Exemplary inert solvents include ethers (tetrahydrofuran, 1,4-dioxane, diethyl ether), acetonitrile or N,N-dimethylformamide. The preferred solvent is N,N-dimethylformamide. Exemplary reaction temperatures are between about 0°C to 154°C, preferably between about 20°C and 65°C.

Compounds of formula IV (especially, where DD is -OSO₂Ph, -OSO₂PhCH₃, -OSO₂CH₃, -OSO₂CF₃) may be prepared from the reaction of a compound of formula VI with ClSO₂Ph, ClSO₂PhCH₃, ClSO₂CH₃ or (CF₃SO₂)₂O in the presence of a base in an inert solvent.

Compounds of formula VI may be prepared from reduction of a compound of formula VII using a suitable reducing agent in an inert solvent.

Compounds of formula VII are either commercially available or available by means known to one skilled in the art.

Compounds of formula VIII may be prepared via lithiation of a compound of formula IX wherein AA is hydrogen or a halogen (chloro, bromo, iodo), and reacting the resulting aryl lithium with an appropriate borate derivative.

Compounds of formula IX may be prepared via the protection of the nitrogen in a compound of formula XI. Exemplary nitrogen protecting groups and methods of protecting the nitrogen are similar to those for protecting amines, such as those described in T. W. Greene and P. G. M. Wuts, Protecting Groups in Organic Synthesis, John Wiley & Sons, Inc, New York, 1991.

Compounds of formula XI may be prepared from the reaction of a compound of formula XII with a compound R₃-NH₂.

Compounds of the formula XII are either commercially available or available by means known to one skilled in the art.

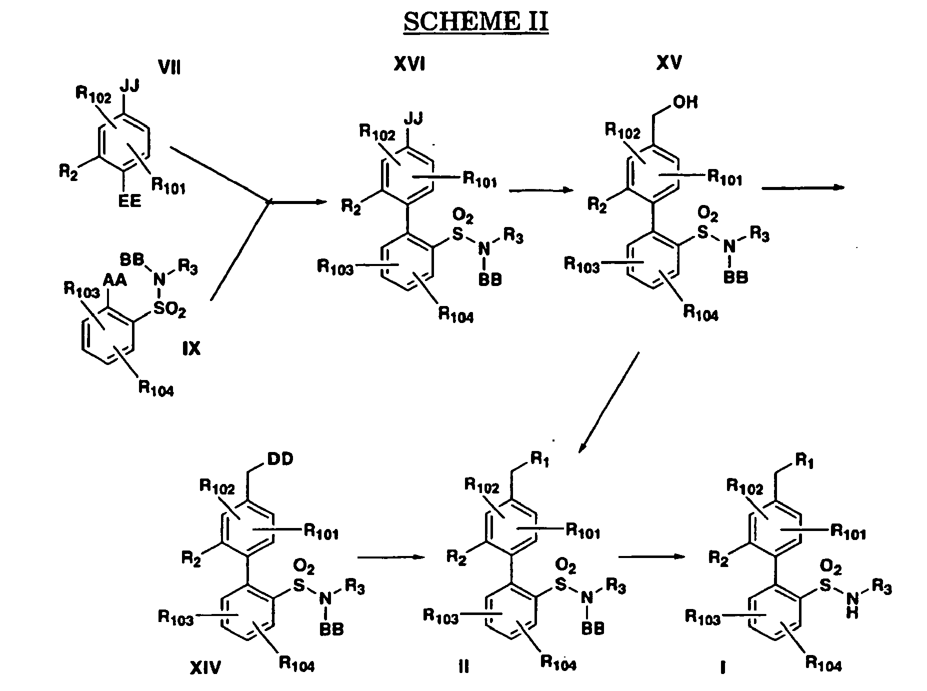

Compounds of formula I may be prepared from the deprotection of a compound of formula II as described in Scheme I.

Compounds of formula II may be prepared from a compound of formula XIV via displacement of the leaving group (DD) by the conjugate base of a compound R₁-H, wherein R₁ is as previously defined, using a base in an inert solvent. Exemplary bases include sodium carbonate, potassium carbonate, cesium carbonate, sodium hydride, potassium hydride, or alkyl lithiums. The preferred base is sodium hydride. Exemplary inert solvents include ethers (tetrahydrofuran, 1,4-dioxane, diethyl ether), or N,N-dimethylformamide. The preferred solvent is N,N-dimethylformamide. Exemplary reaction temperatures are between about 0°C to 154°C, preferably between about 25°C and 110°C.

Compounds of formula II may also be prepared via a Mitsunobu reaction between a compound of formula XV and the conjugate acid R₁-H using a phosphine and oxidizing agent in an inert solvent. Exemplary phosphines include trialkylphosphines, triarylphosphines and polymer supported triarylphosphines. The preferred phosphine is triphenylphosphine. Exemplary oxidizing reagents include diethyl azodicarboxylate, diisopropyl azodicarboxylate, or carbon tetrabromide. The preferred oxidizing reagent is diethyl azodicarboxylate. Exemplary inert solvents include ethers (tetrahydrofuran, 1,4-dioxane, diethyl ether), acetonitrile or N,N-dimethylformamide. The preferred solvent is N,N-dimethylformamide. Exemplary reaction temperatures are between about 0°C to 154°C, preferably between about 20°C and 65°C.

Compounds of formula XIV may be prepared from compounds of formula XV using methods well known in the art. For example, compounds of formula XIV (DD = Br) may be prepared by the treatment of compound XV with carbon tetrabromide and triphenylphosphine in a suitable solvent such as toluene or tetrahydrofuran.

Compounds of formula XV may be prepared from reduction of a compound of formula XVI using a suitable reducing agent in an inert solvent. R₂ is preferably not an amide, an ester, a carboxylic acid or an aldehyde during this operation.

Compounds of formula XVI may be prepared from a palladium catalyzed coupling of a compound of formula VII with a compound of formula IX in the presence of a suitable base and an inert solvent as described in Scheme I.

Compounds of formula VII are available by means known to one skilled in the art.

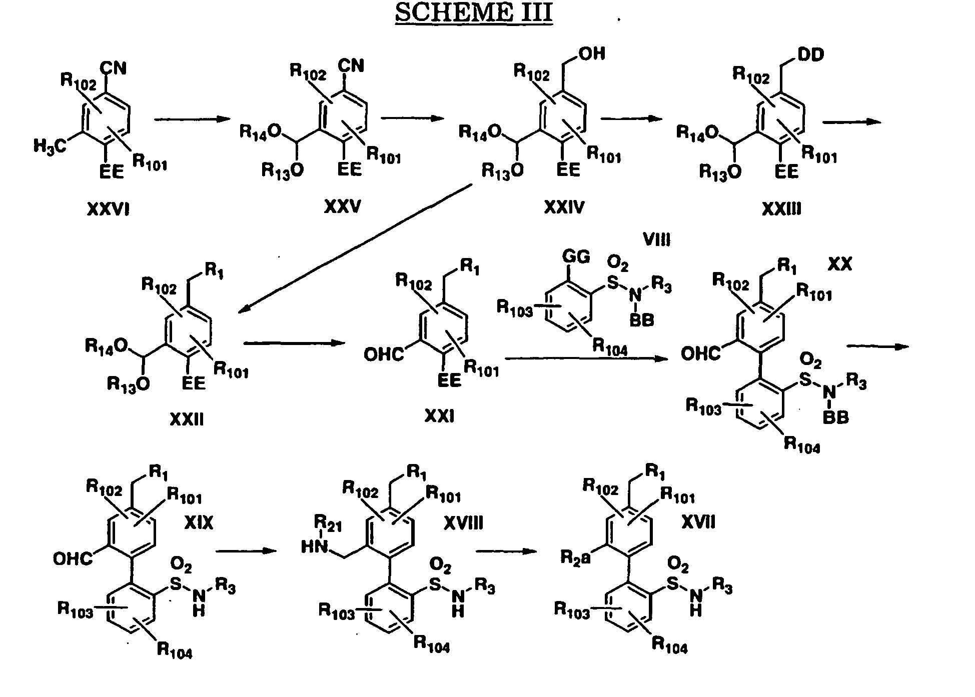

Compounds of formula XVII (which are compounds of formula I wherein R₂ is R₂ₐ where R₂ₐ is -CH₂N(R₂₁)(C=O)N(R₁₉)R₂₀, -CH₂N(R₂₁)(C=O)OR₁₈ or -CH₂N(R₂₁)(C=O)R₂₂), may be prepared from compounds of formula XVIII (also compounds of formula I, where R₂ is -CH₂NHR₂₁) by reaction of a compound of formula XVIII with an active ester (i.e., from a carboxylic acid such as R₂₂COOH in the presence of a suitable coupling agent such as dicyclohexylcarbodimide (DCC)), or an acid chloride (i.e., R₂₂(C=O)Cl), or an isocyanate (i.e., R₁₉N=C=O), or a chloroformate (i.e., R₁₈O(C=O)Cl) in the presence of a suitable base such as triethylamine and catalyst such as 4-dimethylaminopyridine in an inert solvent. This step may, for example, be conducted combinatorially and a library of such compounds created.

Compounds of formula XVIII may be prepared from reductive amination of compounds of formula XIX (compounds of formula I where R₂ is -CHO) using a primary amine such as R₂₁NH₂, in the presence of a suitable reducing agent such as sodium triacetoxyborohydride in an inert solvent.

Compounds of formula XIX may be prepared by deprotection of compounds of formula XX wherein BB is a suitable nitrogen protecting group as described in Scheme I.

Compounds of formula XX may be prepared from a palladium catalyzed coupling of a compound of formula XXI with a compound of formula VIII in the presence of a suitable base and an inert solvent as described in Scheme I.

Compounds of formula XXI may be prepared in two steps from a compound of formula XXIII, first by displacement of the leaving group (DD) by the conjugate base of a compound R₁-H, wherein R₁ is as previously defined using a suitable base in an inert solvent as described in Scheme I to provide a compound of formula XXII. Subsequent deprotection of the acetal in a compound of formula XXII using methods known in the art is employed to provide a compound of formula XXI.

Compounds of formula XXII may also be prepared via a Mitsunobu reaction between a compound of formula XXIV and the conjugate acid R₁-H using a phosphine and oxidizing agent in an inert solvent. Exemplary phosphines include trialkylphosphines, triarylphosphines and polymer supported triarylphosphines. Exemplary oxidizing reagents include diethyl azodicarboxylate, diisopropyl azodicarboxylate, or carbon tetrabromide. Exemplary inert solvents include ethers (tetrahydrofuran, 1,4-dioxane, diethyl ether), acetonitrile or N,N-dimethylformamide.

Compounds of formula XXIII may be prepared from compounds of formula XXIV using methods well known in the art. For example, compounds of formula XXIII (DD = Br) may be prepared by the treatment of compound XXIV with carbon tetrabromide and triphenylphosphine in a suitable solvent such as toluene or tetrahydrofuran.

Compounds of formula XXIV may be prepared in two steps from compound XXV via a partial reduction of the nitrile group to the aldehyde using a suitable reducing agent such as diisobutylaluminum hydride, with subsequent reduction of the aldehyde to hydroxymethyl using an agent such as sodium borohydride.

Methods for the preparation of compounds XXV and XXVI are known in the art [H.-Y. Zhang, et al., Tetrahedron, *50*, 11339-11362 (1994)].

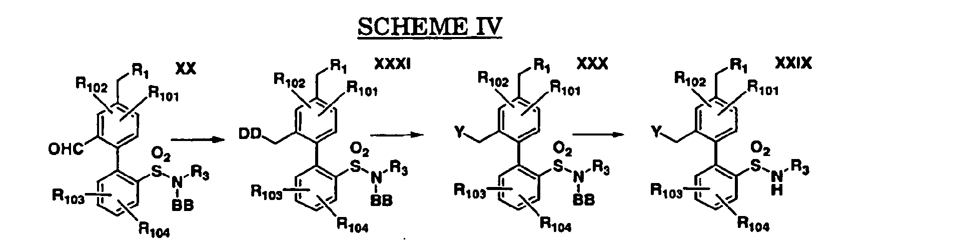

Compounds of formula XXIX (which are compounds of formula I where R₂ is -CH₂Y) may be prepared from the deprotection of a compound of formula XXX such as is described in Scheme I.

Compounds of formula XXX may be prepared from a compound of formula XXXI via displacement of the leaving group (DD) by the conjugate base of a compound Y-H, wherein Y is as previously defined using a base in an inert solvent. Exemplary bases include sodium carbonate, potassium carbonate, cesium carbonate, sodium hydride, potassium hydride, or alkyl lithiums. The preferred base is sodium hydride.

Compounds of formula XXXI may be prepared from compounds of formula XX using methods well known in the art. For example, compounds of formula XXXI (DD = Br) may be prepared from compound XX in two steps: first by reducing the aldehyde to a hydroxymethyl group using a suitable reducing agent such as sodium borohydride, and second, conversion of the hydroxymethyl group to the bromomethyl function using carbon tetrabromide and triphenylphosphine in a suitable solvent such as toluene or tetrahydrofuran.

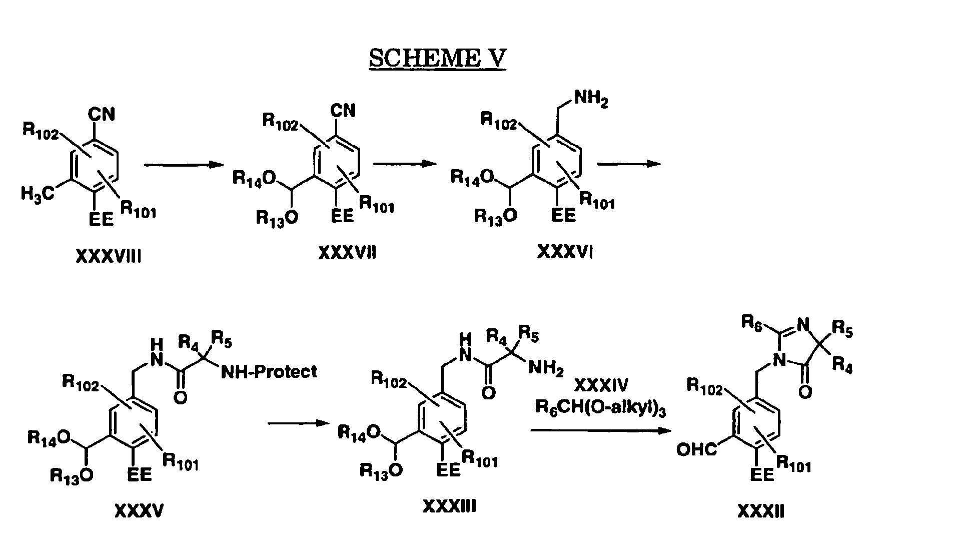

Compounds of formula XXXII (which may be employed, for example, in the methods of the preceding Schemes) may be prepared by cyclization of compounds of formula XXXIII in the presence of orthoester XXXIV using a catalytic amount of a weak acid such as acetic acid. Exemplary reaction temperatures are between about 25°C to 154°C, preferably between about 60°C and 110°C.

Compounds of formula XXXIII (e.g., where R₁₃ and R₁₄, together with the atoms to which they are bonded, form the five-membered ring 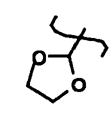 ) may be prepared from compounds of formula XXXVI in two steps: (1) acylation of compound XXXVI with an N-protected amino acid in the presence of a suitable coupling agent such as dicyclohexylcarbodimide (DCC) or (benzotriazol-1-yloxy)tripyrrolidinophosphonium hexafluorophosphate (PyBOP) in a suitable solvent such as N,N-dimethylformamide, and (2) removal of the protecting group. Suitable conditions and suitable nitrogen protecting groups and the corresponding deprotection conditions may be found in T. W. Greene and P. G. M. Wuts, Protecting Groups in Organic Synthesis, John Wiley & Sons, Inc, New York, 1991, pp. 309-405.

Compounds of formula XXXVI may be prepared via reduction of a compound of formula XXXVII using an appropriate reducing agent such as diborane or lithium aluminum hydride in an appropriate solvent such as tetrahydrofuran.

Compounds of formula XXXVII may be prepared from compounds of formula XXXVIII as described in Scheme III.

Compounds of formula XXXVIII may be prepared by methods known in the art.

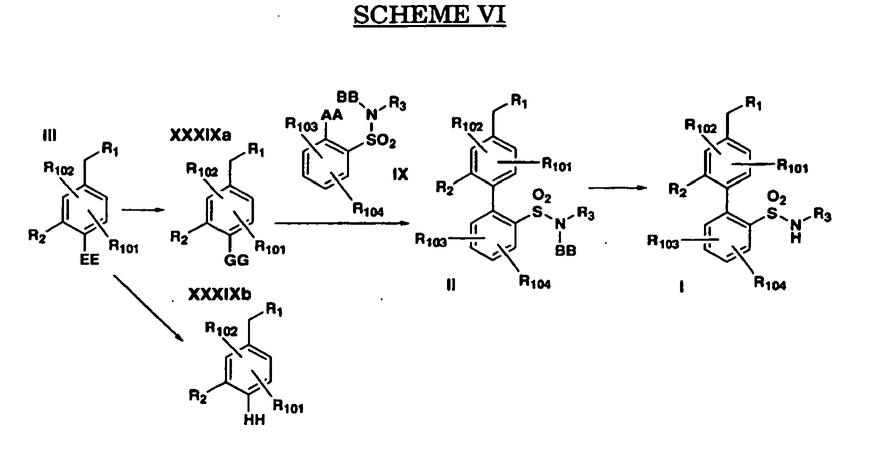

Compounds of formula I may be prepared from the deprotection of a compound of formula II as described in Scheme I.

Compounds of formula II may be prepared by a palladium catalyzed coupling of a compound of formula IX (as described in Scheme I) wherein AA here is -OSO₂CF₃ or a halogen (chlorine, bromine, or iodine; preferably bromine or iodine) with a compound of formula XXXIXa, wherein GG is a boronic acid or ester, in the presence of a base and an inert solvent as described in Scheme I.

Compounds of formula II may also be prepared by a palladium or nickel catalyzed coupling of a compound of formula IX (as described in Scheme I) wherein AA is a halogen (chlorine, bromine, or iodine; preferably bromine or iodine) with a compound of formula XXXIXb wherein HH is a suitable metal atom bearing appropriate ligands. Exemplary metal atoms include tin, zinc, magnesium, and lithium. Exemplary catalysts include tetrakis(triphenylphosphine)palladium(0) and dichlorobis(triphenylphosphine)nickel(II).

Compounds of formula XXXIXa or XXXIXb may be prepared via lithiation of a compound of formula III wherein EE is a halogen (chlorine, bromine, or iodine; preferably bromine or iodine), then reacting the resulting aryl lithium with an appropriate borate derivative or with an appropriate zinc, tin, or magnesium reagent.

Compounds of formula III may be prepared by the methods described in Scheme I.

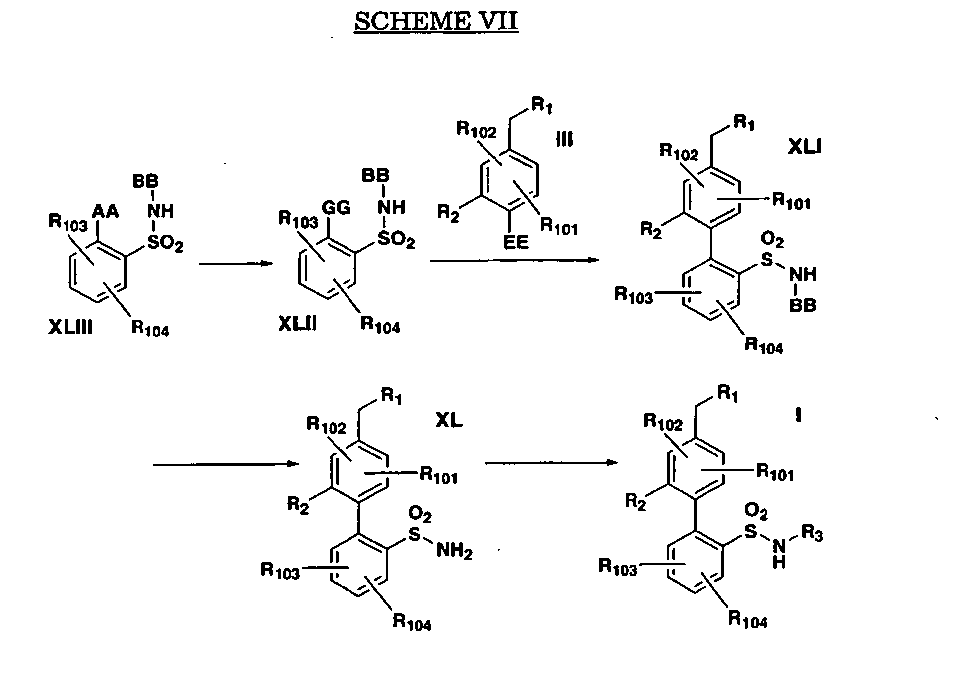

Compounds of formula I may be prepared from the thermal reaction of a compound of formula XL with a heterocyclic compound of formula R₃-X, wherein X is a halogen (fluorine, chlorine, bromine, or iodine), in the presence of a base and an inert solvent. Exemplary bases include sodium hydride, potassium carbonate, potassium hydride, and potassium bis(trimethylsilyl)amide, preferably sodium hydride. Exemplary solvents include N,N-dimethylformamide and N,N-dimethylacetamide. Exemplary reaction temperatures are between about 80°C and 150°C, preferably between 110°C and 130°C.

Compounds of formula I may also be prepared from the reaction of a compound of formula XL with a heterocyclic compound of formula R₃-X, wherein X is a halogen (chlorine, bromine, or iodine), in the presence of a palladium catalyst, a phosphine ligand, a base, and an inert solvent. Exemplary palladium catalysts include palladium acetate and tris(dibenzylideneacetone)palladium(0), and the preferred palladium catalyst is palladium acetate. The preferred phosphine ligand is 2,2'-bis(diphenylphosphino)-1,1'-binaphthyl. Exemplary bases include sodium hydride and sodium t-butoxide. The preferred base is sodium hydride. Exemplary reaction temperatures are between about 20°C and 110°C, preferably between 85°C and 110°C.

Compounds of formula XL may be prepared by deprotection of a compound of formula XLI, wherein BB is a suitable nitrogen protecting group. Exemplary conditions for protection and deprotection of nitrogen functionalities may be found in T. W. Greene and P. G. M. Wuts, Protecting Groups in Organic Synthesis, John Wiley & Sons, Inc., New York, 1991, pp. 309-405. The preferred protecting group BB for Scheme VII is *tertiary*-butyl. Exemplary deprotection conditions include the use of acids, such as trifluoroacetic acid.

Compounds of formula XLI may be prepared by a palladium catalyzed coupling of a compound of formula III (as described in Scheme I) wherein EE is a halogen (chlorine, bromine, or iodine; preferably bromine or iodine) with a compound of formula XLII, wherein GG is a boronic acid or ester, in the presence of a base and an inert solvent as described in Scheme I.

Compounds of formula XLII may be prepared via the lithiation of a compound of formula XLIII in an inert solvent, followed by reacting the resulting aryl lithium with an appropriate borate derivative. Exemplary reagents for the lithiation reaction include *n*-butyllithium and t-butyllithium. Exemplary solvents include ethers such as tetrahydrofuran, either alone or in combination with hydrocarbon solvents such as hexane. The preferred solvent is a mixture of tetrahydrofuran and hexane.

Compounds of formula XLIII are either commercially available or available by means known to one skilled in the art.

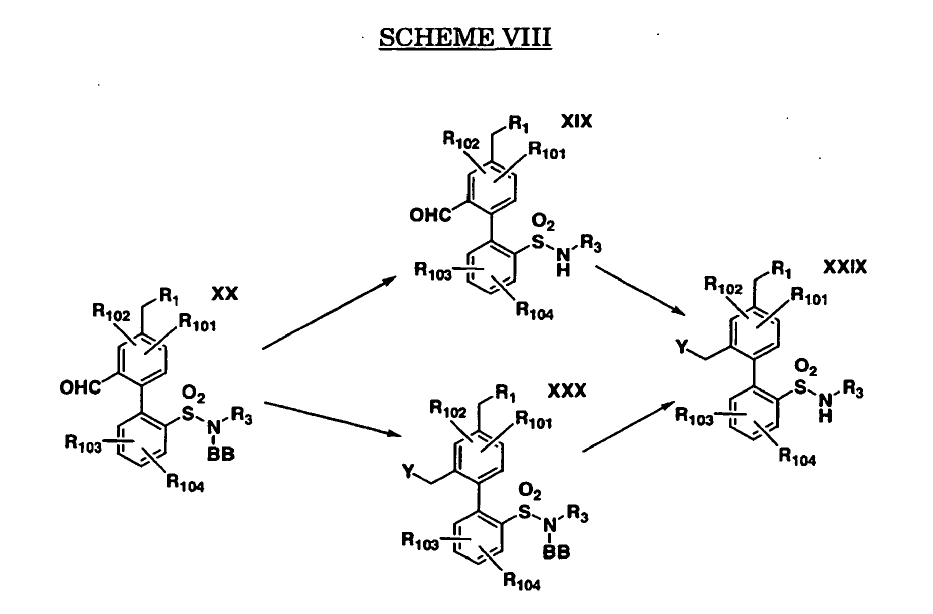

Compounds of formula XXIX (which are certain compounds of formula I where R₂ is -CH₂Y, as described in Scheme IV) may be prepared from a compound of formula XIX (which is a compound of formula I where R₂ is -CHO, as described in Scheme III) via a two step process: 1) reductive amination of XIX in the presence of a primary amine R₃₀NH₂, wherein R₃₀ is carboxyalkyl, alkoxycarbonylalkyl, hydroxyalkyl, or aminoalkyl, using a suitable reducing agent such as sodium triacetoxyborohydride, yields an intermediate amine; 2) subsequent cyclization using an appropriate cyclization reagent yields a compound of formula XXIX. When R₃₀ is carboxyalkyl, appropriate cyclization reagents include carbodiimides such as diisopropylcarbodiimide. When R₃₀ is hydroxyalkyl or aminoalkyl, appropriate cyclization reagents include phosgene and 1,1'-carbonyldiimidazole. When R₃₀ is alkoxycarbonylalkyl, appropriate cyclization reagents include tertiary amine bases such as triethylamine and N,N-diisopropylethylamine.

Compounds of formula XIX may be prepared via deprotection of a compound of formula XX as described in Scheme III.

Compounds of formula XXIX may also be prepared by deprotection of a compound of formula XXX as described in Scheme I.

Compounds of formula XXX may be prepared from a compound of formula XX, using the two step process described above for the formation of compounds of formula XXIX from a compound of formula XIX. 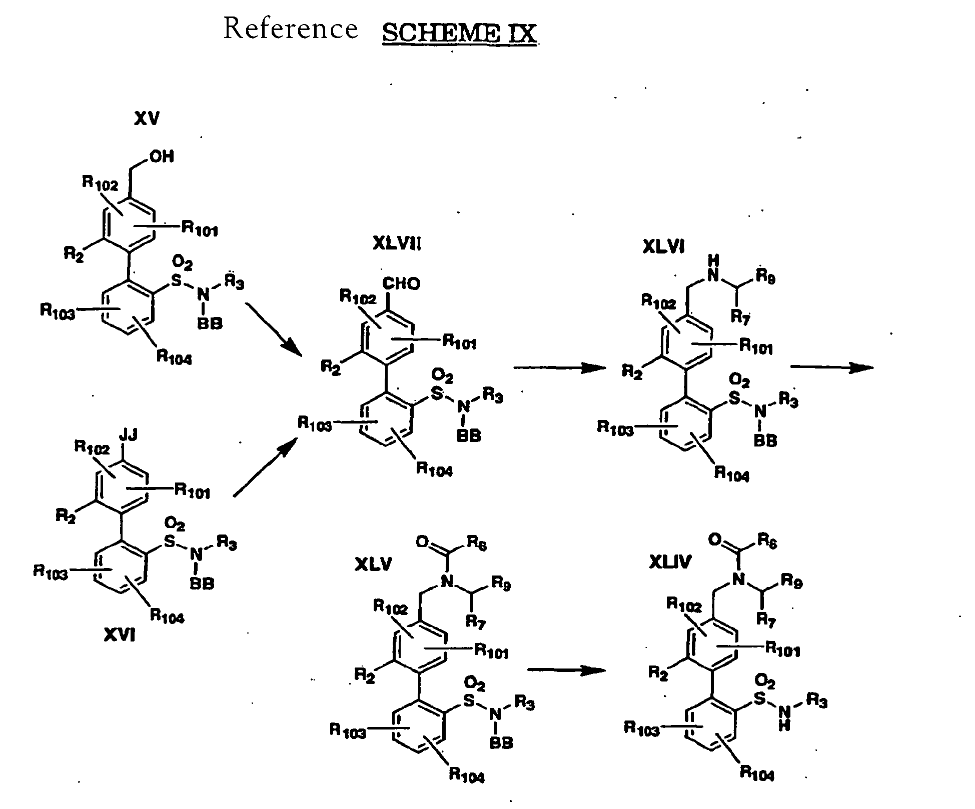 Reference Compounds of formula XLIV (which are compounds of formula I not encompassed in the scope of the claims wherein R₁ is D as defined for formula I) may be prepared from the deprotection of a compound of formula XLV as described in Scheme I.

Compounds of formula XLV may be prepared via the acylation of a compound of formula XLVI using either a carboxylic acid such as R₆COOH in the presence of a suitable coupling agent such as dicyclohexylcarbodiimide, or the corresponding acid chloride or acid anhydride in the presence of a suitable base such as triethylamine.

Compounds of formula XLVI may be prepared from reduction of a compound of formula XLVII in the presence of a primary amine such as H₂NCHR₇R₉ in the presence of a suitable reducing agent such as sodium triacetoxyborohydride.

Compounds of formula XLVII may be prepared via reduction of a compound of formula XVI, as described in Scheme II, wherein JJ is -CN, or -CO₂R₂₀ wherein R₂₀ is hydrogen or C₁ to C₃ alkyl, using means known to one skilled in the art.

Compounds of formula XLVII may also be prepared via oxidation of a compound of formula XV, as defined in Scheme II, using means known to one skilled in the art. 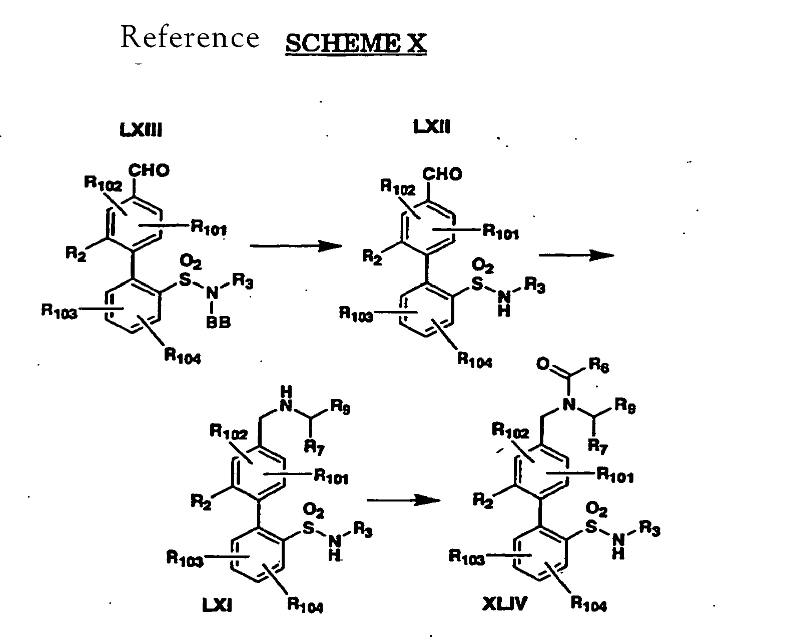 Reference Compounds of formula XLIV (which are compounds of formula I not encompassed in the scope of the claims wherein R₁ is D as defined for formula I) may be prepared by the acylation of a compound of formula LXI using either a carboxylic acid such as R₆COOH in the presence of a suitable coupling agent such as dicyclohexylcarbodiimide, or the corresponding acid chloride or acid anhydride in the presence of a suitable base such as triethylamine.

Compounds of formula LXI may be prepared by the reduction of a compound of formula LXII in the presence of a primary amine such as H₂NCHR₇R₉ in the presence of a suitable reducing agent such as sodium triacetoxyborohydride.

Compounds of formula LXII may be prepared via deprotection of a compound of formula LXIII (which is a compound of formula XVI wherein JJ is CHO), as described in Scheme I.

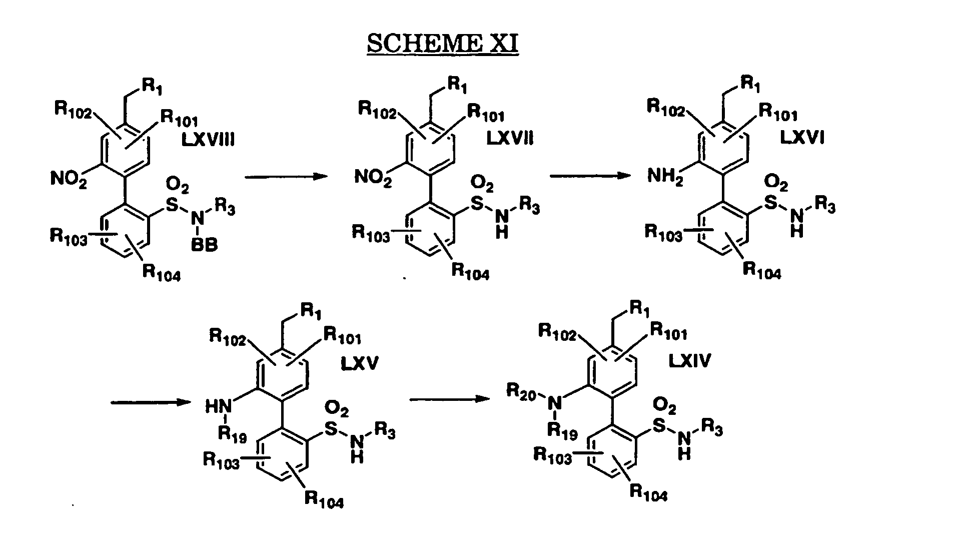

Compounds of formula LXIV (which are compounds of formula I wherein R₂ is -N(R₁₉)₂₀) may be prepared via reduction of a compound of formula LXV in the presence of an aliphatic, aromatic, or heteroaromatic aldehyde using a suitable reducing agent such as sodium triacetoxyborohydride.

Compounds of formula LXV (which are compounds of formula I wherein R₂ is -NHR₁₉) may be similarly prepared via reduction of a compound of formula LXVI in the presence of an aliphatic, aromatic, or heteroaromatic aldehyde using a suitable reducing agent such as sodium triacetoxyborohydride.

Compounds of formula LXVI (which are compounds of formula I wherein R₂is -NH₂) may be prepared by reduction of a compound of formula LXVII using a suitable reducing agent such as tin (II) chloride dihydrate in a suitable solvent such as ethyl acetate.

Compounds of formula LXVII (which are compounds of formula I wherein R₂ is -NO₂) may be prepared by deprotection of a compound of formula LXVIII as described in Scheme I.
Compounds of formula LXVIII (which are compounds of formula II wherein R₂ is -NO₂) may be prepared by the methods described for the preparation of compounds of formula II in Schemes I and II.

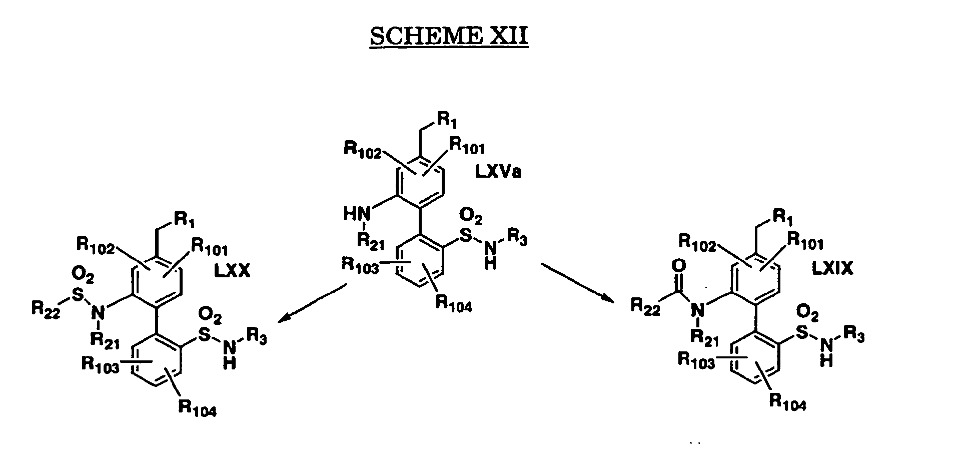

Compounds of formula LXIX (which are compounds of formula I wherein R₂ is -N(R₂₁)(C=O)R₂₂) may be prepared via acylation of a compound of formula LXVa (prepared as described in Scheme XI for a compound of formula LXV) using either a carboxylic acid such as R₂₂COOH in the presence of a suitable coupling agent such as dicyclohexylcarbodiimide, or the corresponding acid chloride or acid anhydride in the presence of a suitable base such as triethylamine.

Compounds of formula LXX (which are compounds of formula I wherein R₂ is -N(R₂₁)SO₂R₂₂) may be prepared via sulfonylation of a compound of formula LXVa using either a sulfonyl chloride such as R₂₂SO₂Cl or the corresponding sulfonic anhydride, in the presence of a suitable base such as triethylamine.

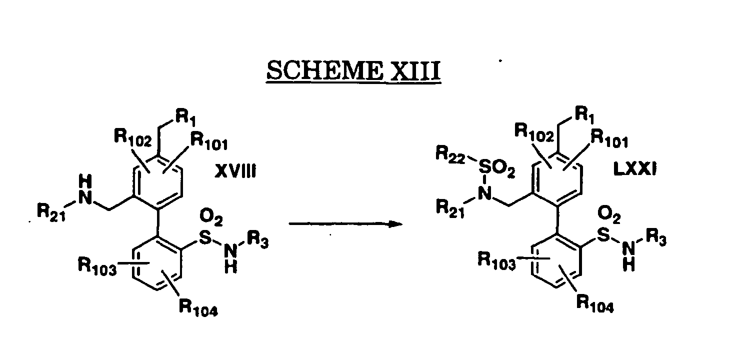

Compounds of formula LXXI (which are compounds of formula I wherein R₂ is -CH₂N(R₂₁)(SO₂R₂₂)) may be prepared via sulfonylation of a compound of formula XVIII (prepared as described in Scheme III) using either a sulfonyl chloride such as R₂₂SO₂Cl or the corresponding sulfonic anhydride in the presence of a suitable base such as triethylamine.

The present invention further provides the following novel compounds, which may be employed as intermediates in the preparation of compounds of the formula I and salts thereof: 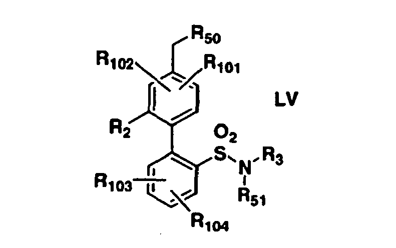 wherein R₂, R₃, R₁₀₁, R₁₀₂, R₁₀₃, and R₁₀₄ are as defined for a compound of formula I, R₅₀ is hydroxy, chloro, bromo, iodo, -OSO₂-alkyl, or -OSO₂-aryl, and R₅₁ is hydrogen, -CH₂OCH₂CH₂OCH₃, -CH₂OCH₂CH₂Si(CH₃)₃, - CH₂OCH₃, -CH₂OCH₂-aryl, or other suitable nitrogen protecting group; 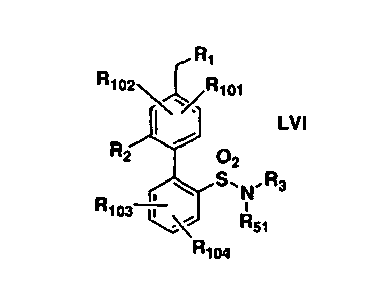 wherein R₁, R₂, R₃, R₁₀₁, R₁₀₂, R₁₀₃, and R₁₀₄ are as defined for a compound of formula I and BB is -CH₂OCH₂CH₂OCH₃, -CH₂OCH₂CH₂Si(CH₃)₃, - CH₂OCH₃, -CH₂OCH₂-aryl, or other suitable nitrogen protecting group; and 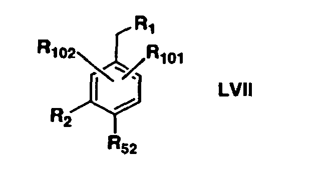 wherein R₁, R₂, , R₁₀₁, and R₁₀₂ are as defined for a compound of formula I, and R₅₂ is chloro, bromo, iodo, or -OSO₂CF₃.

The present invention also provides the following novel method for the preparation of a compound of the formula I or salt thereof, wherein said method comprises at least one of the following steps:
a) displacement of a leaving group R₅₀ via the anion of a compound R₁-H from a compound of the formula 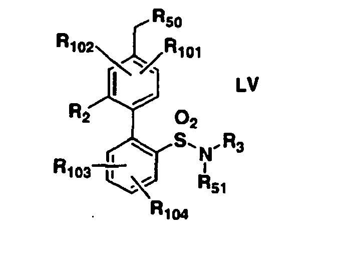 wherein R₁, R₂, R₃, R₁₀₁, R₁₀₂, R₁₀₃, and R₁₀₄ are as defined for a compound of formula I, R₅₀ is hydroxy, chloro, bromo, iodo, -OSO₂-alkyl or -OSO₂-aryl, and R₅₁ is hydrogen or a suitable nitrogen protecting group, using a Mitsunobu reaction or Sₙ1 or Sₙ2 displacement reaction, with removal of said nitrogen protecting group as appropriate;
b) removal of the nitrogen protecting group R₅₁ from a compound of formula 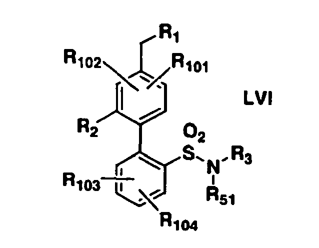 wherein R₁, R₂, R₃, R₁₀₁, R₁₀₂, R₁₀₃, and R₁₀₄ are as defined for a compound of formula I, and R₅₁ is a suitable nitrogen protecting group;
c) organometallic coupling of a compound of formula 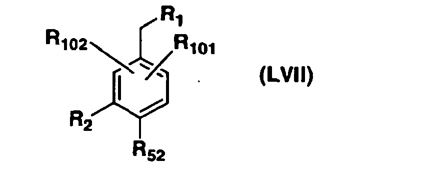 with a compound of formula 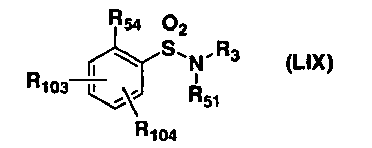 wherein R₁, R₂, R₃, R₁₀₁, R₁₀₂, R₁₀₃, and R₁₀₄ are as defined for a compound of formula I and R₅₁ is a suitable nitrogen protecting group. R₅₂ is chloro, bromo, iodo or -OSO₂CF₃ and R₅₄ is a boronic acid, boronic ester or stannane derivative. Or R₅₂ is a boronic acid, boronic ester or stannane derivative, and R₅₄ is chloro, bromo, iodo or -OSO₂CF₃;
d) acylation of a compound of the formula 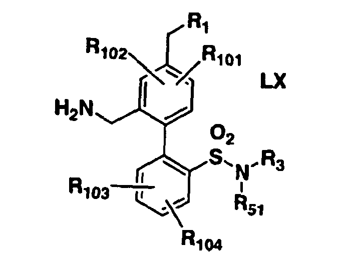 wherein R₁, R₃, R₁₀₁, R₁₀₂, R₁₀₃, and R₁₀₄ are as defined for a compound of formula I, and R₅₁ is hydrogen or a suitable nitrogen protecting group, with an acylating agent of the formula R₅₅-(C=O)R₂₂, R₁₉N=C=O, R₅₅₋CO₂R₁₈, R₅₅SO₂R₂₂, wherein R₁₈, R₁₉ and R₂₂ are as defined for a compound of formula I and R₅₅ is an activating group for an acid, or made using an acid activating agent, with removal of said nitrogen protecting group as appropriate; or
e) reductive amination of a compound of formula 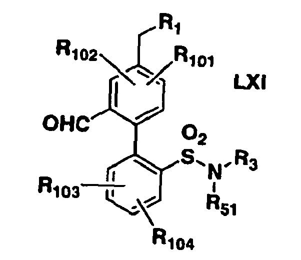 wherein R₁, R₃, R₁₀₁, R₁₀₂, R₁₀₃, and R₁₀₄ are as defined for a compound of formula I, and R₅₁, is hydrogen or a suitable nitrogen protecting group, with an amine of the formula 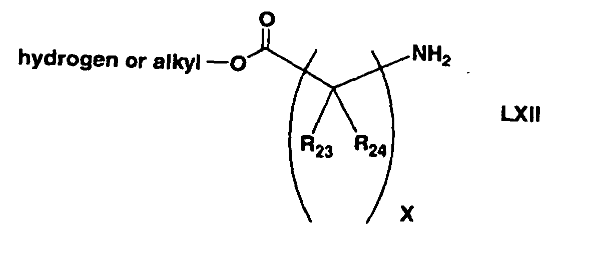 wherein R₂₃, R₂₄, and x are as defined for a compound of formula I, with removal of said nitrogen protecting group as appropriate.

### Utility

The compounds of formula I and salts thereof are antagonists of both endothelin (especially, ET-1) and angiotensin II (especially, subtype AT₁) receptors ("dual angiotensin endothelin receptor antagonists") and are useful in treatment of conditions associated with increased ET levels and/or increased angiotensin II levels and of all endothelin-dependent or angiotensin II-dependent disorders. They are thus useful as antihypertensive agents. By the administration of a composition having one (or a combination) of the compounds of this invention, the blood pressure of a hypertensive mammalian (e.g., human) host is reduced. They are also useful in portal hypertension, hypertension secondary to treatment with erythropoietin and low renin hypertension.

The compounds of the present invention are also useful in the treatment of disorders related to renal, glomerular and mesangial cell function, including acute (such as ischemic, nephrotoxic, or glomerulonephritis) and chronic (such as diabetic, hypertensive or immune-mediated) renal failure, diabetic nephropathy, glomerular injury, renal damage secondary to old age or related to dialysis, nephrosclerosis (especially hypertensive nephrosclerosis), nephrotoxicity (including nephrotoxicity related to imaging and contrast agents and to cyclosporine), renal ischemia, primary vesicoureteral reflux, glomerulosclerosis and the like. The compounds of this invention are also useful in the treatment of disorders related to paracrine and endocrine function. The compounds of this invention are also useful in the treatment of diabetic nephropathy, hypertension-induced nephropathy, and IGA-induced nephropathy.

The compounds of the present invention are also useful in the treatment of endotoxemia or endotoxin shock as well as hemorrhagic shock. The compounds of the present invention are also useful in alleviation of pain associated cancer, such as the pain associated with prostate cancer, and bone pain associated with bone cancer. The compounds of the present invention are further useful in the prevention and/or reduction of end-organ damage associated the cell-poliferative effects of endothelin.

The compounds of the present invention are also useful in hypoxic and ischemic disease and as anti-ischemic agents for the treatment of, for example, cardiac, renal and cerebral ischemia and reperfusion (such as that occurring following cardiopulmonary bypass surgery), coronary and cerebral vasospasm, and the like.

In addition, the compounds of this invention are also useful as antiarrhythmic agents; anti-anginal agents; anti-fibrillatory agents; antiasthmatic agents; anti-atherosclerotic and anti-arteriosclerotic agents (including anti-transplantation arteriosclerotic agents); additives to cardioplegic solutions for cardiopulmonary bypasses; adjuncts to thrombolytic therapy; and anti-diarrheal agents. The compounds of this invention may be useful in therapy for myocardial infarction; therapy for peripheral vascular disease (e.g., Raynaud's disease, intermittent claudication and Takayashu's disease); treatment of cardiac hypertrophy (e.g., hypertrophic cardiomyopathy); treatment of primary pulmonary hypertension (e.g., plexogenic, embolic) in adults and in the newborn and pulmonary hypertension secondary to heart failure, radiation and chemotherapeutic injury, or other trauma; treatment of central nervous system vascular disorders, such as stroke, migraine and subarachnoid hemorrhage; treatment of central nervous system behavioral disorders; treatment of gastrointestinal diseases such as ulcerative colitis, Crohn's disease, gastric mucosal damage, ulcer, inflammatory bowel disease and ischemic bowel disease; treatment of gall bladder or bile duct-based diseases such as cholangitis; treatment of pancreatitis; regulation of cell growth; treatment of benign prostatic hypertrophy; restenosis following angioplasty or following any procedure including transplantation and stenting; therapy for congestive heart failure including inhibition of fibrosis; inhibition of left ventricular dilatation, remodeling and dysfunction; and treatment of hepatotoxicity and sudden death. The compounds of this invention are useful in the treatment of sickle cell disease including the initiation and/or evolution of the pain crises of this disease; treatment of the deleterious consequences of ET-producing tumors such as hypertension resulting from hemangiopericytoma; treatment of early and advanced liver disease and injury including attendant complications (e.g., hepatotoxicity, fibrosis and cirrhosis); treatment of spastic diseases of the urinary tract and/or bladder; treatment of hepatorenal syndrome; treatment of immunological diseases involving vasculitis such as lupus, systemic sclerosis, mixed cryoglobulinemia; and treatment of fibrosis associated with renal dysfunction and hepatotoxicity. The compounds of this invention are useful in therapy for metabolic and neurological disorders; cancer; insulin-dependent and non insulin-dependent diabetes mellitus; neuropathy; retinopathy; epilepsy; hemorrhagic and ischemic stroke; bone remodeling; psoriasis; and chronic inflammatory diseases such as arthritis, rheumatoid arthritis, osteoarthritis, sarcoidosis and eczematous dermatitis (all types of dermatitis).

The compounds of this invention are additionally useful in the treatment of disorders involving bronchoconstriction and disorders of chronic or acute pulmonary inflammation such as chronic obstructive pulmonary disease (COPD) and adult respiratory distress syndrome (ARDS).

The compounds of this invention are also useful in the treatment of sexual dysfunction in both men (erectile dysfunction, for example, due to diabetes mellitus, spinal cord injury, radical prostatectomy, psychogenic etiology or any other cause) and women by improving blood flow to the genitalia, especially, the corpus cavemosum.

The compounds of this invention are also useful in the treatment of dementia, including Alzheimer's dementia, senile dementia and vascular dementia.

Additionally the compounds of the present invention are further useful in the reduction of general morbidity and/or mortality as a result of the above utilities.

The present invention thus provides methods for the treatment of all endothelin-dependent or angiotensin II-dependent disorders, comprising the step of administering to a subject in need thereof at least one compound of the formula I in an amount effective therefor. Other therapeutic agents such as those described below may be employed with the inventive compounds in the present methods. In the methods of the present invention, such other therapeutic agent(s) may be administered prior to, simultaneously with or following the administration of the compound(s) of the present invention.

The effective amount of a compound of the present invention may be determined by one of ordinary skill in the art, and includes exemplary dosage amounts for a human of from about 0.1 to about 100 mg/kg, preferably about 0.2 to about 50 mg/kg and more preferably from about 0.5 to about 25 mg/kg of body weight (or from about 1 to about 2500 mg, preferably from about 5 to about 500 mg) of active compound per day, which may be administered in a single dose or in the form of individual divided doses, such as from 1 to 4 times per day. It will be understood that the specific dose level and frequency of dosage for any particular subject may be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats and the like, subject to endothelin-dependent or angiotensin II-dependent disorders.

The present invention also provides pharmaceutical compositions comprising at least one of the compounds of the formula I capable of treating an endothelin-dependent or angiotensin II-dependent disorder in an amount effective therefor, and a pharmaceutically acceptable vehicle or diluent. The compositions of the present invention may contain other therapeutic agents as described below, and may be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation or called for by accepted pharmaceutical practice.

The compounds of the formula I may be administered by any suitable means, for example, orally, such as in the form of tablets, capsules, granules or powders; sublingually; buccally; parenterally, such as by subcutaneous, intravenous, intramuscular, or intrasternal injection or infusion techniques (e.g., as sterile injectable aqueous or non-aqueous solutions or suspensions); nasally such as by inhalation spray; topically, such as in the form of a cream or ointment; or rectally such as in the form of suppositories; in dosage unit formulations containing non-toxic, pharmaceutically acceptable vehicles or diluents. The present compounds may, for example, be administered in a form suitable for immediate release or extended release. Immediate release or extended release may be achieved by the use of suitable pharmaceutical compositions comprising the present compounds, or, particularly in the case of extended release, by the use of devices such as subcutaneous implants or osmotic pumps. The present compounds may also be administered liposomally. For example, the active substance can be utilized in a composition such as tablet, capsule, solution or suspension containing about 5 to about 500 mg per unit dosage of a compound or mixture of compounds of formula I or in topical form for wound healing (0.01 to 5% by weight compound of formula I, 1 to 5 treatments per day). They may be compounded in a conventional manner with a physiologically acceptable vehicle or carrier, excipient, binder, preservative, stabilizer, flavor, etc., or with a topical carrier. The compounds of formula I can also be formulated in compositions such as sterile solutions or suspensions for parenteral administration. About 0.1 to 500 milligrams of a compound of formula I may be compounded with a physiologically acceptable vehicle, carrier, excipient, binder, preservative, stabilizer, etc., in a unit dosage form as called for by accepted pharmaceutical practice. The amount of active substance in these compositions or preparations is preferably such that a suitable dosage in the range indicated is obtained.

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations may also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g., Carbopol 934). Lubricants, glidants, flavors, coloring agents and stabilizers may also be added for ease of fabrication and use.

Exemplary compositions for nasal aerosol or inhalation administration include solutions in saline which may contain, for example, benzyl alcohol or other suitable preservatives, absorption promoters to enhance bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid.

Exemplary compositions for rectal administration include suppositories which may contain, for example, a suitable non-irritating excipient, such as cocoa butter, synthetic glyceride esters or polyethylene glycols, which are solid at ordinary temperatures, but liquify and/or dissolve in the rectal cavity to release the drug.

Exemplary compositions for topical administration include a topical carrier such as Plastibase (mineral oil gelled with polyethylene). For example, the compounds of the invention may be administered topically to treat peripheral vascular diseases and as such may be formulated as a cream or ointment.

The compounds of the present invention may be employed alone or in combination with each other and/or other suitable therapeutic agents useful in the treatment of endothelin-dependent or angiotensin II-dependent disorders. For example, the compounds of this invention can be formulated in combination with endothelin converting enzyme (ECE) inhibitors, such as phosphoramidon; thromboxane receptor antagonists such as ifetroban; potassium channel openers; thrombin inhibitors (e.g., hirudin and the like); growth factor inhibitors such as modulators of PDGF activity; platelet activating factor (PAF) antagonists; anti-platelet agents such as GPIIb/IIIa blockers (e.g., abciximab, eptifibatide, and tirofiban), P2Y(AC) antagonists (e.g., clopidogrel, ticlopidine and CS-747), and aspirin; anticoagulants such as warfarin, low molecular weight heparins such as enoxaparin, Factor VIIa inhibitors, and Factor Xa inhibitors such as those described in U.S. Serial No. 09/496,571 filed February 2, 2000 (attorney docket HA 723); renin inhibitors; angiotensin converting enzyme (ACE) inhibitors such as captopril, zofenopril, fosinopril, ceranapril, alacepril, enalapril, delapril, pentopril, quinapril, ramipril, lisinopril and salts of such compounds; neutral endopeptidase (NEP) inhibitors; vasopepsidase inhibitors (dual NEP-ACE inhibitors) such as omapatrilat and gemopatrilat; HMG CoA reductase inhibitors such as pravastatin, lovastatin, atorvastatin, simvastatin, NK-104 (a.k.a. itavastatin, or nisvastatin or nisbastatin) and ZD-4522 (a.k.a. rosuvastatin, or atavastatin or visastatin); squalene synthetase inhibitors; fibrates; bile acid sequestrants such as questran; niacin; anti-atherosclerotic agents such as ACAT inhibitors; MTP inhibitors such as those described in U.S. Serial No. 09/007,938 filed January 16, 1998 (attorney docket HX 91); calcium channel blockers such as amlodipine besylate; potassium channel activators; alpha-adrenergic agents, beta-adrenergic agents such as carvedilol and metoprolol; antiarrhythmic agents; diuretics, such as chlorothiazide, hydrochlorothiazide, flumethiazide, hydroflumethiazide, bendroflumethiazide, methylchlorothiazide, trichloromethiazide, polythiazide or benzothiazide as well as ethacrynic acid, tricrynafen, chlorthalidone, furosemide, musolimine, bumetanide, triamterene, amiloride and spironolactone and salts of such compounds; thrombolytic agents such as tissue plasminogen activator (tPA), recombinant tPA, streptokinase, urokinase, prourokinase and anisoylated plasminogen streptokinase activator complex (APSAC); anti-diabetic agents such as biguanides (e.g. metformin), glucosidase inhibitors (e.g., acarbose), insulins, meglitinides (e.g., repaglinide), sulfonylureas (e.g., glimepiride, glyburide, and glipizide), biguanide/glyburide combinations such as those described in U.S. Serial No. 09/432,465 filed November 3, 1999 (attorney docket LA 46) and U.S. Serial No. 09/460,920 filed December 14, 1999 (attorney docket LA 46a); thiozolidinediones (e.g. troglitazone, rosiglitazone and pioglitazone), and PPAR-gamma agonists; mineralocorticoid receptor antagonists such as spironolactone and eplerenone; growth hormone secretagogues such as those described in U.S. Serial No. 09/417,180 filed October 12, 1999 (attorney docket LA 25) and U.S. Serial No. 09/506,749 filed February 18, 2000 (attorney docket LA 26); aP2 inhibitors such as those described in U.S. Serial No. 09/391,053 filed September 7, 1999 (attorney docket LA 24a) and U.S. Serial No. 09/390,275 filed September 7, 1999 (attorney docket LA 24b); digitalis; ouabian; non-steroidal antiinflammatory drugs (NSAIDS) such as aspirin and ibuprofen; phosphodiesterase inhibitors such as PDE III inhibitors (e.g., cilostazol) and PDE V inhibitors (e.g., sildenafil); protein tyrosine kinase inhibitors; antiinflammatories; antiproliferatives such as methotrexate, FK506 (tacrolimus, Prograf), mycophenolate and mofetil; chemotherapeutic agents; immunosuppressants; anticancer agents and cytotoxic agents (e.g., alkylating agents, such as nitrogen mustards, alkyl sulfonates, nitrosoureas, ethylenimines, and triazenes); antimetabolites such as folate antagonists, purine analogues, and pyrimidine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids (e.g., cortisone), estrogens/antiestrogens, androgens/antiandrogens, progestins, and luteinizing hormone-releasing hormone anatagonists, octreotide acetate; microtubule-disruptor agents, such as ecteinascidins or their analogs and derivatives; microtubule-stabilizing agents such as paclitaxel (Taxol®), docetaxel (Taxotere®), and epothilones A-F or their analogs or derivatives; plant-derived products, such as vinca alkaloids, epipodophyllotoxins, taxanes; and topoisomerase inhibitors; prenyl-protein transferase inhibitors; and miscellaneous agents such as, hydroxyurea, procarbazine, mitotane, hexamethylmelamine, platinum coordination complexes such as cisplatin and carboplatin); cyclosporins; steroids such as prednisone or dexamethasone; gold compounds; cytotoxic drugs such as azathiprine and cyclophosphamide; TNF-alpha inhibitors such as tenidap; anti-TNF antibodies or soluble TNF receptor such as etanercept (Enbrel) rapamycin (sirolimus or Rapamune), leflunimide (Arava); and cyclooxygenase-2 (COX-2) inhibitors such as celecoxib (Celebrex) and rofecoxib (Vioxx).

If formulated as a fixed dose, such combination products employ the compounds of this invention within the dosage range described below and the other pharmaceutically active agent within its approved dosage range. The compounds of this invention may also be formulated with, or useful in conjunction with, antifungal and immunosuppressive agents such as amphotericin B, cyclosporins and the like to counteract the glomerular contraction and nephrotoxicity secondary to such compounds. The compounds of this invention may also be used in conjunction with hemodialysis.

The above other therapeutic agents, when employed in combination with the compounds of the present invention, may be used, for example, in those amounts indicated in the Physicians' Desk Reference (PDR) or as otherwise determined by one of ordinary skill in the art.

The following assays may be employed in ascertaining the degree of activity of a compound ("drug") as an endothelin and angiotensin II receptor antagonist. Compounds described in the following Examples have been tested in these assays, and have shown activity.

### ET_{A/B} Attached cell binding assay

CHO-K1 cells expressing either the human endothelin A or endothelin B receptor were cultured in Ham's F12 media (Gibco/BRL, Grand Island, NY) with 10% fetal bovine serum (Hyclone), supplemented with 300 µg/mL Geneticin (G-418 Gibco BRL Products, Grand Island, NY) and maintained at 37°C with 5% CO₂ in a humidified incubator. Twenty four hours prior to assay, the cells were treated with 0.25% trypsin-EDTA and were seeded in Falcon, 96 well tissue culture plates at a density of 1.8 x 10⁴ cells/ well (the monolayer should reach 80-90% confluency by the day of assay).

In the attached cell assay, culture media was aspirated from each well and the monolayers were washed with 50 µl of PBS (Mg⁺⁺, Ca⁺⁺ free). The binding assay was performed in a total volume of 125 µl consisting of assay buffer (50 mM Tris, pH 7.4, including 1% BSA, and 2 µM phosphoramidon), and 25 µl of either 500 nM ET-1 (to define nonspecific binding) or competing drug. The reaction was initiated with the addition of 25 µl of 0.25 nM [¹²⁵I]-ET-1 (New England Nuclear). Incubation was carried out with gentle orbital shaking, at 4°C, reaching equilibrium at 4 hours. The reaction was terminated by aspiration of the reaction buffer and two subsequent washes with cold PBS (Mg⁺⁺, Ca⁺⁺ free). The cells were dissociated by the addition of 100 µl of 0.5N NaOH followed by incubation for 40 minutes. Samples were then transferred from the 96 well format into tubes for counting in a Cobra gamma counter (Packard). Data was analyzed with curve fitting software by Sigma plot.

### RASMC binding assay

Assays were conducted in a total volume of 250 µL in 96 well microtitre plates. The incubation mixture contained 50 µL [125]I-Sar-Ile-Angiotensin II (0.2 nM), 25 µL of drug dissolved in DMSO, or angiotensin II (1 µM) to define non-specific binding. Binding to rat aortic smooth muscle cells (RASMCs) was conducted in RPMI media (Gibco BRL Products, Grand Island, NY) containing 0.1% BSA for 2 hours at room temperature with continuous shaking. Unbound radioligand was washed from the wells. The RASMCs with bound radioligand are lysed with 1% Triton X and 0.1% BSA in distilled water for 15 minutes at room temperature with continuous shaking. The solution in each well was transferred to tubes and placed in a gamma counter.

Compounds within the scope of this invention include compounds that have an IC₅₀ concentration of less than 100 micromolar versus either or both [125]I-Sar-Ile-Angiotensin II or [¹²⁵I]-ET-1, ideally against both ligands. Preferred compounds within the scope of this invention are compounds that have an IC₅₀ concentration of less than 5 micromolar versus either or both [125]I-Sar-Ile-Angiotensin II or [¹²⁵I]-ET-1, ideally against both ligands. More preferred compounds within the scope of this invention are compounds that have an IC₅₀ concentration of less than 1 micromolar versus either or both [125]I-Sar-Ile-Angiotensin II or [¹²⁵I]-ET-1, ideally against both ligands.

All documents cited in the present specification are incorporated herein by reference in their entirety.

The following Examples illustrate embodiments of the present invention, and are not intended to limit the scope of the claims. Abbreviations employed herein are defined below. Compounds of the Examples are identified by the example and step in which they are prepared (for example, **"1A"** denotes the title compound of step A of Example 1), or by the example only where the compound is the title compound of the example (for example, "**4**" denotes the title compound of Example 4). Compounds prepared for use as synthetic intermediates are identified by the Preparation number and step in which they appear, prefaced by the letter "**P**." For example, **"P1A"** denotes the compound generated in step A of Preparation 1, while **"P1"** denotes the title compound of Preparation 1.

### Abbreviations

Ac = acetyl
(S)-BINAP = (S)-(-)2,2'-bis(diphenylphosphino)-1,1'-binaphthyl
BOC = t-butoxycarbonyl
*n*-Bu = *n*-butyl
BSA = bovine serum albumin
CDI = 1,1' carbonyldiimidazole
d = days
DBU = 1,8-diazabicyclo[5.4.0]undec-7-ene
DIBAL-H = diisobutylaluminum hydride
DMF = N,N-dimethylformamide
DMSO = dimethylsulfoxide
EDCI = 1-ethyl-3-[3-(dimethylamino)propyl]carbodiimide hydrochloride
EDTA = ethylenediaminetetraacetic acid
eq = equivalents
Et = ethyl
ET = endothelin
ET-1 = endothelin-1
EtOAc = ethyl acetate
EtOH = ethanol
h = hours
Me = methyl
MEM = methoxyethoxymethyl
MeOH = methanol
min = minutes
mp = melting point
Ms = methanesulfonyl
NBS = N-bromosuccinimide
PBS = phosphate buffered saline
Ph = phenyl
*n*-Pr = *n*-propyl
SEM = 2-(trimethylsiloxy)ethoxymethyl
Rochelle's salt = potassium sodium tartrate tetrahydrate
RT = room temperature
TFA = trifluoroacetic acid
THF = tetrahydrofuran

### General Methods

The following General Methods were employed in the Preparations and Examples.

### General Method 1: Suzuki Coupling of Aryl Bromides with Arylboronic Acids

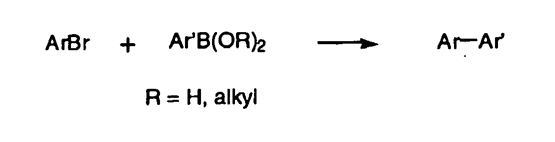

A solution of 1.0 eq of an arylboronic acid (or ester) and the appropriate aryl bromide (1.0 eq) in 2:1 toluene:ethanol (0.1 M concentration for each reagent) was sparged with nitrogen for 15 minutes. Tetrakis (triphenylphosphine)palladium (0) (0.05 eq) and 2 M aqueous sodium carbonate (3 eq) were added and the mixture was heated at 85°C for 3 h under a nitrogen atmosphere. The mixture was cooled and ethyl acetate and water were added. The organic layer was washed once with saturated aqueous sodium carbonate, dried over sodium sulfate, and concentrated. The residue was chromatographed on silica gel using hexanes/ethyl acetate as eluant to yield the biaryl product.

Arylboronic acids used: [2-[[(3,4-dimethyl-5-isoxazolyl)[(2-methoxyethoxy)-methyl]amino]-sulfonyl]phenyl]boronic acid (or the corresponding SEM-protected compound, both of which were prepared as described in U.S. Patent No. 5,612,359); [2-[[(4,5-dimethyl-3-isoxazolyl)[(2-methoxyethoxy)methyl]amino]-sulfonyl]phenyl]boronic acid (prepared as described in U.S. Patent No. 5,612,359 and U.S. Patent Application Serial No. 09/013,952, filed January 27, 1998); [2-*(N-tert-*butylsulfamoyl)phenyl]boronic acid (prepared according to Chang, L. L. et al., *J. Med Chem.,* 38, 3741-3758 (1995)).

Arylboronate ester used: N-[(2-methoxyethoxy)methyl]-N-(3,4-dimethyl-5-isoxazolyl)-2-(4,4,5,5-tetramethyl-1,3,2-dioxaborolan-2-yl)benzenesulfonamide (prepared as described in WO 97/29747).

### General Method 2: Conversion of Primary Alcohols to Alkyl Bromides

RCH₂OH → RCH₂Br

To a 0.2 M solution of the alcohol in DMF at 0 °C was added carbon tetrabromide (1.5 eq) followed by triphenylphosphine (1.5 eq). The mixture was stirred at 0 °C for 4 h, diluted with 10 parts 2:1 hexanes/ethyl acetate, and washed with water and brine. The solution was dried over sodium sulfate and concentrated, and the residue chromatographed on silica gel using hexanes/ethyl acetate as eluant to yield the alkyl bromide product.

### General Method 3: Conversion of Primary Alcohols to Alkyl Methanesulfonates

RCH₂OH → RCH₂OMs

To a 0.15 M solution of the alcohol in dichloromethane at 0 °C was added *N,N*-diisopropylethylamine (1.5 eq) followed by methanesulfonyl chloride (1.1 eq). The mixture was stirred at 0 °C for 1 to 3 h, and was then treated with 10% aqueous potassium dihydrogensulfate. The aqueous layer was extracted once with dichloromethane and the combined organic layers were dried over sodium sulfate and concentrated to yield the crude alkyl methanesulfonate.

### General Method 4: Alkylation of Heterocycles or Aliphatic Alcohols

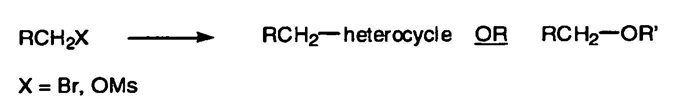

Sodium hydride (60% dispersion in mineral oil, 1.2 eq) was added at 0 °C to a 1.0 M solution or suspension of an appropriate heterocycle or alcohol (1.5 eq) in DMF. The mixture was allowed to warm to RT, was stirred for 20 min, and was then cooled back to 0 °C. To the heterocycle mixture was added a solution of the appropriate alkyl bromide or alkyl methanesulfonate (1.0 eq) in a minimal amount of DMF. The resultant mixture was allowed to warm to RT and was stirred for 16-24 h. The reaction mixture was diluted with EtOAc and washed with water and brine. The organic layer was dried over sodium sulfate and concentrated, and the residue chromatographed on silica gel with hexanes/ethyl acetate as eluant to yield the alkylation product.

### Heterocycles Used:

| | | |
|---|---|---|
| 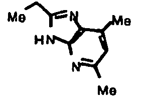 | 2-ethyl-5,7-dimethyl-3H-imidazo[4,5-b]pyridine | Senanayake, C. H., et. al. *Heterocycles* **1996**, *42*, 821-830. |
| 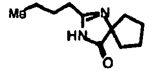 | 2-butyl-1,3-diazaspiro[4.4]non-1-en-4-one | Bernhart, C. A., et. al. J. *Med. Chem. ,* **1993,** *36*, 3371-3380. |
| 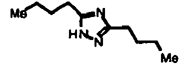 | 3,5-di-n-butyl-1,2,4-triazole | Reitz, D.B., et al. *Biorganic & Medicinal chemistry Letters,* **1994,** 4(1), 99-104 |
| 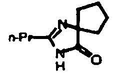 | 2-propyl-1,3-diazaspiro[4.4]non-1-en-4-one | Bernhart, C. A., et. al. *J*. *Med. Chem.,* **1993**, *36*, 3371-3380. |
| 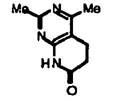 | 7-oxo-2,4-dimethyl-5,6,7,8-tetrahydropyrido[2,3-d]pyrimidine | Hullar, T. L.; French, W. C. *J. Med. Chem.,* **1969**,*12*, 424-426. |
| 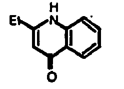 | 2-ethyl-4(1H)-quinolone | Bradbury, R.H.; et. al. *J*. *Med. Chem.* **1992**, *35*, 4027-4038. |
| 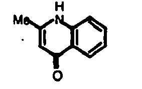 | 2-methyl-4(1H)-quinolone | commercially available |
| 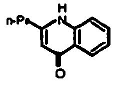 | 2-propyl-4(1H)-quinolone | Bradbury, R.H.; et. al. *J*. *Med. Chem.* **1992**,*35*, 4027-4038. |
| 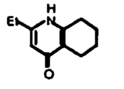 | 2-ethyl-5,6,7,8-tetrahydro-4(1H)-quinolone | Bradbury, R.H.; et. al. *J*. *Med. Chem.* **1993**, *36*, 1245-1254. |
| 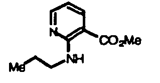 | methyl 2-(N-propylamino)pyridine-3-carboxylate | De, B.; et. al. *J. Med. Chem.* **1992**, *35*, 3714-3717 |
| 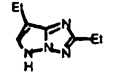 | 2,7-diethyl-5H-pyrazolo[1,5-b] [1,2,4]triazole | U,S, Patent No. 5,475,114 |
| 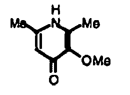 | 3-methoxy-2,6-dimethyl-4(4H)-pyridinone | Voss, G.; et. al. *Liebigs Ann Chem.* **1982**, 1466-1477 |
| 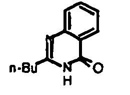 | 2-butyl-4(3H)-quinazolinone | Allen, E. C. ; et. al *Biorganic & Medicinal Chemistry Let.* **1993,** *3(6),* 1293-1298 |
| 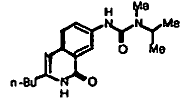 | N- (2-butyl-3,4-dihydro-4-oxo-6- quinazolinonyl)-N'-isopropyl-N'methylurea | Laszlo, S. E.; et al *Biorganic & Medicinal Chemistry Let.* **1993,** *3(6)*, 1299-1304 |

### General Method 5: Reductive Amination

ArCHO + RNH₂ [or RNH₂•HCl] → ArCH₂-NHR

To a mixture of an aromatic aldehyde (1.0 eq) and a primary amine (1.2 eq) in dichloromethane (0.1 M aldehyde concentration) was added 4 Å molecular sieves (5g per mmol aldehyde). [Alternately, a primary amine hydrochloride (1.2 eq) and triethylamine (1.2 eq) could be substituted for the primary amine free base.] The mixture was stirred vigorously for 1 h, after which sodium triacetoxyborohydride (1.5 eq) was added. The mixture was stirred vigorously at RT, while the course of the reaction was monitored by HPLC. If the reaction had not reached completion within several hours, additional sodium triacetoxyborohydride (1.0 eq) was added and monitoring was continued. When the reaction was complete the mixture was filtered through celite, aqueous sodium bicarbonate solution was added to the filtrate, and the aqueous layer was extracted with dichloromethane. The combined organic extracts were dried over sodium sulfate and evaporated. The crude residue was carried on without further purification.

In general, reductive amination with a 4-aminobutanoic acid resulted in a lactam product. In a few cases, cyclization was promoted by treatment of a 0.1 M solution of the crude amino acid product in dichloromethane with 1.0 eq of diisopropylcarbodiimide for 1 h at RT.

### General Method 6: Amine Acylation

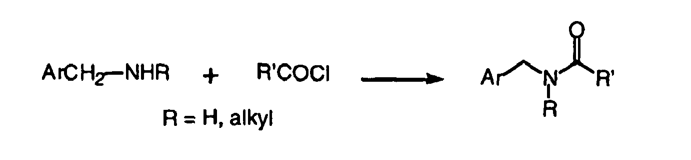

A 0.15 M solution of a primary or secondary amine (1.0 eq) and *N,N*-diisopropylethylamine (2.0 eq) in dichloromethane was treated at RT with an acyl chloride (1.5 eq). After 1.5 h, methanol (10 eq) was added, followed by aqueous sodium carbonate solution. The aqueous layer was extracted with dichloromethane and the combined organic extracts were combined, dired over sodium sulfate, and concentrated. The residue was chromatographed on silica gel with hexanes/ethyl acetate as eluant to provide the product tertiary amide.

### General Method 7: Hydrolysis of SEM or MEM Sulfonamide Protecting Groups Using Hydrochloric Acid/Ethanol

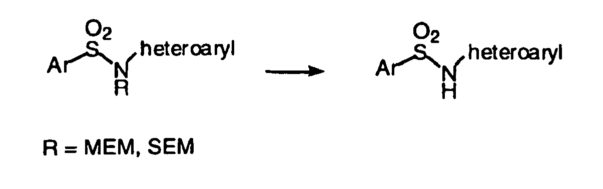

To a 0.1 M solution of a SEM- or MEM-protected N-heteroaryl sulfonamide in one volume of 95% EtOH was added an equal volume of 6N aqueous HCl, and the resulting solution was heated at reflux for 1 h. The reaction mixture was concentrated and the pH of the solution was adjusted to pH 8 using aqueous sodium bicarbonate solution. It was then reacidified to pH 5 with glacial acetic acid. The mixture was extracted with three portions of ethyl acetate. The combined organic extracts were washed with water and brine, dried over sodium sulfate, and concentrated. The residue was purified by reverse-phase preparative HPLC, or by silica gel chromatography using chloroform/methanol or hexanes/acetone as eluant.

### General Method 8: Hydrolysis of SEM or MEM Sulfonamide Protecting Groups Using Hydrogen Chloride in Dioxane/Alcohol

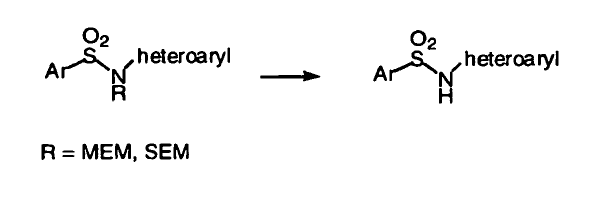

A solution of a SEM- or MEM-protected N-heteroaryl sulfonamide in one volume of absolute methanol or ethanol was treated with two volumes of 4 N hydrogen chloride/dioxane solution (final substrate concentration 0.05 M). The resulting solution was heated at 55 °C for 16 h and was then concentrated. The residue was purified by reverse-phase preparative HPLC, or by extraction with ethyl acetate from aqueous potassium phosphate adjusted to pH 5-6, followed by silica gel chromatography.

### General Method 9: Cleavage of SEM or MEM Sulfonamide Protecting Groups Using Trimethylsilyl Iodide

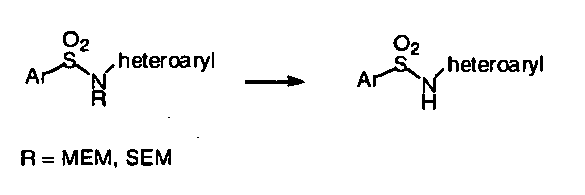

To a 0.1 M solution of of a SEM- or MEM-protected N-heteroaryl sulfonamide in acetonitrile was added trimethylsilyl chloride (8 eq) followed by sodium iodide (8 eq). The mixture was stirred at RT for 30 min and was then poured onto water and ethyl acetate. The organic layer was washed with saturated sodium sulfite and brine, and was then dried over sodium sulfate and concentrated. The residue was purified by reverse-phase preparative HPLC or by silica gel chromatography.

### General Method 10: Cleavage of SEM Sulfonamide Protecting Groups Using Tetrabutylammonium Fluoride

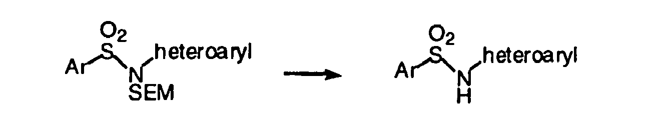

To a 0.05 M solution of a SEM-protected N-heteroaryl sulfonamide in THF was added freshly activated 4Å molecular sieves (20 g per mmol sulfonamide), followed by tetrabutylammonium fluoride (1.0 M solution in THF, 3 eq). The mixture was heated at 55°C for 1-2 h, then was cooled and filtered through celite. The filter cake was rinsed with methanol, then aqueous potassium dihydrogen phosphate solution was added to the filtrate and the mixture partially concentrated. The residue was adjusted to pH 4-5 using dilute hydrochloric acid, and the mixture was extracted with two portions of ethyl acetate. The combined organic extracts were dried over sodium sulfate and concentrated. The residue was purified by reverse-phase preparative HPLC or by silica gel chromatography.

### General Method 11: Reduction of Aryl Aldehydes to Benzylic Alcohols Using Sodium Borohydride

ArCHO → ArCH₂-OH

Sodium borohydride (0.5 eq) was added at 0 °C to a 0.2 M solution of an aromatic aldehyde in absolute ethanol or methanol. The mixture was allowed to warm to RT and stirred for 1-2 h. Aqueous potassium dihydrogen phosphate solution (or dilute hydrochloric acid) was added and the mixture was stirred for an additional 15 min. The mixture was partially concentrated and the residue partitioned between ethyl acetate and water. The aqueous layer was extracted twice with ethyl acetate and the combined organic extracts were dried over sodium sulfate and concentrated. The crude benzylic alcohol was either used directly or was purified by silica gel chromatography using hexanes/ethyl acetate as eluant.

### General Method 12: Amide Formation using 1,1'-Carbonyldiimidazole

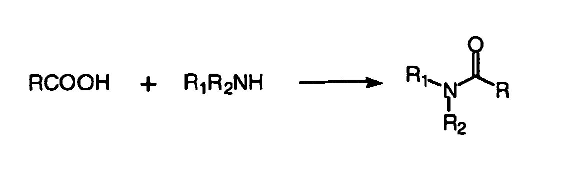

1,1'-Carbonyldiimidazole (2.0 eq) was added to a 0.1 M solution or suspension of an appropriate carboxylic acid (1.0 eq) in THF. The mixture was heated at 50 °C for 1h, and was then cooled to RT. An appropriate amine (5-10 eq) was then added, and the miture was stirred at RT for 12 h. Ethyl acetate and aqueous sodium bicarbonate solution were added and the aqueous layer was extracted with ethyl acetate. The combined organic layers were dried over sodium sulfate and concentrated. The residue was purified by reverse-phase preparative HPLC or by silica gel chromatography.

### General Method 13: Benzylic Bromination Using N-Bromosuccinimide

ArCH₃ → ArCH₂-Br

To a 0.4 M solution of a methyl-substituted aromatic compound in carbon tetrachloride was added N-bromosuccinimide (1.05 eq) and benzoyl peroxide (0.03 eq), and the mixture was heated at reflux for 8-16 h. The mixture was cooled and filtered and the filtrate concentrated. The residue was purified by trituration with 3:1 hexanes/ethyl acetate, or by silica gel chromatography using hexanes/ethyl acetate as eluant to provide the mono-brominated product.

### General Method 14: Reduction of an Aromatic Nitrile to an Aromatic Aldehyde Using DIBAL-H

ArCN → ArCHO

DIBAL-H (1.5 M solution in toluene, 1.5 eq) was added dropwise at 0 °C to a 0.5 M solution of an aromatic nitrile (1.0 eq) in toluene or 9:1 toluene/dichloromethane. The solution was stirred at 0 °C for 1-4 h, and was then treated with excess methanol. After 15 min, 2N hydrochloric acid was added and the mixture was stirred vigorously for an additional 15 min. The layers were separated, and the aqueous layer was extracted with ethyl acetate. The combined organic layers were dried over sodium sulfate and concentrated to yield the crude aldehyde, which was either carried on crude or purified via silica gel chromatography using hexanes/ethyl acetate as eluant.

### General Method 15: Ester Hydrolysis Using Lithium Hydroxide

RCOOR' → RCOOH

A 0.25 M solution of an alkyl ester in 1:1 THF/water was treated with lithium hydroxide hydrate (1.5 eq) at RT. The mixture was stirred for 8-16 h and was then acidified with dilute hydrochloric acid. The product was either isolated by direct filtration from the reaction mixture, or by extraction with ethyl acetate, followed by drying of the organic layers with sodium sulfate, concentration, and silica gel chromatography using methanol/chloroform or hexanes/acetone as eluant.

### General Method 16: Displacement of a Benzylic Bromide or Mesylate with Cyanide

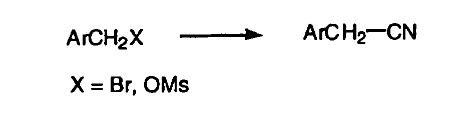

Sodium cyanide (1.2 eq) was added at RT to a 1.0 M solution of a benzylic bromide or mesylate in DMF. The mixture was stirred at RT for 16 h. The reaction mixture was diluted with ethyl acetate and partitioned against aqueous sodium bicarbonate. The organic layer was dried over sodium sulfate and concentrated, and the residue chromatographed on silica gel with hexanes/ethyl acetate as eluant to yield the nitrile product.

### General Method 17: Swern Oxidation of a Benzylic Alcohol to an Aromatic Aldehyde

ArCH₂―OH → ArCHO

Oxalyl chloride (1.5 eq) was added dropwise to a solution of DMSO (2.0 eq) in dichloromethane at -78 °C. After 5 min, a solution of benzylic alcohol substrate (1.0 eq) in dichloromethane was added and the mixture (0.2 M final substrate concentration) was stirred at -78 °C for 15 min. Triethylamine (4.0 eq) was added and the mixture was stirred and allowed to warm to RT. Aqueous sodium bicarbonate solution was added, the layers were separated, and the aqueous layer was extracted with one portion of dichloromethane. The combined organic layers were dried over sodium sulfate, concentrated, and the residue was purified by silica gel chromatography using hexanes/ethyl acetate as eluant.

### General Method 18: Reduction of an Aromatic Nitro Group to an Aromatic Amine Using Tin (II) Chloride Dihydrate

ArNO₂ → ArNH₂

Tin (II) chloride dihydrate (4.0 eq) was added to a 0.05 M solution of an aromatic nitro compound in ethyl acetate and the resulting mixture was heated at 70 °C for 45 min. The mixture was cooled, half-saturated aqueous sodium carbonate solution was added, and the layers were separated. The aqueous layer was extracted once with ethyl acetate, and the combined organic layers were dried over sodium sulfate and concentrated. The residue was chromatographed on silica gel using hexanes/ethyl acetate as eluant to provide the product aromatic amine.

### General Method 19: Hydrolysis of a 2-Aryl-1,3-Dioxolane to an Aromatic Aldehyde

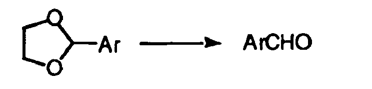

A 0.2 M solution of a 2-aryl-1,3-dioxolane (1.0 eq) in THF was treated with 1N hydrochloric acid (1.5 eq), and the resulting solution was heated at 55 °C for 16 h. The mixture was cooled and neutralized with aqueous sodium bicarbonate solution, then extracted with three portions of ethyl acetate. The combined organic extracts were dried over sodium sulfate and concentrated, and the crude aldehyde was used directly with no further purification.

### General Method 20: Ester Formation Using 1,1'-Carbonyldiimidazole and DBU

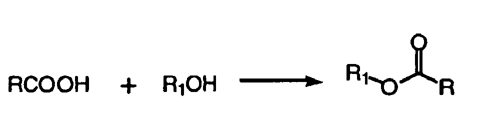

1,1'-Carbonyldiimidazole (2.0 eq) was added to a 0.1 M solution or suspension of an appropriate carboxylic acid (1.0 eq) in THF. The mixture was heated at 50 °C for 1h. An appropriate alcohol (3.0 eq) was then added, followed by DBU (3.0 eq). The mixture was heated at 50 °C for 16 h and was then cooled. Ethyl acetate and 35% aqueous citric acid solution were added, and the organic layer was dried over sodium sulfate and concentrated. The residue was purified by reverse-phase preparative HPLC or by silica gel chromatography.

### General Method 21: Phase-Transfer Alkylation of Imidazoles

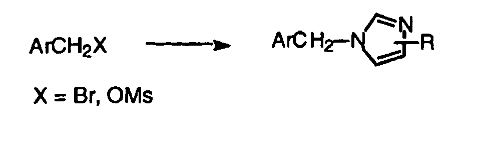

A solution of an appropriate imidazole (0.1 M) in toluene was treated with 50% aqueous sodium hydroxide solution (0.5 ml per mmol imidazole), tetrabutylammonium hydrogen sulfate (0.05 eq), and an appropriate benzylic alkyl bromide or mesylate (0.95 eq). The mixture was stirred vigorously at 40°C for 24 h and was then cooled and filtered. Water was added and the aqueous layer was extracted with two portions of ethyl acetate. The combined organic extracts were dried over sodium sulfate and concentrated to provide the crude product, which was either purified by silica gel chromatography or was carried on crude. Imidazole used: 2-propyl-4,5,6,7-tetrahydro-8-oxocycloheptimidazole (Yanagisawa, T.; et. al. *Biorg. Med. Chem. Lett.* **1993**, *3*, 1559-1564).

### General Method 22: Imidazole or Phenol Alkylation

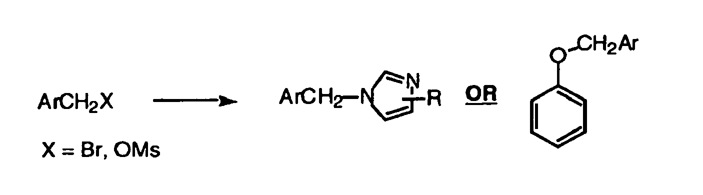

A solution of an appropriate imidazole (0.5 M) in DMF was treated with potassium carbonate (2.0 eq) and a benzylic alkyl bromide or mesylate (1.0 eq) at RT. The mixture was stirred at RT for 16 to 24 h. The solvent was evaporated and the residue was partitioned between ethyl acetate and water. The ethyl acetate layer was dried over sodium sulfate and concentrated to provide the crude product, which was purified by silica gel chromatography or was carried on crude. When mixtures of N-1 and N-3 alkylation products were obtained, the regiochemistry of the alkylation was determined by NOESY spectroscopy.

### Imidazoles Used:

| | | |
|---|---|---|
| 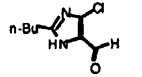 | 2-n-butyl-4-chloro-5-formylimidazole | Watson, S.P. *Synth. Comm.,* **1992**, *22*, 2971-2977 |
| 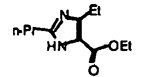 | ethyl 2-n-propyl-4-ethylimidazole-5-carboxylate | Carini, D. J., WO 92/00977 |
| 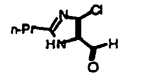 | 2-n-propyl-4-chloro-5-formylimidazole | Watson, S.P. *Synth. Comm.,* **1992**, *22*,2971-2977 |

### General Method 23: Cleavage of SEM Sulfonamide Protecting Groups Using Cesium Fluoride

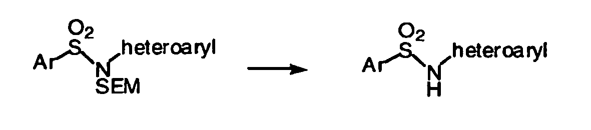

To a 0.05 M solution of a SEM-protected *N*-heteroaryl sulfonamide in DMF was added cesium fluoride (5.0 eq), and the resulting mixture was heated at 130°C for 3 h. The reaction mixture was cooled and the solvent evaporated. Aqueous potassium dihydrogen phosphate solution was added (pH 4-5) and the mixture was extracted with three portions of ethyl acetate. The combined organic extracts were dried over sodium sulfate and concentrated. The residue was purified by reverse-phase preparative HPLC or by silica gel chromatography.

### General Method 24: Sulfonylation of Aromatic Amines

ArNH₂ → ArNHSO₂R

To a 0.1 M solution of an aromatic amine (1.0 eq.) in dichloromethane at -30 °C was added triethylamine (2.6 eq), followed by a sulfonyl chloride (1.4 eq). The mixture was allowed to warm to RT over 3 hr. Aqueous sodium bisulfate was added (final pH 5) and the aqueous layer was extracted with dichloromethane. The combined organic extracts were washed with water and brine, and were then dried over sodium sulfate and concentrated. The residue was chromatographed on silica gel using dichloromethane/methanol as eluant.

### General Method 25: Oxidation of Aromatic Aldehydes to Carboxylic Acids

ArCHO → ArCOOH

A 0.1 M solution of an aromatic aldehyde in 1:1 THF/water was treated at 0 °C with sulfamic acid (1.5 eq) and sodium chlorite (1.5 eq). After 1 h aqueous potassium bisulfate solution was added and the mixture was extracted with ethyl acetate. The combined organic extracts were washed with brine, dried over sodium sulfate and concentrated to provide the crude carboxylic acid, which was used without further purification.

### General Procedure: Purification by Anion Exchange Chromatography

Anion exchange chromatography was performed on Varian SAX cartridges (acetate form, 1.5-3 g) or United Chemical Technologies CUQAX13M6-AC cartridges (acetate form, 3 g). Following a methanol rinse, the cartridge was loaded with a dichloromethane solution of crude product. Elution of impurities with dichloromethane, followed by elution of the desired product with 1-3% TFA in dichloromethane or dichloromethane/methanol, provided the purified product.

### General Procedure: Purification by Reverse-Phase Preparative HPLC

Reverse-phase preparative HPLC was performed with Shimadzu 8A liquid chromatographs using YMC S5 ODS columns (20 x 100, 20 x 250, or 30 x 250 mm). Gradient elution was performed with methanol/water mixtures in the presence of 0.1% TFA. In some cases a product eluting as a TFA salt was subsequently converted to the corresponding free base by extraction from aqueous sodium bicarbonate or sodium carbonate solution.

### Analytical HPLC Methods Employed in Characterization of Examples

Analytical HPLC was performed on Shimadzu LC10AS liquid chromatographs using the following methods:
- A.: Linear gradient of 0 to 100% solvent B over 4 min, with 1 min hold at 100% B;
UV visualization at 220 nm
Column: YMC S5 ODS Ballistic 4.6 x 50 mm
Flowrate: 4 ml/min
Solvent A: 0.1% trifluoroacetic acid, 90% water, 10% methanol
Solvent B: 0.1% trifluoroacetic acid, 90% methanol, 10% water
- B.: Linear gradient of 0 to 100% solvent B over 30 min, with 5 min hold at 100% B;
UV visualization at 254 nm
Column: YMC S3 ODS 6 x 150 mm
Flowrate: 1.5 ml/min
Solvent A: 0.2% phosphoric acid, 90% water, 10% methanol
Solvent B: 0.2% phosphoric acid, 90% methanol, 10% water
- C.: Linear gradient of 0 to 100% solvent B over 4 min, with 1 min hold
at 100% B
UV visualization at 220 nm
Column: YMC S5 ODS Ballistic 4.6 x 50 mm
Flowrate: 4 ml/min
Solvent A: 0.2% phosphoric acid, 90% water, 10% methanol
Solvent B: 0.2% phosphoric acid, 90% methanol, 10% water
- D.: Linear gradient of 45 to 100% solvent B over 2 min, with 1 min hold
at 100% B;
UV visualization at 220 nm
Column: Phenomenex Primesphere 4.6 x 30 mm
Flowrate: 5 ml/min
Solvent A: 0.2% phosphoric acid, 90% water, 10% methanol
Solvent B: 0.2% phosphoric acid, 90% methanol, 10% water
- E.: Same conditions as (B), but with a linear gradient of 40 to 100% solvent B over 30 min, with 5 min hold at 100% B
- F.: Same conditions as (B), but with a linear gradient of 70 to 100% solvent B over 30 min, with 5 min hold at 100% B
- G.: Same conditions as (D), but with a linear gradient of 40 to 100% solvent B over 2 min, with 1 min hold at 100% B
- H.: Linear gradient of 0 to 100% solvent B over 2 min, with 1 min hold at 100% B;
UV visualization at 220 nm
Column: Phenomenex Primesphere 4.6 x 30 mm
Flowrate: 5 ml/min
Solvent A: 0.1% trifluoroacetic acid, 90% water, 10% methanol
Solvent B: 0.1% trifluoroacetic acid, 90% methanol, 10% water
- I.: Same conditions as (B), but with a linear gradient of 50 to 100%
solvent B over 30 min, with 5 min hold at 100% B
- J.: Same conditions as (C), but with a linear gradient of 0 to 100% solvent B over 8 min, with 1 min hold at 100% B
- K.: Same condidtions as (D), but with a linear gradient of 0 to 100% solvent B over 2 min, with a 1 minute hold at 100% B.

### Preparations

### Preparation 1:

### N-(3,4-Dimethyl-5-isoxazolyl)-4'-formyl-2'-(hydroxymethyl)-N-[(2-trimethylsiloxy)ethoxymethyl][1,1'-biphenyl]-2-sulfonamide

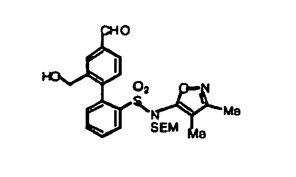

### A. 4-Bromo-3-(bromomethyl)benzonitrile

The product was prepared according to General Method 13 starting from 12.0 g 4-bromo-3-methylbenzonitrile. Partial purification of the crude product was performed by trituration with 3:1 hexanes/ethyl acetate to afford 7.3 g of a slightly yellow solid, which was contaminated with approximately 20 mol% of the starting material.

### B. 4-Bromo-3-(acetoxymethyl)benzonitrile

A mixture of **P1A** (7.3 g), potassium acetate (3.4 g), and DMF (10 ml) was stirred at RT for 16 h. Ethyl acetate was added and the mixture was washed with four portions of water, followed by one portion of brine. The ethyl acetate layer was dried over sodium sulfate and concentrated. The solid residue was partially purified by crystallization from ethyl acetate, yielding 4.5 g of a slightly yellow solid.

### C. 4-Bromo-3-(hydroxymethyl)benzaldehyde

**P1B** (4.4 g) was treated with DIBAL-H according to General Method 14, using 3.5 eq of the reducing agent rather than 1.5 eq. The crude product was an orange oil (4.8 g), judged by ¹H NMR to be approximately 75% pure by weight.

### D. N-(3,4-Dimethyl-5-isoxazolyl)-4'-formyl-2'-(hydroxymethyl)-N-[(2-trimethylsiloxy)ethoxymethyl][1,1'-biphenyl]-2-sulfonamide

**P1C** (4.7 g) was subjected to Suzuki coupling according to General Method 1, yielding 7.6 g of the product as an orange oil following silica gel chromatography (2:1 hexanes/ethyl acetate eluant).

### Preparation 2:

### N-(3,4-Dimethyl-5-isoxazolyl)-4'-bromomethyl-2'-(methoxymethyl)-N-[(2-methoxyethoxy)methyl] [1,1'-biphenyl]-2-sulfonamide

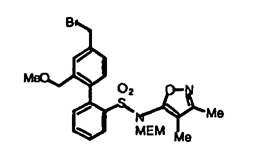

### A. 4-Bromo-3-(bromomethyl)benzonitrile

The product was prepared according to General Method 13 starting from 19.6 g 4-bromo-3-methylbenzonitrile. After cooling the mixture was filtered and the filtrate was washed with H₂O and brine, dried and concentrated. The residue was chromatographed on silica gel using 100:3 and then 100:10 hexane/EtOAc to afford **P2A** (16 g, 58%). R_{f}=0.15, silica gel, 10:1 hexane/EtOAc.

### B. 4-Bromo-3-(Methoxymethyl)benzonitrile

To a solution of **P2A** (6.95 g, 25.28 mmol) in 10 ml DMF, NaOMe (25 wt. % in MeOH, 6.94 ml, 30.3 mmol) was added dropwise. The reaction mixture was stirred at RT for 3 h. Ethyl acetate (100 ml) and hexanes (50 ml) were added, and the mixture was washed twice with water and once with brine. The organic layer was dried over sodium sulfate and concentrated. The residue was chromatographed on silica gel using 100:6 hexane/EtOAc to afford **P2B** (4.70 g, 82%). R_{f}=0.5, silica gel, 5:1 hexane/EtOAc.

### C. 4-Bromo-3-(methoxymethyl)benzaldehyde

**P2B** (7.0 g) was treated with DIBAL-H according to General Method 14, using THF instead of toluene as solvent. The crude product was purified by silica gel chromatography using **11:1** hexanes/ethyl acetate as eluant to give 6.2 g **P2C** as a colorless gum. R_{f}=0.4, silica gel, 5:1 Hexane/EtOAc.

### D. N-(3,4-Dimethyl-5-isoxazolyl)-4'-formyl-2'-(methoxymethyl)-N-[(2-methoxyethoxy)methyl][1,1'-biphenyl]-2-sulfonamide

**P2C** (6.2 g) was subjected to Suzuki coupling according to General Method 1, giving **P2D** as an oil in 83% yield after silica gel chromatography.

### E. N-(3,4-Dimethyl-5-isoxazolyl)-4'-hydroxymethyl-2'-(methoxymethyl)-N-[(2-methoxyethoxy)methyl] [1,1'-biphenyl]-2-sulfonamide

**P2D** (2.8 g) was reduced with sodium borohydride according to General Method 11, to provide 2.8 g **P2E**.

### F. N-(3,4-Dimethyl-5-isoxazolyl)-4'-bromomethyl-2'-(methoxymethyl)-N-[(2-methoxyethoxy)methyl][1,1'-biphenyl]-2-sulfonamide

**P2E** (2.8 g) was treated with triphenylphosphine and carbon tetrabromide according to General Method 2, providing the title compound (2.3 g) in 72% yield.

### Preparation 3:

### 2'-Cyano N-(3,4-dimethyl-5-isoxazolyl) -4'-(hydroxymethyl)-N-[(2-trimethylsiloxy)ethoxymethyl][1,1'-biphenyl]-2-sulfonamide

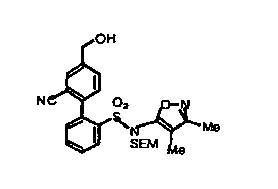

### A. 4'-(Acetoxymethyl)-N-(3,4-dimethyl-5-isoxazolyl) -2'-formyl-N-[(2-trimethylsiloxy)ethoxymethyl][1,1'-biphenyl]-2-sulfonamide acetoxime

Hydroxylamine hydrochloride (1.13 g) was added to a solution of 7.0 g **P4** in 20 ml pyridine and the mixture was stirred at RT for 2 h. Acetic anhydride (5.1 ml) was added and the mixture was stirred for 1 h at **RT**. Ethanol (5 ml) was added and the mixture was concentrated under reduced pressure. The residue was taken up in ethyl acetate and washed twice with 0.1 N hydrochloric acid, twice with half-saturated aqueous sodium carbonate solution, and once with brine. The ethyl acetate layer was dried over sodium sulfate and concentrated to provide **P3A** as an orange oil.

### B. 4'-(Acetoxymethyl)-2-cyano-N-(3,4-dimethyl-5-isoxazolyl)-N-[(2-trimethylsiloxy)ethoxymethyl][1,1'-biphenyl]-2-sulfonamide

**P3A** was dissolved in 75 ml acetonitrile, DBU (4.0 ml) was added, and the mixture was stirred at RT for 14 h. The mixture was concentrated, and the residue was taken up in ethyl acetate and washed twice with 0.1 N hydrochloric acid, then once with half-saturated aqueous sodium carbonate solution. The ethyl acetate layer was dried over sodium sulfate and concentrated to give **P3B** as an orange oil.

### C. 2'-Cyano N-(3,4-dimethyl-5-isoxazolyl) -4'-(hydroxymethyl)-N-[(2-trimethylsiloxy)ethoxymethyl][1,1'-biphenyl]-2-sulfonamide

**P3B** was dissolved in 150 ml methanol, potassium carbonate (1.5 g) was added, and the mixture was stirred at RT for 2 h. 2N Hydrochloric acid (5.5 ml) was added and the mixture was concentrated. The residue was taken up in ethyl acetate and partitioned against aqueous sodium bicarbonate solution. The ethyl acetate layer was dried over sodium sulfate and concentrated to yield 5.7 g of the crude title compound, which was used without further purification.

Preparations 4 through 22 were performed by application of the General Methods and are listed in the following Table.

**Preparations by General Methods**

| No. | Structure | Name | Starting Material | Starting Material Reference | General Methods Applied (yield,%) |
|---|---|---|---|---|---|
| **P4** | 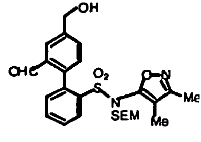 | N-(3,4-Dimethyl-5-iso-xazolyl)-2'-formyl-4'-(hydroxy-methyl)-N-[[2-(tri-methylsilyl)ethoxy]-methyl][1,1'-biphenyl]-2-sulfonamide | 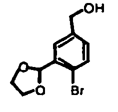 | Zhang, H.-Y. et al., *Tetrahedron,* **1994,** *50*, 11339-11362. | 19 (95); 1 (81) |
| **P5** | 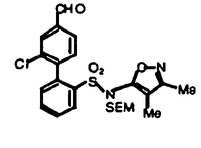 | 2'-Chloro-N-(3,4-dimethyl-5-iso-xazolyl)-4'-formyl-N-[[2-(trimethylsilyl)-ethoxy]-methyl] [1,1'-biphenyl]-2-sulfonamide | 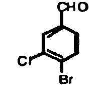 | Casida, J. E.; Elliott, M.; Pullmann, D. A., EP 294229 | 1 (83) |
| **P6** | 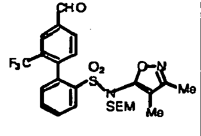 | N-(3,4-Dimethyl-5-isoxazolyl)-4'-formyl-2'-(trifluoromethyl)-N-[[2-(tri-methylsilyl)ethoxy] methyl] [1,1'-biphenyl]-2-sulfonamide | 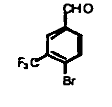 | DoAmaral, J. R.; French, F. A.; Blanz, E. J. Jr.; French, D. A. *J*. *Med. Chem.* **1971**, *9*, 862-866. | 1 (11) |
| **P7** | 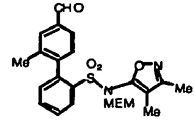 | N-(3,4-Dimethyl-5-isoxazolyl)-4'-formyl-N-[(2-methoxy-ethoxy)-methyl]-2'-methyl[1,1'-bi-phenyl]-2-sulfonamide | 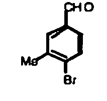 | Pine, S. H.; et. al. *J. Org. Chem.* **1971**, *36*, 984-91. | 1 (77) |
| **P8** | 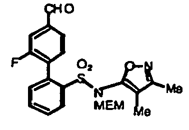 | N-(3,4-Dimethyl-5-isoxazolyl)-2'-fluoro-4'-formyl-N-[(2-methoxyethoxy)-methyl] [1,1'-biphenyl]-2-sulfonamide | 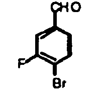 | Palmer, C. J.; Casida, J. E.; Larkin, J. P. US Patent 5,061,726 | 1 (50) |
| **P9** | 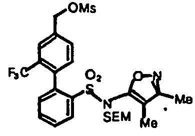 | 2'-[[(3,4-Dimethyl-5-isoxazolyl)[[2-(tri-methylsilyl)ethoxy] methyl] amino] sulfonyl]-2-(trifluoromethyl)[1,1'-biphenyl]-4-methanol methanesulfonate | **P6** | | 11 (90); 3 (75) |
| **P1 0** | 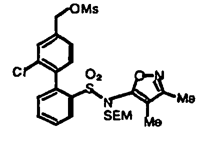 | 2-Chloro-2'-[[(3,4-dimethyl-5-isoxazolyl)[[2-(tri-methylsilyl)ethoxy] methyl]amino]sulfonyl] [1,1'-biphenyl]-4-methanol methanesulfonate | **P5** | | 11 (90); 3 (83) |
| **P1 1** | 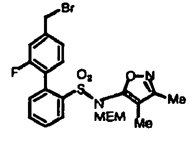 | 4'-(Bromomethyl)-N-(3,4-dimethyl-5-isoxazolyl)-2'-fluoro-N-[(2-methoxy-ethoxy)methyl] [1,1'-biphenyl]-2-sulfonamide | **P8** | | 11 (98); 2 (67) |
| **P1 2** | 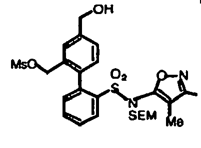 | 2'-[[(3,4-Dimethyl-5-isoxazolyl)[[2-(tri-methylsilyl)ethoxy] methyl]amino]-sulfonyl]-4-(hydroxy-methyl)[1,1'-biphenyl]-2-methanol methanesulfonate | **P1** | | 3 (99); 11 (76) |
| **P1 3** | 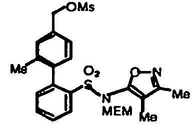 | 4'-[(Methane-sulfonyloxy)methyl] -N-(3,4-dimethyl-5-isoxazolyl)-N-[(2-methoxyethoxy)-methyl]-2'-methyl[1,1'-biphenyl]-2-sulfonamide | **P7** | | 11; 3 (62) |
| **P1 4** | 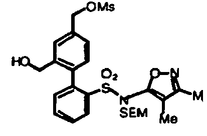 | 2'-[[(3,4-Dimethyl-5-isoxazolyl)[[2-(trimethylsilyl)ethoxy] methyl]amino]sulfonyl]-2-(hydroxy-methyl)[1,1'-biphenyl]-4-methanol methanesulfonate | **P4** | | 3 (90); 11 (35) |
| **P1 5** | 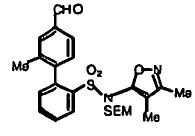 | N-(3,4-Dimethyl-5-isoxazolyl)-4'-formyl-2'-methyl-N-[[2-(trimethylsilyl)-ethoxy]methyl] [1,1'-biphenyl]-2-sulfonamide | 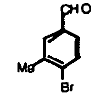 | Pine, S. H.; et. al. *J. Org. Chem.* **1971**, *36*, 984-91. | 1 (52) |
| **P1 6** | 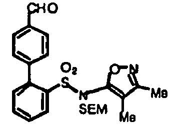 | N-(3,4-Dimethyl-5-isoxazolyl)-4'-formyl-N-[[2-(tri-methylsilyl)ethoxy]-methyl][1,1'-biphenyl]-2-sulfonamide | 4-bromo-benzaldehyde | | 1(67) |
| **P1 7** | 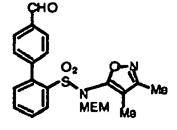 | N-(3,4-Dimethyl-5-isoxazolyl)-4'-formyl-N-[[2-methoxy-ethoxy]methyl][1,1'-biphenyl]-2-sulfonamide | 4-bromo-benzaldehyde | | 1 (80) |
| **P1 8** | 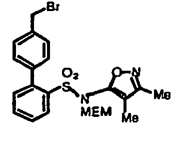 | 4'-Bromomethyl-N-(3,4-dimethyl-5-isoxazolyl)-N-(2-methoxyethoxy-methyl) [1,1'-biphenyl]-2-sulfonamide | **P17** | | 11,2 (80) |
| **P1 9** | 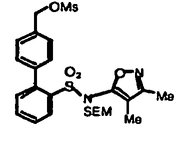 | N-(3,4-Dimethyl-5-isoxazolyl)-4'-[(methanesulfonyloxy)methyl]-N-[ [2-(trimethylsilyl)-ethoxy]methyl][1,1'-biphenyl]-2-sulfonamide | **P16** | | 11 (83); 3 (90) |
| **P2 0** | 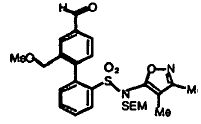 | N-(3,4-Dimethyl-5-isoxazolyl)-4'-formyl-N-[[2-(trimethylsilyl)ethoxy]-methyl][1,1'-biphenyl]-2-sulfonamide | **P2C** | | 1 (72) |
| **P2 1** | 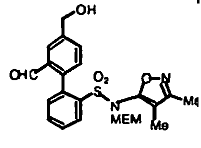 | N-(3,4-Dimethyl-5-isoxazolyl)-2'-formyl-4'-(hydroxymethyl)-N-[[2-methoxy-ethoxy]methyl][1,1'-biphenyl]-2-sulfonamide | 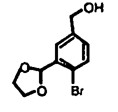 | Zhang, H.-Y. et al., *Tetrahedron,* **1994,** *50*, 11339-11362. | 19 (95); 1 (77) |
| **P2 2** | 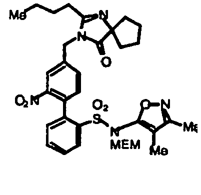 | 4'-[(2-Butyl-4-oxo-1,3-diazaspiro-[4.4]non-1-en-3-yl)methyl]-N-(3,4-di-methyl-5- isoxazolyl)-N-[[2-methoxy-ethoxy]methyl]-2'-nitro[1,1'-biphenyl]-2-sulfonamide | 4-bromo-3-nitro-toluene | | 13 (53); 4 (74); 1 (50) |

### Examples

### Reference Example 1

### 4'-[(2-Butyl-4-oxo-1,3-diazaspiro[4,4]non-1-en-3-yl)methyl]-N-(4,5-dimethyl]-3-isoxazolyl)[1,1'-biphenyl]-2-sulfonamide

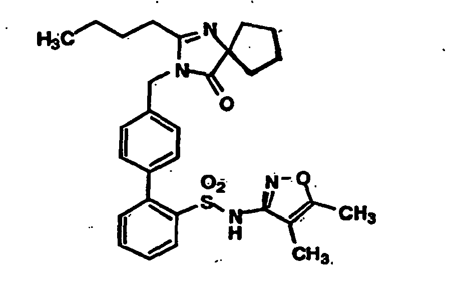

### A. N-(4,5-Dimethyl-3-isoxazolyl)-4'-(hydroxymethyl)-N-[(2-methoxyethoxy)methyl][1,1'-biphenyl]-2-sulfonamide

4-Bromobenzyl alcohol (750 mg, 4.0 mmol) was coupled with [2-[[(4,5-dimethyl-3-isoxazolyl)[(2-methoxyethoxy)methyl]amino]sulfonyl]phenyl]boronic acid (1.0 g, 2.7 mmol) according to General Method 1. The crude residue was chromatographed on silica gel using 3:4 hexane/EtOAc to afford **1A** (730 mg, 66%) as a colorless gum: R_{f}=0.26, silica gel, 2:3 hexane/EtOAc.

### B. 4'-Bromomethyl-N-(4,5-dimethyl-3-isoxazolyl)-N-[(2-methoxyethoxy)methyl][1,1'-biphenyl]-2-sulfonamide

**1A** (730 mg, 1.64 mmol) was converted to the corresponding bromide according to General Method 2. The crude residue was chromatographed on silica gel using 4:1 hexane/EtOAc to afford **1B** (750 mg, 90%) as a colorless gum: R_{f}=0.66, silica gel, 1:1 hexane/EtOAc.

### C. 4'-[(2-Butyl-4-oxo-1,3-diazaspiro[4.4]non-1-en-3-yl)methyl]-N-(4,5-dimethyl-3-isoxazolyl)-N-(2-methoxyethoxymethyl)[1,1'-biphenyl]-2-sulfonamide

**1B** (255 mg, 0.5 mmol was reacted with 2-butyl-1,3-diazaspiro[4.4]non-1-en-4-one hydrochloride according to General Method 4. The crude residue was chromatographed on silica gel using 3:4 hexane/EtOAc to afford **1C** as a gum: R_{f}=0.32, silica gel, 1:1 hexane/EtOAc.

### D. 4'-[(2-Butyl-4-oxo-1,3-diazaspiro[4.4]non-1-en-3-yl)methyl]-N-(4,5-dimethyl-3-isoxazolyl)[1,1'-biphenyl]-2-sulfonamide

**1C** was subjected to deprotection according to General Method 7. The crude residue was purified by preparative HPLC to provide the title compound as a white solid (130 mg, 49%, for two steps): mp 77-81°C. Analysis calculated for C₂₉H₃₄N₄O₄S **•** 1.0H₂O: Calc'd: C, 63.02; H, 6.57; N, 10.14; S, 5.80. Found: C, 62.75; H, 6.16; N, 9.85; S, 5.54.

### Reference Example 2

### 4'-[(2-Butyl-4-oxo-1,3-diazaspiro[4,4]non-1-en-3-yl)methyl-N-(3,4-dimethyl-5-isoxazolyl)-2'-[(methylamino)methyl][1,1'-biphenyl]-2-sulfonamide

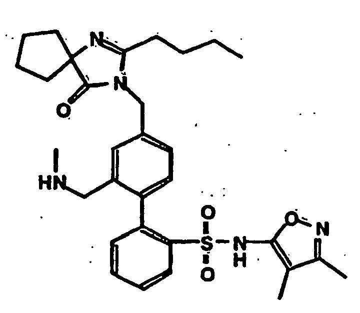

### A. 4-Bromo-3-formyl-benzonitrile

To a solution of 4-bromo-3-methyl benzonitrile (14.0 g, 71.4 mmol) in carbon tetrachloride (200 mL), N-bromosuccinimide (13.98 g, 78.55 mmol) and benzoyl peroxide (700 mg) were added, and the mixture was heated at reflux for 8 h while illuminating the solution with a sun lamp. The mixture was cooled and filtered. The filtrate was concentrated to provide a light yellow solid (21 g) which was used in the next step without any further purification.

To a solution of the crude compound (21 g) obtained above in anhydrous DMSO (30 mL) under argon, was added anhydrous trimethylamine N-oxide (6.97 g, prepared as described in Soderquist et al., *Tetrahedron Letters,* 27, 3961 (1986)), and the mixture was stirred at 55°C for 48 h. The mixture was then cooled and added to ice/water (150 mL) and the resulting aqueous mixture was extracted with EtOAc (3 x 100 mL). The combined organic extracts were washed once with brine (100 mL), dried and evaporated. The residue was chromatographed on silica gel using 8:1 hexanes/EtOAc to afford **2A** as a white solid (6.1 g, 47% for two steps).

### B. 4'-Cyano-2'-formyl-N-(3,4-dimethyl-5-isoxazolyl)-N-(2-methoxyethoxymethyl) [1,1'-biphenyl]-2-sulfonamide

**2A** (3.0 g, 14 mmol) was subjected to Suzuki coupling with N-[(2-methoxyethoxy)methyl]-N-(3,4-dimethyl-5-isoxazolyl)-2-(4,4,5,5-tetramethyl-1,3,2-dioxaborolan-2-yl)benzenesulfonamide according to General Method 1. The crude residue was chromatographed on silica gel using 2:1 hexane/EtOAc to afford **3B** (4.5 g, 68%) as a colorless gum.

### C. 4'-Cyano-2'-(N-methylaminomethyl)-N-(3,4-dimethyl-5-isoxazolyl)-N-(2-methoxyethoxymethyl)[1,1'-biphenyl]-2-sulfonamide

To **2B** (2.2 g, 4.69 mmol), MeNH₂ (8.03 M in EtOH, 2.33 mL, 18.74 mmol) and 3A molecular sieves in CH₂Cl₂ (47 mL), glacial acetic acid (1.13 g, 18.74 mmol) was added followed by NaB(AcO)₃H (3.98 g, 18.74 mmol). The reaction mixture was stirred at RT overnight, diluted with EtOAc and filtered through celite. The filtrate was washed with H₂O, dried and concentrated. The residue was chromatographed on silica gel using 100:5 CH₂Cl₂/MeOH to afford 2C (1.24 g, 55%) as a colorless gum: R_{f}=0.2, silica gel, 100:5 CH₂Cl₂/MeOH.

### D. 2'-(N-t-Butoxycarbonyl-N-methylaminomethyl)-4'-cyano-N-(3,4-dimethyl-5-isoxazolyl)-N-(2-methoxyethoxymethyl) [1,1'-biphenyl]-2-sulfonamide

To **2C** (1.3 g, 2.68 mmol), triethylamine (434 mg, 4.3 mmol) and 4-dimethylaminopyridine (33 mg, 0.27 mmol) in THF (10 mL) was added a solution of di-t-butyl dicarbonate (703 mg, 3.22 mmol) in THF (10 mL) dropwise. The reaction mixture was stirred at RT for 3 h. 10% aqueous NH₄Cl was added, and the mixture was extracted with EtOAc. The extracts were washed with H₂O and brine, dried and concentrated. The residue was chromatographed on silica gel using 7:4 hexane/EtOAc to afford **2D** (1.1 g, 70%) as a colorless gum: R_{f}=0.57, silica gel, 2:3 hexane/EtOAc.

### E. 2'-(N-t-Butoxycarbonyl-N-methylaminomethyl)-N-(3,4-dimethyl-5-isoxazolyl)-4'-formyl-N-(2-methoxyethoxymethyl) [1,1'-biphenyl]-2-sulfonamide

To **2D** (1.03 g, 1.76 mmol) in **THF** (18 mL), diisobutylaluminum hydride (1M in CH₂Cl₂, 5.29 mL, 5.29 mmol) was added dropwise. The reaction mixture was stirred at RT for 4 h. MeOH (20 mL) was added, and the mixture was stirred for 20 min. The mixture was then added into H₂O and extracted with EtOAc. The combined extracts were washed with H₂O and brine, dried and concentrated. The residue was chromatographed on silica gel using 5:6 hexane/EtOAc to afford **2E** (325 mg, 31%) as a colorless gum: R_{f}=0.37, silica gel, 1:1 hexane/EtOAc.

### F. 2'-(N-t-Butoxycarbonyl-N-methylaminomethyl)-4'-Hydroxymethyl-N-(3,4-dimethyl-5-isoxazolyl)-N-(2-methoxyethoxymethyl)[1,1'-biphenyl]-2-sulfonamide

**2E** was reduced using sodium borohydride according to General Method 11 to provide **2F**, which was used in the next reaction step without any purification.

### G. 4'-Bromomethyl-2'-(N-t-butoxycarbonyl-N-methylaminomethyl)-N-(3,4-dimethyl-5-isoxazolyl)-N-(2-methoxyethoxymethyl) [1,1'-biphenyl]-2-sulfonamide

**2F** was treated with carbon tetrabromide and triphenylphosphine according to General Method 2 to provide **2G** in 78% yield.

### H. 2'-(N-t-Butoxycarbonyl-N-methylaminomethyl)-4'-[(2-butyl-4-oxo-1,3-diazaspiro[4.4]non-1-en-3-yl)methyl]-N-(3,4-dimethyl-5-isoxazolyl)-N-(2-methoxyethoxymethyl) [1,1'-biphenyl]-2-sulfonamide

**2G** was treated with 2-*n*-butyl-1,3-diazaspiro [4,4]non-1-en-4-one hydrochloride according to General Method 4 to provide **2H** in 79% yield.

### I. 4'-[(2-Butyl-4-oxo-1,3-diazaspiro[4,4]non-1-en-3-yl)methyl]-N-(3,4-dimethyl-5-isoxazolyl)-2'-[(methylamino)methyl][1,1'-biphenyl]-2-sulfonamide

**2H** (170 mg, 0.22 mmol) was deprotected according to General Method 7 to provide the title compound in 67% yield: R_{f}=0.39, silica gel, 10:1 CH₂Cl₂/MeOH; mp: 194-200 °C; LRMS (m/z) 578 (MH⁺).

### Reference Example 3

### 4'-[(2-Butyl-4-ozo-1.3-diazaspiro[4.4]non-1-en-3-yl)methyl]-2'-formyl-N-(3,4-dimethyl-5-isoxazolyl)-[1,1'-biphenyl]1-2-sulfonamide

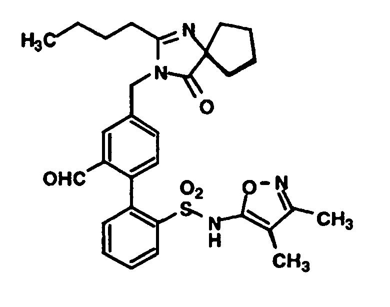

### A. 4'-Cyano-2'-(1.3-dioxolan-2-yl)-N-(3,4-dimethyl-5-isoxazolyl)-N-(2-methoxyethoxymethyl) [1,1'-biphenyl]-2-sulfonamide

A mixture of **2B** (1.28 g, 2.73 mmol), ethylene glycol (1.69 g, 27.3 mmol) and p-toluenesulfonic acid (38 mg) in toluene (30 mL) was heated at 130°C for 5 h, while a Dean-Stark water separator was used. After cooling, the mixture was diluted with EtOAc. The organic liquid was separated and washed with H₂O and brine, dried and concentrated. The residue was chromatographed on silica gel using 5:4 hexane/EtOAc to afford **3A** (1.1 g, 79%) as a colorless gum: R_{f}=0.57, silica gel, 1:2 hexane/EtOAc.

### B. 2'-(1,3-Dioxolan-2-yl)-4'-formyl-N-(3,4-dimethyl-5-isoxazolyl)-N-(2-methoxyethoxymethyl) [1,1'-biphenyl]-2-sulfonamide

To **3A** (1.1 g, 2.14 mmol) in THF (21 mL) at 0°C was added DIBAL-H (1M in CH₂Cl₂, 4.28 mL 4.28 mmol) dropwise. The reaction was stirred at RT overnight. MeOH (20 mL) was added and the reaction was stirred for 5 min. The mixture was poured into cold 0.1 N HC1 solution (150 mL), shaken for 5 min, and then extracted with 3:1 EtOAc/hexane. The combined organic extracts were washed with H₂O and brine, dried and concentrated. The residue was chromatographed on silica gel using 3:4 hexane/EtOAc to afford **3B** (710 mg, 64%) as a colorless gum: R_{f}=0.45, silica gel, 2:3 hexane/EtOAc.

### C. 2'-(1,3-Dioxolan-2-yl)-4'-hydroxymethyl-N-(3,4-dimethyl-5-isoxazolyl)-N-(2-methoxyethoxymethyl) [1,1'-biphenyl]-2-sulfonamide

**3B** (710 mg, 1.4 mmol) was subjected to sodium borohydride reduction according to General Method 11 to afford **3C**, which was used for the next reaction step without further purification.

### D. 4'-Bromomethyl-2'-(1,3-dioxolan-2-yl)-N-(3,4-dimethyl-5-isoxazolyl)-N-(2-methoxyethoxymethyl) [1,1'-biphenyl]-2-sulfonamide

**3C** was treated with carbon tetrabromide and triphenylphosphine according to General Method 2. The crude residue was chromatographed on silica gel using 3:2 hexane/EtOAc to afford **3D** (750 mg, 94%) as a colorless gum: R_{f}=0.74, silica gel, 1:2 hexane/EtOAc.

### E. 4'-[(2-Butyl-4-oxo-1,3-diazaspiro[4.4]non-1-en-3-yl)methyl]-2'-(1,3-dioxolan-2-yl)-N-(3,4-dimethyl-5-isoxazolyl)-N-(2-methoxyethoxymethyl)[1,1'-biphenyl]-2-sulfonamide

**3D** (750 mg, 1.3 mmol) was treated with 2-n-butyl-1,3-diazaspiro[4.4]non-1-en-4-one hydrochloride (387 mg, 1.68 mmol) according to General Method 4. The crude residue was chromatographed on silica gel using 100:1.7 CH₂Cl₂/MeOH to afford **3E** as a gum (830 mg, 93%): R_{f}=0.40, silica gel, 100:5 CH₂Cl₂/MeOH.

### F. 4'-[(2-Butyl-4-oxo-1,3-diazaspiro[4.4]non-1-en-3-yl)methyl]-2'-formyl-N-(3,4-dimethyl-5-isoxazolyl)-[1,1'-biphenyl]-2-sulfonamide

3E (830 mg, 1.20 mmol) was subjected to deprotection according to General Method 7. The crude residue was chromatographed on silica gel using 100:1.5 and then 100:4 CH₂Cl₂ /MeOH to afford the title compound as a gum (480 mg, 72%): R₁=0.16, silica gel, 100:5 CH₂Cl₂/MeOH.

### Reference Example 4

### 4'-[(2-Butyl-4-oxo-1,3-diazaspiro[4,4]non-1-en-3-yl)methyl]-N-(3,4-dimethyl-5-isoxazolyl)-2'-[(3,3-dimethyl-2-oxo-1-pyrrolidinyl)methyl][1,1'-biphenyl]-2-sulfonamide

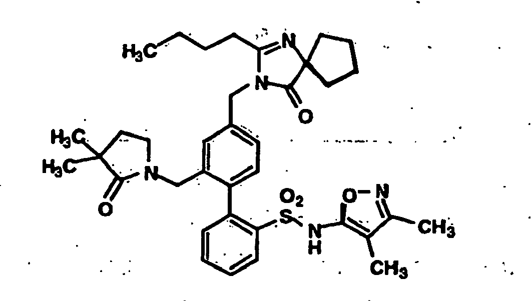

To **3F** (110 nig, 0.20 mmol) in CH₂Cl₂ (4 mL) was added 4-amino-2,2-dimethylbutanoic acid hydrochloride (98 mg, 0.59 mmol) [Scheinmann, et al., *J. Chem. Research* (*S*), 414-415 (1993)] and 3Å molecular sieves, followed by glacial acetic acid (35 mg, 0.59 mmol) and then sodium acetate (48 mg, 0.59 mmol). The mixture was stirred for 8 minutes, and NaB(AcO)₃H (124 mg, 0.59 mmol) was then added. The reaction mixture was stirred at RT for 2 h, diluted with EtOAc and filtered through celite. The filtrate was washed with H₂O and brine, dried and concentrated. This material was dissolved in CH₂Cl₂ (6 mL) and 1,3-diisopropylcarbodiimide (32 mg, 0.25 mmol) was added. The reaction mixture was stirred at RT for 2 h and diluted with CH₂Cl₂, washed with H₂O and brine, dried and concentrated. The residue was purified by preparative HPLC to provide the title compound as a white solid (40 mg, 31%, for two steps): mp 104-110°C. Analysis calculated for C₃₆H₄₅N₅O₅S •0.8 H₂O: Calc'd: C, 64.13; H, 6.97; N, 10.39; S, 4,75. Found: C, 64.18; H, 6.60;
N, 10.23; S, 4.50.

### Reference Example 5

### 4'-[(2-Butyl-4-ozo-1,3-diazaspiro[4.4]non-1-en-3-yl)methyl]-2'-formyl-N-(3,4-dimethyl-5-isoxazolyl)-[1,1'-biphenyl]-2-sulfonamide (Alternative Preparation for 3F)

### A. 2-[(2'-Bromo-5'-formyl)phenyl)]-1,3-dioxolane

DIBAL-H (1.0 M solution in toluene, 445 mL, 445 mmol, 1.1 eq) was added over 30 minutes to a solution of 2-[(2'-bromo-5'-cyano)phenyl)]-1,3-dioxolane (103 g, 404 mmol, 1.0 eq) [Zhang, H.-Y. et al., *Tetrahedron*, 50, 11339-11362 (1994)] in toluene (2.0 L) at -78 °C. The solution was allowed to warm to 0 °C. After 1 hour, a solution of Rochelle's salt (125 g) in water (200 mL) was added, and the mixture was allowed to warm to room temperature and was stirred vigorously for 16 h. The organic layer was concentrated and the residue partitioned between ethyl acetate (1 L) and 1 N hydrochloric acid (800 mL). The organic layer was washed with saturated aqueous sodium bicarbonate (800 mL), dried over sodium sulfate, and then concentrated to give 70.5 g of crude **5A** as a yellow solid, which was used without further purification.

### B. 2-[(2'-Bromo-5'-hydroxymethyl)phenyl)]-1,3-dioxolane

Sodium borohydride (3.66 g, 96.7 mmol, 0.5 eq) was added to a solution of crude **5A** (49.7 g, approximately 193 mmol, 1.0 eq) in absolute ethanol (1300 mL) at 0 °C. After 2 hours, a solution of 10% aqueous sodium dihydrogen phosphate (50 mL) was added and the mixture was stirred and allowed to warm to room temperature. The mixture was concentrated, then partitioned between ethyl acetate (800 mL) and saturated aqueous sodium bicarbonate (500 mL). The organic layer was dried over sodium sulfate and concentrated to give 49.0 g of crude **5B** as a yellow oil, which was used without further purification.

### C. 2-[(2'-Bromo-5'-bromomethyl)phenyl)]-1,3-dioxolane

Triphenylphosphine (52.7 g, 199 mmol, 1.05 eq) was added in portions over 15 minutes to a solution of crude **5B** (49.0 g, approximately 189 mmol, 1.0 eq) and carbon tetrabromide (69.0 g, 208 mmol, 1.1 eq) in THF at 0°C. After 2 hours, saturated aqueous sodium bicarbonate solution (20 mL) was added, and the mixture was allowed to warm to room temperature and was then concentrated. Ether (500 mL) was added, and the resulting mixture was filtered. The filtrate was dried over magnesium sulfate and concentrated. The residue was chromatographed on silica gel (8:1 hexanes/ethyl acetate as eluant) to give **5C** as a white solid (31.1 g, 51% yield from 2-[(2'-bromo-5'-cyano)phenyl)]-1,3-dioxolane).

### D. 2-(1,3-Dioxolan-2-yl)-4-[(2-n-butyl-4-oxo-1,3-diazaspiro[4,4]non-1-en-3-yl)methyl]bromobenzene

Sodium hydride (60% dispersion in mineral oil, 9.65 g, 241 mmol, 2.5 eq) was added in portions over 15 minutes to a mixture of 2-*n*-butyl-1,3-diazaspiro[4.4]non-1-en-4-one hydrochloride (18.7 g, 96.5 mmol, 1.0 eq) in DMF (400 mL) at 0°C. The mixture was stirred and allowed to warm to room temperature over 15 minutes. To this mixture was added via canula a solution of 5C (31.1 g, 96.5 mmol, 1.0 eq) in DMF (100 mL). After 14 hours, the mixture was concentrated *in vacuo* and partitioned between ethyl acetate (500 mL) and 10% aqueous sodium dihydrogen phosphate (300 mL). The organic layer was dried over sodium sulfate and concentrated to give crude **5D** as an orange oil (42.7 g), which was used without further purification.

### E. 4-[(2-n-Butyl-4-oxo-1,3-diazaspiro[4.4]non-1-en-3-yl)methyl]-2-formyl-bromobenzene

A solution of crude **5D** (6.0 g, approximately 13.6 mmol, 1.0 eq) in THF (180 mL) and 1N hydrochloric acid (30 mL) was heated at 65°C for 1.5 hours. The mixture was cooled and then treated with saturated aqueous sodium carbonate solution (75 mL) and ethyl acetate (200 mL). The organic layer was removed and dried over sodium sulfate, concentrated, and then further dried azeotropically with toluene to give **5E** as a crude yellow oil (8.2 g) which contained a small amount of toluene. This material was used without further purification.

### F. 4'-[(2-Butyl-4-oxo-1,3-diazaspiro[4.4]non-1-en-3-yl)methyl]-2'-formyl-N-(3,4-dimethyl-5-isoxazolyl)-N-(2-methoxyethoxymethyl) [1,1'-biphenyl]-2-sulfonamide

Palladium catalyzed Suzuki coupling of **5E** and [2-[((3,4-dimethyl-5-isoxazolyl)[(2-methoxyethoxy)methyl]amino]sulfonyl]phenyl]boronic acid was performed according to General Method **1** to yield **5F** in 60% yield.

### G. 4'-[(2-Butyl-4-oxo-1,3-diazaspiro[4,4]non-1-en-3-yl)methyl]-2'-formyl-N-(3,4-dimethyl-5-isoxazolyl)-[1,1'-biphenyl]-2-sulfonamide

Deprotection of 5F according to General Method 7 provided the title compound (**5G = 3F)** in 73% yield: R_{f}=0.2 (silica gel using CH₂Cl₂/MeOH [100:5]).

### Reference Example 6

### 4'-[(2-Butyl-4-oxo-1,3-diazaspiro[4,4]non-1-en-3-yl)methyl]-N-(3,4-dimethyl-5-isoxazolyl)-2'-[(3,3-dimethyl-2-oxo-1-pyrrolidinyl)methyl][1,1'-biphenyl]-2-sulfonamide(Alternative Preparation for 4)

The title compound (**6** = **4**) was obtained as a white solid from **5G** using a procedure similar to that described in Example 4 (945 mg, 35%): mp 104-110°C. Analysis calculated for C₃₆H₄₅N₅O₅S • 0.8 H₂O: Calc'd: C, 64.13; H, 6.97; N, 10.39; S, 4,75. Found: C, 64.18; H, 6.60; N, 10.23; S, 4.50.

### Reference Example 7

### 4'-[(2-Butyl-4-oxo-1,3-diazaspiro[4.4]non-1-en-3-yl)methyl]-N-(3,4-dimethyl-5-isoxazolyl)-2'-[(3-methyl-2-oxo-1-imidazolidinyl)methyl][1,1'-biphenyl]-2-sulfonamide

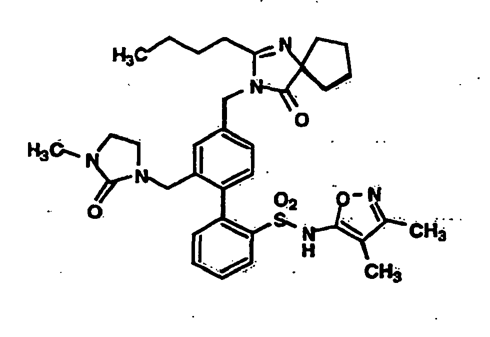

### A. 4'-[(2-Butyl-4-oxo-1,3-diazaspiro[4,4]non-1-en-3-yl)methyl]-N-(3,4-dimethyl-5-isoxazolyl)-N-(2-methoxyethoxymethyl)-2'-[(3-methyl-2-oxo-1-imidazolidinyl)methyl] [1,1'-biphenyl]-2-sulfonamide

To a solution of 0.3 g (0.46 mmol) of **5F** in CH₂Cl₂ (15 mL) was added 3Å molecular sieves (1 mL), N-methyl ethylenediamine (0.051 g, 0.69 mmol) and glacial acetic acid (0.828 g, 1.38 mmol), and the resulting mixture was stirred under argon for 15 min. Sodium triacetoxyborohydride (0.292 g, 1.38 mmol) was then added to the mixture, and the resulting mixture was stirred at room temperature for 3 h. The solution was then filtered through celite, and the celite was washed with CH₂Cl₂ (25 mL). The combined filtrates were washed with water (2x50 mL), dried and evaporated to afford a colorless gum (0.32 g). This gum was redissolved in CH₂Cl₂ (10 mL), and carbonyldiimidazole was then added (0.112 g, 0.69 mmol). The resulting mixture was stirred at room temperature for 24 h. The mixture was then washed with water (15 mL) and dried and evaporated to provide a colorless gum. Purification on silica gel using 1:1 hexane: EtOAc then provided **7A** (0.17 g, 50%) as a colorless gum.

### B. 4'-[(2-Butyl-4-oxo-1,3-diazaspiro[4.4]non-1-en-3-yl)methyl]-N-(3,4-dimethyl-5-isoxazolyl)-2'-[(3-methyl-2-oxo-1-imidazolidinyl)methyl] [1,1'-biphenyl]-2-sulfonamide

To a solution of **7A** (0.165 g, 0.225 mmol) in 95% EtOH (2 mL) was added 6N aqueous hydrochloric acid (2 mL), and the resulting solution was heated at reflux for 1 h. The mixture was then neutralized with aqueous sodium bicarbonate to pH 7 and then reacidified to pH 6 using aqueous sodium bisulfate. The mixture was then extracted with .EtOAc (3 x 30 mL). The combined organic extracts were then washed once with water and dried and evaporated. The residue was purified on silica gel using 5% MeOH in methylene chloride to provide the title compound (0.13 g, 89%) as a light yellow solid: ¹H NMR (CDCl₃) δ 0.89 (t, J=7.0 Hz, 3H), 1.26-1.60 (m, 4H), 1.87 (s, 3H), 1.97 (m, 8H), 2.16 (s, 3H), 2.35 (t, J=7.6 Hz, 2H), 2.72 (s, 3H), 3.29 (br s, 4H), 4.10 (s, 2H), 4.72 (m, 2H), 7.13-7.92 (m, 7H).

### Reference Example 8

### 4'-[(2-Butyl-4-oxo-1,3-diazaspiro[4.4]non-1-en-3-yl)methyl]-2'-[(3,3-dimethyl-2-oxo-1-pyrrolidinyl)methyl-N-(2-pyrazinyl) [1,1'-biphenyl]-2-sulfonamide

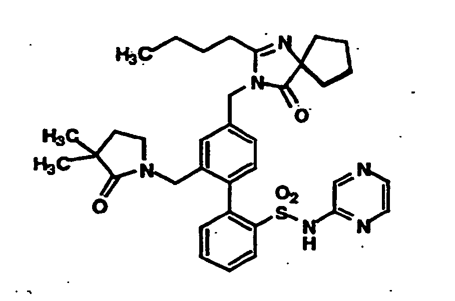

### A. N-tert-Butyl-4'-[(2-n-butyl-4-oxo-1,3-diazaspiro[4.4]non-1-en-3-yl)methyl]-2'-formyl[1,1'-biphenyl]-2-sulfonamide

Crude **5E** (8.3 g, approximately 13.6 mmol) was subjected to Suzuki coupling with [2-(*N-tert-*butylsulfamoyl)phenyl]boronic acid according to General Method 1. The crude residue was chromatographed on triethylamine-deactivated silica gel (1:1 hexanes/ethyl acetate eluant) to yield **8A** (5.03 g, 71% from **5C**) as an orange oil.

### B. N-tert-Butyl-4'-[(2-n-butyl-4-oxo-1,3-diazaspiro[4,4]non-1-en-3-yl)methyl]-2'-[(3,3-dimethyl-2-oxo-1-pyrrolidinyl)methyl][1,1'-biphenyl]-2-sulfonamide

A mixture of **8A** (2.0 g, 3.81 mmol, 1.0 eq), 4-amino-2,2-dimethylbutanoic acid hydrochloride (1.28 g, 7.64 mmol, 2.0 eq), freshly-activated 3Å molecular sieves (5.0 g), and methanol (35 mL) was stirred at room temperature. Solid 85% potassium hydroxide (225 mg) was added to adjust the pH to 6.5. After 1.5 hours, sodium cyanoborohydride (120 mg, 1.91 mmol, 1.5 eq) was added, and the mixture was stirred for an additional two hours. Solid 85% potassium hydroxide (640 mg) was added, the mixture was filtered, and the filtrate concentrated. To the residue was added dry dichloromethane (35 mL) and EDCI (1.10 g, 5.72 mmol, 1.5 eq) and the mixture was stirred at room temperature for 17 hours. Saturated aqueous sodium carbonate solution (100 mL) was added and the aqueous layer was extracted with dichloromethane (3 x 150 mL). The combined organic extracts were dried over sodium sulfate and concentrated to give **8B** (2.50 g) as a crude yellow oil used directly without further purification.

### C. 4'-[(2-n-Butyl-4-oxo-1,3-diazaspiro[4.4]non-1-en-3-yl)methyl]-2'-[(3,3-dimethyl-2-oxo-1-pyrrolidinyl)methyl][1,1'-biphenyl]-2-sulfonamide

Crude **8B** (2.50 g) was dissolved in trifluoroacetic acid (16 mL) and the resulting solution was stirred at room temperature for 7 hours. The resulting mixture was concentrated, and saturated aqueous sodium bicarbonate solution was added to the residue. This mixture was extracted with ethyl acetate (3 x 50 mL), and the combined organic extracts were dried over sodium sulfate and concentrated. Silica gel chromatography of the residue (5% methanol in dichloromethane as eluant) gave **8C** (0.98 g, 45% from **8A**) as a yellow solid.

### D. 4'-[(2-Butyl-4-oxo-1,3-diazaspiro[4.4]non-1-en-3-yl)methyl]-2'-[(3,3-dimethyl-2-oxo-1-pyrrolidinyl)methyl]-N-(2-pyrazinyl) [1,1'-binhenyl]-2-sulfonamide

A solution of **8C** (110 mg, 0.19 mmol, 1.0 eq) in DMF (2.5 mL) was treated at room temperature with sodium hydride (60% dispersion in mineral oil, 17 mg, 0.43 mmol, 2.2 eq). After 10 minutes, 2-chloropyrazine (68 µl, 0.76 mmol, 4.0 eq) was added via syringe, and the mixture heated at 120 °C for 2 hours and 130 °C for 6 hours. The solvent was evaporated, and the residue partially purified by preparative reverse-phase HPLC. The fractions containing product were evaporated, and the residue partitioned between dichloromethane and 10% aqueous sodium dihydrogen phosphate. The aqueous phase was adjusted to pH 5 and extracted with two additional portions of dichloromethane. The combined organic extracts were dried over sodium sulfate, evaporated, and the residue chromatographed on silica gel (3% methanol in dichloromethane as eluant) to yield still impure product (70 mg). This material was subjected to ion-exchange chromatography to give the pure title compound (8 mg, 6%) as a white powder after lyophilization: ¹H NMR (CDCl₃, 400 MHz) δ 8.52 (s, 1H), 8.29 (dd, J=1 and 8 Hz, 1H), 8.16 (d, J=3 Hz, 1H), 7.97 (s, 1H), 7.60 (dt, J=1 and 7 Hz, 1H), 7.55 (dt, J=1 and 7 Hz, 1H), 7.20 (dd, J=1 and 7 Hz, 1H), 7.01 (s, 2H), 6.96 (s, 1H), 4.60 (AB quartet, J=16 Hz, 2 H), 4.15 (AB quartet, J=16 Hz, 2 H), 3.16 (m, 2H), 2.34 (dd, J=7 and 8 Hz, 2H), 1.97 (m, 6H), 1.87 (t, J=7 Hz, 2H), 1.82 (m, 2H), 1.61 (m, 2H), 1.36 (sextet, J=7 Hz, 2 H), 1.17 (s, 3H), 1.16 (s, 3H), 0.90 (t, J=7 Hz, 3H); LRMS m/z 643 (ESI+ mode).

### Reference Example 9

### 4'-[(2-Butyl-4-oxo-1,3-diazaspiro[4.4]non-1-en-3-yl)methyl]-N-(3-chloro-2-pyrazinyl)-2'-[(3,3-dimethyl-2-oxo-1-pyrrolidinyl)methyl][1,1'-biphenyl]-2-sulfonamide

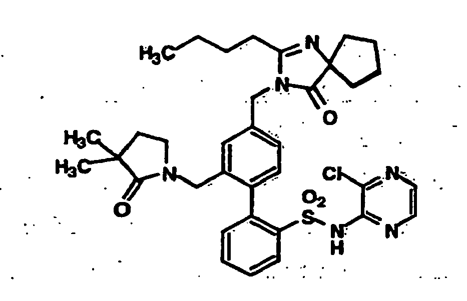

A solution of **8C** (100 mg, 0.18 mmol, 1.0 eq) in DMF (1.8 mL) was treated at room temperature with sodium hydride (60% dispersion in mineral oil, 8.5 mg, 0.21 mmol, 1.2 eq). After 10 minutes, 2,3-dichloropyrazine (79 mg, 0.53 mmol, 3.0 eq) was added via syringe, and the mixture heated at 60°C for 3 hours, then at 85°C for 14 hours, and then at 120°C for 5 hours. Workup and purification as described in Example 8 yielded the title compound (7.4 mg, 10%) as a white powder after lyophilization: ¹H NMR (CDCl₃, 400 MHz) δ 8.40 (dd, J=1 and 8 Hz, 1H), 8.0 (m, 2H), 7.68 (dt, J= 1 and 7 Hz, 1H), 7.61 (dt, J=1 and 7 Hz, 1H), 7.22 (dd, J=1 and 7 Hz, 1H), 7.16 (s, 1H), 7.05 (d, J =8 Hz, 1H), 6.89 (d, J=8 Hz, 1H), 6.85 (br s, 2H), 4.87 (AB quartet, J=16 Hz, 2 H), 4.31 (d, J=16 Hz, 1H), 4.15 (d, J=16 Hz, 1H), 3.16 (m, 2H), 2.82 (t, J=7 Hz, 2H), 1.90-2.20 (m, 8H), 1.82 (m, 2H), 1.66 (m, 2H), 1.38 (sextet, J=7 Hz, 2H), 1.22 (s, 3H), 1.14 (s, 3H), 0.92 (t, J=7 Hz, 3H) ppm; LRMS m/z 677, 679 (ESI+ mode); m/z 675, 677 (ESI- mode).

### Reference Example 10

### 4'-[(2-Butyl-4-oxo-1.3-diazaspiro[4,4]non-1-en-3-yl)methyl]-N-(3,4-dimethyl-5-isoxaolyl)-2'-[(2-oxo-1-pyrrolidinyl)methyl][1,1'-biphenyl]-2-sulfonamide

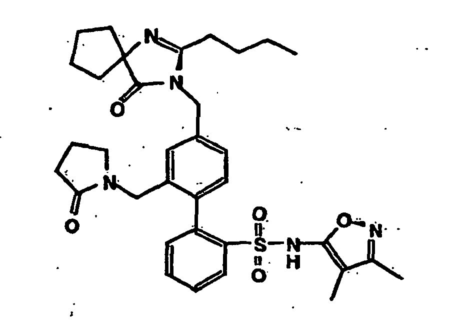

The title compound was prepared in 51% yield starting from 3F and 4-aminobutyric acid as described in Example 4 as a white solid: mp 95-100°C; ¹H NMR (CDCl₃) 8 0.90 (t, J=7.3 Hz, 3H), 1.36 (m, 2H), 1.61 (m, 2H), 1.82-2.10 (m, 15H), 2.17 (s, 3H), 2.36 (m, 2H), 3.41 (m, 2H), 4.20 (m, 2H), 4.72 (s, 2H), 7.00-7.90 (m, 7H).

### Reference Example 11

### 4'-[(2-Butyl-4-oxo-1,3-diazaspiro[4,4]non-1-en-3-yl)methyl]-2'-[(3,3-dimethyl-2-oxo-1-pyrrolidinyl)methyl]-N-(3,6-dimethyl-2-pyrazinyl)[1,1'-biphenyl]-2-sulfonamide

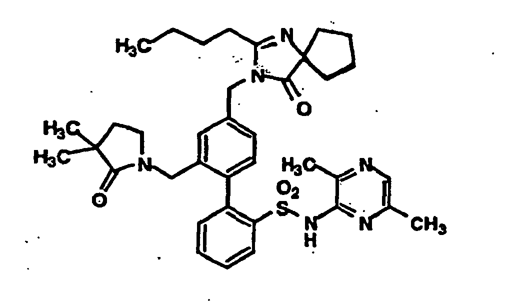

A mixture of **8C** (200 mg, 0.36 mmol, 1.0 eq), palladium acetate (4 mg, 0.02 mmol, 0.05 eq), (S)-BINAP (2.5 mg, 0.02 mmol, 0.05 eq), and toluene (5 mL) was sparged with nitrogen for 10 minutes. 2-Chloro-3,6-dimethylpyrazine (83 µl, 0.71 mmol, 2.0 eq) was added via syringe, followed by sodium hydride (60% dispersion in mineral oil, 28 mg, 0.71 mmol, 2.0 eq). The mixture was heated at 80°C for 15 hours. After cooling, the mixture was diluted with ethyl acetate and partitioned against 10% aqueous sodium phosphate adjusted to pH 5-6, and the aqueous layer was extracted with two additional portions of ethyl acetate. The combined organic extracts were dried over sodium sulfate, concentrated, and evaporated to a yellow oil, which was chromatographed on silica gel (1:3 hexanes/ethyl acetate eluant) to yield the title compound (50 mg, 21%) as an off-white solid: ¹H NMR (CD₈OD, 400 MHz) δ 8.14 (d, J=7 Hz, 1H), 7.75 (br s, 1H), 7.49 (m, 2H), 7.11 (dd, J = 1 and 7 Hz, 1H), 6.87 (s, 2H), 6.70 (s, 1H), 4.65 (br s, 2H), 4.11 (s, 2H), 3.95 (m, 2H), 2.92 (m, 2H), 2.29 (br s, 2H), 2.17 (s, 3H), 2.06 (s, 3H), 1.79 (m, 6H), 1.68 (m, 2H), 1.65 (m, 2H), 1.41 (m, 2H), 1.17 (m, 2H), 1.03 (s, 3H), 1.00 (s, 3H), 0.72 (t, J = 7 Hz, 3H); LRMS m/z 671 (ESI+ mode); m/z 669 (ESI- mode).

### Reference Example 12

### N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N,N,N'-trimethylurea

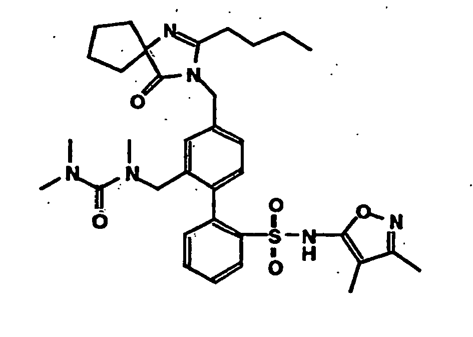

To **2I** (34.7 mg, 0.06 mmol) in CH₂Cl₂ (0.6 mL) and DMF (0.15 mL) was added dimethylcarbamyl chloride (6.5 mg, 0.06 mmol) followed by triethyl amine (6.7 mg, 0.066 mmol). The resulting mixture was stirred at RT for two days. The mixture was purified by anion-exchange chromatography to provide the title compound (90%). Characterization recorded with Example 73:

### Reference Example 13

### N-[[4-[(2-Butyl-4-oxo-1,3-diazaspiro[4,4]non-1-en-3-yl)methyl]-2'-[[(3,4-dimethyl-5-isoxazolyl)amino]sulfonyl][1,1'-biphenyl]-2-yl]methyl]-N'-(1,1-dimethylethyl)-N-methylurea

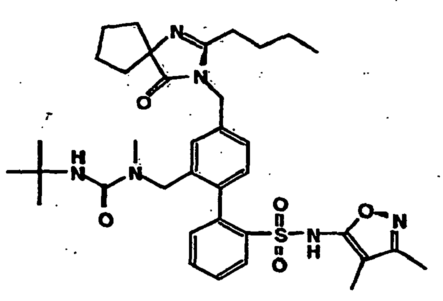

To **2I** (34.7 mg, 0.06 mmol) in CH₂Cl₂ (0.6 mL) and DMF (0.15 mL), *t*-butylisocyanate (6.0 mg, 0.06 mmol) was added, and the resulting mixture was stirred at RT for two days. The mixture was was purified by anion-exchange chromatography to provide the title compound (90%). Characterization recorded with Example 74.

### Reference Example 14

### [[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]methylcarbamic acid ethyl ester

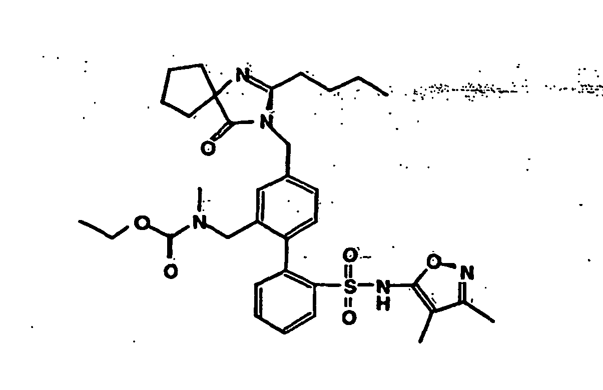

The title compound was prepared from **2I** and ethyl chloroformate using a procedure similar to the one described in Example 12 (90%). Characterization recorded with Example 75.

### Reference Example 15

### [[4-[(2-Butyl-4-oxo-1,3-diazasipiro[4.4]non-1-en-3-yl)methyl]-2'-[[(3,4-dimethyl-5-isoxazolyl)amino]sulfonyl][1,1'-biphenyl]-2-yl]methyl]methylcarbamic acid 2-methylpropyl ester

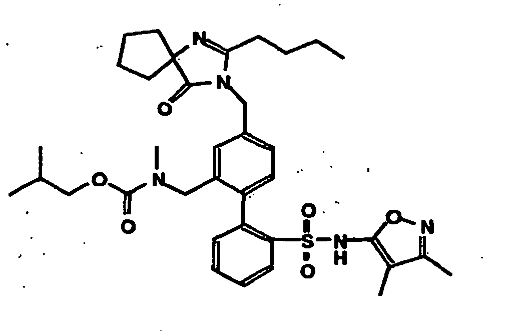

The title compound was prepared from **2I** and isobutyl chloroformate using a procedure similar to the one described in Example 12 (90%). Characterization recorded with Example 76.

### Reference Example 16

### 4'-[(2-Butyl-4-oxo-1,3-diazaspiro[4,4]non-1-en-3-yl)methyl]-2'-[(3,3-dimethyl-2-oxo-1-pyrrolidinyl)methyl]-N-(3-methoxy-2-pyrazinyl)[1,1'-biphenyl]-2-sulfonamide

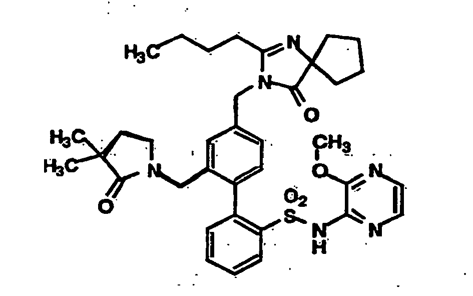

The title compound was prepared from **8C** (486 mg, 0.86 mmol) and 2-chloro-3-methoxypyrazine (250 mg, 1.72 mmol) [Uchimaru, F. et al., *Chem. Pharm. Bull.,* 20, 2204-2208 (1972)] using a procedure similar to the one used in Example 9. Preparative reverse-phase HPLC of the crude product after extractive workup gave the title compound (24 mg, 4%) as an off-white solid after lyophilization: ¹H NMR (CDCl₃, 400 MHz) δ 10.8 (br s, 2H), 8.36 (dd, J=1 and 8 Hz, 1H), 7.50-7.65 (m, 4H), 7.19 (dd, J=1 and 7 Hz, 1H), 7.15 (s, 1H), 7.01 (s, 2H), 4.92 (AB quartet, J=16 Hz, 2H), 4.20 (AB quartet, J=16 Hz, 2H), 3.99 (s, 3H), 3.18 (m, 2H), 2.82 (m, 2H), 2.18 (m, 4H), 1.96 (m, 4H), 1.82 (m, 2H), 1.63 (m, 2H), 1.32 (m, 2H), 1.21 (s, 3H), 1.12 (s, 3H), 0.87 (t, 3H, J=7 Hz, 3H); LRMS m/z 673 (ESI+ mode); m/z 671 (ESI- mode); HPLC retention time 25.52 minutes (Method B).

### Reference Example 17

### 4'-[(2-Butyl-4-oxo-1,3-diazaspiro[4.4]non-1-en-3-yl)methyl]-2'-formyl-N-(4,5-dimethyl-3-isoxazolyl)-[1,1'-biphenyl]-2-sulfonamide

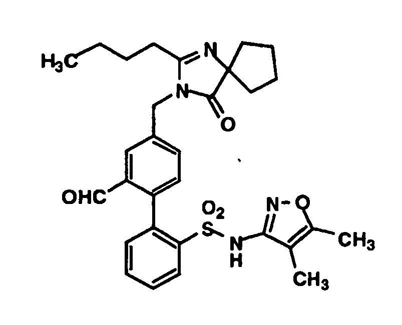

### A. 4'-[(2-Butyl-4-oxo-1,3-diazaspiro[4.4]non-1-en-3-yl)methyl]-2'-formyl-N-(4,5-dimethyl-3-isoxazolyl)-N-(2-methoxyethoxymethyl) [1,1'-biphenyl]-2-sulfonamide

Palladium catalyzed Suzuki coupling of **5E** and [2-[[(4,5-dimethyl-3-isoxazolyl)[(2-methoxyethoxy)methyl]amino]sulfonyl)phenyl]boronic acid was performed according to General Method 1 to afford 17A (81%) following silica-gel chromatography.

### B. 4'-[(2-Butyl-4-oxo-1,3-diazaspiro[4.4]non-1-en-3-yl)methyl]-2'-formyl-N-(4,5-dimethyl-3-isoxazolyl)-[1,1'-biphenyl]-2-sulfonamide

Treatment of **17A** with 6N aqueous hydrochloric acid according to General Method 7 provided the title compound (85%): Rf = 0.38, 5% MeOH in methylene chloride.

### Reference Example 18

### 4'-[(2-Butyl-4-oxo-1,3-diazaspiro[4.4]non-1-en-3-yl)methyl]-N-(4,5-dimethyl-3-isoxazolyl)-2'-[(2-oxo-1-pyrrolidinyl)methyl][1,1'-biphenyl]-2-sulfonamide

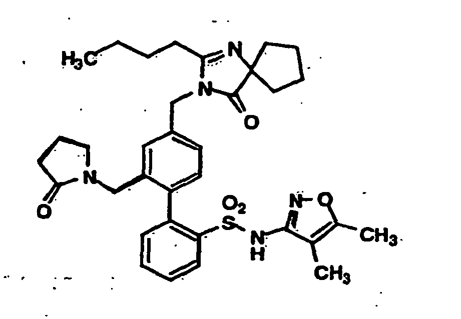

The title compound was obtained as a white solid using **17B** and 4-aminobutyric acid as described in Example 4 (25%): mp 97-103°C; ¹H NMR (CDCl₃) δ 0.90 (t, J=7 Hz, 3H), 1.36 (m, 2H), 1.62 (m, 2H), 1.80-2.10 (m, 13H), 2.26 (s, 3H), 2.39 (m, 4H), 3.31 (m, 2H), 4.20 (s, 2H), 4.73 (s, 2H), 7.05-8.10 (m, 7H).

### Reference Example 19

### (S)-N-[[2'-[[(3,4-Dimethyl-5-isoxazolyl)amino]sulfonyl]][1,1'-biphenyl]-4-yl]methyl]-N-[2-methyl-1-(3-methyl-1,2,4-oxadiazol-5-yl)propyl]pentanamide

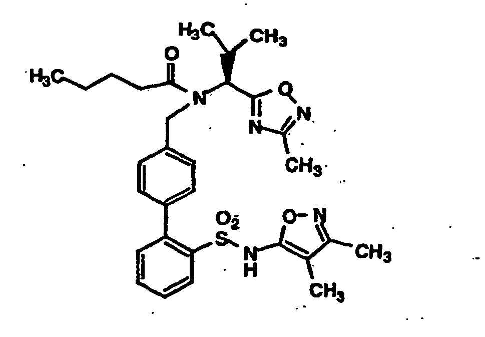

### A. (S)-5-(1-amino-2-methylpropyl)-3-methyl-1,2,4-oxadiazole

A solution of BOC-L-valine (4.34 g, 20.0 mmol) in dichloromethane (20 mL) was cooled to 0°C and treated dropwise with a solution of dicyclohexylcarbodiimide (2.06 g, 10.0 mmol) in dichloromethane (5 mL). After 1 hour the white solid which had formed was removed by filtration and the filtrate concentrated under reduced pressure. The residue was dissolved in pyridine (15 mL) and treated with acetamidoxime (488 mg, 6.6 mmol) in pyridine (5 mL) and the mixture heated at reflux for 1 hour. The pyridine was evaporated under reduced pressure and the residue partitioned between ethyl acetate and water. The organic phase was washed several times with water, dried over magnesium sulfate and concentrated. The residue was chromatographed on silica gel using 10% ethyl acetate in hexanes to elute the product (0.62 g, 25%) as a white solid.

A solution of the above product in 3 M hydrochloric acid (10 mL) was stirred at room temperature for 1 hour. The mixture was evaporated to dryness under reduced pressure and then azeotroped with toluene. This gave **19A** as a white solid (100%).

### B. 2'-[[(3,4-Dimethyl-5-isoxazolyl)amino]sulfonyl][1,1'-biphenyl]-4-carboxaldehyde

**P17** (0.35 g, 0.79 mmol) was subjected to deprotection according to General Method 7. Extraction and concentration gave 200 mg (71%) of **19B** as a yellow solid which was used without purification.

### C. (S)-[[1-[2'-[(3,4-Dimethyl-5-isoxazolyl)amino]sulfonyl][1,1'-biphenyl]-4-ylmethylamino]-2-methylpropyl]-3-methyl-1,2,4-oxadiazole

A mixture of **19B** (100 mg, 0.28 mmol) and **19A** (53 mg, 0.28 mmol) in dichloroethane (8 mL) was treated with sodium triacetoxyborohydride (74 mg, 0.35 mmol) in one portion and the mixture stirred under argon at room temperature. After 1 hour more sodium triacetoxyborohydride (32 mg, 0.15 mmol) was added to the mixture which stirred for 45 minutes more and the mixture was adjusted to pH 6 with saturated sodium bicarbonate solution. The mixture was diluted with 50 mL dichloromethane and the organic phase washed with water, dried over magnesium sulfate and concentrated to give crude 19C (110 mg, 79%) which was used without purification in the next step.

### D. (S)-N-[[2'-[[(3,4-Dimethyl-5-isoxazolyl)amino]sulfonyl][1,1'-biphenyl]-4-yl]methyl]-N-[2-methyl-1-(3-methyl-1,2,4-oxadiazol-5-yl)propyl]pentanamide

A mixture of **19C** (100 mg, 0.20 mmol), dichloromethane (5 mL) and triethylamine (70 mL, 0.5 mmol) was treated with valeryl chloride (60 mg; 0.5 mmol) and the mixture stirred under argon for 16 hours. The reaction mixture was concentrated under reduced pressure and the residue treated with 2 M sodium hydroxide (1 mL) and 2-propanol (2 mL) and stirred at room temperature for 1.5 hours. Citric acid was added to adjust the pH to 5-6 and the mixture extracted with dichloromethane. The residue obtained after drying the extract over magnesium sulfate and concentrating under reduced pressure was chromatographed on silica gel using 30 to 50% ethyl acetate in hexanes to elute the title product (73 mg, 63%) as a white foam: ESIMS (NH₃) m/z 580 (MH⁺ 100), 597 (M + NH₄⁺, 20), 1176 (2M +NH₄⁺, 10); HRMS calcd for C₃₀H₃₇N₅O₅S (MH⁺) 580.2593, found 580.2619.

### Reference Example 20

### N-(3,4-Dimethyl-5-isoxazolyl)-2'-[(3,3-dimethyl-2-oxo-1-pyrrolidinyl)methyl]-4'-[(2-ethyl-5,7-dimethyl-3H-imidazo[4,5-b]pyridin-3-yl)methyl][1,1-biphenyl]-2-sulfonamide

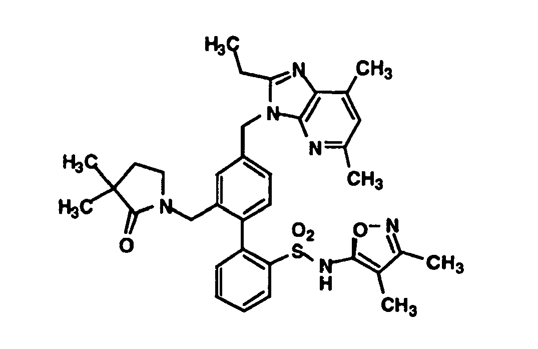

### A. N-(3,4-Dimethyl-5-isoxazolyl)-2'-[(3,3-dimethyl-2-oxo-1-pyrrolidinyl)methyl]-4'-hydroxymethyl-N-(2-methoxyethoxymethyl)[1,1'-biphenyl]-2-sulfonamide

Preparation **P21** (4.25 g, 9.0 mmol) was reacted with ethyl 4-amino-2,2-dimethylbutanoate hydrochloride (2.13 g, 10.9 mmol) according to General Method 5. The crude residue was chromatographed on silica gel (5% methanol in dichloromethane eluant) to yield 3.05 g of **20A** (59%) as an orange oil.

### B. N-(3,4-Dimethyl-5-isoxazolyl)-2'-[(3,3-dimethyl-2-oxo-1-pyrrolidinyl)methyl]-4'-[(2-ethyl-5,7-dimethyl-3H-imidazo[4,5-b]pyridine-3-yl)methyl]-N-(2-methoxyethoxymethyl)[1,1'-biphenyl]-2-sulfonamide

A solution of **20A** (500 mg, 0.87 mmol, 1.0 eq), triphenylphosphine (344 mg, 1.3 mmol, 1.5 eq), and 2-ethyl-5,7-dimethyl-3H-imidazo[4,5-b]pyridine (184 mg, 1.1 mmol, 1.2 eq) in tetrahydrofuran (5 mL) was treated at 0°C with diethylazodicarboxylate (206 µl, 1.3 mmol, 1.5 eq). The mixture was allowed to warm to room temperature, was stirred for 16 hr, and then concentrated. The residue was chromatographed on silica gel (3:2 hexanes/acetone as eluant) to give 320 mg of a mixture containing **20B** and 2-ethyl-5,7-dimethyl-3H-imidazo[4,5-b]pyridine (approximately 3:1 ratio by weight).

### C. N-(3,4-Dimethyl-5-isoxazolyl)-2'-[(3,3-dimethyl-2-oxo-1-pyrrolidinyl)methyl]-4'-[(2-ethyl-5,7-dimethyl-3H-imidazo[4,5-b]pyridine-3-yl)methyl][1,1'-biphenyl]-2-sulfonamide

**20B** (320 mg) was deprotected according to General Method 8 (ethanol). The crude residue was purified by reverse-phase preparative HPLC followed by extraction (3x50 mL ethyl acetate) of the product from brine adjusted to pH 4 with hydrochloric acid, to provide 135 mg of the title compound (24% from **20A**) as a white solid after lyophilization; mp 95-104 °C; MS m/e 641 (ESI+ mode); MS m/e. 639 (ESI- mode); HPLC retention time 3.43 minutes (Method C).

### Reference Example 21

### N-(3,4-Dimethyl-5-isoxazolyl)-4'-[2-(2-methoxyethyl)-4-oxo-1,3-diazaspiro[4.4]non-1-en-3-yl)methyl][1,1'-biphenyl]-2-sulfonamide

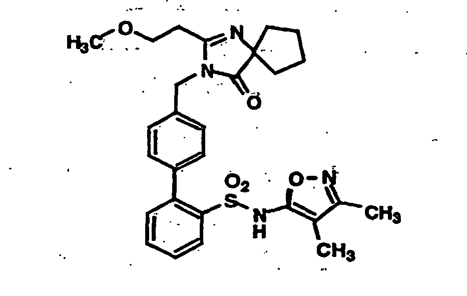

### A. Methyl 1-[3-methoxy-1-oxopropyl)amino]cyclopentane-1-carboxylate

3-Methoxypropanoic acid (1.65 mL, 17.6 mmol, 2.1 eq) was added to a mixture of EDCI (1.77 g, 9.24 mmol, 1.1 eq), triethylamine (3.5 mL, 25.2 mmol., 3.0 eq), 4-dimethylaminopyridine (20 mg, 0.18 mmol, 0.02 eq), and dichloromethane (100 mL) at room temperature. After 5 minutes, methyl 1-aminocyclopentane-1-carboxylate hydrochloride (1.50 g, 8.4 mmol, 1.0 eq) was added and the mixture was stirred at room temperature for 18 hours. The reaction mixture was partitioned against 1N hydrochloric acid and the aqueous phase was extracted once with dichloromethane. The combined organic extracts were dried over sodium sulfate and concentrated. Silica gel chromatography of the residue (1:3 hexanes/ethyl acetate as eluant) yielded 1.30 g of **21A** (67%) as a colorless oil.

### B. 1-[(3-Methoxy-1-oxopropyl)amino]cyclopentane-1-carboxylic acid

A mixture of **21A** (1.25 g, 5.5 mmol, 1.0 eq), lithium hydroxide hydrate (300 mg, 7.1 mmol, 1.3 eq), THF (10 mL), and water (10 mL) was stirred at room temperature for 5.5 hours. 1N hydrochloric acid (10 mL) was added and the mixture was saturated with solid sodium chloride, then extracted with ethyl acetate (3x30 mL). The combined organic extracts were dried over sodium sulfate and concentrated to provide 0.95 g of **21B** (80%) as a white solid.

### C. 1-[(3-Methoxy-1-oxopropyl)amino]cyclopentane-1-carboxamide

A suspension of **21B** (0.95 g, 4.4 mmol, 1.0 eq) in THF (20 mL) was treated with 1,1'-carbonyldiimidazole (930 mg, 5.7 mmol, 1.3 eq) at room temperature. After 30 minutes, the mixture was cooled to -78 °C and ammonia gas was introduced. The resulting heterogeneous mixture was allowed to warm to room temperature and was stirred for 16 hours. The solvent was evaporated and 1N hydrochloric acid saturated with sodium chloride was added to the residue. The aqueous mixture was extracted with ethyl acetate (6x50 mL), and the combined organic layers were dried over sodium sulfate and concentrated to yield 500 mg of 21C (53%) as a white solid.

### D. 2-(2-Methoxyethyl)-1,3-diazaspiro[4.4]non-1-en-4-one

A solution of **21C** (460 mg, 2.15 mmol, 1.0 eq), potassium hydroxide (288 mg, 4.30 mmol, 2.0 eq) and methanol (15 mL) was heated at reflux for 20 hours. The mixture was cooled, solid ammonium chloride was added, and the solvent was evaporated. Water was added and the mixture was extracted with ethyl acetate (3x30 mL). The combined organic extracts were dried over sodium sulfate, concentrated, and the residue chromatographed on silica gel (4% methanol in dichloromethane as eluant) to yield 162 mg of **21D** (38%) as an amber oil.

### E. N-(3,4-Dimethyl-5-isoxazolyl)-4'-[2-(2-methoxyethyl)-4-oxo-1,3-diazaspiro[4.4]non-1-en-3-yl)methyl][1,1'-biphenyl]-2-sulfonamide

**21D** (75 mg, 0.38 mmol, 1.0 eq) was alkylated with **P18** (195 mg, 0.38 mmol, 1.0 eq) according to General Method 4. The resulting crude orange oil was dissolved in methanol (7 mL). Concentrated hydrochloric acid (7 mL) was added and the solution was heated at 55°C for 14 hours. The reaction mixture was concentrated and then partitioned between ethyl acetate and pH 5 sodium phosphate buffer. The organic layer was dried over sodium sulfate and concentrated to provide a crude residue. The title compound (7 mg, 3% yield) was obtained as a white powder following silica gel chromatography (4% methanol in chloroform as eluant), preparative reverse-phase HPLC, and lyophilization: MS m/e 537 (ESI+mode); MS m/e 535 (ESI- mode); HPLC retention time 3.35 minutes (Method A).

### Reference Example 22

### N-(3,4-Dimethyl-5-isoxazolyl)-4'-[2-(ethoxymethyl)-4-oxo-1,3-diazaspiro[4.4]non-1-en-3-yl)methyl][1.1'-biphenyl]-2-sulfonamide

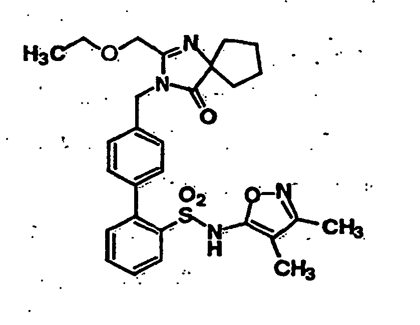

### A. Methyl 1-[(2-ethoxyethanoyl)amino]cyclopentane-1-carboxylate

2-Ethoxyacetic acid (3.3 mL, 35 mmol, 2.1 eq) was added to a mixture of EDCI (3.38 g, 18 mmol, 1.1 eq), triethylamine (7.0 mL, 50 mmol, 3.0 eq), 4-dimethylaminopyridine (20 mg, 0.18 mmol, 0.01 eq), and dichloromethane (100 mL) at room temperature. After 5 minutes, methyl 1-aminocyclopentane-1-carboxylate hydrochloride (3.0 g, 17 mmol, 1.0 eq) was added and the mixture was stirred at room temperature for 18 hours. The solvent was evaporated and the residue partitioned between ether and 1N hydrochloric acid. The organic layer was washed once with 1N hydrochloric acid, then twice with saturated aqueous sodium bicarbonate, and once with brine. The organic layer was dried over magnesium sulfate and concentrated to give 2.0 g of **22A** (52%) as a pink oil.

### B. 2-(Ethoxymethyl)-1,3-diazaspiro[4.4]non-1-en-4-one

A mixture of **22A** (2.0 g, 8.7 mmol, 1.0 eq), lithium hydroxide hydrate (440 mg, 10.5 mmol, 1.2 eq), THF (10 mL), and water (3 mL) was stirred at room temperature for 16 hours. 2N hydrochloric acid (6 mL) was added and the solvents were evaporated. The residue was dried azeotropically with toluene, then dissolved in THF (15 mL) and treated with 1,1'-carbonyldiimidazole (2.8 g, 17.3 mmol, 2.1 eq) at room temperature. After 2 hours, the mixture was cooled to -78 °C and ammonia gas was introduced. The resulting mixture was allowed to warm to room temperature and was stirred for 16 hours, after which the solvent was evaporated. The residue was suspended in methanol (15 mL), solid potassium hydroxide (2.9 g, 43 mmol, 5 eq) was added, and the mixture was heated at reflux for 72 hours. After cooling, the mixture was treated with solid ammonium chloride and the solvent was evaporated. Water was added and the mixture was extracted with ethyl acetate (3x30 mL). The combined organic extracts were dried over sodium sulfate, concentrated, and the residue chromatographed on silica gel (4% methanol in chloroform as eluant) to yield 370 mg of **22B** (22%) as an orange oil.

### C. N-(3,4-Dimethyl-5-isoxazolyl)-4'-[2-(ethoxymethyl)-4-oxo-1,3-diazaspiro[4.4]non-1-en-3-yl)methyl][1,1'-biphenyl]-2-sulfonamide

The title compound was prepared from **22B** (80 mg, 0.41 mmol) and **P18** (209 mg, 0.41 mmol) according to the procedure described in Example 61, Step E, substituting ethanol for methanol in the deprotection reaction. The crude residue was chromatographed twice on silica gel (4% methanol in chloroform as eluant, followed by 25:75:1 hexanes/ethyl acetate/ acetic acid as eluant), then was further purified by reverse-phase preparative HPLC to provide 42 mg of the title compound (19%) as a white solid after lyophilization; MS m/e 537 (ESI+ mode); MS m/e 535 (ESI- mode); HPLC retention time 3.51 minutes (Method A).

### Reference Example 23

### 4'-[(2-Ethyl-5,7-dimethyl-3H-imidazo[4,5-b]pyridin-3-yl)methyl]-2'-[(2-oxo-1-pyrrolidinyl)methyl]-N-(3,4-dimethyl-5-isoxazolyl)[1,1-biphenyl]-2-sulfonamide

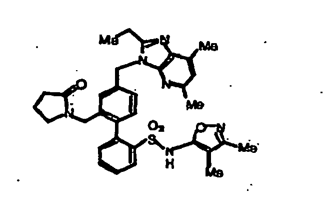

### A. 2'-Formyl-N-(3,4-dimethyl-5-isoxazolyl)-4'-[(methanesulfonyl)oxy]-N-(2-methoxyethoxymethyl)[1,1'-biphenyl]-2-sulfonamide

**P21** (2.4 g) was converted to the corresponding mesylate according to General Method 3. The crude product (2.7 g) was carried on without further purification.

### B. 4'-[(2-Ethyl-5,7-dimethyl-3H-imidazo[4,5-b]pyridin-3-yl)methyl]-2'-formyl-N-(3,4-dimethyl-5-isoxazolyl)-N-(2-methoxyethoxymethyl)[1,1'-biphenyl]-2-sulfonamide

**23A** (2.7 g) was used to alkylate 2-ethyl-5,7-dimethyl-3H-imidazo[4,5-b]pyridine according to General Method 4. The crude product was chromatographed on silica gel using 1:3 hexanes/ethyl acetate as eluant to provide 1.8 g **23B** as a colorless oil.

### C. 4'-[(2-Ethyl-5,7-dimethyl-3H-imidazo[4,5-b]pyridin-3-yl)methyl]-2'-[(2-oxo-1-pyrrolidinyl)methyl]-N-(3,4-dimethyl-5-isoxazolyl)-N-(2-methoxyethoxymethyl)[1,1'-biphenyl]-2-sulfonamide

**23B** (1.5 g) was subjected to reductive amination with ethyl 4-aminobutanoate hydrochloride according to General Method 5. The reaction mixture was allowed to stir for 24 h to allow time for cyclization of the amino ester to the corresponding lactam. The crude product after workup was purified by silica gel chromatography (1:1 hexanes/acetone eluant) to provide **23C** (0.50 g) as a yellow oil.

### D. 4'-[(2-Ethyl-5,7-dimethyl-3H-imidazo[4,5-b]pyridin-3-yl)methyl]-2'-[(2-oxo-1-pyrrolidinyl)methyl]-N-(3,4-dimethyl-5-isoxazolyl)[1,1'-biphenyl]-2-sulfonamide

**23C** (0.50 g) was subjected to sulfonamide deprotection using HCl/dioxane/ethanol according to General Method 8. The crude product was purified by silica gel chromatography (90:9:1 dichloromethane/methanol/ammonium hydroxide eluant), and the purified product was partitioned between ethyl acetate and pH 5 potassium phosphate buffer. The ethyl acetate layer was dried over sodium sulfate and concentrated to provide 310 mg of the title compound as a white solid; mp 98-102 °C; MS m/e 613 (ESI+ mode); HPLC retention time 3.09 min (Method A); HPLC purity 97%.

### Reference Example 24

### 4'-[(2-Ethyl-5,7-dimethyl-3H-imidazo[4,5-b]pyridin-3-yl)methyl]-2'-[(3-methyl-2-oxo-1-imidazolidinyl)methyl]-N-(3,4-dimethyl-5-isoxazolyl)[1,1'-biphenyl]-2-sulfonamide

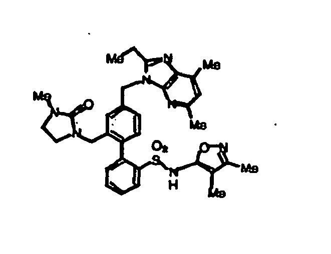

### A. 4'-[(2-Ethyl-5,7-dimethyl-3H-imidazo[4.5-b]pyridin-3-yl)methyl]-2'-[(3-methyl-2-oxo-1-imidazolidinyl)methyl]-N-(3,4-dimethyl-5-isoxazolyl)-N-(2-methoxyethoxymethyl)[1.1'-biphenyl]-2-sulfonamide

**23B** (2.0 g) was subjected to reductive amination with *N-*methylethylenediamine according to General Method 5. The crude product following extractive workup was dissolved in dichloromethane (25 ml) and treated with CDI (0.77 g). The mixture was stirred at RT for 24 h, and was then washed once with water and once with brine. The dichloromethane layer was dried over sodium sulfate and concentrated. The residue was chromatographed on silica gel using 95:5 chloroform/methanol as eluant to give **24A** (0.53 g) as a slightly yellow oil.

### B. 4'-[(2-Ethyl-5,7-dimethyl-3H-imidazo[4,5-b]pyridin-3-yl)methyl]-2'-[(3-methyl-2-oxo-1-imidazolidinyl)methyl]-N-(3,4-dimethyl-5-isoxazolyl)[1,1'-biphenyl]-2-sulfonamide

**24A** (0.50 g) was subjected to sulfonamide deprotection according to General Method 8. The crude product was purified by reverse-phase preparative HPLC to yield 140 mg of the title compound as a white solid following lyophilization; MS m/e 628 (ESI+ mode); HPLC retention time 3.03 min (Method A); HPLC purity 97%.

### Reference Example 25

### (S)-2-[N-[2'-[[N-(3-Methyl-5-isoxazolyl)amino]sulfonyl][1.1'-biphenyl]-4-yl]methyl]-N-(1-oxopentyl)amino]-3,N-dimethylbutanamide

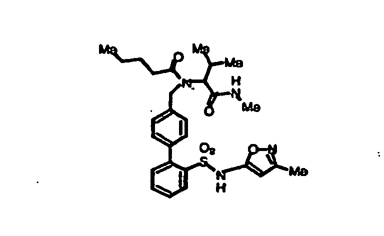

### A. N-(3-Methyl-5-isoxazolyl)-2-bromobenzenesulfonamide

5-Amino-3-methylisoxazole (3.84 g) was added at RT in portions to a solution of 2-bromobenzenesulfonyl chloride (10.0 g) in pyridine (40 ml). The mixture was heated at 60 °C for 16 h, then the solvent was evaporated. The residue was taken up in ethyl acetate and washed three times with 1 N hydrochloric acid. The ethyl acetate layer was dried over sodium sulfate and concentrated to give **25A** (8.8 g).

### B. N-[(2-Trimethylsilyl)ethoxymethyl]-N-(3-methyl-5-isoxsazolyl)-2-bromobenzenesulfonamide

2-(Trimethylsilyl)ethoxymethyl chloride (5.2 ml) was added to a mixture of **25A** (8.8 g), potassium carbonate (7.7 g), and DMF (40 ml) at 0 °C. The mixture was allowed to warm to RT and was then stirred for 16 h. The solvent was evaporated, and the residue was taken up in ethyl acetate and washed with water and brine. The ethyl acetate layer was dried over sodium sulfate and concentrated. The residue was purified by silica gel chromatography using 3:2 hexanes/ethyl acetate as eluant to provide **25B** (6.6 g) as an oil.

### C. 4'-Formyl-N-(3-methyl-5-isoxazolyl)-N-[(2-trimethylsilyl)ethoxymethyl][1,1'biphenyl]-2-sulfonamide

**25B** (2.0 g) and 4-formylphenylboronic acid (1.5 g) were subjected to Suzuki coupling according to General Method 1. The crude product was chromatographed on silica gel using 9:1 hexanes/ethyl acetate as eluant to yield 0.75 g 25C as a colorless oil.

### D. (S)-2-[N-[2'-[[N-(3-Methyl-5-isoxazolyl)-N-[(2-trimethylsilyl)ethoxymethyl]amino]sulfonyl] [1,1'-biphenyl]-4-yl] methyl]-amino]-3,N-dimethylbutanamide

**25C** (0.75 g) was subjected to reductive amination with L-valine N-methyl amide hydrochloride according to General Method 5. Crude **25D** (0.93 g) was obtained as an orange oil.

### E. (S)-2-[N-[[2'-[[N-(3-Methyl-5-isoxazolyl)-N-[(2-trimethylsilyl)ethoxymethyl]amino]sulfonyl][1,1'-biphenyl]-4-yl]methyl]-N-(1-oxopentyl)amino]-3,N-dimethylbutanamide

**25D** (0.93 g) was subjected to acylation with valeryl chloride according to General Method 6. The crude product was chromatographed on silica gel using 2:3 hexanes/ethyl acetate as eluant to yield 0.78 g **25E** as a colorless oil.

### F. (S)-2-[N-[2'-[[N-(3-Methyl-5-isoxazolyl)amino]sulfonyl][1,1'-biphenyl]-4-yl]methyl]-N-(1-oxopentyl)amino]-3,N-dimethylbutanamide

**25E** (0.78g) was deprotected with HCl/methanol according to General Method 8 The crude product was purified by silica gel chromatography using 3:7 hexanes/ethyl acetate as eluant, providing the title compound (210 mg) as a white solid; MS m/e 541 (ESI+ mode); HPLC retention time 31.32 min (Method B); HPLC purity >98%.

### Reference Example 26

### (S)-2[N-[2'-[[N-(4-Bromo-3-methyl-5-isoxazolyl)amino]sulfonyl][1,1'-biphenyl]4-yl]methyl]-N-(1-oxopentyl)amino]-3,N-dimethylbutanamide

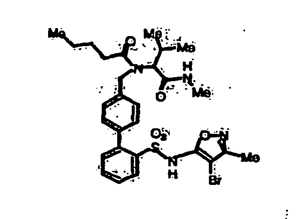

A solution of **25** (75 mg) in chloroform (1.5 ml) was treated with NBS (25 mg) at RT. After 1h; the mixture was diluted with dichloromethane and partitioned against water. The organic layer was dried over sodium sulfate and concentrated, and the residue was chromatographed on silica gel using 3:7 hexanes/ethyl acetate as eluant to provide the title compound (25 mg) as a white solid; MS m/e 619, 621 (ESI+ mode); HPLC retention time 31.83 min (Method B); HPLC purity >99%.

### Reference Example 27

### 4'-[(2-Butyl-4-oxo-1,3-diazaspiro[4.4]non-1-en-3-yl)methyl] -N-(3,4-dimethyl-5-isoxazolyl)-2'-propyl[1.1'-biphenyl]-2-sulfonamide

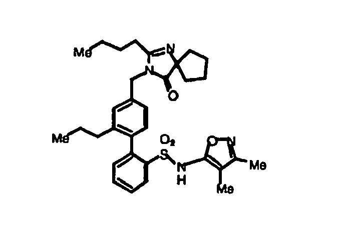

### A. 4-Bromo-3-(1-propen-1-yl)benzonitrile

n-Butyllithium (2.5M solution in hexane, 7.6 ml, 19 mmol) was added dropwise to a solution of ethyltriphenylphosphonium bromide (6.42 g, 17.3 mmol) in 100 ml of 1:1 THF/ether at -15°C. The mixture was stirred for 3h at RT and then was cooled to -50 °C. **2A** (4.0 g, 19.0 mmol) in THF (10 ml) was added and the mixture was allowed to warm to RT and was stirred for 16 h. The mixture was added to water and extracted with EtOAc (3 x 50 mL) and the combined organic extracts were washed with water, dried over magnesium sulfate, and evaporated. The residue was chromatographed on silica gel using 95:5 hexane/EtOAc to afford **27A** as an E/Z mixture (3.5 g, 83%).

### B. 4-Bromo-3-propylbenzonitrile

A mixture of **27A** (1.5 g) and 150 mg of PtO₂ in 40 ml EtOH was hydrogenated at 35 PSI for 40 min. Filtration and concentration gave 1.44 g of **27B** (85%).

### C. 4-Bromo-3-propylbenzaldehyde

**27B** (1.44 g) was treated with DIBAL-H according to General Method 14 to provide crude **27C** (1.4 g, 97%) as an oil.

### D. N-(3,4-Dimethyl-5-isoxazolyl)-4'-formyl-2'-propyl-N-[(2-methoxyethoxy)methyl][1,1'-biphenyl]-2-sulfonamide

**27C** (1.4 g) was subjected to Suzuki coupling according to General Method 1 to provide **27D** (27%) as an oil.

### E. N-(3,4-Dimethyl-5-isoxazolyl)-4'-hydroxymethyl-2'-propyl-N-[(2-methoxyethoxy)methyl][1,1'-biphenyl]-2-sulfonamide

**27D** (810 mg) was reduced with sodium borohydride in methanol according to General Method 11 to provide **27E** (32%) as an oil.

### F. N-(3,4-Dimethyl-5-isoxazolyl)-4'-(methanesulfonyl)oxymethyl-2'-propyl-N-[(2-methoxyethoxy)methyl][1,1'-biphenyl]-2-sulfonamide

**27E** (250 mg) was converted to the corresponding methanesulfonate ester according to General Method 3 to provide **27F** (68%) as an oil.

### G. 4'-[(2-Butyl-4-oxo-1,3-diazaspiro[4.4]non-1-en-3-yl)methyl]-2'-propyl-N-[(2-methoxyethoxy)methyl]-N-(3,4-dimethyl-5-isoxazolyl)[1,1'-biphenyl]-2-sulfonamide

**27F** (100 mg) was used to alkylate 2-butyl-1,3-diazaspiro[4.4]non-1-en-4-one according to General Method 4. **27G** (100 mg, 85%) was produced as an oil.

### H. 4'-[(2-Butyl-4-oxo-1,3-diazaspiro[4.4]non-1-en-3-yl)methyl]-2'-propyl-N-(3,4-dimethyl-5-isoxazolyl)[1,1'-biphenyl]-2-sulfonamide

**27G** (100 mg) was deprotected according to General Method 7. The crude product was purified by preparative HPLC to provide the title compound (57 mg, 66%) as a solid; MS m/e 577 (ESI+ mode); HPLC retention time 29.11 min (Method B); HPLC purity >98%.

### Reference Example 28

### 4'-[(7-Methoxycarbonyl-2-ethoxybenzimidazol-1-yl)methyl]-N-(3,4-dimethyl-5-isoxazolyl)-[1,1'-biphenyl]-2-sulfonamide

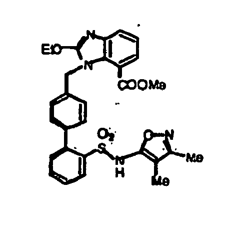

### A. N-(2-Methoxycarbonyl-6-nitrophenyl)-4-bromobenzylamine.

Triethylamine (5.2 ml, 37 mmol) was added to a mixture of methyl 2-chloro-3-nitrobenzoate (3.2 g, 15 mmol) and 4-bromobenzylamine hydrochloride (3.4 g, 15 mmol) in acetonitrile (75 ml). The mixture was heated at reflux for 48 hr, then was cooled and concentrated. 10% Aqueous sodium dihydrogen phosphate solution was added and the mixture was extracted with 2 portions of ethyl acetate. The combined organic extracts were dried over sodium sulfate and concentrated, and the residue was crystallized from ethyl acetate to give **28A** (3.2 g) as a yellow solid.

### B. 4'-[(2-Methoxycarbonyl-6-nitrophenvl)aminomethyl]-N-(3,4-dimethyl-5-isoxazolyl)-N-(2-trimethylsiloxymethyl)[1,1'-biphenyl]-2-sulfonamide

**28A** (3.5 g, 9.6 mmol) was subjected to Suzuki coupling according to General Method 1, providing **28B** as a yellow oil (3.1 g) following silica gel chromatography using 5:1 hezanes/ethyl acetate as eluant.

### C. 4'-[(2-Methoxycarbonyl-6-aminophenyl)aminomethyl]-N-(3,4-dimethyl-5-isoxazolyl)-N-(2-trimethylsiloxymethyl)[1,1'-biphenyl]-2-sulfonamide

**28B** (2.4 g, 3.6 mmol) was treated with tin (II) chloride dihydrate (3.3g) in ethyl acetate (80 ml) according to General Method 18. The crude product was purified by silica gel chromatography using 2:1 hexanes/ethyl acetate as eluant to provide **28C** (1.3 g) as a pale yellow oil.

### D. 4'-[(7-Methoxycarbonyl-2-ethoxybenzimidazol-1-yl)methyl]-N-(3.4-dimethyl-5-isoxazolyl)-N-(2-trimethylsiloxymethyl)[1.1'-biphenyl]-2-sulfonamide

**28C** (1.3 g), tetraethylorthocarbonate (6 ml), and acetic acid (0.2 ml) was heated under a nitrogen atmosphere at 70°C for 2 h. The mixture was cooled and concentrated, and the residue was chromatographed on silica gel using 1:1 hexanes/ethyl acetate as eluant to provide **28D** (1.1 g) as a brown oil.

### E. 4'-[(7-Methoxycarbonyl-2-ethoxybenzimidazol-1-yl)methyl]-N-(3,4-dimethyl-5-isoxazolyl)-[1,1'-biphenyl)-2-sulfonamide

**28D** (1.1 g) was subjected to sulfonamide deprotection using TBAF in THF according to General Method 10. The crude product was purified by silica gel chromatography using 2:1 hexanes/acetone as eluant to provide 0.75 g of the title compound as a white solid; mp 105-110 °C; MS m/e 561 (ESI+ mode); HPLC retention time 3.96 min (Method A); HPLC purity 96%.

### Reference Example 29

### 4'-[(7-Carboxy-2-ethoxybenzimidazol-1-yl)methyl]-N-(3,4-dimethyl-5-isoxazolyl)-[1,1'-biphenyl]-2-sulfonamide

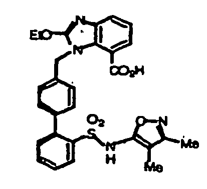

28 (0.70 g) was subjected to ester hydrolysis according to General Method 15 to provide the crude product (0.66 g). Purification of a portion by reverse-phase preparative HPLC provided the title compound (9 mg, white solid); MS m/e 547 (ESI+ mode); HPLC retention time 3.79 min (Method A); HPLC purity 91%.

### Reference Example 30

### 4'-[(7-Methoxycarbonyl-2-ethylbenzimidazol-1-yl)methyl]-N-(3,4-dimethyl-5-isoxazolyl)-[1,1'-biphenyl]-2-sulfonamide

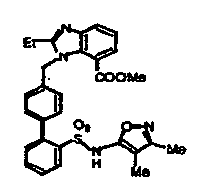

A solution of **28C** (3.8 g, 5.9 mmol) and triethylamine (1.7 ml, 12 mmol) in dichlroromethane (25 ml) was treated at 0 °C with propionyl chloride (0.67 ml, 10 mmol), and the mixture was allowed to come to RT. After 2.5 h, aqueous sodium bicarbonate solution was added to the mixture and the aqueous layer was extracted with two portions of dichloromethane. The combined organic extracts were dried over sodium sulfate and concentrated to provide an orange oil,

To this residue was added methanolic hydrogen chloride (prepared from 100 ml methanol and 11 ml (200 mmol) acetyl chloride), and the resulting solution was heated at 50°C for 16 h. The mixture was cooled and concentrated, and the residue was extracted with two portions of ethyl acetate from sodium phosphate buffer adjusted to pH 4. The combined ethyl acetate extracts were dried over sodium sulfate and concentrated. The residue was chromatographed on silica gel using 1:2 hexanes/acetone as eluant to provide the title compound (2.6 g) as a slightly orange solid; MS m/e 545 (ESI+ mode); HPLC retention time 3.32 min (Method C); HPLC purity 95%.

### Reference Example 31

### 4'-[(7-Carboxy-2-ethylbenzimidazol-1-yl)methyl]-N-(3,4-dimethyl-5-isoxazolyl)-[1,1'-biphenyl]-2-sulfonamide

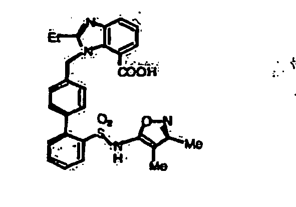

**30** (2.6 g, 4.8 mmol) was subjected to ester hydrolysis according to General Method 15. The THF was evaporated and the residue was treated with 6 ml of 2N hydrochloric acid, resulting in the precipitation of a white solid. The solid was collected on a filter, rinsed with water, and dried to provide 2.4 g of the title compound; MS m/e 531 (ESI+mode); HPLC retention time 2.94 min (Method A); HPLC purity 95%.

### Reference Example 32

### 2'-[(3,3-Dimethyl-2'-oxopyrrolidin-1-yl)methyl]-4'-[(2-ethoxy-7-(methoxycarbonyl)benzimidazol-1-yl)methyl]-N-(3,4-dimethyl-5-isoxazolyl)-[1,1'-biphenyl]-2-sulfonamide

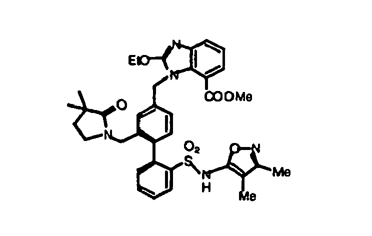

### A. 2-[(5'-Aminomethyl-2'-bromo)phenyl)]-1,3-dioxolane

Borane•THF (100 ml of a 1.0 M solution in THF, 100 mmol) was added at 0 °C to a solution of 2-[(2'-Bromo-5'-cyano)phenyl)]-1,3-dioxolane [Zhang, H.-Y. et al., *Tetrahedron,* 50, 11339-11362 (1994)] (10.8 g, 43 mmol) in 25 ml THF. The mixture was allowed to warm to RT and was stirred for 18 h. After cooling to 0 °C, the mixture was treated carefully with 10 ml methanol and was then evaporated. The residue was taken up in 150 ml ethyl acetate and was washed with 1N aqueous sodium hydroxide, followed by water and brine. The organic layer was dried over magnesium sulfate and concentrated to give crude **32A** (11.0 g) as an amber oil, which was estimated (HPLC) to be 80% pure.

### B. N-(2-Methoxycarbonyl-6-nitrophenyl)-4-bromo-3-(1,3-dioxolan-2-yl)benzylamine

Triethylamine (4.8 ml, 34 mmol) was added to a mixture of methyl 2-chloro-3-nitrobenzoate (4.9 g, 23 mmol) and **32A** (5.9 g of a 65% pure mixture, 15 mmol) in acetonitrile (150 ml). The mixture was heated at reflux for 24 h and was then cooled and concentrated. Ethyl acetate was added and the solution was washed twice with 10% aqueous potassium dihydrogen phosphate solution and once with aqueous sodium bicarbonate solution. The organic layer was concentrated and the residue purified by silica gel column chromatography using 3:1 hexanes/ethyl acetate as eluant, followed by trituration with 3:1 hexanes/ethyl acetate. **32B** was a yellow solid (6.6 g).

### C. N-(2-Methoxycarbonyl-6-nitrophenyl)-4-bromo-3-(formyl)benzylamine

**32B** (6.6 g, 15 mmol) was subjected to acetal hydrolysis according to General Method 19, providing **32C** as a crude yellow solid following extractive workup.

### D. 2'-Formyl-4'-[(2-methoxycarbonyl-6-nitrophenyl)aminomethyl]-N-(3,4-dimethyl-5-isoxazolyl)-N-(2-trimethylsiloxymethyl)[1,1'-biphenyl]-2-sulfonamide

Crude **32C** was subjected to Suzuki coupling according to General Method 1, providing **32D** (5.9 g) as a yellow oil following silica gel chromatography using 2:1 hexanes/ethyl acetate as eluant.

### E. 2'-[(3,3-Dimethyl-2-oxopyrrolidin-1-yl)methyl]-4'-[(2-methoxycarbonyl-6-nitrophenyl)aminomethyl]-N-(3,4-dimethyl-5-isoxazolyl)-N-(2-trimethylsiloxymethyl) [1,1'-biphenyl]-2-sulfonamide

**32D** (4.3 g) was subjected to reductive amination with ethyl 4-amino-2, 2-dimethylbutanoate hydrochloride according to General Method 5. The reaction mixture was allowed to stir for 60 h at RT to allow time for cyclization of the amino ester to the corresponding lactam. The crude product after workup was purified by silica gel chromatography, using 3:2 hexanes/ethyl acetate as eluant, to provide **32E** (2.8 g) as a yellow oil.

### F. 2'-[(3,3-Dimethyl-2-oxopyrrolidin-1-yl)methyl]-4'-[(2-methoxycarbonyl-6-aminophenyl)aminomethyl]-N-(3,4-dimethyl-5-isoxazolyl)-N-(2-trimethylsiloxymethyl)[1,1'-biphenyl]-2-sulfonamide

**32E** (2.8 g, 3.6 mmol) was treated with tin (II) chloride dihydrate (3.2 g) in ethyl acetate (200 ml) according to General Method 18. The crude product (3.5 g) was used without further purification.

### G. 2'-[(3,3-Dimethyl-2-oxopyrrolidin-1-yl)methyl]-4'-[((7-methoxycarbonyl)benzimidazol-1-yl)methyl]-N-(3,4-dimethyl-5-isoxazolyl)-N-(2-trimethylsiloxymethyl)[1,1'-biphenyl]-2-sulfonamide

A mixture of **32F** (1.5 g, 2.0 mmol), tetraethylorthocarbonate (6 ml), and acetic acid (0.15 ml) was heated under a nitrogen atmosphere at 70°C for 2 h. The mixture was cooled and concentrated, and the residue chromatographed on silica using 1:2 hexanes/ethyl acetate as eluant to provide **32G** (0.80 g) as a yellow oil.

### H. 2'-[(3,3-Dimethyl-2-oxopyrrolidin-1-yl)methyl]-4'-[(2-ethoxy-7-(methoxycarbonyl)benzimidazol-1-yl)methyl]-N-(3,4-dimethyl-5-isoxazolyl)-[1,1'-biphenyl]-2-sulfonamide

**32G** (0.80 g) was subjected to sulfonamide deprotection using TBAF in THF according to General Method 10. The crude product was purified by silica gel chromatography using 3:2 hexanes/acetone as eluant to provide 0.55 g of the title compound as a white solid; mp 101-103 °C (decomp); MS m/e 686 (ESI+ mode); HPLC retention time 3.91 min (Method A); HPLC purity >98%.

### Reference Example 33

### 2'-[(3,3-Dimethyl-2-oxopyrrolidin-1-yl)methyl]-4'-[(2-ethoxy-7-(carboxy)benzimidazol-1-yl)methyl]-N-(3,4-dimethyl-5-isoxazolyl)-[1,1'-biphenyl]-2-sulfonamide

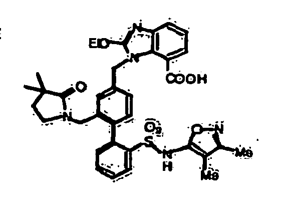

**32** (0.31 g) was subjected to ester hydrolysis according to General Method 15. Purification by reverse-phase preparative HPLC provided the title compound (14 mg) as a white solid, MS m/e 672 (ESI+ mode); HPLC retention time 3.61 min (Method A); HPLC purity 82%.

### Reference Example 34

### 2'-[(3,3-Dimethyl-2-oxopyrrolidin-1-yl)methyl]-4'-[(2-ethoxy-7-(N-methylcarbamoyl)benzimidazol-1-yl)methyl]-N-(3,4-dimethyl-5-isoxazolyl)-[1,1'-biphenyl]-2-sulfonamide

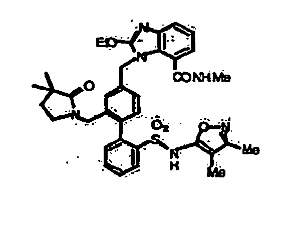

**33** (80 mg) was subjected to amide formation according to General Method 12 using methylamine as the amine component. The product was purified by reverse-phase preparative HPLC: white solid (7 mg); MS m/e 685 (ESI+ mode); HPLC retention time 3.39 min (Method A); HPLC purity 81%.

### Reference Example 35

### 2'-[(3,3-Dimethyl-2-oxopyrrolidin-1-yl)methyl]-4'-[(2-ethoxy-7-(N,N-dimethylcarbamoyl)benzimidazol-1-yl)methyl]-N-(3,4-dimethyl-5-isoxazolyl)-[1,1'-biphenyl]-2-sulfonamide

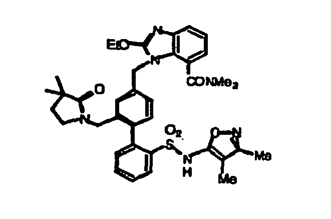

33 (80 mg) was subjected to amide formation according to General Method 12 using dimethylamine as the amine component. The crude product was subjected to reverse-phase preparative HPLC to give the title compound as a white solid (6 mg); MS m/e 699 (ESI+ mode); HPLC retention time 3.46 min (Method A); HPLC purity 66% [contaminant (34%) is the corresponding imidazolin-2-one].

### Reference Example 36

### 4'-[(2-Ethylquinolin-4-yl)oxymethyl]-N-(1,3,5-trimethylpyrazol-4-yl) [1,1'-biphenyl]-2-sulfonamide

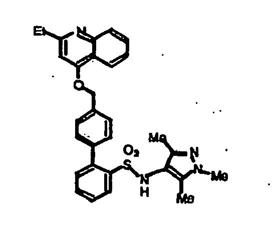

### A. 4-[(4-Bromophenyl)methoxy]-2-ethylquinoline

A mixture of 2-ethyl-4-quinolone (1.0 g, 5.8 mmol), 4-bromobenzyl bromide (1.7 g, 6.9 mmol), potassium carbonate (1.6 g, 11.6 mmol), and DMF (10 ml) was stirred at RT for 16 h. Ethyl acetate (100 ml) was added and the mixture was washed four times with water, then once with brine. The organic layer was dried over sodium sulfate and evaporated, and the residue was triturated with 1:1 hexanes/ethyl acetate to provide 1.6 g **36A** as a white solid.

### B. 4-[(2-Ethylquinolin-4-yl)oxymethyl]-N-(tert-butyl)-[1,1'-biphenyl]-2-sulfonamide

A mixture of **36A** (1.5 g, 4.4 mmol) and [2-(*N*-*tert-*butylsulfamoyl)phenyl]boronic acid (2.3 g, 8.7 mmol) was subjected to Suzuki coupling according to General Method 1. The crude product was chromatographed on silica gel using 1:1 hexanes/ethyl acetate as eluant to provide the **36B** (1.8 g) as a yellow solid.

### C. 4'-[(2-Ethylquinolin-4-yl)oxymethyl]-[1,1'-biphenyl]-2-sulfonamide

A solution of **36B** (1.8 g, 3.8 mmol) in 4 ml dichloromethane and 8 ml TFA was stirred at RT for 14 h, then was heated- at reflux for 8 h. The solvent was evaporated and the residue partitioned between ethyl acetate and aqueous sodium bicarbonate. A small amount of a solid precipitate was retained with the organic layer. The organic layer was washed twice with water, then was concentrated. The crude product was crystallized from 1:1 toluene/ethyl acetate to provide **36C** (1.2 g) as a yellow solid.

### D. 4'-[(2-Ethylquinolin-4-yl)oxymethyl] [1,1'-biphenyl]-2-sulfonic acid

A suspension of **36C** (1.1 g, 2.7 mmol) in 25 ml acetonitrile was treated at 0 °C with nitrosonium tetrafluoroborate (370 mg, 3.2 mmol). After 30 min the mixture was allowed to warm to RT and was stirred at RT for 4 h. The mixture was concentrated to provide **36D** (1.2 g) as a crude white solid.

### E. 4'-[(2-Ethylquinolin-4-yl)oxymethyl]-N-(1,3,5-trimethylpyrazol-4-yl)[1,1'-biphenyl]-2-sulfonamide

A suspension of **36D** (100 mg, 0.24 mmol) in thionyl chloride (4 ml) was treated with DMF (10 µl). The resulting mixture was refluxed for 45 min, then the solvent was evaporated. The residue was twice taken up in toluene and evaporated to dryness. Pyridine (2.5 ml) and 4-amino-1,3,5-trimethylpyrazole (90 mg, 0.72 mmol) were added and the mixture was stirred at RT for 14 h, then the solvent was evaporated. The residue was purified by silica gel chromatography (100:1 chloroform/methanol eluant), followed by silica gel thin-layer chromatography (1:1 hexanes/acetone eluant) to provide the title compound (24 mg) as an amorphous white solid: mp 212-215 °C (decomp); MS m/e 527 (ESI+ mode); HPLC retention time 3.18 min (Method C); HPLC purity 94%.

### Reference Example 37

### 4'-[(2-Ethylquinolin-4-yl)oxymethyl]-N-(3-methylisoxazol-5-yl) [1,1'-biphenyl]-2-sulfonamide

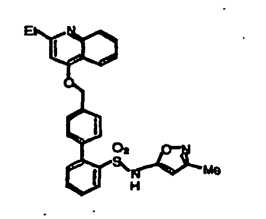

A suspension of the product of **36D** (370 mg, 0.88 mmol) was subjected to the procedure used for Example 36, Step E, substituting 5-amino-3-methylisoxazole as the amine component. The crude product was purified by reverse-phase preparative HPLC to provide the title compound (8 mg) as an amorphous tan solid: MS m/e 500 (ESI+ mode); HPLC retention time 3.32 min (Method A); HPLC purity >98%.

### Reference Example 38

### 4'-[(5-Acetyl-2-n-propyl-4-chloroimidazol-1-yl)methyl]-N-(3,4-dimethyl-5-isoxazolyl)-[1,1'-biphenyl]-2-sulfonamide

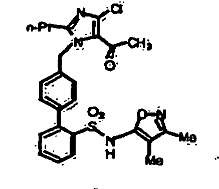

### A. 5-(1-Hydroxyethyl)-2-n-propyl-4-chloromidazole

A solution of 2-n-propyl-4-chloroimidazole-5-carboxaldehyde (Watson, S.P. *Synth. Comm.,* **1992**, *22*, 2971-2977) (1.5 g, 8.7 mmol) in THF (50 ml) was treated dropwise at 0 °C with methylmagnesium bromide (8.7 ml of a 3.0 M solution in ether). Upon completion of the addition, the mixture was allowed to warm to RT and was stirred for 2h. Tne mixture was cooled again to 0 °C and was quenched with the addition of 1N hydrochloric acid. The mixture was adjusted to pH 8-9 by the addition of aqueous dipotassium hydrogen phosphate solution, then was partitioned against ethyl acetate. The ethyl acetate layer and an accompanying precipitate were collected and the solvent was evaporated to provide 38A (1.6 g) as a slightly yellow solid.

### B. 5-Acetyl-4-chloro-2-n-propyl-imidazole

A mixture of **38A** (1.6 g), activated manganese dioxide (5.7 g), and dioxane (20 ml) was heated at 55°C for 48 h. The mixture was cooled and filtered through celite, and the filter cake was rinsed with dichloromethane. The combined filtrates were evaporated and the residue was chromatographed on silica gel using 3:1 hexanes/ethyl acetate as eluant. The product was further purified by trituration with 9:1 hexanes/ethyl acetate, providing 0.6 g **38B** as an orange solid.

### C. 4'-[(5-Acetyl-2-n-propyl-4-chloroimidazol-1-yl)methyl]-N-(3,4-dimethyl-5-isoxazolyl)-N-(2-trimethylsiloxymethyl)[1,1'-biphenyl]-2-sulfonamide

**38B** (148 mg, 0.79 mmol) was alkylated with **P19** (300 mg, 0.53 mmol) according to General Method 22. The crude product was purified by silica gel chromatography using 2:1 hexanes/ethyl acetate as eluent to provide 38C (89 mg) as a yellow oil.

### D. 4'-[(5-Acetyl-2-n-propyl-4-chloroimidazol-1-yl)methyl]-N-(3,4-dimethyl-5-isoxazolyl)-[1,1'-biphenyl]-2-sulfonamide

Deprotection of 38C (60 mg) according to General Method 8, followed by preparative thin-layer chromatography using 1:1 hexanes/aceteone as eluant, provided the title compound (24 mg) as a white solid: MS m/e 528 (ESI+ mode); HPLC retention time 3.75 min (Method A); HPLC purity 98%.

### Reference Example 39

### 4'-[(5-Methoxycarbonyl-2-n-propyl-4-chloroimidazol-1-yl)methyl]-N-(3,4-dimethyl-5-isoxazolyl)-[1,1'-biphenyl]-2-sulfonamide

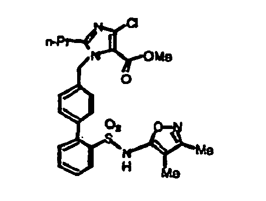

### A. 4'-[( 5-Formyl-2-n-propyl-4-chloroimidazol-1-yl)methyl]-N-(3,4-dimethyl-5-isoxazolyl)-N-(2-trimethylsiloxymethyl)[1,1'-biphenyl]-2-sulfonamide

**P19** (300 mg) was used to alkylate 2-n-propyl-4-chloroimidazole-5-carboxaldehyde according to General Method 22. The crude product was chromatographed on silica gel using 4:1 hexanes/ethyl acetae as eluant to provide 39A (200 mg) as a yellow oil.

### B. 4'-[(5-Carboxy-2-n-propyl-4-chloroimidazol-1-yl)methyl]-N-(3,4-dimethyl-5-isoxazolyl)-N-(2-trimethylsiloxmethyl)[1,1'-biphenyl]-2-sulfonamide

Sodium chlorite (19 mg, 0.21 mmol) was added to a mixture of **39A** (90 mg, 0.14 mmol) and sulfamic acid (20 mg, 0.21 mmol) in 1:1 THF/water (8 ml) at 0 °C. The mixture was stirred at 0 °C for 1h, then saturated potassium bisulfate solution was added. The aqueous layer was extracted twice with ethyl acetate. The combined organic extracts were washed once with brine, then were dried over sodium sulfate and concentrated to provide **39B** (62 mg) as a yellow oil.

### C. 4'-[( 5-Carboxy-2-n-propyl-4-chloroimidazol-1-yl)methyl]-N-(3,4-dimethyl-5-isoxazolyl) 1,1'-biphenyl]-2-sulfonamide

**39B** (62 mg) was deprotected according to General Method 8, using water in place of an alcohol as co-solvent. Crude **39C** (54 mg) was a yellow oil.

### D. 4'-[( 5-Methoxycarbonyl-2-n-propyl-4-chloroimidazol-1-yl)methyl]-N-(3,4-dimethyl-5-isoxazolyl) 1,1'-biphenyl]-2-sulfonamide

**39C** (54 mg) was subjected to ester formation according to General Method 20. Reverse-phase preparative HPLC provided the title compound (9 mg) as a white solid: MS m/e 544 (ESI+ mode); HPLC retention time 3.94 min (Method A); HPLC purity >98%

### Reference Example 40

### 4'-[(5-(N,N-dimethylcarbamoyl)-2-n-propyl-4-chloroimidazol-1-yl)methyl]-N-(3,4-dimethyl-5-isoxazolyl)-[1,1'-biphenyl]-2-sulfonamide

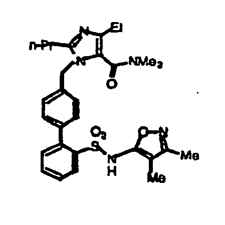

### A. 4'-[(5-Ethoxycarbonyl-2-n-propyl-4-ethylimidazol-1-yl)methyl]-N-(3,4-dimethyl-5-isoxazolyl)-N-(2-methoxyethoxymethyl)[1,1'-biphenyl]-2-sulfonamide

Ethyl 2-n-propyl-4-ethylimidazole-5-carboxylate (94 mg, 0.45 mmol) was alkylated with **P18** (380 mg, 0.37 mmol), according to General Method 22 to provide crude **40A** as a 3:1 mixture of N-1 and N-3 regioisomeric alkylation products.

### B. 4'-[(5-Carboxy-2-n-propyl-4-ethylimidazol-1-yl)methyl]-N-(3,4-dimethyl-5-isoxazolyl)-[1,1'-biphenyl]-2-sulfonamide

A solution of **40A** (0.45 g) in dioxane (3 ml) and 6N hydrochloric acid (3 ml) was heated at 70 °C for 2 h. The mixture was cooled to RT and made basic (pH > 14) with the addition of 45% aqueous potassium hydroxide solution. Additional dioxane and water were added to obtain a clear solution and the mixture was stirred at RT for 16 h, followed by heating at 70 °C for 3 h. The pH was adjusted to 2-3 with the addition of 6N hydrochloric acid and solid trisodium phosphate, and the mixture was extracted with three portions of ethyl acetate. The combined organic extracts were dried over sodium sulfate and evaporated to give **40B** (0.33 g) as a crude oil.

### C. 4'-[(5-(N,N-dimethylcarbamoyl)-2-n-propyl-4-ethylimidazol-1-yl)methyl]-N-(3,4-dimethyl-5-isoxazoly)-[1,1'-biphenyl]-2-sulfonamide

**40B** (110 mg) was subjected to amide formation according to General Method 12 using dimethylamine as the amine component. The crude material was subjected to reverse-phase preparative HPLC to provide the title compound (19 mg) as a white solid: MS m/e 550 (ESI+ mode); HPLC retention time 19.54 min (Method B); HPLC purity 74%. The contaminant (24%) is the isomeric product arising from N-3 alkylation of the imidazole in Step A (HPLC retention time 19.77 min).

### Reference Example 41

### 4'-[(2-Butyl-4-oxo-1,3-diazaspiro[4.4]non-1-en-3-yl)methyl]-N-(3,4-dimethyl-5-isoxazolyl)-2'-hydroxymethyl[1.1'-biphenyl]-2-sulfonamide

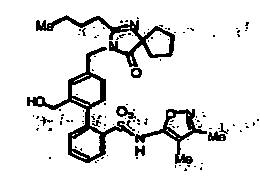

### A. 4'-[(2-Butyl-4-oxo-1,3-diazaspiro[4,4]non-1-en-3-yl)methyl]-N-(3,4-dimethyl-5-isoxazolyl)-N-[(2-trimethylsilylethoxy)methyl]-2'-hydroxymethyl[1,1'-biphenyl]-2-sulfonamide

**P14** (243 mg, 0.41 mmol) was used to alkylate 2-butyl-4-oxo-1,3-diazaspiro[4.4]non-1-ene hydrochloride according to General Method 4. **41A** (100 mg, 35% yield) was isolated as a slightly yellow oil after silica gel chromatography using 1:1 hexanes/ethyl acetate as eluant.

### B. 4'-[(2-Butyl-4-oxo-1,3-diazaspiro[4.4] non-1-en-3-yl)methyl]-N-(3,4-dimethyl-5-isoxazolyl)-2'-hydroxymethyl[1,1'-biphenyl]-2-sulfonamide

Deprotection of **41A** (100 mg, 0.14 mmol) according to General Method 8 (ethanol) gave the title compound as white solid in 46% yield following silica gel chromatography (96:4 methanol/chloroform eluant): MS m/e 565 (ESI+ mode); HPLC retention time 3.21 min (Method A); HPLC purity >98%.

### Reference Example 42

### 4'-[(2-Butyl-4-oxo-1,3-diazaspiro[4.4]non-1-en-3-yl)methyl]-N-(3.4-dimethyl-5-isoxazolyl)-2'-ethoxymethyl[1.1'-biphenyl]-2-sulfonamide

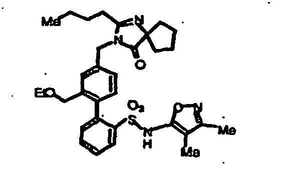

### A. 4'-[(2-Butyl-4-oxo-1,3-diazaspiro[4,4]non-1-en-3-yl)methyl]-N-(3,4-dimethyl-5-isoxazolyl)-N-[(2-methoxyethoxy)methyl]-2'-hydroxymethyl[1,1'-biphenyl]-2-sulfonamide

Triethylsilane (6 ml) and TFA (6 ml) were added to a solution of 5F (960 mg, 1.5 mmol) in 15 ml dichloromethane at RT. The mixture was stirred at RT for 2 h and was then concentrated. The residue was taken up in ethyl acetate and was washed successively with aqueous sodium bicarbonate, water, and brine. The organic layer was dried over sodium sulfate and concentrated. The residue was chromatographed on silica gel using 100:2 dichloromethane/methanol to afford **42A** (740 mg, 77%) as a colorless gum. Rf=0.13, silica gel, 100:5 dichloromethane/methanol.

### B. 4'-[(2-Butyl-4-oxo-1,3-diazaspiro[4,4]non-1-en-3-yl)methyl]-N-(3,4-dimethyl-5-isoxazolyl)-N-[(2-methoxyethoxy)methyl]-2'-ethoxymethyl[1,1'-biphenyl]-2-sulfonamide

A mixture of **42A** (100 mg, 0.15 mmol), iodoethane (960 mg, 6.1 mmol) and silver (I) oxide (180 mg, 0.77 mmol) in 0.7 ml DMF was heated at 40 °C for 16 h. Additional iodoethane (190 mg, 1.2 mmol) and silver (I) oxide (71 mg, 0.31 mmol) were added and the reaction mixture was heated at 40 °C for an additional 4 h. The mixture was diluted with 1:4 hexanes/ethylacetate and was then washed with water and brine. The organic layer was dried-over sodium sulfate and was then concentrated. The residue was chromatographed on silica gel using 200:3 dichloromethane/methanol as eluant to afford **42B** (51mg, 49%) as a colorless gum. Rf=0.35, silica gel, 100:5 dichloromethane/methanol.

### C. 4'-[(2-Butyl-4-oxo-1,3-diazaspiro[4,4]non-1-en-3-yl)methyl]-N-(3,4-dimethyl-5-isoxazolyl)-2'-ethoxymethyl[1,1'-biphenyl]-2-sulfonamide

**42B** (51 mg) was deprotected according to General Method 7 to afford the title compound in 80% yield following preparative reverse-phase HPLC purification: white solid; m.p. 74-80°C (amorphous); 1H NMR (CDCl₃)δ0.87(tr, J=7Hz, 3H), 0.99(tr, J=7Hz, 3H), 1.32 (m, 2H), 1.59(m, 2H), 1.75-2.02(m, 11H), 2.16(s, 3H), 2.35(m, 2H), 3.38 (m, 2H), 4.23(m, 2H), 4.73(s, 2H), 7.11-7.85 (m, 7H); MS m/e 593 (ESI+ mode); HPLC retention time 18.22 min. (Method E); HPLC purity >97%.

### Reference Example 43

### 4'-[(2-Butyl-4-oxo-1,3-diazaspiro[4.4]non-1-en-3-yl)methyl] -N-(3,4-dimethyl-5-isoxazolyl)-2'-propyl[1,1'-biphenyl]-2-sulfonamide

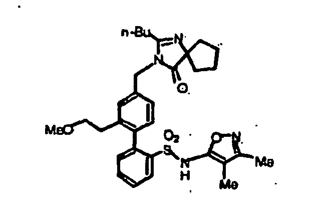

### A. 4'-[(2-Butyl-4-oxo-1,3-diazaspiro[4.4]non-1-en-3-yl)methyl]-N-[(2-methoxyethoxy)methyl]-N-(3,4-dimethyl-5-isoxazolyl)-2'-(2-methoxyvinyl)[1,1'-biphenyl]-2-sulfonamide

**5F** was treated with methoxymethyltriphenylphosphonium bromide according to the procedure used in Example 27, Step A. The product (34%) was generated as a mixture of E and Z isomers:

### B. 4'-[(2-Butyl-4-oxo-1,3-diazaspiro[4.4]non-1-en-3-yl)methyl]-N-(3,4-dimethyl-5-isoxazolyl)-2'-methoxyethyl [1,1'-biphenyl]-2-sulfonamide

**43A** (18 mg) was treated with triethylsilane and TFA according to the procedure of Example 42, Step B, to provide the title compound (6 mg, 45%) as an oil: MS m/e 593 (ESI+ mode); HPLC retention time 24.74 min. (Method B); HPLC purity >98%.

### Reference Example 44

### 4'-[(2-Butyl-4-oxo-1,3-diazaspiro[4.4]non-1-en-3-yl)methyl]-2'-[(3,3-dimethyl-2-oxo-1-pyrrolidinyl)methyl]-N-(3-methoxy-5-methyl-2-pyrazinyl)[1,1'-biphenyl]-2-sulfonamide

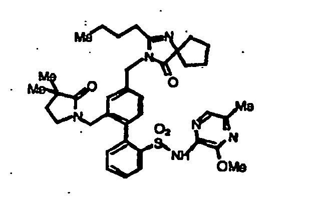

### A. N-(3-Methoxy-5-methyl-2-pyrazinyl)-2-bromobenzenesulfonamide

2-amino-3-methoxy-5-methylpyrazine (1.50 g, 10.8 mmol; synthesized according to Bradbury, R. H., et. al. *J. Med. Chem.* **1997**,*40*, 996-1004) and 2-bromobenzenesulfonyl chloride (2.80 g, 11.0 mmol) were reacted according to the procedure of Example 25, Step A. **44A** was a pink solid, 2.0 g (52%).

### B. N-(3-Methoxy-5-methyl-2-pyrazinyl)-N-[2-(trimethylsilyl)ethoxymethyl]-2-bromobenzenesulfonamide

**44A** (2.0 g) was reacted with 2-(trimethylsilyl)ethoxymethyl chloride (1.15 ml) according to the procedure of Example 25, Step B. The crude residue was chromatographed on silica gel using 4:1 hexanes/ethyl acetate to give **44B** (2.37 g, 86%) as a yellow oil.

### C. 4'-[(2-Butyl-4-oxo-1,3-diazaspiro[4.4]non-1-en-3-yl)methyl]-2'-(1,3-dioxolan-2-yl)-N-[2-(trimethylsilyl)ethoxymethyl]-N-(3-methoxy-5-methyl-2-pyrazinyl)[1,1'-biphenyl]-2-sulfonamide

A solution of **5E** (2.0 g, 4.6 mmol) in ether (45 ml) was treated at -78 °C with t-butyllithium (1.7 M in pentane, 5.9 ml, 10.1 mmol). After stirring at -78 °C for 10 min, the mixture was treated with trimethylborate (1.3 ml, 11.5 mmol) and was then allowed to warm to RT. The mixture was concentrated and the residue azeotroped twice with methanol to produce a pale yellow solid (3.5 g).

The crude solid was subjected to Suzuki coupling with **44B** (2.37 g) according to General Method 1. Silica gel chromatography using 1:1 hexanes/ethyl acetate as eluant provide 1.45 g **44C** (39%) as a yellow oil.

### D. 4'-[(2-Butyl-4-oxo-1,3-diazaspiro[4.4]non-1-en-3-yl)methyl]-2'-formyl-N-(3-methoxy-5-methyl-2-pyrazinyl)[1,1'-biphenyl]-2-sulfonamide

**44C** (1.45 g, 1.9 mmol) was treated with 2M sulfuric acid (11 ml) and ethanol (11 ml) at RT for 6 h. Aqueous sodium bicarbonate was added (final pH 7) and the mixture was extracted with ethyl acetate. The combined organic extracts were dried over sodium sulfate and concentrated to provide crude **44D** (0.91 g) as an off-white solid.

### E. 4'-[(2-Butyl-4-oxo-1,3-diazaspiro[4,4]non-1-en-3-yl)methyl]-2'-[(3,3-dimethyl-2-oxo-1-pyrrolidinyl)methyl]-N-(3-methoxy-5-methyl-2-pyrazinyl)[1,1'-biphenyl]-2-sulfonamide

**44D** (0.91 g) was reacted with ethyl 4-amino-2,2-dimethylbutanoate hydrochloride according to General Method 5. The crude residue was purified by silica gel column chromatography using 1:3 hexanes/ethyl acetate as eluant to provide the title compound (230 mg, 18% over two steps) as a yellow solid: MS m/e 687 (ESI+ mode); HPLC retention time 3.58 min (Method A); HPLC purity 95%.

### Reference Example 45

### 4'-[(2-Butyl-4-oxo-1,3-diazaspiro[4,4]non-1-en-3-yl)methyl]-N-(4-bromo-3-methyl-5-isoxazolyl)2'-[(3,3-dimethyl-2-oxo-1-pyrrolidinyl)methyl][1,1'-biphenyl]-2-sulfonamide

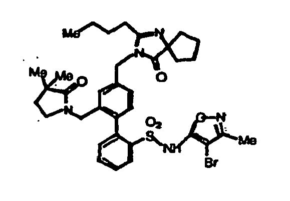

### A. N-(2-methoxyethoxymethyl)-N-(3-methyl-5-isoxazolyl)-2-bromobenzenesulfonamide

**25A** (10.0 g, 31.5 mmol) was reacted with MEM chloride according to the procedure of Example 25, Step B. The crude residue was chromatographed on silica gel using 2:1 hexanes/ethyl acetate to give 4.8 g **45A** (38%) as a yellow oil.

### B. [2-[[(3-methyl-5-isoxazolyl)[(2-methoxyethoxy)-methyl]amino] sulfonyl]phenyl]boronic acid

n-Butyllithium (1.35 M solution in hexanes, 9.7 ml, 13 mmol) was added dropwise over 5 min to a 0.2 M solution of the**45A** (4.8 g, 12 mmol) in THF at -90 °C. After 10 min, trimethylborate (1.6 ml, 14 mmol) was added and the mixture was allowed to warm to RT and stirred for 30 min. The mixture was cooled to 0 °C and treated with 21 ml of 3N hydrochloric acid, after which it was allowed to warm to RT over 30 min. Brine was added and the mixture was extracted with dichloromethane. The combined extracts were dried over sodium sulfate and concentrated to give **45B** (5.3 g) as a yellow oil.

### C. 4'-[(2-Butyl-4-oxo-1,3-diazaspiro[4.4]non-1-en-3-yl)methyl]-N-(2-methoxyethoxymethyl)-N-(3-methyl-5-isoxazolyl)-2'-formyl][1,1'-biphenyl]-2-sulfonamide

**45B** (4.5 g) was subjected to Suzuki cooupling with 5E according to General Method 1. Silica gel chromatography using 1:2 hexanes/ethyl acetate as eluant provide **45C** (1.30 g, 17%) as a yellow oil.

### D. 4'-[(2-Butyl-4-oxo-1,3-diazaspiro[4.4]non-1-en-3-yl)methyl]-N-(3-methyl-5-isoxazolyl)-N-[2-methoxyethoxy)methyl]-2'-[(3,3-dimethyl-2-oxo-1-pyrrolidinyl)methyl][1,1'-biphenyl]-2-sulfonamide

**45C** (420 mg, 0.66 mmol) was reacted with ethyl 4-amino-2,2-dimethylbutanoate hydrochloride and sodium cyanoborohydride using a procedure similar to that of Example 8, Step B. The crude residue was chromatographed on silica gel using 1:3 hexanes/ethyl acetate as eluant to provide **45D** (150 mg) as a yellow oil.

### E. 4'-[(2-Butyl-4-oxo-1,3-diazaspiro[4,4]non-1-en-3-yl)methyl]-N-(3-methyl-5-isoxazolyl)-2'-[(3,3-dimethyl-2-oxo-1-pyrrolidinyl)methyl][1,1'-biphenyl]-2-sulfonamide

**45D** (110 mg) was deprotected according to General Method 7. The crude residue was chromatographed on silica gel using 95:5 chloroform/methanol as eluant to provide **45E** (50 mg) as a white solid.

### F. 4'-[(2-Butyl-4-oxo-1,3-diazaspiro[4.4]non-1-en-3-yl)methyl]-N-(4-bromo-3-methyl-5-isoxazolyl)-2'-[(3,3-dimethyl-2-oxo-1-pyrrolidinyl)methyl][1,1'-biphenyl]-2-sulfonamide

**45E** (33 mg) was brominated using NBS according to the procedure used for Example 26. Preparative TLC purification of the crude residue provided the title compound (4 mg) as a white powder: MS m/e 724, 726 (ESI+ mode); HPLC retention time 3.53 min (Method A); HPLC purity 98%.

### Reference Examples 46 to 97

The following compounds **46** to **97** were prepared by a solution phase combinatorial chemistry method using **2I** and the corresponding carboxylic acid in the presence of diisopropylcarbodiimide. The products were purified by ion-exchange chromatography according to the General Method. HLPC retention times were determined using HPLC Method C.

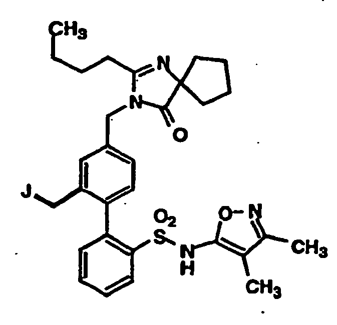

| Ex. No. | Compound Name | J | HPLC Retention Time (min) | LRMS m/z [MH⁺] |
|---|---|---|---|---|
| 46 | 4'-[(2-Butyl-4-oxo-1,3-diazaspiro[4.4] non-1-en-3-yl)methyl]-N-(3,4-dimethyl-5-isoxazolyl)-2'-[(formylmethylamino)-methyl] [1,1'-biphenyl]-2-sulfonamide | 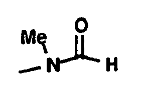 | 3.23 | 606 |
| 47 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N-methylpropanamide | 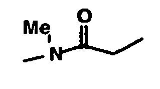 | 3.28 | 634 |
| 48 | N-[[4-[(2-Butyl-4-oxo-1,3-diazaspiro [4.4] non-1-en-3-yl)methyl]-2'-[[(3,4-dimethyl-5-isoxazolyl)amino]sulfonyl] [1,1'-biphenyl]-2-yl]methyl]-N-methylcyclopropanecarbox-amide | 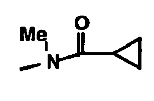 | 3.33 | 646 |
| 49 | N-[[4-[(2-Butyl-4-oxo-1,3-diazaspiro [4.4] non-1-en-3-yl)methyl]-2'-[[(3,4-dimethyl-5-isoxazolyl)amino]sulfonyl][1,1'-biphenyl]-2-yl]methyl]-N,2-dimethylpropanamide | 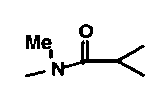 | 3.41 | 648 |
| 50 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N-methylbutanamide | 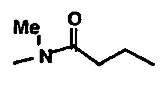 | 3.45 | 648 |
| 51 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl] methyl]-2-methoxy-N-methylacetamide | 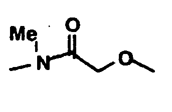 | 3.29 | 650 |
| 52 | N-[[4-[(2-Butyl-4-oxo-1,3-diazaspiro[4.4]non-1-en-3-yl)methyl]-2'-[ [[(3,4-dimethyl-5-isoxazolyl)amino]sulfonyl] [1,1'-biphenyl]-2-yl]methyl]-N-methyl-4-pentynamide | 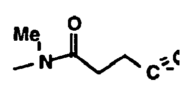 | 3.32 | 658 |
| 53 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N-methylcyclobutanecarbox-amide | 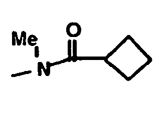 | 3.50 | 660 |
| 54 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N,3-dimethylbutanamide | 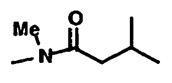 | 3.57 | 662 |
| 55 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N,2,2-trimethylpropanamide | 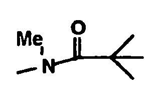 | 3.80 | 662 |
| 56 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-3-methoxy-N-methylpropanamide | 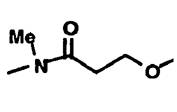 | 3.38 | 664 |
| 57 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-2-ethoxy-N-methylacetamide | 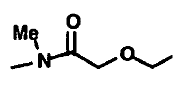 | 3.25 | 664 |
| 58 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N-methyl-2-furancarboxamide | 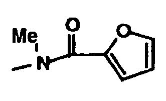 | 3.30 | 672 |
| 59 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N,4-dimethylpentanamide | 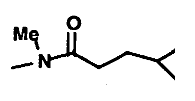 | 3.95 | 676 |
| 60 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N-methylbenzamide | 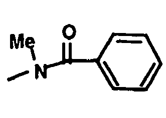 | 3.66 | 682 |
| 61 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl] methyl]-N-methyl-3-thiophenecarboxamide | 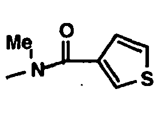 | 3.59 | 688 |
| 62 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl] methyl]-N-methylcyclopentaneacet-amide | 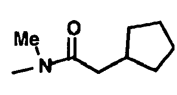 | 3.82 | 688 |
| 63 | N-[[4-[(2-Butyl-4-oxo-1,3-diazaspiro[4.4]non-1-en-3-yl)methyl]-2'-[((3,4-dimethyl-5-isoxazolyl)amino]sulfonyl] [1,1'-biphenyl]-2-yl]methyl]-N-methylcyclohexanecarbox-amide | 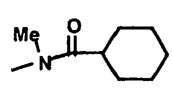 | 3.76 | 688 |
| 64 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N,3-dimethylbenzamide | 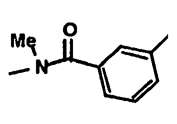 | 3.82 | 696 |
| 65 | N-[[4-[(2-Butyl-4-oxo-1,3-diazaspiro[4.4]non-1-en-3-yl)methyl]-2'-[[(3,4-dimethyl-5-isoxazolyl)amino] sulfonyl][1,1'-biphenyl]-2-yl]methyl]-N-methylbenzeneacetamide | 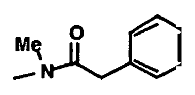 | 3.78 | 696 |
| 66 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-2-fluoro-N-methylbenzamide | 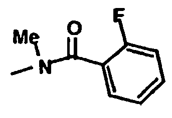 | 3.46 | 700 |
| 67 | N-[[4-[(2-Butyl-4-oxo-1,3-diazaspiro [4.4] non-1-en-3-yl)methyl]-2'-[[(3,4-dimethyl-5-isoxazolyl)amino]sulfonyl][1,1'-biphenyl]-2-yl]methyl]-3-fluoro-N-methylbenzamide | 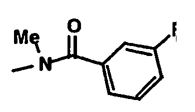 | 3.53 | 700 |
| 68 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-4-fluoro-N-methylbenzamide | 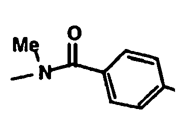 | 3.72 | 700 |
| 69 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N-methylcyclohexaneacet-amide | 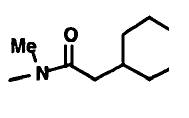 | 3.96 | 702 |
| 70 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-2-fluoro-N-methylbenzeneacetamide | 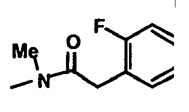 | 3.79 | 714 |
| 71 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl] methyl]-3-fluoro-N-methylbenzeneacetamide | 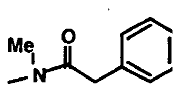 | 3.82 | 714 |
| 72 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl] methyl]-4-fluoro-N-methylbenzeneacetamide | 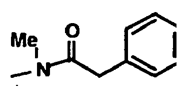 | 3.82 | 714 |
| 73 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N,N',N'-trimethylurea | 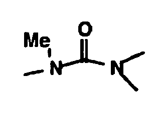 | 3.44 | 649 |
| 74 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N'-(1,1-dimethylethyl)-N-methylurea | 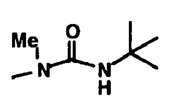 | 3.74 | 677 |
| 75 | [[4-[(2-Butyl-4-oxo-1,3-diazaspiro[4.4] non-1-en-3-yl)methyl]-2'-[[(3,4-dimethyl-5-isoxazolyl)amino]sulfonyl] [1,1'-biphenyl]-2-yl]methyl]methylcarbamic acid ethyl ester | 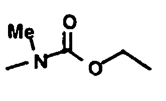 | 3.69 | 650 |
| 76 | [[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]methylcarbamic acid 2-methylpropyl ester | 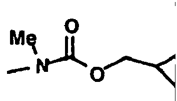 | 4.01 | 678 |
| 77 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N,3,3-trimethylbutanamide | 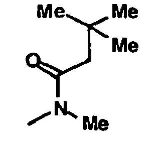 | 3.85 | 676 |
| 78 | N-[[4-[(2-Butyl-4-oxo-1,3-diazaspiro [4.4] non-1-en-3-yl)methyl]-2'-[[(3,4-dimethyl-5-isoxazolyl)amino]sulfonyl] [1,1'-biphenyl]-2-yl]methyl]-N-methyl-2-pyridinecarboxamide | 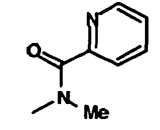 | 3.33 | 683 |
| 79 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N-methyl-3-pyridinecarboxamide | 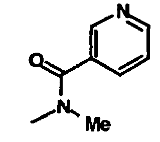 | 3.16 | 683 |
| 80 | N- [[4- [(2-Butyl-4-oxo-1,3-diazaspiro[4.4]non-1-en-3-yl)methyl]-2'- [[(3,4-dimethyl-5-isoxazolyl)amino]sulfonyl] [1,1'-biphenyl]-2-yl]methyl]-N-methyl-2-pyrazinecarboxamide | 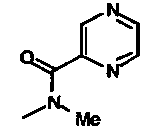 | 3.27 | 684 |
| 81 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N,1-dimethyl-1H-pyrrole-2-carboxamide | 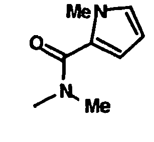 | 3.64 | 685 |
| 82 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N-methyl-1,2,3-thiadiazole-4-carboxamide | 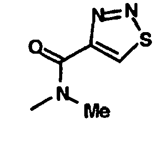 | 3.36 | 690 |
| 83 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N,5-dimethyl-2-pyrazinecarboxamide | 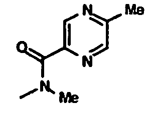 | 3.37 | 698 |
| 84 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N,3,5-trimethyl-4-isoxazolecarboxamide | 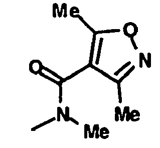 | 3.42 | 701 |
| 85 | N-[[4-[(2-Butyl-4-oxo-1,3-diazaspiro [4.4] non-1-en-3-yl)methyl]-2'-[[(3,4-dimethyl-5-isoxazolyl)amino]sulfonyl][1,1'-biphenyl]-2-yl]methyl]-N,3-dimethyl-2-thiophenecarboxamide | 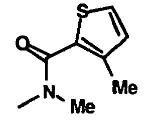 | 3.66 | 702 |
| 86 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-N,5-dimethyl-2-thiophenecarboxamide | 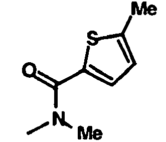 | 3.72 | 702 |
| 87 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-3-cyano-N-methylbenzamide | 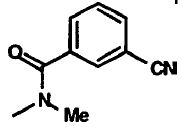 | 3.45 | 707 |
| 88 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-4-cyano-N-methylbenzamide | 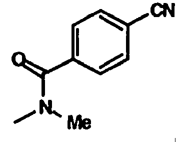 | 3.49 | 707 |
| 89 | N-[[4-[(2-Butyl-4-oxo-1,3-diazaspiro[4.4] non-1-en-3-yl)methyl]-2'-[[(3,4-dimethyl-5-isoxazolyl)amino)sulfonyl] [1,1'-biphenyl]-2-yl]methyl]-2-methoxy-N-methylbenzamide | 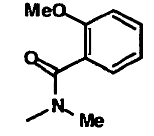 | 3.63 | 712 |
| 90 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl] methyl]-2-chloro-N-methylbenzamide | 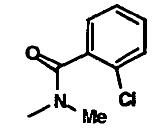 | 3.77 | 717 |
| 91 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-3-chloro-N-methylbenzamide | 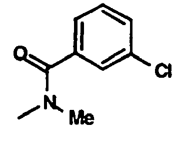 | 3.84 | 717 |
| 92 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-4-chloro-N-methylbenzamide | 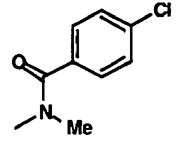 | 3.87 | 717 |
| 93 | N-[[4-[(2-Butyl-4-oxo-1,3-diazaspiro[4,4]non-1-en-3-yl)methyl]-2'-[[(3,4-dimethyl-5-isoxazolyl)amino]sulfonyl] [1,1'-biphenyl]-2-yl]methyl]-2,3-difluoro-N-methylbenzamide | 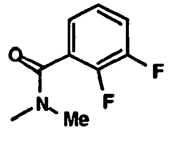 | 3.66 | 718 |
| 94 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-3,4-difluoro-N-methylbenzamide | 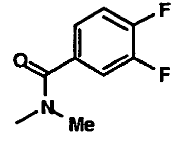 | 3.76 | 718 |
| 95 | N-[[4-[(2-Butyl-4-oxo-1,3-diazaspiro[4.4]non-1-en-3-yl)methyl]-2'-[[(3,4-dimethyl-5-isoxazolyl)amino]sulfonyl][1,1'-biphenyl]-2-yl]methyl]-3,5-difluoro-N-methylbenzamide | 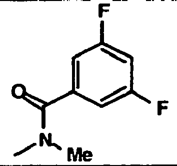 | 3.76 | 718 |
| 96 | 4-Acetyl-N-[[4-[(2-butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-N-methylbenzamide | 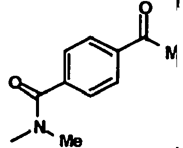 | 3.48 | 724 |
| 97 | N-[[4-[(2-Butyl-4-oxo-1,3-diazaspiro[4.4]non-1-en-3-yl)methyl]-2'-[[(3,4-dimethyl-5-isoxazolyl)amino]sulfonyl] [1,1'-biphenyl]-2-yl]methyl]-3-ethoxy-N-methyl-2-thiophenecarboxamide | 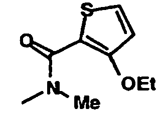 | 3.63 | 732 |

The following examples were synthesized by combinations of the General Methods.

| **EXAMPLE** | **STRUCTURE** | **NAME** | **STARTING MATERIAL** | **General Methods Applied (yield,%)** | **M/z (MH )** | **HPLC % Purity** | **HPLC ret time, min (method)** |
|---|---|---|---|---|---|---|---|
| 98 * | 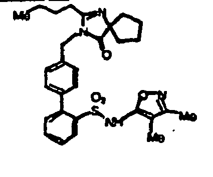 | 4'-[(2-Butyl-4-oxo-1,3-diazaspiro[4.4]no n-1-en-3-yl)methyl]-N-(3,4-dimethyl-5-isoxazolyl)[1,1'-biphenyl]-2-sulfonamide | **P18** | 4, 7 (32) | 535 | >98 | 15.00 |
| | | | | | | | (I) |
| 99 * | 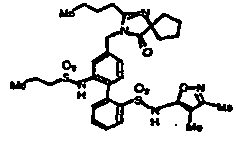 | 4'-[(2-Butyl-4-oxo-1,3-diazaspiro[4.4]no n-1-en-3-yl)methyl]-N-(3,4-dimethyl-5-isoxazolyl)-2'-[(propylsulfonyl)a mino][1,1'-biphenyl]-2-sulfonamide | **123** | 24(67) | 656 | 98 | 14.58 |
| | | | | | | | (E) |
| 100 * | 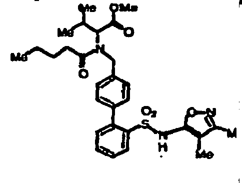 | N-[[2'-[[(3,4-Dimethyl-5-soxazolyl)amino] sulfonyl][1,1'-biphenyl]-4-yl]methyl]-N-(1-oxopentyl)-L-valine methyl ester | **P17** | 5,6 (16); | 556 | >98 | 16.27 |
| | | | | 9(60) | | | (F) |
| 101 * | 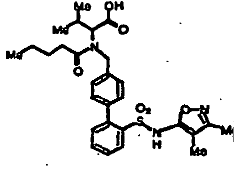 | N-[[2'-[[(3,4-Dimethyl-5-isoxazolyl)amino] sulfonyl][1,1'-biphenyl]-4-yl]methyl]-N-(1-oxopentyl)-L-valine | **100** | 15 (40) | 542 | >98 | 13.55 |
| | | | | | | | (F) |
| 102 * | 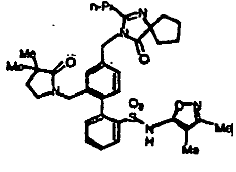 | N-(3,4-Dimethyl-5-isoxazolyl)-2'-[(3,3-dimethyl-2-oxo-1-pyrrolidinyl)meth yl]-4'-[(2-propyl-4-oxo-1,3-diazaspiro[4.4]no n-1-en-3-yl)methyl][1,1'-biphenyl]-2-sulfonamide | **5C** | 4(65); | 646 | 97 | 7.92 |
| | | | | 19,1 (70); | | | (E) |
| | | | | 5 (36); | | | |
| | | | | 9(50) | | | |
| 103 * | 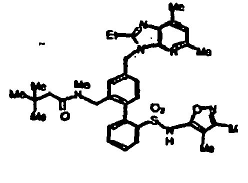 | N-[[2'-[[(3,4-Dimethyl-5-isoxazolyl)amino] sulfonyl]-4-[(2-ethyl-5,7-dimethyl-3H-imidazo[4,5-b]pyridin-3-yl)methyl][1,1'-biphenyl]-2-yl]methyl]-N-3,3-trimethylbutana mide | **2G** | 4 (32); | 657 | >98 | 25.45 |
| | | | | 7,6 (80) | | | (B) |
| 104 * | 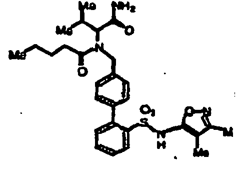 | N²-[[2'-[[(3,4-Dimethyl-5-isoxazolyl)amino] sulfonyl][1,1'-biphenyl]-4-yl]methyl]-N²-(1-oxopentyl)-L-valinamide | **101** | 12, NH, (70) | 541 | 98 | 11.39 |
| | | | | | | | (B) |
| 105 * | 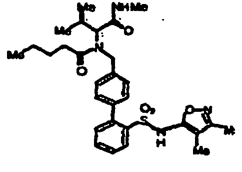 | N²-[[2'-[[(3,4-Dimethyl-5-isoxazolyl)amino] sulfonyl][1,1'-biphenyl]-4-yl)methy])-N-methyl-N²-(1-oxopentyl)-L-valinamide | **101** | 12, MeNH, (67) | 555 | 98 | 11.27 |
| | | | | | | | (F) |
| 106 * | 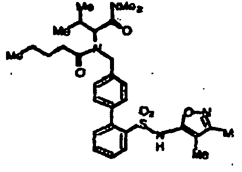 | N²-[[2'-[[(3,4-Dimethyl-5-isoxazolyl)amino] sulfonyl][1,1'-biphenyl]-4-yl]methyl]-N,N-dimethyl-N²-(1-oxopentyl)-L-valinamide | **101** | 12. Me₂NH (12) | 569 | 94 | 12.28 |
| | | | | | | | (F) |
| 107 * | 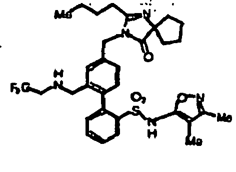 | 4'-[(2-Butyl-4-oxo-1,3-diazaspiro[4.4]no n-1-en-3-yl)methyl]-N-(3,4-dimethyl-5-isoxazolyl)-2'-[[(2,2,2-trifluoroethyl)ami no)methyl][1,1'-biphenyl]-2-sulfonamide | **P4** | 3 (90); | 646 | >99 | 3.46 |
| | | | | 4 (37); | | | (A) |
| | | | | 5 (56); | | | |
| | | | | 8, EtOH (31) | | | |
| 108 * | 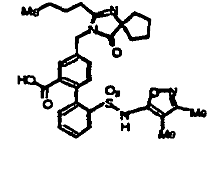 | 4-[(2-Butyl-4-oxo-1,3-diazaspiro[4.4]no n-1-en-3-yl)methyl]-2'-[[(3,4-dimethyl-5-isoxazolyl)amino] sulfonyl][1,1'-biphenyl]-2-carboxylic acid | **110** | 15(62) | 578 | 95 | 3.16 |
| | | | | | | | (A) |
| 109 * | 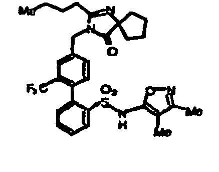 | 4'-[(2-Butyl-4-oxo-1,3-diazaspiro[4.4]no n-1-en-3-yl)methyl]-N-(3,4-dimethyl-5-isoxazolyl)-2'-(trifluoromethyl)[ 1,1'-biphenyl]-2-sulfonamide | **P9** | 4 (60); | 603 | 94 | 2.33 |
| | | | | 10(90) | | | (D) |
| 110 * | 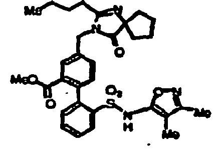 | 4-[(2-Butyl-4-oxo-1,3-diazaspiro[4.4]no n-1-en-3-yl)methyl]-2'-[[(3,4-dimethyl-5-isoxazolyl)amino] sulfonyl][1,1'-biphenyl)-2-carboxylic acid methyl ester | methyl 2-bro-mo-5-methyl-benzo-ate | 13,4 (61); | 593 | 96 | 3.42 |
| | | | | 1 (50); | | | (A) |
| | | | | 8, MeOH (34) | | | |
| 111 * | 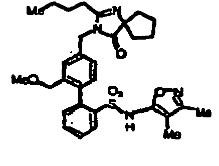 | 4'-[(2-Butyl-4-oxo-1,3-diazaspiro[4.4]no n-1-en-3-yl)methyl]-N-(3,4-dimethyl-5-isoxazolyl)-2'-(methoxymethyl)[ 1,1'-biphenyl]-2-sulfonamide | **P2** | 4 (53); | 579 | >98 | 16.26 |
| | | | | 7 (58) | | | (E) |
| 112 * | 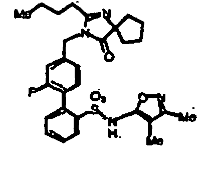 | 4'-[(2-Butyl-4-oxo-1,3-diazaspiro[4.4]no n-1-en-3-yl)methyl)-N-(3,4-dimethyl-5-isoxazolyl)-2'-fluoro[1,1'-biphenyl]-2-sulfonamide | **P11** | 4 (82); | 553 | >99 | 25.90 |
| | | | | 7(85) | | | (B) |
| 113 * | 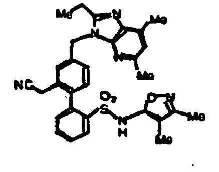 | 2'-(Cyanomethyl)-N-(3,4-dimethyl-5-isoxazolyl)-4'-[(2-ethyl-5,7-dimethyl-3H-imidazo[4,5-b]pyridin-3-yl)methyl][1,1'-biphenyl]-2-sulfonamide | **P12** | 16,3,4 (35); | 555 | 97 | 3.16 |
| | | | | 8, EtOH (81) | | | (A) |
| 114 * | 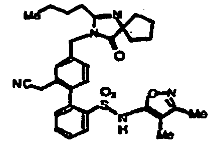 | 4'-[(2-Butyl-4-oxo-1,3-diazaspiro[4.4]no n-1-en-3-yl)methyl]-2'-(cyanomethyl)-N-(3,4-dimethyl-5-isoxazolyl)[1,1'-biphenyl]-2-sulfonamide | **P12** | 16,3,4 (35); | 574 | 96 | 3.36 |
| | | | | 8, EtOH (52) | | | (A) |
| 115 * | 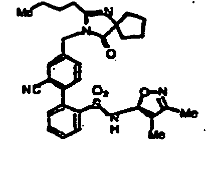 | 4'-[(2-Butyl-4-oxo-1,3-diazaspiro[4.4]no n-1-en-3-yl)methyl]-2'-cyano-N-(3,4-dimethyl-5-isoxazolyl)[1,1'-biphenyl]-2-sulfonamide | **P3** | 3(88); | 560 | >97 | 3.31 |
| | | | | 4 (45); | | | (C) |
| | | | | 10 (40) | | | |
| 116 * | 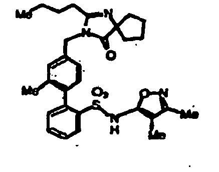 | 4'-[(2-Butyl-4-oxo-1,3-diazaspiro[4,4]no n-1-en-3-yl)methyl]-N-(3,4-dimethyl-5-isoxazolyl)-2'-methyl(1,1'-biphenyl]-2-sulfonamide | **P7** | 11 (83); | 549 | 98 | 27.21 |
| | | | | 2 (87); | | | (B) |
| | | | | 4(70); | | | |
| | | | | 1(35); | | | |
| | | | | 7(20) | | | |
| 117 * | 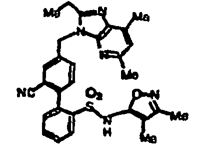 | 2'-Cyano-N-(3,4-dimethyl-5-isoxazolyl)-4'-[(2-ethyl-5,7-dimethyl-3H-imidazo[4,5-b]pyridin-3-yl)methyl][1,1'-biphenyl]-2-sulfonamide | **P3** | 3 (88); | 541 | 98 | 3.10 |
| | | | | 4 (45); | | | (A) |
| | | | | 10 (38) | | | |
| 118 * | 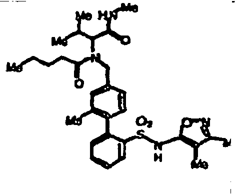 | N²-[[2'-[[(3,4-Dimethyl-5-soxazolyl)amino] sulfonyl]-2-methyl[1,1'-biphenyl]-4-yl]methyl]-N-methyl-N²-(1-oxopentyl)-L-valinamide | **P15** | 5 (39); | 569 | >99 | 2.15 |
| | | | | 6 (92); | | | (H) |
| | | | | 10 (30) | | | |
| 119 * | 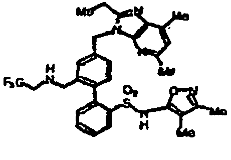 | N-(3,4-Dimethyl-5-isoxazolyl)-4'-[(2-ethyl-5,7-dimethyl-3H-imidazo[4,5-b]pyridin-3-yl)methyl]-2'-[[(2,2,2-trifluoroethyl]ami no]methyl][1,1'-biphenyl]-2-sulfonamide | **P4** | 3 (90); | 627 | 89 | 3.10 |
| | | | | 4 (49); | | | (A) |
| | | | | 5 (83); | | | |
| | | | | 10 (15) | | | |
| 120 * | 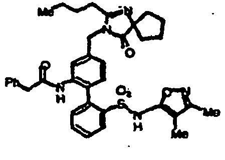 | N-[4-[(2-Butyl-4-oxo-1,3-diazaspiro[4.4]no n-1-en-3-yl)methyl)-2'-[[(3,4-dimethyl-5-isoxazolyl)amino] sulfonyl] [1,1'-biphenyl]-2-yl]benzene-acetamide | **122** | 6 (27) | 668 | 97 | 14.97 |
| | | | | | | | (I) |
| 121 * | 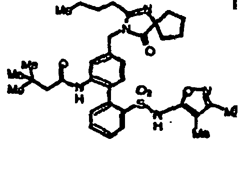 | N-[4-[(2-Butyl-4-oxo-1,3-diazaspiro[4.4]no n-1-en-3-yl)methyl]-2'-[[(3,4-dimethyl-5-isoxazolyl)amino] sulfonyl][1,1'-biphenyl]-2-yl]-3,3-dimethylbutanamide | **122** | 6 (36) | 648 | >98 | 16.38 |
| | | | | | | | (I) |
| 122 * | 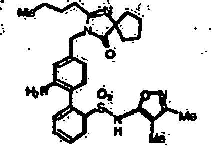 | 2'-Amino-4'-[(2-butyl-4-oxo-1,3-diazaspiro[4.4]no n-1-en-3-yl)methyl]-N-(3,4-dimethyl-5-isoxazolyl)[1,1'-biphenyl]-2-sulfonamide | **123** | 18 (75) | 550 | | 8.39 |
| | | | | | | | (I) |
| 123 * | 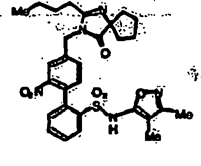 | 4'-[(2-Butyl-4-oxo-1,3-diazaspiro[4.4]no n-1-en-3-yl)methyl]-N-(3,4-dimethyl-5-isoxazolyl)-2'-nitro[1,1'-biphenyl]-2-sulfonamide | **P22** | 7 (93) | 580 | >98 | 11.17 |
| | | | | | | | (I) |
| 124 * | 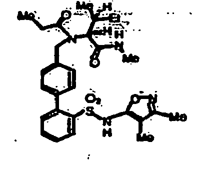 | N²-[[2'-[[(3,4-Dimethyl-5-isoxazolyl)amino] sulfonyl][1,1'-biphenyl]-4-yl]methyl]-N-methyl-N²-(1-oxopropyl)-L-isoleucinamide ; | **P16** | 5 (85); | 541 | 96 | 1.00 |
| | | | | 6, 10 (62) | | | (G) |
| 125 * | 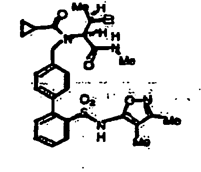 | N²-(Cyclopropylcarbo nyl)-N²-[[2'-[[3,4-dimethyl-5-isoxazolyl)amino] sulfonyl][1,1'-biphenyl]-4-yl]methyl]-N-methyl-L-isoleuomamide | **P16** | 6,10 (62) | 553 | 96 | 1.09 |
| | | | | | | | (G) |
| 126 * | 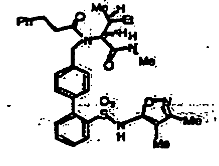 | N²-[[2'-[[(3,4-Dimethyl-5-isoxazolyl)amino] sulfonyl][1,1'-biphenyl]-4-yl]methyl]-N-methyl-N²-(1-oxo-3-phenylpropyl)-L-isoleucinamide | **P16** | 5 (85); | 617 | 95 | 1.63 |
| | | | | 6, 10 (64) | | | (G) |
| 127 * | 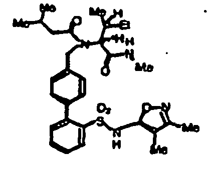 | N'-[[2'-[[(3,4-Dimethyl-5-isoxazolyl)amino] sulfonyl][1,1'-biphenyl]-4-yl]methyl]-N-methyl- N²-(3-methyl-1-oxobutyl)-L-isoleucinamide | **P16** | 5 (85); | 569 | 98 | 1.47 |
| | | | | 6,10 (58) | | | (G) |
| 128 * | 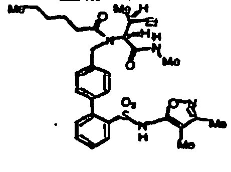 | N²-[[2'-[[(3,4-Dimethyl-5-isoxazolyl)amino] sulfonyl][1,1'-biphenyl]-4-yl]methyl]-N-methyl- N²-(1-oxohexyl)-L-isoleucinamide | **P16** | 5(85); | 583 | 98 | 1.74 |
| | | | | 6,10 (75) | | | (G) |
| 129 * | 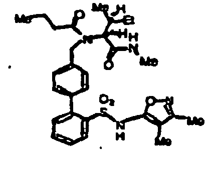 | N²-[[2'-[[(3,4-Dimethyl-5-isoxazolyl)amino] sulfonyl][1,1'-biphenyl]4-yl]methyl]-N-methyl- N²-(1-oxobutyl)-L-isoleucinamide | **P16** | 5 (85); | 555 | 95 | 1.31 |
| | | | | 6, 10 (62) | | | (G) |
| 130 * | 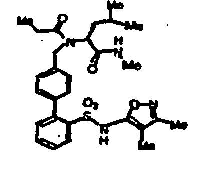 | N²-[[2'·[[(3,4-Dimethyl-5-isoxazolyl)amino] sulfonyl][1,1'-biphenyl]-4-yl]methyl]-N-methyl. N²-(1-oxopropyl)-L-leucinamide | **P16** | 5(83); | 541 | 90 | 1.01 |
| | | | | 6,10 (75) | | | (G) |
| 131 * | 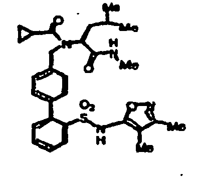 | N²-(Cyclopropylcarbo nyl)- N²-[[2'-[[(3,4-dimethyl-5-isoxazolyl)amino] sulfonyl] [1,1'-biphenyl]-4-yl]methyl]-N-methyl-L-, leucinamide | **P16** | 5(83); | 553 | 95 | 1.11 |
| | | | | 6,10 (79) | | | (G) |
| 132 * | 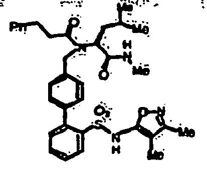 | N²-[[2'-[[(3,4-Dimethyl-5-isoxazolyl)amino] sulfonyl][1,1'-biphenyl]-4-yl]methyl]-N-methyl- N²-(1-oxo-3-phenylpropyl)-L-leucinamide | **P16** | 5 (83); | 617 | 95 | 1.08 |
| | | | | 6,10 (20) | | | (G) |
| 133 * | 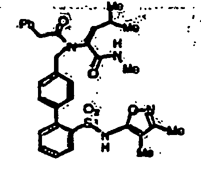 | N²-[[2'-[[(3,4-Dimethyl-5-isoxazolyl)amino] sulfonyl][1,1'-biphenyl]-4-yl]methyl]-N-methyl- N²-(phenylacetyl)-L-leucinamide | **P16** | 5 (83); | 603 | 95 | 1.68 |
| | | | | 6,10 (20) | | | (G) |
| 134 * | 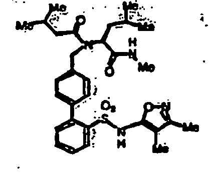 | N²-[[2'-[[(3,4 Dimethyl-5-isoxazolyl)amino] sulfonyl][1,1'-bipheny]-4-yl]methyl]-N-methyl-N²-(3-methyl-1-oxobutyl)-L-leucinamide | **P16** | 5 (83); | 569 | 90 | 1.51 |
| | | | | 6,10 (62) | | | (G) |
| 135 * | 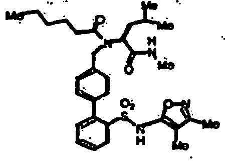 | N²-[[2'-[[(3,4-Dimethyl-5-isoxazolyl)amino]-sulfonyl][1,1'-biphenyl]-4-yl]methyl]-N-methyl-N²-(1'-oxohexyl)-L-leucinamide | **P16** | 5(83); | 583 | 97 | 1.76 |
| | | | | 6,10 (47) | | | (G) |
| 136 * | 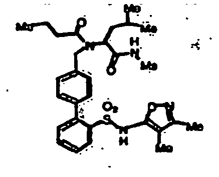 | N²-[[2'[[(3,4-Dimethyl-5-isoxazolyl)amino] sulfonyl][1,1'-biphenyl]-4-yl]methyl]-N-methyl-N²-(1 oxobutyl)-L leucinamide | **P16** | 5 (83); | 555 | 94 | 1.29 |
| | | | | 6, 10 (64) | | | (G) |
| 137 * | 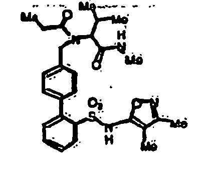 | N²-[[2'-[[(3,4-Dimethyl-5-isoxazolyl)amino] sulfonyl][1,1'-biphenyl]-4-yl]methyl]-N-methyl- N²-(1-oxopropyl)-L-valinamide | **P16** | 5 (89); | 527 | 90 | 0.73 |
| | | | | 6,10 (39) | | | (G) |
| 138 * | 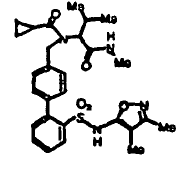 | N²- (Cyclopropylcarbo nyl)- N²-[[2'-[[(3,4-dimethyl-5-isoxazolyl)amino] sulfonyl][1,1'-biphenyl]-4-yl]methyl]-N-methyl-L-valinamide | **P16** | 5 (89); | 539 | 98 | 0.89 |
| | | | | 6,10 (66) | | | (G) |
| 139 * | 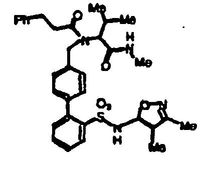 | N²-[[2'-[[(3,4- Dimethyl-5-isoxazolyl)amino] sulfonyl][1,1'-biphenyl]-4-yl]methyl]-N-methyl- N²-(1-oxo-3-phenylpropyl)-L-valinamide | **P16** | 5(89); | 603 | 95 | 1.63 |
| | | | | 6,10(6) | | | (G) |
| 140 * | 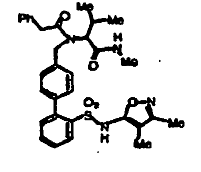 | N²-[[2'-[[(3,4- Dimethyl-5-isoxazolyl)amino] sulfonyl][1,1'-biphenyl]-4-yl]methyl]-N-methyl-N²-(phenylacetyl)-L-valinamide | **P16** | 5 (89); | 589 | 93 | 1.53 |
| | | | | 6,10 (54) | | | (G) |
| 141 * | 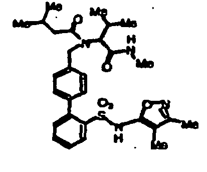 | N²-[[2'-[[(3,4- Dimethyl-5-isoxazolyl)amino] sulfonyl][1,1'-biphenyl]-4-yl]methyl]-N-methyl- N²-(3-methyl-1-oxobutyl)-L-valinamide | **P16** | 5(89); | 555 | 98 | 1.37 |
| | | | | 6, 10 (45) | | | (G) |
| 142 * | 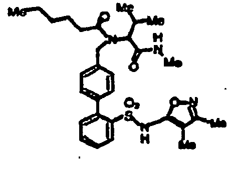 | N²-[[2'-[[(3,4- Dimethyl-5-isoxazolyl)amino] sulfonyl][1,1'-biphenyl]-4-yl]methyl]-N-methyl- N²-(1-oxohexyl)-L-valinamide | **P16** | 5(89); | 569 | 98 | 1.58 |
| | | | | 6,10 (61) | | | (G) |
| 143 * | 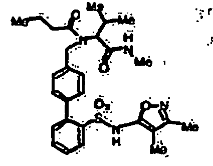 | N²-[[2'-[[(3,4-Dimethyl-5-isoxazolyl)amino] sulfonyl][1,1'-biphenyl]-4-yl]methyl]-N-methyl- N²-(1-oxobutyl)-L-valinamide | **P16** | 5 (89); | 541 | 98 | 1.65 |
| | | | | 6, 10 (43) | | | (G) |
| 144 * | 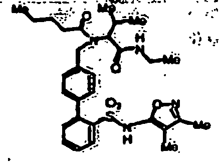 | N²-[[2'-[[(3,4-Dimethyl-5-isoxazolyl)amino] sulfonyl][1,1'-biphenyl]-4-yl]methyl]-N-ethyl-N²-(1-oxopentyl)-L-valinamide | **P17** | 5 (87), | 569 | >97 | 12.94 |
| | | | | 6 (93); | | | (F) |
| | | | | 9 (65); | | | |
| | | | | 15 (85); | | | |
| | | | | 12 (65) | | | |
| 145 * | 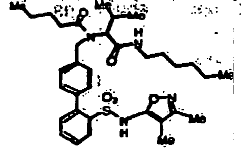 | N²-[[2'-[[(3,4-Dimethyl-5-isoxazolyl)amino] sulfonyl][1,1'-biphenyl]-4-yl]methyl]-N-hexyl-N²-(1-oxopentyl)-L-valinamide | **P17** | 5 (87); | 625 | >98 | 22.19 |
| | | | | 6 (93); | | | (F) |
| | | | | 9(65); | | | |
| | | | | 15(85); | | | |
| | | | | 12 (65) | | | |
| 146 * | 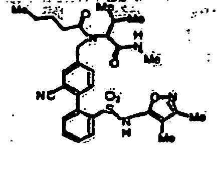 | N²-[[2'-[[(3,4-Dimethyl-5-isoxazolyl)amino] sulfonyl]-2-cyano[1,1'-biphenyl]-4-yl]methyl]-N-methyl-N²-(1-oxopentyl)-L-valinamide | **P3** | 17 (58); | 580 | >98 | 3.78 |
| | | | | 5,6 (49); | | | (C) |
| | | | | 10 (14) | | | |
| 147 * | 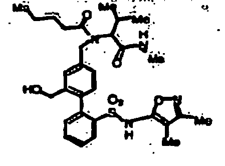 | N²-[[2'-[[(3,4-Dimethyl-5-isoxazolyl)amino] sulfonyl]-2-hydroxymethyl[1, 1'-biphenyl]-4-yl]methyl]-N-methyl-N²-(1-oxopentyl)-L-valinamide | **P1** | 5 (77); | 585 | 94 | 3.82, |
| | | | | 6(92); | | | 3.90 |
| | | | | 10(17) | | | (A) |
| | | | | | | | (two diastere-omers) |
| 148 * | 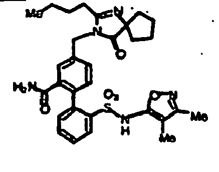 | 4-[(2-Butyl-4-oxo-1,3-diazaspiro[4,4]no n-1-en-3-yl)methyl]-2'-[[(3,4-dimethyl-5-isoxazolyl)amino] sulfonyl][1,1'-biphenyl]-2-carboxamide | **108** | 12 (33) | 578 | 99 | 3.16 |
| | | | | | | | (A) |
| 149 * | 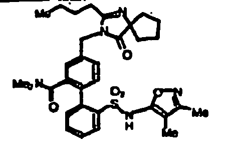 | N,N-Dimethyl-4-[(2-butyl-4-oxo-1,3-diazaspiro[4.4]no n-1-en-3-yl)methyl]-2'-[[(3,4-dimethy-5-isoxazolyl)amino] sulfonyl][1,1'-biphenyl]-2-carboxamide | **108** | 12 (61) | 606 | 99 | 3.22 |
| | | | | | | | (A) |
| 150 * | 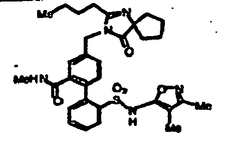 | N-Methyl-4-[(2-butyl-4-oxo-1,3-diazaspiro[4.4]no n-1-en-3-yl)methyl]-2'-[[(3,4-dimethyl-5-isoxazolyl)amino] sulfonyl][1,1'-biphenyl]-2-carboxamide | **108** | 12 (93) | 592 | 99 | 3.17 |
| | | | | | | | (A) |
| 151 * | 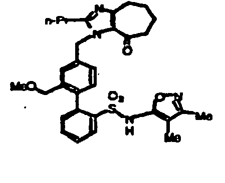 | N-(3,4-Dimethyl-5-isoxazolyl)-4'-[(1,4,5,6,7,8-hexahydro-8-oxo-2-propyl-1-cycloheptimidazol yl)methyl]-2'-(methoxymethyl)[ 1,1'-biphenyl]-2-sulfonamide | **P2** | 11 (87); | 577 | >98 | 11.58 |
| | | | | 3 (38); | | | (E) |
| | | | | 8(42) | | | |
| 152 * | 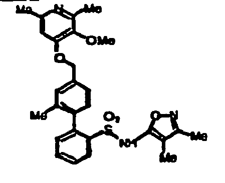 | N-(3,4-Dimethyl-5-isoxazolyl)-4'-[[(3-methoxy-2,6-dimethyl-4-pyridinyl)oxy]met hyl]-2'-methyl[1,1'-biphenyl]-2-sulfonamide | **P13** | 4 (20); | 508 | >98 | 22.83 |
| | | | | 7 (27) | | | (B) |
| 153 * | 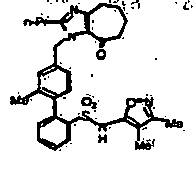 | N-(3,4-Dimethyl 5-isoxazolyl)-4'-[(1,4,5,6,7,8-hexahydro-8-oxo-2-propyl-1-cycloheptimidazol yl)methyl]-2'-methyl[1,1'-biphenyl]-2-sulfonamide | **P13** | 21(60); | 547 | >98 | 23.29 |
| | | | | 7 (20) | | | (B) |
| 154 * | 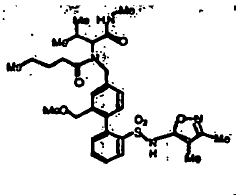 | N²-[[2'-[[(3,4-Dimethyl-5-isoxazolyl)amino] sulfonyl]-2-(methoxymethyl)[ 1,1'-biphenyl]-4-yl]methyl]-N-methyl-N²-(1-oxopentyl)-L-valinamide | **P20** | 5 (90); | 599 | >98 | 12.71, |
| | | | | 6 (90); | | | 12.95 |
| | | | | 7 (42) | | | (F) |
| | | | | | | | migrates as diastere-omers |
| 155 * | 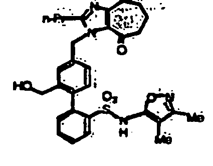 | N-(3,4-Dimethyl-5-isoxazolyl)-4'-[(1,4,5,6,7,8-hexahydro-8-oxo-2-propyl-1-cycloheptimidazol yl)methyl]-2'-(hydroxymethyl)[1,1' -biphenyl]-2-sulfonamide | **P14** | 21(29); | 563 | >98 | 2.99 |
| | | | | 8, EtOH (32) | | | (A) |
| 156 * | 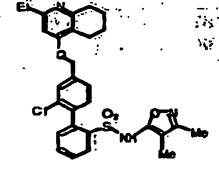 | 2'-Chloro-N-(3,4-dimethyl-5-isoxazolyl)-4'-[[(5,6,7,8-tetrahydro-2-ethyl-4-quinolinyl)oxy]me thyl][1,1'-biphenyl]-2-sulfonamide | **P10** | 4,8(33) | 553 | 97 | 1.69 |
| | | | | | | | (D) |
| 157 * | 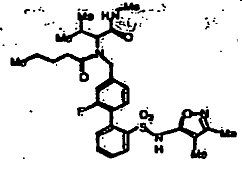 | N²-[[2'-[[(3,4-Dimethyl-5-isoxazolyl)amino] sulfonyl]-2-fluoro[1,1'-biphenyl]-4-yl]methyl]-N-methyl-N²-(1-axopentyl)-L-valinamide | **P8** | 6(34); | 573 | >98 | 30.02 |
| | | | | 7(45) | | | (B) |
| 158 * | 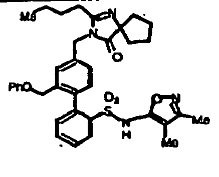 | 4'-[(2-Butyl-4-oxo-1,3-diazaspiro[4.4]no n-1-en-3-yl)methyl]-N-(3,4-dimethyl-5-isoxazolyl)-2'-(phenoxymethyl)[ 1,1'-biphenyl]-2-sulfonamide | **42A** | 3 (70); | 641 | 94 | 19.91 |
| | | | | 22 (68); | | | (I) |
| | | | | 7 (76) | | | |
| 159 * | 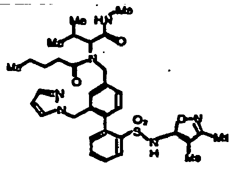 | N²-[[2'-[[(3,4-Dimethyl-5-isoxazolyl)amino] sulfonyl]-2-(1H-pyrazol-1-ylmethyl)[1,1'-biphenyl]-4-yl]methyl]-N-methyl-N²-(1-oxopentyl)-L-valinamide | **P1** | 5 (77); | 635 | 95 | 4.08 |
| | | | | 6 (92); | | | (A) |
| | | | | 3(99); | | | |
| | | | | 4 (69); | | | |
| | | | | 23 (18) | | | |
| 160 * | 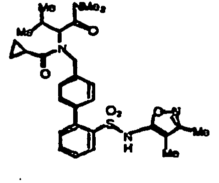 | N²-(Cyclopropylcarbo nyl)-N²-[[2'-[[(3,4-dimethyl-5-isoxazolyl)amino] sulfonyl][1,1'-biphenyl]-4-yl]methyl]-N,N-dimethyl-L-valinamide | **P16** | 5 (85); | 553 | 88 | 2.58 |
| | | | | 6,10 (28) | | | (D) |
| 161 * | 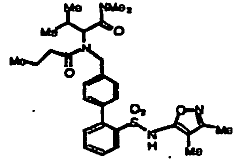 | N²-[[2'-[[(3,4-Dimethyl-5-isoxazolyl)amino] sulfonyl][1,1'-biphenyl]-4-yl]methyl]-N,N-dimethyl-N²-(1-oxobutyl)-L-valinamide | **P16** | 5 (85); | 555 | 93 | 2.82 |
| | | | | 6, 10 (33) | | | (D) |
| 162 * | 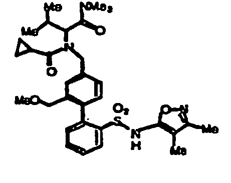 | N²-(Cyclopropylcarbo nyl)-N²-[[2'-[[(3,4-dimethyl-5-isoxazolyl)amino] sulfonyl]-2-(methoxymethyl)[ 1,1'-biphenyl]-4-yl]methyl]-N,N-dimethyl-L- valinamide | **P2** | 5(50); | 597 | >98 | 2.50, |
| | | | | 6, 8, EtOH (56) | | | 2.63 |
| | | | | | | | (D) |
| | | | | | | | (migrates as diastere-omers) |
| 163 * | 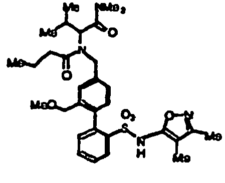 | N²-[[2'-[((3,4-Dimethyl-5-isoxazolyl)amino] sulfonyl]-2-(methoxymethyl)[ 1,1' -biphenyl]-4-yl]methyl]-N,N-dimethyl-N²-(1-oxobutyl)-L-valinamide | **P2** | 5 (50); | 599 | 96 | 2.92, |
| | | | | 6, 8, EtOH (59) | | | 3.02 |
| | | | | | | | (D) |
| | | | | | | | (migrates as diastere-omers) |
| 164 * | 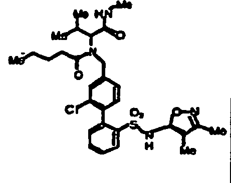 | N²-[[2-Chloro-2'-[[(3,4-dimethyl-5-isoxazolyl)amino] sulfonyl][1,1'-biphenyl]-4-yl]methyl]-N- methyl-N²-(1-oxopentyl)-L-valinamide | **P5** | 5 (69); | 590 | 96 | 3.03 |
| | | | | 6,10 (50) | | | (D) |
| 165 * | 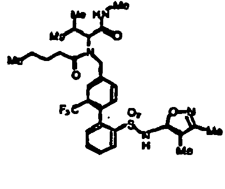 | N²-[[2'-[[(3,4-Dimethyl-5-isoxazolyl)amino] sulfonyl]-2-(trifluoromethyl)[ 1,1'-biphenyl]-4-yl]methyl]-N-methyl-N²-(1-oxopentyl)-L-valinamide | **P6** | 5 (80); | 623 | 95 | 2.93, |
| | | | | 6,10 (83) | | | 3.05 |
| | | | | | | | (D) |
| | | | | | | | (migrates as diastere-omers) |
| 166 * | 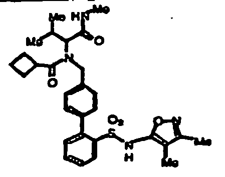 | N²-(Cyclobutylcarbon yl)-N²-[[2'-[[(3,4-dimethyl-5-isoxazolyl)amino] sulfonyl][1,1'-biphenyl)-4-yl]methyl]-N-methyl-L-valinamide | **P16** | 5 (85); | 553 | 98 | 2.82 |
| | | | | 6,10 (35) | | | (D) |
| 167 * | 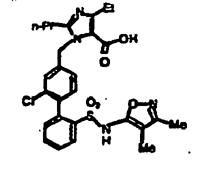 | 1-[[2-Chloro-2'-[[(3,4-dimethyl-5-isoxazolyl)amino] sulfonyl][1,1'-biphenyl]-4-yl]methy]-4-ethyl-2-propyl-1H-imidazole-5-carboxylic acid | **P10** | 22 (60); | 558 | >98 | 3.04 |
| | | | | 8, EtOH (90); | | | (A) |
| | | | | 15 (99) | | | |
| 168 * | 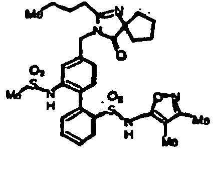 | 4'-[(2-Butyl-4-oxo-1,3-diazaspiro[4.4]no n-1-en-3-yl)methyl]-N-(3,4-dimethyl-5-isoxazolyl)-2'-(methylsulfonyl)amino] [1,1'-biphenyl]-2-sulfonamide | **122** | 24(25) | 628 | >98 | 11.48 (E) |
| | | | | | | | |
| 169 * | 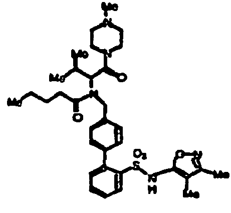 | (S)-N-[[2'-[[(3,4-Dimethyl-5-isoxazolyl)amino] sulfonyl][1,1'-biphenyl]-4-yl]methyl]-N-[2-methyl-1-[(4-methyl-1-piperazinyl)carbo nyl]propyl]pentan amide | **101** | 12 (43) | 624 | >98 | 28.26 (B) |
| | | | | | | | |
| 170 * | 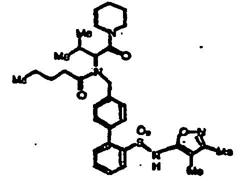 | (S)-N-[[2'-[[(3,4-Dimethyl-5-isoxazolyl)amino] sulfonyl][1,1'-biphenyl]-4-yl]methyl]-N-(1-piperidinyl)carbo nyl]propyl]pentan amide | **101** | 12 (30) | 609 | >98 | 34.45 (B) |
| | | | | | | | |
| 171 * | 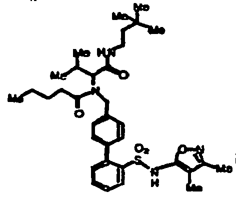 | N-(3,3-Dimethylbutyl)-N²-[[2'-[[(3,4-dimethyl-5-isoxazolyl)amino] sulfonyl][1,1'-biphenyl]-4-yl]methyl]-N²-(1-oxopentyl)-L-valinamide | **101** | 12 (38) | 625 | >98 | 33.97 (B) |
| | | | | | | | |
| 172 * | 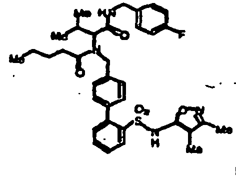 | N²-[[2'-[[(3,4-Dimethyl-5-isoxazolyl)amino] sulfonyl][1,1'-biphenyl]-4-yl]methyl]-N-[(4-fluorophenyl)methyl]-N²-(1-oxopentyl)-L-valinamide | **101** | 12(38) | 649 | >98 | 32.90 (B) |
| | | | | | | | |
| 173 * | 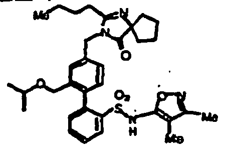 | 4'-[(2-Butyl-4-oxo-1,3-diazaspiro[4.4]no n-1-en-3-yl)methyl]-N-(3,4-dimethyl-5-isoxazolyl)-2'-[(methylethoxy)methyl][1,1'-biphenyl]-2-sulfonamide | **42A** | 2 (67); | 607 | 97 | 13.59 |
| | | | | 4 (51); | | | (E) |
| | | | | 7 (62) | | | |
| 174 * | 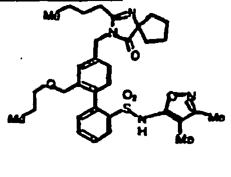 | 4'-[(2-Butyl-4-oxo-1,3-diazaspiro[4.4]no n-1-en-3-yl)methyl]-N-(3,4-dimethyl-5-isoxazolyl)-2'-(propoxymethyl)[ 1,1'-bipbenyl]-2-sulfonamide | **42A** | 2 (67); | 607 | >97 | 20.61 |
| | | | | 4 (49); | | | (E) |
| | | | | 7(68) | | | |
| 175 * | 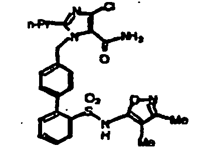 | 4-Chloro-1-[[2'- [[(3,4-dimethyl-5-isoxazolyl)amino] sulfonyl][1,1'-biphenyl]-4-yl]methyl]-2-propyl-1H-imidazole-5-carboxamide | **P19** | 22(87); | 529 | >98 | 3.69 |
| | | | | 25 (47); | | | (A) |
| | | | | 8,H₂O (99); | | | |
| | | | | 12 (35) | | | |
| 176 * | 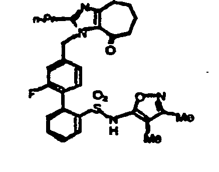 | N-(3,4-Dimethyl-5-isoxazolyl)-2'-fluoro-4'-[(1,4,5,6,7,8-hexahydro-8-oxo-2-propyl-1-cycloheptimidazol yl)methyl][1,1'-biphenyl]-2-sulfonamide | **P8** | 21 (15); | 551 | >98 | 5.78 |
| | | | | 7 (16) | | | (J) |
| 177 * | 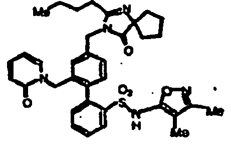 | 4'-[(2-Butyl-4-oxo-1,3-diazaspiro[4.4]no n-1-en-3-yl)methyl]-2'-[(1,2-dihydro-2-oxo-1-pyridinyl)methyl] -N-(3,4-dimethyl-5-isoxazolyl)[1,1'-biphenyl]-2-sulfonamide | **42A** | 2 (67); | 642 | >97 | 13.59 |
| | | | | 4(72); | | | (E) |
| | | | | 7(21) | | | |
| 178 * | 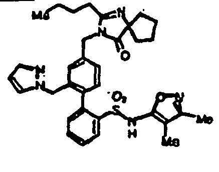 | 4'-[(2-Butyl-4-oxo-1,3-diazaspiro[4.4]no n-1-en-3-yl)methyl]-N-(3,4-dimethyl-5-isoxazolyl)-2'-(1H-pyrazol-1-ylmethyl)[1,1'-biphenyl]-2-sulfonamide | **P1** | 3 (98); | 615 | 97 | 3.42 |
| | | | | 4, 11 (35); | | | (A) |
| | | | | 3, 4, 7 (13) | | | |
| 179 * | 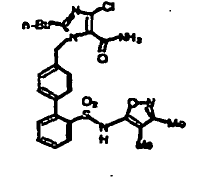 | 2-Butyl-4-chloro-1-[[2'-[[(3,4-dimethyl-5-isoxazolyl)amino] sulfanyl][1,1'-biphenyl]-4-yl]methyl]-1H-imidazole-5-carboxamide | **P19** | 22(43); | 543 | 94 | 3.82 |
| | | | | 25; 8, H₂O; | | | (A) |
| | | | | 12 (19) | | | |
| 180 * | 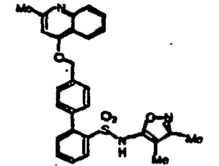 | N-(3,4-Dimethyl-5-isoxazolyl)-4'-[[(2-methyl-4-quinolinyl)oxy]methyl][1,1'-biphenyl]-2-sulfonamide | **P18** | 4(79); | 500 | 98 | 22.4 |
| | | | | 8, EtOH (26) | | | (B) |
| 181 * | 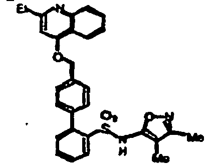 | N-(3,4-Dimethyl-5-isoxazolyl)-4'-[[(2-ethyl4-quinolinyl)oxy]methyl][1,1'-biphenyl]-2-sulfonamide | **P18** | 4 (49); | 514 | 93 | 23.25 |
| | | | | 8, EtOH (30) | | | (B) |
| 182 * | 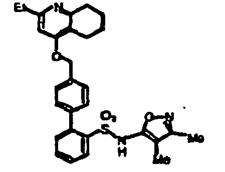 | N-(3,4-Dimethyl-5-isoxazolyl)-4'-[[(2-ethyl-5,6,7,8-tetrahydro-4-quinolinyl)oxy]methyl][1,1'-biphenyl]-2-sulfonamide | **P18** | 4(79); | 518 | 93 | 3.49 |
| | | | | 8, EtOH (3) | | | (A) |
| 183 * | 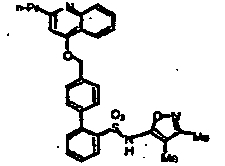 | N-(3,4-Dimethyl-5-isoxazolyl)-4'-[[(2-propyl-4-quinolinyl)oxy]methyl][1,1'-biphenyl]-2-sulfonamide | **P18** | 4(47); | 528 | 97 | 3.50 |
| | | | | 8, EtOH (20) | | | (A) |
| 184 * | 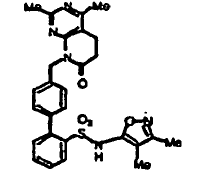 | N-(3,4-Dimethyl-5-isoxazolyl)-4'-[(5,6,7,8-tetrahydro-2,4-dimethyl-7-oxopyrido[2,3-d]pyrimidin-8-yl)methyl][1,1'-biphenyl]-2-sulfonamide | **P18** | 4 (79); | 518 | >98 | 3.05 |
| | | | | 8, EtOH (20) | | | (A) |
| 185 * | 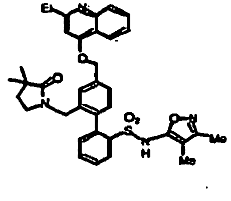 | 4'-[[(2-Ethyl-4-quinolinyl)oxy]me thyl]-N-(3,4-dimethyl-5-isoxazolyl)-2'-[(3,3-dimethyl-2-oxo-1-pyrrolidinyl)meth yl][1,1'-biphenyl]-2-sulfonamide | **P18** | 4 (20); | 639 | 93 | 3.43 |
| | | | | 8, EtOH (33) | | | (A) |
| 186 * | 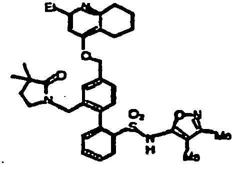 | N-(3,4-Dimethyl-5-isoxazolyl)-2'-[(3,3-dimethyl-2-oxo-1-pyrrolidinyl)meth yl]-4'-[[(5,6,7,8-tetrahydro-2-ethyl-4-quinolinyl)oxy]me thyl][1,1'-biphenyl]-2-sulfonamide | **P18** | 4 (63); | 643 | 92 | 3.79 |
| | | | | 8, EtOH (8) | | | (C) |
| 187 * | 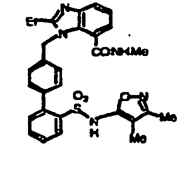 | 3-[[2'-[[(3,4-Dimethyl-5-isoxazolyl)amino] sulfonyl][1,1'-biphenyl]-4-yl]methyl]-2 ethyl-N-methyl-3H-benzimidazole-4-carboxamide | **31** | 12 (12) | 544 | 92 | 2.80 |
| | | | | | | | (A) |
| 188 * | 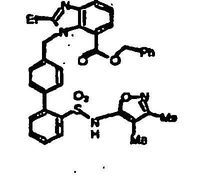 | 1-[[2'-[[(3,4-Dimethyl-5-isoxazolyl)amino] sulfonyl][1,1'-biphenyl]-4-yl]methyl]-2-ethyl-1H- benzimidazole-7-carboxylic acid phenylmethyl ester | **31** | 20 (55) | 621 | 96 | 3.95 |
| | | | | | | | (C) |
| 189 * | 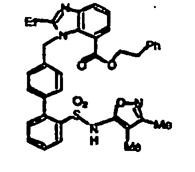 | 1-[[2'-[[(3,4-Dimethyl-5-isoxazolyl)amino] sulfonyl][1,1'-biphenyl]-4-yl]methyl]-2-ethyl-1H-benzimidazole-7-carboxylic acid 2-phenylethyl ester | **31** | 20 (12) | 635 | >98 | 4.03 |
| | | | | | | | (C) |
| 190 * | 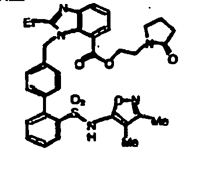 | 1-[[2'-[[(3,4-Dimethyl-5-isoxazolyl)amino]sulfonyl] [1,1'-biphenyl]-4-yl]methyl]-2-ethyl-1H-benzimidazole-7-carboxylic acid 2-(2-oxo-1-pyrrolidinyl)ethyl ester | **31** | 20 (45) | 642 | >98 | 3.15 |
| | | | | | | | (C) |
| 191 * | 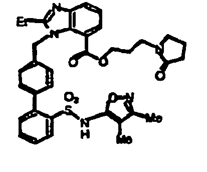 | 1-[[2'-[[(3,4-Dimethyl-5-isoxazolyl)amino] sulfonyl][1,1'-biphenyl]-4-yl]methyl]-2-ethyl-1H-benzimidazole-7-carboxylic acid 3-(2-oxo-1-pyrrolidinyl)propylester | **31** | 20 (30) | 656 | >98 | 3.21 |
| | | | | | | | (C) |
| 192 * | 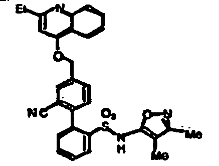 | 2'-Cyano-N-(3,4-dimethyl-5-isoxazolyl)-4'-[[(2-ethyl-4-quinolinyl)oxy]me thyl][1,1'-biphenyl]-2- sulfonamide | **P3** | 3,4 (59); | 539 | >98 | 3.01 |
| | | | | 10 (60) | | | (C) |
| 193 * | 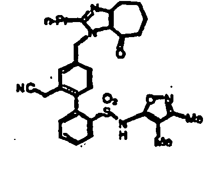 | 2'-(Cyanomethyl)-N-(3,4-dimethyl-5-isoxazolyl)-4'-[(1,4,5,6,7,8-hexahydro-8-oxo-2-propyl-1-cycloheptimidazol yl)methyl][1,1'-biphenyl]-2-sulfonamide | **P12** | 16,3 (73); | 572 | 96 | 2.95 |
| | | | | 21 (46); | | | (A) |
| | | | | 8, EtOH(16) | | | |
| 194 * | 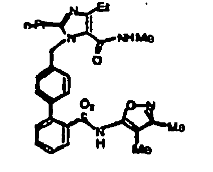 | 3-[[(2'-[[(3,4-Dimethyl-5-isoxazolyl)amino] sulfonyl][1,1'-biphenyl]-4-yl]methyl]-5-ethyl-N-methyl-2-propyl-3H-imidazole-4-carboxamide | **40B** | 12, MeNH₂ (12) | 536 | >98 | 2.88 |
| | | | | | | | (C) |
| 195 * | 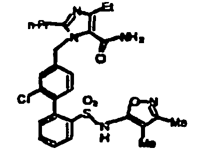 | 1-[{2-Chloro-2'-[[(3,4-dimethyl-5-isoxazolyl)amino] sulfonyl][1,1'-biphenyl]-4-yl]methyl]-4-ethyl-2-propyl-1H-imidazole-5-carboxamide | **167** | 12, NH, (17) | 557 | >98 | 3.08 |
| | | | | | | | (A) |
| 196 * | 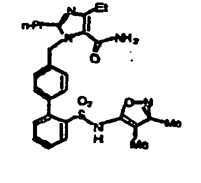 | 3-[[2'-[[(3,4-Dimethyl-5-isoxazolyl)amino] sulfonyl][1,1'-biphenyl]-4-yl]methyl]-5-ethyl-2-propyl-3H-imidazole-4-carboxamide | **40B** | 12, NH₂ (16) | 522 | >98 | 2.79 |
| | | | | | | | (C) |
| 197 * | 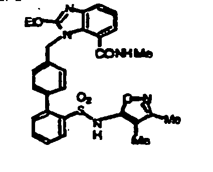 | 3-[[2'-[[(3,4-Dimethyl-5-isoxazolyl)amino] sulfonyl][1,1'-biphenyl)-4-yl]methyl]-2- ethoxy-N-methyl-3H-benzimidazole-4-carboxamide | **29** | 12, MeNH₂ (32) | 560 | >98 | 3.31 |
| | | | | | | | (A) |
| 198 * | 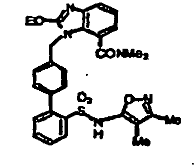 | 3-[[2'-[[(3,4-Dimethyl-5-isoxazolyl)amino] sulfonyl][1,1'-biphenyl]-4-yl]methyl]-2-ethoxy-N,N-dimethyl-3H-benzimidazole-4-carboxamide | **29** | 12, Me₂NH (5) | 574 | >98 | 3.42 |
| | | | | | | | (A) |
| 199 * | 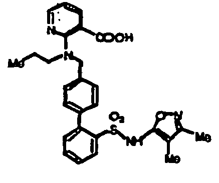 | 2-[[[2'-[[(3,4- Dimethyl-5-isoxazolyl)amino] sulfonyl][1,1'-biphenyl]-4-yl]methyl]propyla mino]-3-pyridinecarboxyli c acid | **P18** | 4, 15, 7 (34) | 521 | 99 | 25.4 |
| | | | | | | | (B) |
| 200 * | 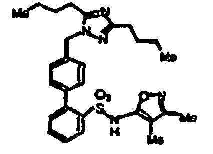 | 4'-[(3,5-Dibutyl- 1H-1,2,4-triazol-1-yl)methyl]-N-(3,4-dimethyl-5-isoxazolyl)[1,1'-biphenyl]-2-sulfonamide | **P18** | 4, 7 (77) | 522 | 99 | 28.4 |
| | | | | | | | (B) |
| 201 * | 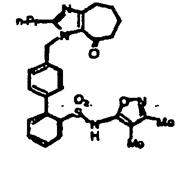 | N-(3,4-Dimethyl-5-isoxazolyl)-4'-[(1,4,5,6,7,8-hexahydro-8-oxo-2-propyl-1-cycloheptimidazol yl)methyl][1,1'-biphenyl]-2-sulfonamide | **P18** | 21,7 (55) | 533 | 99 | 21.5 |
| | | | | | | | (B) |
| 202 * | 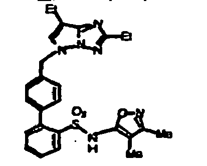 | 4'-[(2,7-Diethyl-5H-pyrazolo[1,5-b][1,2,4]triazol-5-yl)methyl]-N-(3,4-dimethyl-5-isoxazolyl)[1,1'-biphenyl]-2-sulfonamide | **P18** | 4,7 (33) | 505 | 99 | 21.8 |
| | | | | | | | (B) |
| 203 * | 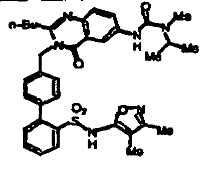 | N-[2-Butyl-3-[[2'-[[(3,4-dimethyl-5-isoxazolyl)amino] sulfonyl][1,1'-biphenyl]-4-yl]methyl]-3,4-dihydro-4-oxo-6-quinazolinyl]-N'-methyl-N'-(1-methylethyl)urea | **P18** | 4,7 (71) | 657 | 99 | 28.7 |
| | | | | | | | (B) |
| 204 * | 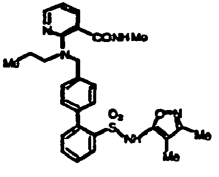 | 2-[[[2'-[[(3,4- Dimethyl-5-isoxazolyl)amino] sulfonyl][1,1'-biphenyl]-4-yl]methyl]propyla mino]-N-methyl-3-pyridinecarboxamide | **199** | 12 (98) | 534 | 99 | 23.8 |
| | | | | | | | (B) |
| 205 * | 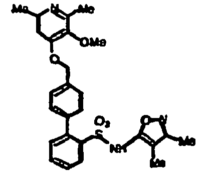 | 4'-[[(3-Methoxy-2,6-dimethyl-4-pyridinyl)oxy]met hyl]-N-(3,4-dimethyl-5-isoxazolyl)[1,1'-biphenyl]-2, sulfonamide | **P18** | 4 (59); | 494 | 98 | 6.99 |
| | | | | 8(74) | | | (I) |
| 206 * | 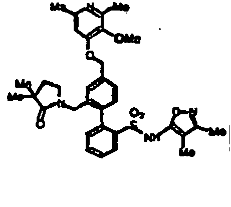 | N-(3,4-Dimethyl-5-isoxazolyl)-2'-[(3,3-dimethyl-2-oxo-1-pyrrolidinyl)meth yl]-4'-[[(3-methoxy-2,6-dimethyl-4-pyridinyl)oxy]methyl][1,1'-biphenyl]-2-sulfonamide | **20A** | 2 (82); | 619 | 96 | 14.02 |
| | | | | 4(82); | | | (E) |
| | | | | 8 (31) | | | |
| 207 * | 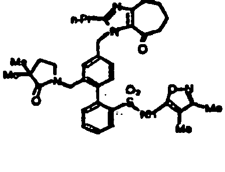 | N-(3,4-Dimethyl-5-isoxazolyl)-2'-[(3,3-dimethyl-2-oxo-1-pyrrolidinyl)meth yl]-4'-[(1,4,5,6,7,8-hexahydro-8-oxo-2-propyl-1-cycloheptimidazol yl)methyl][1,1'-biphenyl]-2-sulfonamide | **20A** | 2 (82); | 658 | 97 | 15.56 |
| | | | | 21,9 (54) | | | (E) |
| 208 * | 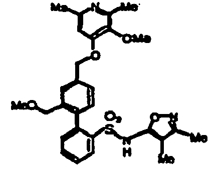 | N-(3,4-Dimethyl-5-isoxazolyl)-4'-[[(3-methoxy-2,6-dimethyl-4-pyridinyl)oxy]methyl]-2'-(methoxymethyl)[1,1'-biphenyl]-2-sulfonamide | **P2** | 4(45); | 538 | 99 | 10.60 |
| | | | | 8 (63) | | | (E) |
| 209 * | 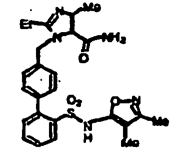 | 3-[[2'-[[(3,4-Dimethyl-5-isoxazolyl)amino] sulfonyl][1,1'-biphenyl]-4-yl]methyl]-2-ethyl-5-methyl-3H-imidazole-4-carboxamide | **P19** | 22 (35); | 494 | >97 | 7.96 |
| | | | | 7(67) | | | (E) |
| 210 * | 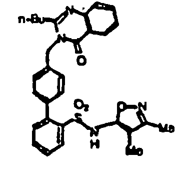 | 4'-[(2-Butyl-3,4-dihydro-4-oxo-3-quinazolinyl)methyl]-N-(3,4-dimethyl-5-isoxazolyl)[1,1'-biphenyl]-2-sulfonamide | **P18** | 4, 7(13) | 543 | 98 | 25.52 |
| | | | | | | | (I) |
| 211 * | 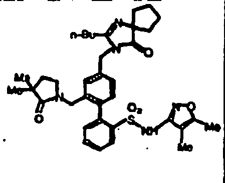 | 4'-[(2-Butyl-4-oxo-1,3-diazaspiro[4.4]no n-1-en-3-yl)methyl]-N-(4,5-dimethyl-3-isoxazolyl)-2'-[(3,3-dimethyl-2-oxo-1-pyrrolidinyl)methyl][1,1'-biphenyl]- 2-sulfonamide | **17** | 5 (36) | 660 | 98 | 14.51 |
| | | | | | | | (I) |
| 212 * | 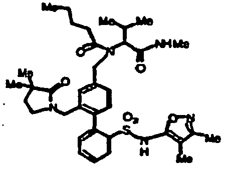 | N²-[[2'-[[(3,4-Dimethyl-5-isoxazolyl)amino] sulfonyl]-2-[(3,3-dimethyl-2-oxo-1-pyrrolidinyl)meth yl][1,1'-biphenyl]-4-yl]methyl]-N- methyl-N²-(1-oxopentyl)-L-valinamide | **20A** | 17 (80); | 680 | 97 | 13.13, |
| | | | | 5 (95); | | | 14.66 |
| | | | | 6 (95); | | | (F) |
| | | | | 7 (42) | | | migrates as diastere-omers |
| 213 * | 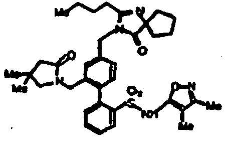 | 4'-[(2-Butyl-4-oxo-1,3-diazaspiro[4.4]no n-1-en-3-yl)methyl)-N-(3,4-dimethyl-5-isoxazolyl)-2'-[(4,4-dimethyl-2-oxo-1-pyrrolidinyl)methyl][1,1'-biphenyl]-2-sulfonamide | **3** | 5 (58) | 660 | >98 | 24.60 |
| | | | | | | | (B) |
| 214 * | 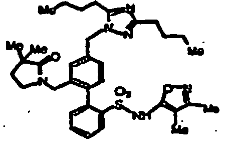 | 4'-[(3,5-Dibutyl-1H-1,2,4-triazol-1-yl)methyl]-N-(3,4-dimethyl-5-isoxazolyl)-2'-[(3,3-dimethyl-2-oxo-1-pyrrolidinyl)methyl][1,1'-biphenyl]-2-sulfonamide | **20A** | 4(52); | 647 | 97 | 23.53 |
| | | | | 9(57) | | | (I) |
| 215 * | 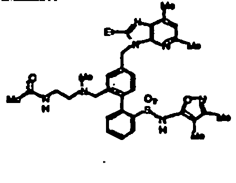 | N-[2-[[[2'-[[(3,4-Dimethyl-5-isoxazolyl)amino] sulfonyl]-4-[(2-ethyl-5,7-dimethyl-3H-imidazo[4,5-b]pyridin-3-yl)methyl][1,1'- bipbenyl]-2-yl)methyl]methylamino]ethyl]acetamide | **23B** | 5,12 (4); | 644 | 89 | 2.69 |
| | | | | 8 (15) | | | (A) |
| 216 * | 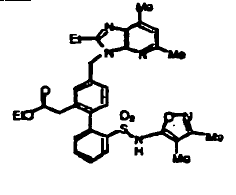 | 4-[(2-Ethyl-5,7-dimethyl-3H-imidazo[4,5-b]pyridin-3-yl)methyl]-2'-[[(3,4-dimethyl-5-isoxazolyl)amino] sulfonyl][1,1'-biphenyl]-2-acetic acid ethyl ester | **113** | 8, EtOH (13) | 602 | 90 | 3.41 |
| | | | | | | | (A) |
| 217 * | 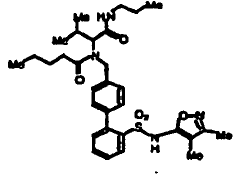 | N²-[[2'-[[(3,4-Dimethyl-5-isoxazolyl)amino] sulfonyl][1,1'-biphenyl]-4-yl]methyl]-N²-(1-oxopentyl)-N-propyl-L-valinamide | **101** | 12 (65) | 583 | >98 | 14.97 |
| | | | | | | | (F) |
| 218 * | 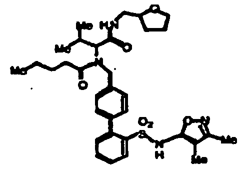 | N²-[[2'-[[(3,4-Dimethyl-5-isoxazolyl)amino] sulfonyl][1,1'-biphenyl]-4-yl]methyl]-N²-(1-oxopentyl)-N-[(tetrahydro-2-furanyl)methyl]-L-valinamide | **101** | 12(43) | 625 | >98 | 14.06 |
| | | | | | | | (F) |
| 219 * | 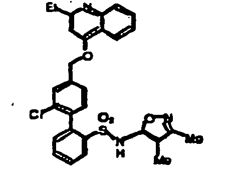 | 2'-Chloro-N-(3,4-dimethyl-5-isoxazolyl)-4'-[[(2-ethyl-4-quinolinyl)oxy]methyl][1,1'-biphenyl]-2-sulfonamide | **P10** | 4(90); | 549 | 98 | 1.68 |
| | | | | 8(65) | | | (D) |
| 220 * | 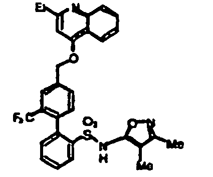 | N-(3,4-Dimethyl-5-isoxazolyl)-4'-[[(2-ethyl-4-quinolinyl)oxy]methyl]-2'-(trifluoromethyl)[ 1,1'-biphenyl]-2-sulfonamide | **P9** | 4, 8 (30) | 582 | 97 | 1.75 |
| | | | | | | | (D) |
| 221 * | 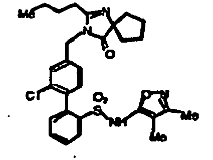 | 4'-[(2-Butyl-4-oxo-1,3-diazaspiro[4.4]no n-1-en-3-yl)methyl]-2'-chloro-N-(3,4-dimethyl-5-isoxazolyl)[1,1'-biphenyl]-2-sulfonamide | **P10** | 4, 8 (42) | 570 | 98 | 2.13 |
| | | | | | | | (D) |
| 222 * | 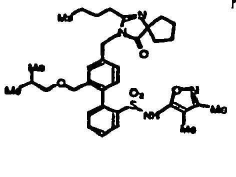 | 4'-[(2-Butyl-4-oxo-1,3-diazaspiro[4.4]no n-1-en-3-yl)methyl]-N-(3,4-dimethyl-5-isoxazolyl)-2'-[(2-methylpropoxy)methyl][1,1'-biphenyl]-2-sulfonamide | **42A** | 2 (67); | 621 | >98 | 23.07 |
| | | | | 4 (49); | | | (B) |
| | | | | 7 (85) | | | |
| 223 * | 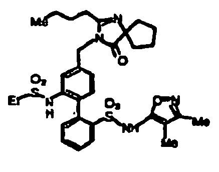 | 4'-[(2-Butyl-4-oxo-1,3-diazaspiro[4.4]no n-1-en-3-yl)methyl]-N-(3,4-dimethyl-5-isoxazolyl)-2'-[(ethylsulfonyl)amino][1,1'-biphenyl]-2-sulfonamide | **123** | 24 (33) | 642 | 98 | 14.32 |
| | | | | | | | (E) |
| 224 * | 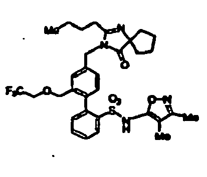 | 4'-[(2-Butyl-4-oxo-1,3-diazaspiro[4,4]non-1-en-3-yl)methyl]-N-(3,4-dimethyl-5-isoxazolyl)-2'-[(2,2,2-trifluoroethoxy)methyl][1,1'-biphenyl]-2-sulfonamide | **P2A** | 4, F₃CCH₂ OH (89); | 647 | >97 | 20.15 |
| | | | | 14 (76); | | | (E) |
| | | | | 1(67); | | | |
| | | | | 11 (95); | | | |
| | | | | 2(90); | | | |
| | | | | 4(85); | | | |
| | | | | 7(85) | | | |
| 225 * | 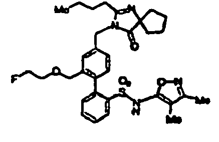 | 4'-[(2-Butyl-4-oxo-1,3-diazaspiro[4.4]no n-1-en-3-yl)methyl]-N-(3,4-dimethyl-5-isoxazolyl)-2'-[(2-fluoroethoxy)methyl][1,1'-biphenyl]-2-sulfonamide | **P2A** | 4, FCH₂C H₂OH (72); | 611 | >97 | 15.94 |
| | | | | 14 (65); | | | (E) |
| | | | | 1 (68); | | | |
| | | | | 11 (95); | | | |
| | | | | 2 (85); | | | |
| | | | | 4 (80); | | | |
| | | | | 7(90) | | | |
| 226 * | 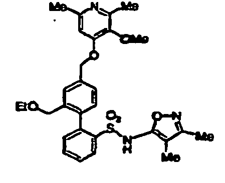 | N-(3,4-Dimethyl-5-isoxazolyl)-4'-[[(3-methoxy-2,6-dimethyl-4-pyridinyl)oxy]methyl]-2'-ethoxymethyl[1,1' -biphenyl]-2-sulfonamide | **P2A** | 4, EtOH (77); | 552 | 97 | 12.60 |
| | | | | 14 (80); | | | (E) |
| | | | | 1 (75); | | | |
| | | | | 11 (95); | | | |
| | | | | 2(77); | | | |
| | | | | 22 (43); | | | |
| | | | | 7(74) | | | |
| 227 * | 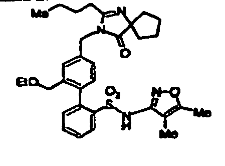 | 4'-[(2-Butyl-4-oxo-1,3-diazaspiro[4.4]non-1-en-3-yl)methyl]-N-(4,5-dimethyl-3-isoxazolyl)-2'-(ethoxymethyl) [1,1'-biphenyl]-2-sulfonamide | **P2A** | 4, EtOH (77); | 593 | >98 | 18.75 |
| | | | | 14 (80); | | | (E) |
| | | | | 1 (70); | | | |
| | | | | 11 (98); | | | |
| | | | | 2(80); | | | |
| | | | | 4(83); | | | |
| | | | | 7(86) | | | |
| 228 * | 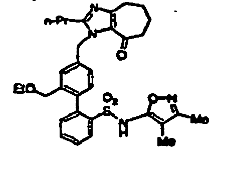 | N-(3,4-Dimethyl-5-isoxazolyl)-4'-[(1,4,5,6,7,8-hexahydro-8-oxo- 2-propyl-1-cycloheptimidazolyl)methyl]-2'- (ethoxymethyl)[1, 1'-biphenyl]-2-sulfonamide | **P2A** | 4, EtOH (77); | 591 | >98 | 20.07 |
| | | | | 14 (80); | | | (B) |
| | | | | 1 (75); | | | |
| | | | | 11 (95); | | | |
| | | | | 2 (77); | | | |
| | | | | 21 (); | | | |
| | | | | 7() | | | |

| | | | | | | | |
|---|---|---|---|---|---|---|---|
| * Reference Compound | | | | | | | |

### Reference Example 229

### 4'-[(2-Butyl-4-oxo-1,3-diazaspiro[4,4]non-1-en-3-yl)methyl]-N-(3,4-dimethylisoxazol-5-yl)-2'-(3,3,3-trifluoropropyl) [1,1'-biphenyl]-2-sulfonamide

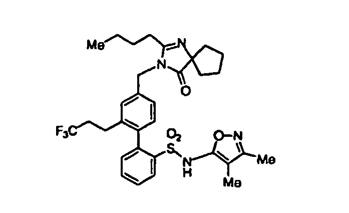

### A. 3-Bromo-4-methoxybenzaldehyde dimethyl acetal

A solution of 3-bromo-4-methoxybenzaldehyde (19.2 g, 89 mmol) and trimethylorthoformate (14.8 ml, 140 mmol) in 150 ml methanol was treated at RT with concentrated sulfuric acid (10 µl). After stirring for 16 h, the mixture was treated with potassium carbonate (70 mg) and the solvent was evaporated. The residue was partitioned between dichloromethane and aqueous sodium bicarbonate. The organic layer was dried over sodium sulfate and concentrated to provide **229A** (22.6 g) as a brown oil, which was used without further purification.

### B. 4-Methoxy-3-(3,3,3-trifluoropropyl)benzaldehyde dimethyl acetal

A solution of **229A** (15.8 g, 60 mmol) in dry THF (120 ml) was added to magnesium turnings (4.1 g, 170 mmol) at RT. The mixture was heated to 60 °C for 5 min, which initiated an exothermic reaction. The heating bath was removed and the mixture was allowed to reflux gently. Upon subsidence of the reaction (approximately 5 min), the mixture was heated to reflux for an additional 20 min and then was allowed to cool to RT. The clear green supernatant was transferred via canula away from excess magnesium and into a separate flask containing copper (I) iodide (575 mg, 3.0 mmol) under a nitrogen atmosphere. To this mixture was added 1-iodo-3,3,3-trifluoropropane (15 g, 67 mmol) and the resulting mixture was allowed to stir for 16 h, during which time a thick white precipitate developed. Aqueous ammonium chloride solution, ethyl acetate, and hexanes were added, and the organic layer was dried over sodium sulfate. Evaporation followed by silica gel chromatography of the residue using 3:1 hexanes/ethyl acetate as eluant provided 7.7 g of a mobile, slightly yellow oil, which was a 6:1 (mol ratio) mixture of **229B** and 4-methoxybenzaldehyde dimethyl acetal, as determined by proton NMR. This mixture was used without further purification.

### C. 4-Methoxy-3-(3,3,3-trifluoropropyl)benzaldehyde

The **229B** mixture (7.7 g) was subjected to hydrochloric acid hydrolysis according to General Method 19. The resulting crude yellow oil (8.0 g) was a 6:1 mixture (mol ratio) of 229C and 4-methoxybenzaldehyde, and was used without further purification.

### D. 4-Hydroxy-3-(3,3,3-trifluoropropyl)benzaldehyde

A solution of the **229C** mixture (8.0 g, 35 mmol) in dichloromethane (5 ml) was treated at 0°C with boron tribromide (55 ml of a 1.0 M solution in dichloromethane). The mixture was allowed to warm to RT and was stirred for 2 h. Aqueous 10% dipotassium hydrogen phosphate solution was added, and the aqueous layer was extracted with two portions of dichloromethane. The combined organic extracts were dried over sodium sulfate and evaporated. The residue was chromatographed on silica gel using 3:1 hexanes/ethyl acetate as eluant to provide 2.0 g of **229D** as an orange solid (contaminated with 4-hydroxybenzaldehyde).

### E. 4-(trifluoromethanesulfonyloxy)-3-(3,3,3-trifluoropropyl)benzaldehyde

A solution of the **229D** mixture (2.0 g, 9 mmol) and N,N-diisopropylethylamine (2.4 ml, 14 mmol) in dichloromethane (40 ml) was treated dropwise at -78°C with trifluoromethanesulfonic anhydride (1.9 ml, 11 mmol). The mixture was stirred at that temperature for 15 min following the completion of the addition. Aqueous sodium bicarbonate solution was added and the mixture was stirred and allowed to warm to RT. The layers were separated and the aqueous layer was extracted with two portions of dichloromethane. The combined organic extracts were dried over sodium sulfate, evaporated, and the residue was chromatographed on silica gel using hexanes/ethyl acetate as eluant to provide first recovered **229D** (540 mg), and then the product fraction (380 mg) as an orange oil. The product fraction was a 2:1 mixture (mol ratio) of **229E** and 4-(trifluormethanesulfonyloxy)benzaldehyde, as determined by proton NMR.

### F. N-(3,4-Dimethyl-5-isoxazolyl)-4'-formyl-2'-(3,3,3-trifluoropropyl)-N-[(2-trimethylsilyl)ethoxymethyl] [1,1'-biphenyl]-2-sulfonamide

A solution of **229E** (380 mg, 1.1 mmol) and [2-[[(3,4-dimethyl-5-isoxazolyl)[(2-(trimethylsilyl)ethoxy)methyl]amino]-sulfonyl]phenyl]boronic acid (925 mg, 2.2 mmol) in dioxane (10 ml) was sparged with nitrogen for 15 min. Tetrakis(triphenylphosphine)palladium(0) (130 mg, 0.11 mmol) was added, followed by powdered potassium phosphate (460 mg, 2.2 mmol). The mixture was heated at 85 °C for 5 h, then the solvent was evaporated. The residue was chromatographed on silica gel using hexanes/ethyl acetate as eluant to provide the product fraction (550 mg) as an orange oil. The product fractionwas a 2:1 mixture (mol ratio) of **229F** and the corresponding 2'-H product, as judged by proton NMR.

### G. N-(3,4-Dimethyl-5-isoxazolyl)-4'-hydroxymethyl-2'-(3,3,3-trifluoropropyl)-N-[(2-trimethylsilyl)ethoxymethyl][1,1'-biphenyl]-2-sulfonamide

The **229F** mixture (550 mg, 0.9 mmol) was subjected to sodium borohydride reduction according to General Method 14. The crude residue was chromatographed directly on silica gel using 3:1 hexanes/ethyl acetate as eluant to provide the product fraction (355 mg) as an amber oil. The product fraction was a 2:1 mixture (mol ratio) of **229G** and the corresponding 2'-H product, as judged by proton NMR.

### H. N-(3,4-Dimethyl-5-isoxazolyl)-4'-(methanesulfonyloxy)methyl-2'-(3,3,3-trifluoropropyl)-N-[(2-trimethylsilyl)ethoxymethyl] [1,1'-biphenyl]-2-sulfonamide

The **229G** mixture (350 mg, 0.63 mmol) was converted to the corresponding methanesulfonate ester according to General Method 3. The entire crude product **229H** was used directly in the next step.

### I. 4'-[(2-Butyl-4-oxo-1,3-diazaspiro[4.4]non-1-en-3-yl)methyl]-N-(3,4-dimethyl-5-isoxazolyl)-2'-(3,3,3-trifluoropropyl)-N-[(2-trimethylsilyl)ethoxymethyl] [1,1'-biphenyl]-2-sulfonamide

**229H** was used to alkylate 2-butyl-1,3-diazaspiro[4.4]non-1-en-4-one according to General Method 4. The crude residue was chromatographed on silica gel using 2:1 hexanes/ethyl acetate as eluant to provide the product fraction (370 mg) as a slightly orange oil. The product fraction was a 2:1 mixture (mol ratio) of **229I** and the corresponding 2'-H product, as judged by proton NMR

### J. 4'-[(2-Butyl-4-oxo-1,3-diazaspiro[4,4]non-1-en-3-yl)methyl]-N-(3,4-dimethylisoxazol-5-yl)-2'-(3,3,3-trifluoropropyl) [1,1'-biphenyl]-2-sulfonamide

The **229I** mixtue was deprotected according to General Method 8. The crude product (which contained the 2'-H contaminant) was purified by reverse-phase preparative HPLC, followed by extraction with ethyl acetate from pH 8 potassium phosphate buffer. Finally, silica gel chromatography using 2:1 hexanes/acetone as eluant provided the title compound (120 mg) as a white foam; LRMS m/z 631 (ESI+ mode); HPLC retention time 3.78 min (Method C); HPLC purity >98%.

### Reference Example 230

### 4'-[(2-Butyl-4-oxo-1,3-diazaspiro[4,4]non-1-en-3-yl)methyl]-N-(3,4-dimethylisoxazol-5-yl)-2'-(3-fluoropropyl) [1,1'-biphenyl]-2-sulfonamide

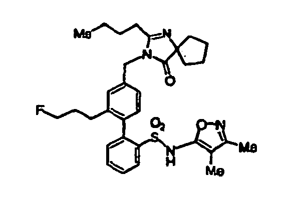

### A. Methyl 3-(2-propenyl)-4-(trifluoromethanesulfonyloxy)benzoate

Methyl 4-hydroxy-3-(2-propenyl)benzoate (12.0 g, 62.4 mmol, prepared according to W.J. Greenlee, et. al., WO 91/11999) was treated with trifluoromethanesulfonic anhydride according to the procedure of Example 229, Step E. The crude product was chromatographed on silica gel using 3:1 hexanes/ethyl acetate as eluant to yield 17.4 g of **230A** as a yellow oil.

### B. Methyl 3-(3-hydroxypropyl)-4-(trifluoromethanesulfonyloxy)benzoate

Borane-THF complex (1.0 M solution in THF, 32 ml,32 mmol) was added to a solution of **230A** (8.5 g, 26 mmol) in THF at 0 °C. The mixture was stirred at RT for 16h and then cooled to 0 °C. A 1.0 M solution of sodium/potassium phoshate buffer (pH 7, 50 ml) was added, followed by 30% aqueous hydrogen peroxide (9.0 ml). The mixture was allowed to warm to RT, then water and EtOAc were added. The organic layer was dried over sodium sulfate and evaporated. The crude product was chromatographed on silica gel using 1:1 hexanes/ethyl acetate as eluant to yield 4.3 g of **230B** as a yellow oil.

### C. N-(3,4-Dimethyl-5-isoxazolyl)-2'-(3-hydroxypropyl)-4'-(methoxycarbonyl)-N-[(2-trimethylsilyl)ethoxymethyl] [1,1'-biphenyl]-2-sulfonamide

Suzuki coupling of 230B (1.3 g, 3.8 mmol) according to the procedure of Example 229, step F, provided 230C (750 mg) as an orange oil following silica gel chromatography using1:1hexanes/ethyl acetate as eluant.

### D. N-(3,4-Dimethyl-5-isoxazolyl)-2'-(3-fluoropropyl)-4'-(methoxycarbonyl)-N-[(2-trimethylsilyl)ethoxymethyl] [1,1'-biphenyl]-2-sulfonamide

A solution of **230C** (750 mg,1.3 mmol) in dichloromethane (3 ml) was treated at -78°C with (diethylamino)sulfur trifluoride (0.21 ml, 1.6 mmol). The mixture was allowed to warm to RT. After 15 min, water was added and the aqueous layer was extracted with two portions of dichloromethane. The organic extracts were dried over sodium sulfate, concentrated, and the residue was chromatographed on silica gel using 2:1 hexanes/ethyl acetate as eluant to provide **230D** (175 mg) as a yellow oil.

### E. N-(3,4-Dimethyl-5-isoxazolyl)-2'-(3-fluoropropyl)-4'-(hydroxymethyl)-N-[(2-trimethylsilyl)ethoxymethyl] [1,1'-biphenyl]-2-sulfonamide

A solution of **230D** (175 mg,0.3 mmol) in THF (5 ml) was treated with DIBAL-H (0.53 ml of a 1.5 M solution in toluene, 0.8 mmol) at -78 °C. The temperature was allowed to rise to -25 °C and the mixture was stirred for 2 h. Water (2 ml) and ether (10 ml) were added and the mixture was stirred at RT for 2 h. The mixture was filtered and the filtrate concentrated to provide crude **230E** (110 mg) as a colorless oil.

### F. N-(3,4-Dimethyl-5-isoxazolyl)-2'-(3-fluoropropyl)-4'-(methanesulfonyloxy)methyl-N-[(2-trimethylsilyl)ethoxymethyl] [1,1'-biphenyl]-2-sulfonamide

**230E** (110 mg,0.2 mmol) was converted to the corresponding methanesulfonate ester according to General Method 3. The entire crude product **230F** was used directly in the next step.

### G. 4'-[(2-Butyl-4-oxo-1,3-diazaspiro[4,4]non-1-en-3-yl)methyl]-N-(3,4-dimethyl-5-isoxazolyl)-2'-(3-fluoropropyl)-N-[(2-trimethylsilyl)ethoxymethyl] [1,1'-biphenyl]-2-sulfonamide

**230F** was used to alkylate 2-butyl-1,3-diazaspiro[4,4]non-1-en-4-one according to General Method 4, to provide **230G** (124 mg) as a colorless oil, which was used without further purification.

### H. 4'-[(2-Butyl-4-oxo-1,3-diazaspiro[4,4]non-1-en-3-yl)methyl]-N-(3,4-dimethylisoxazol-5-yl)-2'-(3-fluoropropyl)[1,1'-biphenyl]-2-sulfonamide

**230G** (124 mg, 0.2 mmol) was deprotected according to General Method 8 (EtOH). The crude product was purified by silica gel chromatography using 1:1 hexanes/ethyl acetate as eluant provided the title compound (23 mg) as a white foam; LRMS m/z 595 (ESI+ mode); HPLC retention time 2.10 min (Method D); HPLC purity >98%.

### Reference Example 231

### 4'-[(2-Butyl-4-oxo-1,3-diazaspiro[4.4]non-1-en-3-yl)methyl]-2'-(1,1-difluoroethyl)-N-(3,4-dimethylisoxazol-5-yl) [1,1'-biphenyl]-2-sulfonamide

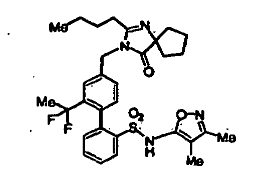

### A. 5-Bromo-2-(trifluoromethanesulfonyloxy)acetophenone

5-Bromo-2-hydroxyacetophenone (3.3 g, 15 mmol) was treated with trifluoromethanesulfonic anhydride according to the procedure of Example 229, Step E. The crude residue was taken up in ether and washed twice with 10% aqueous potassium dihydrogenphosphate solution and once with brine. The ether solution was dried over magnesium sulfate and concentrated to provide crude **231A** (4.7 g) as an orange oil, which was used without further purification.

### B. 3-(1,1,-Difluoroethyl)-4-(trifluoromethanesulfonyloxy)bromobenzene

**231A** (4.4 g, 13 mmol) was treated at RT with neat (diethylamino)sulfur trifluoride (2.5 ml, 19 mmol) and the resulting solution was stirred at RT for 40 h. The mixture was poured onto ice, aqueous sodium bicarbonate solution was added, and the mixture was extracted with three portions of dichloromethane. The combined extracts were dried over sodium sulfate and concentrated, and the residue was chromatographed on silica gel using 5:1 hexanes/ethyl acetate as eluant to provide **231B** ( 4.0 g) as an orange oil.

### C. 3-(1,1,-Difluoroethyl)-4-(trifluoromethanesulfonyloxy)benzaldehyde

A solution of **231B** (3.8 g, 10 mmol) and DMF (1.2 ml, 15 mmol) in dry THF (60 ml) was treated dropwise at -78°C with n-butyllithium (5.0 ml of a 2.5 M solution in hexanes, 13 mmol). 10% aqueous potassium dihydrogenphosphate solution was added and the resulting mixture was allowed to warm to RT. 1:1 hexanes/ethyl acetate and brine were added and the organic layer was collected, dried over sodium sulfate, and evaporated. The residue was chromatographed on silica gel using 3:1 hexanes/ethyl acetate as eluant to provide recovered **231B** (1.4 g), followed by **231C** ( 1.0 g), which was an orange oil.

### D. 2'-(1,1-Difluoroethyl)-N-(3,4-dimethylisoxazol-5-yl)-N-formyl-N-[(2-trimethylsilyl)ethoxymethyl] [1,1'-biphenyl]-2-sulfonamide

Suzuki coupling of **231C** (1.0 g, 3.2 mmol) according to the procedure of Example 229, step F, provided **231D** (640 mg) as an orange oil following silica gel chromatography using 3:1 hexanes/ethyl acetate as eluant.

### E. 2'-(1,1-Difluoroethyl)-N-(3,4-dimethylisoxazol-5-yl)-4'-hydroxymethyl-N-[(2-trimethylsilyl)ethoxymethyl] [1,1'-biphenyl]-2-sulfonamide

**231D** (640 mg, 1.2 mmol) was subjected to sodium borohydride reduction according to General Method 14. The crude residue was chromatographed on silica gel using 1:1 hexanes/ethyl acetate as eluant to provide **231E** (550 mg) as a brown oil.

### F. 2'-(1,1-Difluoroethyl)-N-(3,4-dimethylisoxazol-5-yl)-4'-(methanesulfonyloxy)methyl-N-[(2-trimethylsilyl)ethoxymethyl] [1,1'-biphenyl]-2-sulfonamide

**231E** (360 mg, 0.7 mmol) was converted to the corresponding methanesulfonate ester according to General Method 3. The entire crude product **231F** was used directly in the next step.

### G. 4'-[(2-Butyl-4-oxo-1,3-diazaspiro[4.4]non-1-en-3-yl)methyl]-2'-(1,1-difluoroethyl)-N-(3,4-dimethylisoxazol-5-yl)-N-[(2-trimethylsilyl)ethoxymethyl] [1,1'-biphenyl]-2-sulfonamide

**231F** was used to alkylate 2-butyl-1,3-diazaspiro[4.4]non-1-en-4-one according to General Method 4. The crude residue was chromatographed on silica gel using 1:1 hexanes/ethyl acetate as eluant to provide **231G** (170 mg) as a yellow oil.

### H. 4'-[(2-Butyl-4-oxo-1,3-diazaspiro[4.4]non-1-en-3-yl)methyl]-2'-(1,1-difluoroethyl)-N-(3,4-dimethylisoxazol-5-yl)-[1,1'-biphenyl]-2-sulfonamide

**231G** (150 mg, 0.2 mmol) was deprotected according to General Method 8 (EtOH). The crude product was chromatographed on silica gel using 1:1 hexanes/ethyl acetate as eluant to provide the title compound (36 mg) as a white foam; LRMS m/z 599 (ESI+ mode); HPLC retention time 3.58 min (Method A); HPLC purity >98%.

### Reference Example 232

### 4'-[(2-Butyl-4-oxo-1,3-diazaspiro[4,4]non-1-en-3-yl)methyl]-2'-(2,2,2-trifluoroethyl)-N-(3,4-dimethylisoxazol-5-yl) [1,1'-biphenyl]-2-sulfonamide

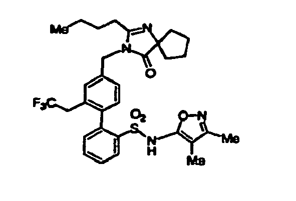

### A. Methyl 4-bromo-3-(bromomethyl)benzoate

Methyl 4-bromo-3-methylbenzoate (20 g, 87 mmol) was brominated according to General Method 13. The crude orange solid was triturated with 4:1 hexanes/ethyl acetate to provide **232A** (14.7 g) as a white solid.

### B. Methyl 4-bromo-3-(2,2,2-trifluoroethyl)benzoate

Methyl 2,2-difluoro-2-(fluorosulfonyl)acetate (3.1 ml, 25 mmol) was added to a mixture of **232A** ( 7.0 g, 22 mmol) and copper(I) iodide (420 mg, 2.2 mmol) in DMF (45 ml) under a nitrogen atmosphere. The mixture was heated to 85 °C for 16 h. The mixture was cooled to RT and the solvent was evaporated. Hexanes and brine were added, and the organic layer was dried over sodium sulfate and concentrated to give a yellow solid (6.0 g), which contained **232A** and **232B**.

The solid was dissolved in DMF (15 ml) and stirred at RT with potassium acetate (1.0 g, 10 mmol) for 3 h. The solvent was evaporated and the residue partitioned between ethyl acetate and water. The organic layer was dried over sodium sulfate and concentrated, and the residue was chromatographed on silica gel using 4:1 hexanes/ethyl acetate as eluant to provide **232B** (3.4 g) as a white solid.

### C. N-(3,4-Dimethylisoxazol-5-yl)-4'-(methoxycarbonyl)- 2'-(2,2,2-trifluoroethyl)-N-[(2-trimethylsilyl)ethoxymethyl) [1,1'-biphenyl]-2-sulfonamide

Suzuki coupling of **232B** (1.8 g, 5.9 mmol) according to the procedure of Example 229, step F, provided **232C** (1.3 g) as a yellow solid following silica gel chromatography using 3:1 hexanes/ethyl acetate as eluant.

### D. N-(3,4-Dimethylisoxazol-5-yl)-4'-hydroxymethyl-2'-(2,2,2-trifluoroethyl)-N-[(2-trimethylsilyl)ethoxymethyl][1,1'-biphenyl]-2-sulfonamide

**232C** (1.3 g, 2.2 mmol) was treated with DIBAL-H according to the procedure of Example 230, step E, to provide **232D** (650 mg) as an oil following silica gel chromatography using 3:1 hexanes/ethyl acetate as eluant.

### E. N-(3,4-Dimethylisoxazol-5-yl)-4'-(methanesulfonyloxy)methyl-2'-(2,2,2-trifluoroethyl)-N-[(2-trimethylsilyl)ethoxymethyl] [1,1'-biphenyl]-2-sulfonamide

**232D** (650 mg, 1.1 mmol) was converted to the corresponding methanesulfonate ester according to General Method 3. The entire crude product **232E** was used directly in the next step.

### F. 4'-[(2-Butyl-4-oxo-1,3-diazaspiro[4.4]non-1-en-3-yl)methyl]-N-(3,4-dimethylisoxazol-5-yl)-2'-(2,2,2-trifluoroethyl)-N-[(2-trimethylsilyl)ethoxymethyl] [1,1'-biphenyl]-2-sulfonamide

**232E** was used to alkylate 2-butyl-1,3-diazaspiro[4.4]non-1-en-4-one according to General Method 4. The crude residue was chromatographed on silica gel using 2:1 hexanes/ethyl acetate as eluant to provide **232F** (440 mg) as a yellow oil.

### G. 4'-[(2-Butyl-4-oxo-1,3-diazaspiro[4.4]non-1-en-3-yl)methyl]-N-(3,4-dimethylisoxazol-5-yl)-2'-(2,2,2-trifluoroethyl) [1,1'-biphenyl]-2-sulfonamide

**232F** was deprotected according to General Method 8 (EtOH). The crude product was purified by reverse-phase preparative HPLC, followed by an extraction with ethyl acetate from pH 5 sodium phoshpate buffer to provide the title compound (78 mg) as a white foam; LRMS m/z 617 (ESI+ mode); HPLC retention time 1.69 min (Method H); HPLC purity >98%.

### Reference Example 233

### 4'-[(2-Butyl-4-oxo-1,3-diazaspiro[4,4]non-1-en-3-yl)methyl]-N-(3,4-dimethylisoxazol-5-yl)-2'-[(2-methyl)propoxy] [1,1'-biphenyl]-2-sulfonamide

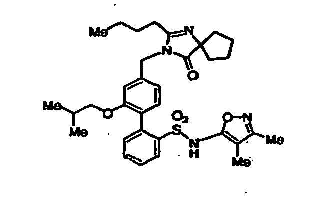

### A. 2-Bromo-5-methylphenol

A solution of sodium nitrite (2.8 g, 41 mmol) in 5 ml water was added rapidly with stirring to an ice-cooled mixture of 6-amino-m-cresol (5.0 g, 41 mmol) and 48% hydrobromic acid (17 ml, 100 mmol). The temperature was kept below 10 °C by addition of ice chips. The diazonium salt solution was then added in portions over a period of 30 min to a boiling mixture of copper(I) bromide (6.4 g, 22 mmol) and 48% hydrobromic acid (5 ml). The resulting mixture was refluxed for an additional 30 min, then was cooled and extracted with ether (2x100ml). The combined organic extracts were washed with water, dried over magnesium sulfate, and evaporated. The residue was chromatographed on silica gel using 98:2 hexanes/ethyl acetate to afford **233A** (1.6 g, 20%) as an oil.

### B. 4-Bromo-3-(2-methylpropoxy)toluene

Isobutyl bromide (0.70 ml, 6.4 mmol) was added to a mixture of **233A** (800 mg, 4.3 mmol), potassium carbonate (1.2 g, 8.5 mmol), and DMF (2 ml). The mixture was stirred for 36 h at 45 °C and was then cooled and concentrated under vacuum. The residue was added to water and extracted with ethyl acetate (3 X 50ml). The combined organic extracts were washed with water and dried over sodium sulfate to give **233B** (960 mg, 93%) as an oil.

### C. 4-Bromomethyl-2-(2-methylpropoxy)-1-bromobenzene

**233B** (960 mg, 3.9 mmol) was subjected to NBS bromination according to General Method 13. The crude **233C** (1.1 g, 86%) was used without further purification.

### D. 4-[(2-Butyl-4-oxo-1,3-diazaspiro[4.4]non-1-en-3-yl)methyl]-2-(2-methylpropoxy)-1-bromobenzene

**233C** (1.1 g, 3.4 mmol) was used to alkylate 2-butyl-1,3-diazaspiro[4.4]non-1-en-4-one according to General Method 4. The crude residue was chromatographed on silica gel using 1:1 hexanes/ethyl acetate as eluant to provide **233D** (550 mg, 40%) as an oil.

### E. 4'-[(2-Butyl-4-oxo-1,3-diazaspiro[4.4]non-1-en-3-yl)methyl]-N-(3,4-dimethylisoxazol-5-yl)-2'-[(2-methyl)propoxy]-N-[(2-trimethylsilyl)ethoxymethyl] [1,1'-biphenyl]-2-sulfonamide

Suzuki coupling of **233D** (550 mg, 1.3 mmol) according to General Method 1 provided **233E** (660 mg, 60%) as an oil following silica gel chromatography using 2:3 hexanes/ethyl acetate as eluant.

### F. 4'-[(2-Butyl-4-oxo-1,3-diazaspiro[4.4]non-1-en-3-yl)methyl]-N-(3,4-dimethylisoxazol-5-yl)-2'-[(2-methyl)propoxy][1.1'-biphenyl]-2-sulfonamide

233E (660 mg) was deprotected according to General Method 8 (EtOH). Water was added to the crude residue and the mixture was extracted with ethyl acetate (3 X 50ml). The combined organic extracts were washed with water and dried and evaporated. The residue was chromatographed on silica gel using 1:1 hexanes/ethyl acetate to provide the title compound (270 mg, 60%) as a white solid, mp 58-61°C ; LRMS m/e 607 (ESI+ mode); HPLC retention time 26.32 min (Method B); HPLC purity >98%.

### Reference Example 234

### 4'-[(2-Butyl-4-oxo-1,3-diazaspiro[4.4]non-1-en-3-yl)methyl]-N(3,4-dimethylisoxazol-5-yl)-2'-(2-methoxyethoxy) [1,1'-biphenyl]-2-sulfonamide

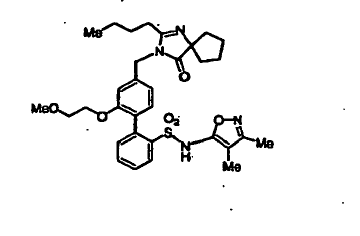

### A. 4-Bromo-3-(2-methoxyethoxy)toluene

**233A** (800 mg, 4.3 mmol) was alkylated with 1-bromo-2-methoxyethane (0.89 g, 6.4 mmol) according to the procedure of Example 233, step B, to give **234A** (840 mg, 81%) as an oil.

### B. 4-Bromomethyl-2-(2-methoxyethoxy)-1-bromobenzene

**234A** (840 mg, 3.4 mmol) was subjected to NBS bromination according to General Method 13. The crude **234B** (510 mg, 46%) was used without further purification.

### C. 4-[(2-Butyl-4-oxo-1,3-diazaspiro[4,4]non-1-en-3-yl)methyl]-2-(2-methoxyethoxy)-1-bromobenzene

**234B** (510 mg, 1.6 mmol) was used to alkylate 2-butyl-1,3-diazaspiro[4.4]non-1-en-4-one according to General Method 4. The crude residue was chromatographed on silica gel using 4:1 hexanes/ethyl acetate as eluant to provide **234C** (600 mg, 88%) as an oil.

### D. 4'-[(2-Butyl-4-oxo-1,3-diazaspiro[4.4]non-1-en-3-yl)methyl]-N-(3,4-dimethylisoxazol-5-yl)-2'-(2-methoxyethoxy)-N-[(2-trimethylsilyl)ethoxymethyl][1,1'-biphenyl]-2-sulfonamide

Suzuki coupling of **234C** (600 mg, 1.4 mmol) according to General Method 1 provided **234D** (550 mg, 54%) as an oil following silica gel chromatography using 2:3 hexanes/ethyl acetate as eluant.

### E. 4'-[(2-Butyl-4-oxo-1,3-diazaspiro[4,4]non-1-en-3-yl)methyl]-N-(3,4-dimethylisoxazol-5-yl)-2'-(2-methoxyethoxy)[1,1'-biphenyl]-2-sulfonamide

**234D** (550 mg, 0.8 mmol) was deprotected according to General Method 8 (EtOH). Water was added to the crude residue and the mixture was extracted with ethyl acetate (3 X 50ml). The combined organic extracts were washed with water and dried and evaporated. The residue was purified by reverse-phase preparative HPLC to provide the title compound (125 mg; 30%) as a white solid, mp 55-58 °C ; MS m/e 609 (ESI+ mode); HPLC retention time 22.75 min (Method B); HPLC purity >98%.

### Reference Example 235

### 4'-[(2-Butyl-4-oxo-1,3-diazaspiro[4.4]non-1-en-3-yl)methyl]-2'-butyl-N-(3,4-dimethylisoxazol-5-yl)[1,1'-biphenyl]-2-sulfonamide

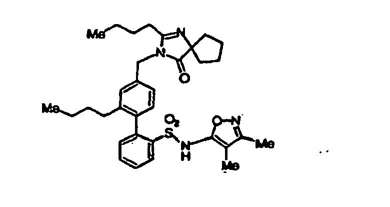

### A. 4-Bromo-3-(1-butenyl)benzonitrile

**2A** (4.0 g, 19 mmol) was reacted with propyltriphenylphosphonium bromide following the procedure of Example 27, step A. The crude residue was chromatographed on silica gel using 9:1 hexanes/ethyl acetate to afford **235A** (2.2 g, 49%) as an E/Z mixture.

### B. 4-Bromo-3-butylbenzonitrile

A mixture of **235A** (2.2 g, 9.3 mmol) and 210 mg of PtO₂ in 40 ml EtOH was hydrogenated at 35 PSI for 40 min. Filtration and concentration gave 1.4 g of **235B** (62%).

### C. 4-Bromo-3-butylbenzaldehyde

**235B** (1.4 g, 5.8 mmol) was treated with DIBAL-H according to General Method 14 to provide crude **235C** (1.2 g, 90%) as an oil.

### D. N-(3,4-Dimethyl-5-isoxazolyl)-2'-butyl-4'-formyl-N-[(2-trimethylsilyl)ethoxymethyl] [1,1'-biphenyl]-2-sulfonamide

235C (1.2 g, 5.1 mmol) was subjected to Suzuki coupling according to General Method 1 to provide 235D as a crude oil.

### E. N-(3,4-Dimethyl-5-isoxazolyl)- 2'-butyl-4'-hydroxymethyl-N-[(2-trimethylsilyl)ethoxymethyl] [1,1'-biphenyl]-2-sulfonamide

**235D** (entire sample) was reduced with sodium borohydride in methanol according to General Method 11 to provide 235E (1.4 g, 50% from **235C**) as an oil.

### F. N-(3,4-Dimethyl-5-isoxazolyl)-4'-(methanesulfonyl)oxymethyl-2'-butyl-N-[(2-trimethylsilyl)ethoxymethyl][1,1'-biphenyl]-2-sulfonamide

**235E** (1.4 g, 2.5 mmol) was converted to the corresponding methanesulfonate ester according to General Method 3 to provide **235F** (1.4 g, 90%) as an oil.

### G. 4'-[(2-Butyl-4-oxo-1.3-diazaspiro[4.4]non-1-en-3-yl)methyl]-2'-butyl-N-[(2-trimethylsilyl)ethoxymethyl]-N-(3,4-dimethyl-5-isoxazolyl)[1,1'-biphenyl]-2-sulfonamide

**235F** (1.3 g, 2.1 mmol) was used to alkylate 2-butyl-1,3-diazaspiro[4.4]non-1-en-4-one according to General Method 4. **235G** (1.3 g, 85%) was produced as an oil.

### H. 4'-[(2-Butyl-4-oxo-1,3-diazaspiro[4.4]non-1-en-3-yl)methyl]-2'-butyl-N-(3,4-dimethyl-5-isoxazolyl)[1,1'-biphenyl]-2-sulfonamide

**235G** (1.2 g, 1.7 mmol) was deprotected according to General Method 7. The crude product was purified by reverse-phase preparative HPLC to provide the title compound (620 mg, 62%) as a solid, mp 58-61 °C; MS m/e 591 (ESI+ mode); HPLC retention time 26.67 min (Method B); HPLC purity >98%.

### Reference Example 236

### 4'-[(2-Butyl-4-oxo-1,3-diazaspiro[4.4]non-1-en-3-yl)methyl]-N-(3-methylisoxazol-5-yl)-2'-trifluoromethyl[1,1'-biphenyl]-2-sulfonamide

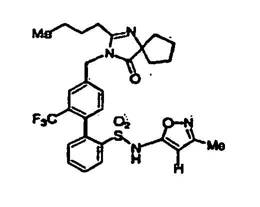

### A. [2-[[(3-methyl-5-isoxazolyl)[[(2-trimethylsilyl)ethoxy]methyl]amino] sulfonyl]phenyl]boronic acid

**25B** (14 g, 31 mmol) was converted to the corresponding boronic acid according to the procedure of Example 45, step B. The crude product **236A** (16.5 g, estimated purity 60%) was produced as an amber oil, and was used without further purification.

### B. 4'-Formyl-N-(3-methyl-5-isoxazolyl)-2'-trifluoromethyl-N-[(2-trimethylsilyl)ethoxymethyl][1,1'-biphenyl]-2-sulfonamide

**236A** (8.0 g, 20 mmol) was subjected to Suzuki coupling with **P6** according to General Method 1. Silica gel chromatography using 2:1 hexanes/ethyl acetate as eluant provided 1.8 g of a mixture of **236B** and a highly crystalline impurity. Trituration with 2:1 hexanes/ethyl acetate to remove this impurity provided **236B** (1.0 g) as an orange oil.

### C. 4'-Hydroxymethyl-N-(3-methyl-5-isoxazolyl)-2'-trifluoromethyl-N-[(2-trimethylsilyl)ethoxymethyl][1,1'-biphenyl]-2-sulfonamide

**236B** (880 mg, 1.6 mmol) was reduced with sodium borohydride (0.3 eq.) in ethanol according to General Method 11. The crude residue was chromatographed on silica gel using 2:1 hexanes/ethyl acetate as eluant to provide **236C** (450 mg) as a yellow oil.

### D. 4'-(Methanesulfonyloxy)methyl-N-(3-methyl-5-isoxazolyl)-2'-trifluoromethyl-N-[(2-trimethylsilyl)ethoxymethyl][1,1'-biphenyl]-2-sulfonamide

**236C** (450 mg) was converted to the corresponding methanesulfonate ester according to General Method 3. The entire crude product **236D** was used in the next reaction step.

### E. 4'-[(2-Butyl-4-oxo-1,3-diazaspiro[4.4]non-1-en-3-yl)methyl] N-(3-methyl-5-isoxazolyl)-2'-trifluoromethyl-N-[(2-trimethylsilyl)ethoxymethyl][1,1'-biphenyl]-2-sulfonamide

**236D** was used to alkylate 2-butyl-1,3-diazaspiro[4.4]non-1-en-4-one according to General Method 4. The crude residue was chromatographed on silica gel using 2:1 hexanes/acetone as eluant to provide **236E** (170 mg) as a yellow oil.

### F. 4'-[(2-Butyl-4-oxo-1,3-diazaspiro[4.4]non-1-en-3-yl)methyl] N-(3-methyl-5-isoxazolyl)-2'-trifluoromethyl [1,1'-biphenyl]-2-sulfonamide

**236E** (100 mg) was deprotected according to General Method 8 (ethanol) to provide the title compound as the hydrochloride salt (77 mg), which required no additional purification: MS m/e 589 (ESI+ mode); HPLC retention time 3.72 min (Method C); HPLC purity 97%.

### Reference Example 237

### N-(4-Bromo-3-methyl-5-isoxazolyl)-4'-[(2-butyl-4-oxo-1.3-diazaspiro[4.4]non-1-en-3-yl)methyl]-2'-trifluoromethyl [1.1'-biphenyl]-2-sulfonamide

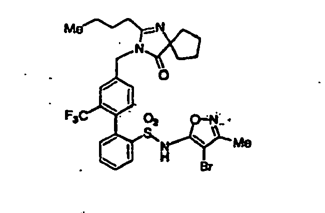

Bromine (33 mg/ml solution in acetic acid, 18 mg, 0.11 mmol) was added in portions to a solution of **236** (53 mg, 0.09 mmol) and sodium acetate (35 mg, 0.42 mmol) in acetic acid (4 ml) at RT. The solvent was evaporated, aqueous potassium phosphate solution was added, and the pH was adjusted to 8. The mixture was extracted with ethyl acetate, and the combined organic layers were dried over sodium sulfate. The residue was chromatographed on silica gel using 1:3 hexanes/acetone as eluant to provide the title compound (31 mg) as a white powder following lyophilization: MS m/e 667, 669 (ESI+ mode); HPLC retention time 3.83 min (Method C); HPLC purity 94%.

### Reference Example 238

### 4'-[(2-Butyl-4-oxo-1,3-diazaspiro[4,4]non-1-en-3-yl)methyl]-N-(4-chloro-3-methyl-5-isoxazolyl)-2'-trifluoromethyl [1,1'-biphenyl]-2-sulfonamide

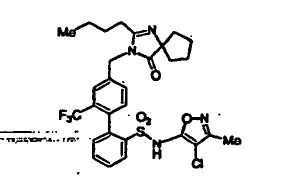

Clorox bleach (5.25% sodium hypochlorite solution, 225 µl) was added in portions to a solution of **236** (32 mg, 0.054 mmol) in THF (2 ml) at RT. The solvent was evaporated and the residue was purified by reverse-phase preparative HPLC, followed by preparative thin-layer silica gel chromatography using 10% methanol in dichloromethane as eluant, providing the title compound (1.0 mg) as a white powder following lyophilization: MS m/e 624 (ESI+ mode); HPLC retention time 3.53 min (Method A); HPLC purity 95%.

### Reference Example 239

### 4'-[(2-Butyl-4-oxo-1,3-diazaspiro[4.4]non-1-en-3-yl)methyl]-N-(3,4-dimethyl-5-isoxazolyl)-2'-(N-methoxy-N-methylaminomethyl) [1,1'-biphenyl]-2-sulfonamide

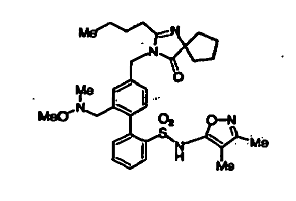

### A N-(3,4-Dimethyl-5-isoxazolyl)-4'-hydroxymethyl-2'-(N-methoxy-N-methylaminomethyl)-N-[(2-methoxyethoxy)methyl] [1,1'-biphenyl]-2-sulfonamide

**P21** (230 mg, 0.48 mmol) was subjected to reductive amination with N-methoxy-N-methylamine according to General Method 5, yielding **239A** (169 mg, 69%) as an oil.

### B. 4'-Bromomethyl-N-(3,4-dimethyl-5-isoxazolyl)-2'-(N-methoxy-N-methylaminomethyl)-N-[(2-methoxyethoxy)methyl] [1,1'-biphenyl]-2-sulfonamide

**239A** (165 mg, 0.33 mmol) was converted to the corresponding bromide according to General Method 2, yielding **239B** (174 mg, 92%) as an oil following silica gel chromatography using hexanes/ethyl acetate as eluant.

### C. 4'-[(2-Butyl-4-oxo-1,3-diazaspiro[4.4]non-1-en-3-yl)methyl]-N-(3,4-dimethyl-5-isoxazolyl)-2'-(N-methoxy-N-methylaminomethyl)-N-[(2-methoxyethoxy)methyl] [1,1'-biphenyl]-2-sulfonamide

**239B** (170 mg, 0,30 mmol) was used to alkylate 2-butyl-1,3-diazaspiro[4.4]non-1-en-4-one according to General Method 4. The crude residue was chromatographed on silica gel using hexanes/ethyl acetate as eluant to provide **239C** ( 155 mg, 72%) as a yellow oil.

### D. 4'-[(2-Butyl-4-oxo-1,3-diazaspiro[4.4]non-1-en-3-yl)methyl]-N-(3,4-dimethyl-5-isoxazolyl)-2'-(N-methoxy-N-methylaminomethyl)[1,1'-biphenyl]-2-sulfonamide

**239C** (150 mg) was deprotected according to General Method 7. The crude product was purified by reverse-phase preparative HPLC to provide the title compound (117 mg, 89%) as a white solid: MS m/e 608 (ESI+ mode); HPLC retention time 17.75 min (Method E ); HPLC purity >97%.

### Reference Example 240

### 4'-[(2-Butyl-4-oxo-1,3-diazaspiro[4.4]non-1-en-3-yl)methyl]-2'-(2,2-difluoroethoxymethyl)-N-(3,4-dimethylisoxazol-5-yl)[1,1'-biphenyl]-2-sulfonamide

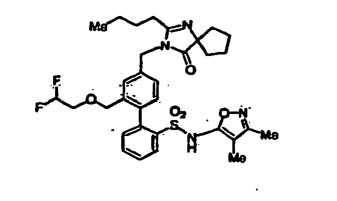

### A. Methyl 4-bromo-3-(hydroxymethyl)benzoate

A mixture of methyl 4-bromo-3-methylbenzoate (168 g, 735 mmol), N-bromosuccuumide (141 g, 792 mmol), benzoyl peroxide (3.5 g, 15 mmol), and carbon tetrachloride (900 ml) was refluxed for 17 h. The mixture was cooled and filtered, and the filtrate was washed once with water and once with brine. The filtrate was then dried over sodium sulfate and concentrated to provide an orange oil (261 g), which was judged (¹H NMR) be about 70 mol% methyl 4-bromo-3-(bromomethyl)benzoate.

The crude orange oil (261 g) was dissolved in 400 ml DMF and treated with potassium acetate (74 g, 750 mmol) at 0 °C. The mixture was allowed to warm to RT and was stirred for 54 h. The solvent was evaporated and the residue was taken up in 1:1 hexanes ethyl acetate and washed twice with half-saturated brine, then once with saturated brine. The organic layer was dried over sodium sulfate and concentrated to provide a new orange oil (208 g), which was judged to contain about 70 mol% methyl 4-bromo-3-(acetoxymethyl)benzoate.

The crude acetate mixture (208 g) was dissolved in methanol (11) and treated with potassium carbonate (12 g, 87 mmol) at 0 °C. The mixture was allowed to warm to RT and was stirred for 18 h. The solvent was evaporated and the residue was treated with 130 ml 1N hydrochloric acid at 0°C. The mixture was extracted once with 1:3 hexanes/ethyl acetate, once with 1:1 hexanes/ethyl acetate, and once with ethyl acetate. The combined organic layers were dried over sodium sulfate and concentrated, and the residue was chromatographed on silica gel using 2:1 hexanes/ethyl acetate. The product fractions were combined, evaporated, and triturated with 1:1 hexanes/ethyl acetate to provide **240A** (102 g) as a white solid.

### B. Methyl 4-bromo-3-[(tetrahydro-2H-pyran-2-yl)oxymethyl]benzoate

p-Toluenesulfonic acid hydrate (50 mg) was added to a solution of **240A** (10 g, 41 mmol) and 3,4-dihydro-2H-pyran (10 g, 120 mmol) in dichloromethane (100 ml) at 0°C. After 1h aqueous sodium bicarbonate solution was added, the layers were separated, and the organic layer was dried over sodium sulfate and concentrated to provide crude **240B** (16 g) as a slightly yellow oil.

### C. N-(3,4-Dimethyl-5-isoxazolyl)-4'-methoxycarbonyl-2'-[(tetrahydro-2H-pyran-2-yl)oxymethyl]-N-[(2-trimethylsilyl)ethoxymethyl][1,1'-biphenyl]-2-sulfonamide

Crude **240B** (10 g, aproximately 25 mmol) was subjected to Suzuki coupling according to General Method 1. Silica gel chromatography using 3:1 hexanes/ethyl acetate as eluant provided **240C** (14 g) as a yellow oil.

### D. N-(3,4-Dimethyl-5-isoxazolyl)-4'-hydroxymethyl-2'-[(tetrahydro-2H-pyran-2-yl)oxymethyl]-N-(2-trimethylsilyl)ethoxymethyl][1,1'-biphenyl]-2-sulfonamide

A solution of **240C** (10.5 g, 17 mmol) in THF (200 ml) was treated with DIBAL-H (23.4 ml of a 1.5 M solution in toluene, 35 mmol) at -78°C. The temperature was allowed to rise to -25 °C and the mixture was stirred for 2 h. Water (10 ml) and ether (100 ml) were added and the mixture was stirred at RT for 2 h. Ethyl acetate and hexanes were added, and the mixture was washed three times with aqueous sodium bicarbonate solution. The organic layer was dried over sodium sulfate and concentrated to provide crude **240D** (9 g) as a deep maroon-colored oil.

### E. N-(3,4-Dimethyl-5-isoxazolyl)-4'-(methanesulfonyloxy)methyl-2'-[(tetrahydro-2H-pyran-2-yl)oxymethyl]-N-[(2-trimethylsilyl)ethoxymethyl][1,1'-biphenyl]-2-sulfonamide

**240D** (4.5 g, 7.5 mmol) was converted to the corresponding methanesulfonate ester according to General Method 3. The entire crude product **240E** was used in the next reaction step.

### F. 4'-[(2-Butyl-4-oxo-1,3-diazaspiro[4.4]non-1-en-3-yl)methyl]-N-(3,4-dimethyl-5-isoxazolyl)-2'-((tetrahydro-2H-pyran-2-yl)oxymethyl]-N-[(2-trimethylsilyl)ethoxymethyl][1,1'-biphenyl]-2-sulfonamide

**240E** was used to alkylate 2-butyl-1,3-diazaspiro[4.4]non-1-en-4-one according to General Method 4. The crude residue was chromatographed on silica gel using 3:2 hexanes/ethyl acetate as eluant to provide **240F** (6.0 g) as a slightly yellow oil.

### G. 4'-[(2-Butyl-4-oxo-1,3-diazaspiro[4.4]non-1-en-3-yl)methyl]-N-(3,4-dimethyl-5-isoxazolyl)-2'-hydroxymethyl-N-[(2-trimethylsilyl)ethoxymethyl][1,1'-biphenyl]-2-sulfonamide

A solution of **240F** (6.0 g, 7.7 mmol) and 2N hydrochloric acid (6 ml, 12 mmol) in methanol (150 ml) was stirred for 16 h at RT. The mixture was neutralized with aqueous sodium bicarbonate solution and the methanol was evaporated. Aqueous sodium bicarbonate solution was added and the mixture was extracted twice with ethyl acetate. The combined organic extracts were dried over sodium sulfate and concentrated to provide **240G** (5.0 g) as a crude orange oil.

### H. 2'-Bromomethyl-4'-[(2-butyl-4-oxo-1,3-diazaspiro[4.4]non-1-en-3-yl)methyl]-N-(3,4-dimethyl-5-isoxazolyl) -N-[(2-trimethylsilyl)ethoxymethyl][1,1'-biphenyl]-2-sulfonamide

**240G** (590 mg, 0.85 mmol) was converted to the corresponding bromide according to General Method 2, providing **240H** (373 mg, 58%) as a yellow oil following silica gel chromatography using hexanes/ethyl acetate as eluant.

### I. 4'-[(2-Butyl-4-oxo-1,3-diazaspiro[4.4]non-1-en-3-yl)methyl]- 2'-(2,2-difluoroethoxymethyl)-N-(3,4-dimethyl-5-isoxazolyl) -N-[(2-trimethylsilyl)ethoxymethyl][1,1'-biphenyl]-2-sulfonamide

**240H** (370 mg, 0.49 mmol) was used to alkylate 2,2-difluoroethanol according to General Method 4. **240I** (209 mg, 56%) was produced as a yellow oil following silica gel chromatography using hexanes/ethyl acetate as eluant.

### J. 4'-[(2-Butyl-4-oxo-1,3-diazaspiro[4.4]non-1-en-3-yl)methyl]-2'-(2,2-difluoroethoxymethyl)-N-(3,4-dimethylisoxazol-5-yl)[1,1'-biphenyl]-2-sulfonamide

**240I** (205 mg, 0.27 mmol) was deprotected according to General Method 7. The crude product was purified by reverse-phase preparative HPLC to provide the title compound (104 mg, 60%) as a white solid: MS m/e 629 (ESI+ mode); HPLC retention time 17.18 min (Method E ); HPLC purity >97%.

### Reference Example 241

### 4'-[(2-Butyl-4-oxo-1,3-diazaspiro[4.4]non-1-en-3-yl)methyl]-N-(3,4-dimethylisoxazol-5-yl)-2'-(2-fluoroethyl)[1,1'-biphenyl]-2-sulfonamide

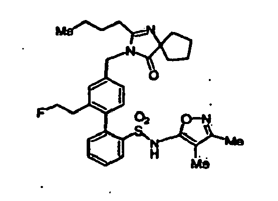

### A. Methyl 3-(2-hydroxethyl)-4-(trifluoromethanesulfonyloxy)benzoate

An ozone/oxygen gas mixture was bubbled through a solution of **230A** (8.3 g, 26 mmol) in methanol (100 ml) at -78 °C until a light blue color persisted. The solution was sparged with nitrogen to remove excess ozone, then triphenylphosphine (10 g, 38 mmol) was added in portions. The cooling bath was removed and the mixture was allowed to warm RT, then was concentrated. Ethanol (100 ml) was added to the residue and the resulting mixture was cooled to 0 °C. Sodium borohydride (1.9 g, 51 mmol) was added. After 1h, the mixture. was allowed to warm to RT and the solvent was evaporated. The residue was taken up in 10% aqueous potassium dihydrogen phosphate solution and was extracted with ethyl acetate. The combined organic extracts were dried over sodium sulfate and concentrated, and the residue chromatographed on silica gel using 1:1 hexanes/ethyl acetate as eluant to yield **241A** (4.5 g) as a colorless oil.

### B. N-(3,4-Dimethyl-5-isoxazolyl)-2'-(2-hydroxethyl)-4'-(methoxycarbonyl)-N-[(2-trimethylsily)ethoxymethyl] [1.1'-biphenyl]-2-sulfonamide

Suzuki coupling of **241A** (4.5 g, 14 mmol) according to the procedure of Example 229, step F, provided **241B** (4.1 g) as a yellow solid following silica gel chromatography using1:1hexanes/ethyl acetate as eluant.

### C. N-(3,4-Dimethyl-5-isoxazolyl)-2'-(2-fluoroethyl)-4'-(methoxycarbonyl)-N-[(2-trimethylsilyl)ethoxymethyl][1.1'-biphenyl]-2-sulfonamide

A mixture of **241B** (1.0 g, 1.8 mmol) and (diethylamino)sulfur trifluoride (0.71 ml, 5.4 mmol) was stirred at RT for 20 h. The mixture was poured onto ice. Aqueous sodium bicarbonate solution was added and the mixture was extracted with two portions of dichloromethane. The organic extracts were dried over sodium sulfate, concentrated, and the residue was chromatographed on silica gel using 1:1 hexanes/ethyl acetate as eluant to provide **241C** (230 mg) as a yellow oil.

### D. N-(3,4-Dimethyl-5-isoxazolyl)-2'-(2-fluoroethyl)-4'-(hydroxymethyl)-N-[(2-trimethylsilyl)ethoxymethyl] [1,1'-biphenyl]-2-sulfonamide

**241C** ( 230 mg, 0.41 mmol) was treated with DIBAL-H according to the procedure of Example 230, step E, to provide crude **241D** (320 mg) as a yellow oil

### E. N-(3,4-Dimethyl-5-isoxazolyl)-2'-(2-fluoroethyl)-4'-(methanesulfonyloxy)methyl-N-[(2-trimethylsilyl)ethoxymethyl] [1,1'-biphenyl]-2-sulfonamide

**241D** (320 mg) was converted to the corresponding methanesulfonate ester according to General Method 3. The entire crude product **241E** was used directly in the next step.

### F. 4'-[(2-Butyl-4-oxo-1,3-diazaspiro[4.4]non-1-en-3-yl)methyl]-N-(3,4-dimethyl-5-isoxazolyl)-2'-(2-fluoroethyl)-N-[(2-trimethylsilyl)ethoxymethyl][1,1'-biphenyl]-2-sulfonamide

**241E** was used to alkylate 2-butyl-1,3-diazaspiro[4.4]non-1-en-4-one according to General Method 4, to provide **241F** (300 mg) as a yellow oil, which was used without further purification.

### G. 4'-[(2-Butyl-4-oxo-1,3-diazaspiro[4.4]non-1-en-3-yl)methyl-N-(3,4-dimethylisoxazol-5-yl)-2'-(2-fluoroethyl) [1,1'-biphenyl]-2-sulfonamide

Crude **241F** (300 mg) was deprotected according to General Method 8 (ethanol). The crude product was purified by silica gel chromatography using hexanes/ethyl acetate as eluant provided the title compound (16 mg) as a tan solid; LRMS m/z 581 (ESI+ mode); HPLC retention time 2.05 min (Method H); HPLC purity 97%.

### Reference Example 242

### 4'-[(2-Butyl-4-oxo-1,3-diazaspiro[4,4]non-1-en-3-yl]methyl]-N-(3,4-dimethylisoxazol-5-yl)-2'-(2-hydroxyethyl)[1,1'-biphenyl]-2-sulfonamide

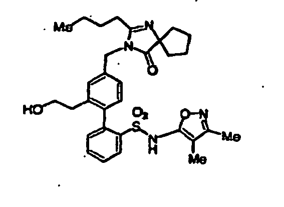

### A. N-(3,4-Dimethyl-5-isoxazolyl)-4'-methoxycarbonyl-2'-[2-[(tetrahydro-2H-pyran-2-yl)oxy]ethyl]-N-[(2-trimethylsilyl)ethoxymethyl][1,1'-biphenyl]-2-sulfonamide

Pyridinium p-toluenesulfonate (2 mg) was added to a solution of **241B** (1.0 g, 1.8 mmol) and 3,4-dihydro-2H-pyran (0.48 ml, 5.4 mmol) in dichloromethane (4 ml) at 0 °C. After 48h aqueous sodium bicarbonate solution was added, the layers were separated, and the organic layer was dried over sodium sulfate and concentrated. The residue was chromatographed on silica gel using 2:1 hexanes/ethyl acetate to provide **242A** (320 mg) as a slightly yellow oil.

### B. N-(3,4-Dimethyl-5-isoxazolyl)-4'-(hydroxymethyl)-2'-[2-[(tetrahydro-2H-pyran-2-yl)oxy]ethyl]-N-[(2-trimethysilyl)ethoxymethyl] [1,1'-biphenyl]-2-sulfonamide

**242A** ( 320 mg, 0.50 mmol) was treated with DIBAL-H according to the procedure of Example 230, step E, to provide crude **242B** (250 mg) as a yellow oil.

### C. N-(3,4-Dimethyl-5-isoxazolyl)-4'-(methanesulfonyloxy)methyl-2'-[2-[(tetrahydro-2H-pyran-2-yl)oxy]ethyl]-N-[(2-trimethysilyl)ethoxymethyl][1,1'-biphenyl]-2-sulfonamide

**242B** (250 mg) was converted to the corresponding methanesulfonate ester according to General Method 3. The entire crude product **242C** was used in the next reaction step.

### D. 4'-[(2-Butyl-4-oxo-1,3-diazaspiro[4.4]non-1-en-3-yl)methyl]-N-(3,4-dimethyl-5-isoxazolyl)-2'-[2-[(tetrahydro-2H-pyran-2-yl)oxy]ethyl]-N-[(2-trimethylsilyl)ethoxymethyl][1,1'-biphenyl]-2-sulfonamide

**242C** was used to alkylate 2-butyl-1,3-diazaspiro[4.4]non-1-en-4-one according to General Method 4. The crude **242D** was used without further purification.

### E. 4'-[(2-Butyl-4-oxo-1,3-diazaspiro[4,4]non-1-en-3-yl)methyl]-N-(3,4-dimethyl-5-isoxazolyl)-2'-(2-hydroxyethyl)[1,1'-biphenyl]-2-sulfonamide

**242D** was deprotected according to General Method 8 (ethanol). The crude product was purified by silica gel chromatography using 3% methanol in dichloromethane as eluant to provide the title compound (45 mg) as a white solid following lyophilization: MS m/e 579 (ESI+ mode); HPLC retention time 1.42 min (Method H ); HPLC purity >98%.

### Reference Example 243

### 4'-[(2-Butyl-4-oxo-1,3-diazaspiro[4,4]non-1-en-3-yl)methyl]-N-(3,4-dimethylisoxazol-5-yl)-2'-(3-methylbutyl)[1,1'-biphenyl]-2-sulfonamide

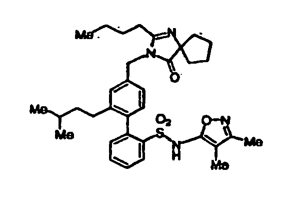

### A. 4-Bromo-3-(3-methyl-1-butenyl)benzonitrile

**2A** (2.0 g, 9.5 mmol) was reacted with isobutyltriphenylphosphonium bromide following the procedure of Example 27, step A. The crude residue was chromatographed on silica gel using 9:1 hexanes/ethyl acetate to afford **243A** (2.2 g, 91%) as an E/Z mixture.

### B. 4-Bromo-3-(3-methylbutyl)benzonitrile

A mixture of **243A** (2.2 g) and 220 mg of PtO₂ in 30 ml EtOH was hydrogenated at 35 PSI for 20 min. Filtration and concentration gave 1.9 g of **243B** (86%).

### C. 4-Bromo-3-(3-methylbutyl)benzaldehyde

**243B** (1.9 g) was treated with DIBAL-H according to General Method 14 to provide crude **243C** (810 mg, 43%) as an oil.

### D. N-(3,4-Dimethyl-5-isoxazolyl)-4'-formyl-2'-(3-methylbutyl)-N-[(2-methoxyethoxy)methyl][1,1'-biphenyl]-2-sulfonamide

**243C** (810 mg) was subjected to Suzuki coupling according to General Method 1 to provide **243D** as a crude oil.

### E. N-(3,4-Dimethyl-5-isoxazolyl)-4'-hydroxymethyl-2'-(3-methylbutyl)-N-[(2-methoxyethoxy)methyl][1,1'-biphenyl]-2-sulfonamide

**243D** (entire sample) was reduced with sodium borohydride in methanol according to General Method 11 to provide 243E (490 mg, 30% from **243C**) as an oil following silica gel chromatography using hexanes/ethyl acetate as eluant.

### F. 4'-Bromomethyl-N-(3,4-dimethyl-5-isoxazolyl)-2'-(3-methylbutyl)-N-[(2-methoxyethoxy) methyl][1,1'-biphenyl]-2-sulfonamide

**243E** (490 mg) was converted to the corresponding bromide according to General Method 2 to provide **243F** (430 mg, 78%) as an oil following silica gel chromatography using hexanes/ethyl acetate as eluant.

### G. 4'-[(2-Butyl-4-oxo-1,3-diazaspiro[4.4]non-1-en-3-yl)methyl]-2'-(3-methylbutyl)-N-[(2-methoxyethoxy)methyl]-N-(3,4-dimethyl-5-isoxazolyl)[1,1'-biphenyl]-2-sulfonamide

**243F** (430 mg) was used to alkylate 2-butyl-1,3-diazaspiro[4.4]non-1-en-4-one according to General Method 4. **243G** (300 mg, 58%) was produced as an oil following silica gel chromatography using hexanes/ethyl acetate as eluant.

### H. 4'-[(2-Butyl-4-oxo-1,3-diazapiro[4,4]non-1-en-3-yl)methyl]-N-(3,4-dimethylisoxazol-5-yl)-2'-(3-methylbutyl)[1,1'-biphenyl]-2-sulfonamide

**243G** was deprotected according to General Method 7. The crude product was purified by reverse-phase preparative HPLC to provide the title compound (165 mg, 63%) as a white solid: mp 50-53 °C; MS m/e 605 (ESI+ mode); HPLC retention time 27.42 min (Method B ); HPLC purity >97%.

### Reference Example 244

### 4'[(2-Butyl-4-oxo-1,3-diazaspiro[4.4]non-1-en-3-yl)methyl]-N-(3,4-dimethylisoxazol-5-yl)-2'-(2-methypropyl)[1.1'-biphenyl]-2-sulfonamide

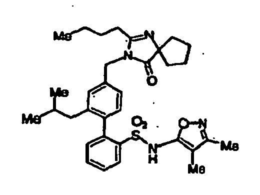

### A. 4-[(2-Methyl-2-propenyl)oxylbenzonitrile

A mixture of 4-cyanophenol (9.0 g, 75 mmol), potassium carbonate (21 g, 150 mmol), and DMF (50 ml) was treated with methallyl bromide (7.8 ml, 77 mmol) at 0 °C. The mixture was stirred at RT for 16 h, then was added to water and extracted with ethyl acetate (3 X 100 ml). The combined organic extracts were washed with water and dried and evaporated to give **244A** (13 g, 99%) as an oil.

### B. 4-Cyano-2-(2-methyl-2-propenyl)phenol

A solution of **244A** (13 g,75 mmol) and BHT (165 mg) in 1,2,4-trichlorobenzene (40 ml) was heated at 200 °C for 5 days. The mixture was cooled and diluted with ethyl acetate, then the organic layer was extracted with 10% NaOH solution (3 X 200ml). The aqueous phase was made acidic with hydrochloric acid and extracted with ether (3 X 100ml). The combined ether extracts were washed with water and dried and evaporated. The residue was chromatographed on silica gel using 9:1 hexanes/ethyl acetate to afford **244C** (5.8 g, 45%) as a solid.

### C. 4-Cyano-2-(2-methylpropyl)phenol

A mixture of **244B** (1.1 g) and 110 mg of PtO₂ in 20 ml EtOH was hydrogenated at 35 PSI for 15 min. Filtration and concentration gave 0.73 g of **244C** (66%).

### D. 4-Hydroxy-3-(2-methylpropyl)benzaldehyde

**244C** (0.73 g) was treated with DIBAL-H according to General Method 14 to provide crude **244D** (530 mg, 72%) as an oil.

### E. 3-(2-Methylpropyl)-4-(trifluoromethanesulfonyloxy)benzaldehyde

**244D** (530 mg) was converted to the corresponding trifluoromethanesulfonate ester according to the procedure of Example 229, step E. The residue was chromatographed on silica gel using 4:1 hexanes/ethyl acetate to give **244E** (295 mg, 33%) as an oil.

### F. N-(3,4-Dimethyl-5-isoxazolyl)-4'-formyl-2'-(2-methylpropyl)-N-[(2-methoxyethoxy)methyl [1,1'-biphenyl]-2-sulfonamide

Suzuki coupling of **244E** (295 mg) according to the procedure of Example 229, step F, provided **244F** (170 mg, 36%) as a yellow solid following silica gel chromatography using hexanes/ethyl acetate as eluant.

### G. N-(3,4-Dimethyl-5-isoxazolyl)-4'-hydroxymethyl-2'-(2-methylpropyl)-N-[(2-methoxyethoxy)methyl][1,1'-biphenyl]-2-sulfonamide

**244F** (170 mg) was reduced with sodium borohydride in methanol according to General Method 11 to provide **244G** (67 mg, 39%) as an oil.

### H. 4'-Bromo-methyl-N-(3,4-dimethyl-5-isoxazolyl)-2'-(2-methylpropyl)-N-[(2-methoxyethoxy)methyl][1,1'-biphenyl]-2-sulfonamide

**244G** (67 mg) was converted to the corresponding bromide according to General Method 2 to provide **244H** (42 mg, 56%) as an oil.

### I. 4'-[(2-Butyl-4-oxo-1,3-diazaspiro[4,4]non-1-en-3-yl)methyl]-2'-(2-methylpropyl)-N-[(2-methoxyethoxy)methyl]-N-(3,4-dimethyl-5-isoxazolyl)[1,1'-biphenyl]-2-sulfonamide

**244H** (42 mg) was used to alkylate 2-butyl-1,3-diazaspiro[4.4]non-1-en-4-one according to General Method 4. **244I** (36 mg, 45%) was produced as an oil following silica gel chromatography using hexanes/ethyl acetate as eluant.

### J. 4'-[(2-Butyl-4-oxo-1,3-diazaspiro[4.4]non-1-en-3-yl)methyl]-2'-(2-methylpropyl)-N-(3,4-dimethyl-5-isoxazolyl)[1,1'-biphenyl]-2-sulfonamide

**244I** (36 mg) was deprotected according to General Method 7. The crude product was purified by reverse-phase preparative HPLC to provide the title compound (25 mg, 86%) as a white solid: mp 58-61 °C; MS m/e 591 (ESI+ mode); HPLC retention time 28.21 min (Method B ); HPLC purity >98%.

### Reference Example 245

### 4'-[[2-(3,3-Difluorobutyl)-4-oxo-1,3-diazaspiro[4.4]non-1-en-3-yl]methyl]-N-(3,4-dimethylisoxazol-5-yl)-2'-(ethoxymethyl)[1.1'-biphenyl]-2-sulfonamide

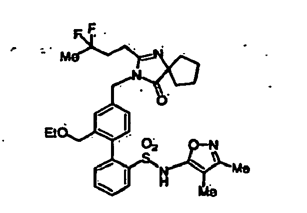

### A. Methyl 1-[(3,3-difluorobutanoyl)amino]cyclopentane-1-carboxylate

A solution of 4,4-Difluoropentanoic acid (600 mg, 4.4 mmol, prepared according to Larsson, U.; Carlson, R.; Leroy, J. *Acta Chem.* Scand. **1993**, *47*, 380-90) in dichloromethane (10 ml) was treated at RT with oxalyl chloride (4.4 ml of a 2.0 M solution in dichloromethane, 8.8 mmol) and DMF (10 µl). After 20 min the mixture was evaporated and 10 ml fresh dichloromethane was added. The mixture was cooled to 0 °C and methyl 1-aminocyclopentane-1-carboxylate hydrochloride (1.6 g, 8.8 mmol) was added, followed by triethylamine (3.6 ml, 26 mmol) and DMAP (10 mg). The mixture was stirred at RT for 3 hours. Aqueous sodium bicarbonate solution was added and the mixture was extracted three times with dichloromethane. The combined organic extracts were dried over sodium sulfate and concentrated. The residue was chromatographed on silica gel using 1:2 hexanes/ethyl acetate as eluant to give **245A** (520 mg, 46%) as an orange oil.

### B. 2-(3,3-Difluorobutyl)-1,3-diazaspiro[4.4]non-1-en-4-one

**245A** (520 mg, 2.0 mmol) was treated according to the procedure of Example 22, step B. The crude residue was chromatographed on silica gel using 1:3 hexanes/ethyl acetate as eluant to yield **245B** (150 mg, 33%) as a yellow oil.

### C. 4'-[[2-(3,3-Difluorobutyl)-4-oxo-1,3-diazaspiro[4,4]non-1-en-3-yl]methyl]-N-(3,4-dimethylisoxazol-5-yl)-2'-(ethoxymethyl)-N-(2-methoxyethoxy)methyl[1,1'-biphenyl]-2-sulfonamide

**245B** (75 mg, 0.42 mmol) was alkylated with 4'-bromomethyl-N-(3,4-dimethyl-5-isoxazolyl)-2'-ethoxymethyl-N-(2-methoxyethoxy)methyl[1,1'-biphenyl]-2-sulfonamide (prepared as described in Example 226) according to General Method 4. Crude 245C (220 mg) was used without further purification.

### D. 4'-[[2-(3,3-Difluorobutyl)-4-oxo-1,3-diazaspiro[4,4]non-1-en-3-yl]methyl]-N-(3,4-dimethylisoxazol-5-yl)-2'-(ethoxymethyl)[1,1'-biphenyl]-2-sulfonamide

**245C** (220 mg) was deprotected according to General Method 8 (EtOH). The crude product was purified by preparative thin-layer silica gel chromatography using 1:1 hexanes/acetone to proviode the title compound (57 mg) as a white lyophilized powder; MS m/e 629 (ESI+ mode); HPLC retention time 3.79 min (Method A); HPLC purity 96%.

### Reference Example 246

### N-(3,4-Dimethyl-5-isoxazolyl)-4'-[[(3-methoxy-2,6-dimethyl-4-pyridinyl)oxy]methyl]-2'-(3,3,3-trifluoropropyl)[1,1'-biphenyl]-2-sulfonamide

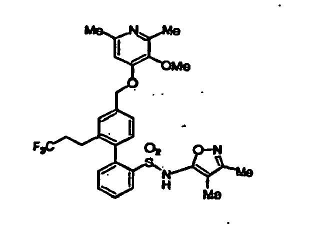

### A. N-(3,4-Dimethyl-5-isoxazolyl)-4'-[[(3-methoxy-2,6-dimethyl-4-pyridinyl)oxy]methyl]-2'-(3,3,3-trifluoropropyl)-N-[(2-trimethylsilyl)ethoxymethyl][1,1'-biphenyl]-2-sulfonamide

**229H** (400 mg, 0.60 mmol) was used to alkylate 3-methoxy-2,6-dimethyl-4-(4H)-pyridinone according to General Method 22. The crude product was purified by silica gel chromatography using 1:2 hexanes/ethyl acetate as eluant to provide **246A** (130 mg) as a slightly yellow oil.

### B. N-(3,4-Dimethyl-5-isoxazolyl)-4'-[[(3-methoxy-2,6-dimethyl-4-pyridinyl)oxy]methyl]-2'-(3,3,3-trifluoropropyl)[1,1'-biphenyl]-2-sulfonamide

**246A** (130 mg, 0.18 mmol) was deprotected according to General Method 8 (EtOH). The crude product was purified by silica gel chromatography using 5% methanol in chloroform as eluant to provide the title compound (82 mg) as a pale orange powder after lyophilization; MS m/e 590 (ESI+ mode); HPLC retention time 3.35 min (Method A); HPLC purity 97%.

### Reference Example 247

### 4'-[(2-Butyl-4-oxo-1,3-diazaspiro[4.4]non-1-en-3-yl)methyl]-N-(3,4-dimethylisoxazol-5-yl)-2'-[(1,1-dimethylethoxy)methyl] [1,1'-biphenyl]-2-sulfonamide

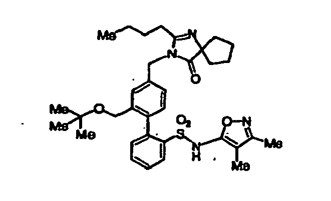

### A. Methyl 4-bromo-3-[(1,1-dimethylethoxy)methyl]benzoate

To a solution of **240A** (4.90 g, 20 mmol) in 40 ml cyclohexane and 20 ml dichloromethane was added t-butyl 2,2,2-trichloroacetimidate (4.81 g; 22 mmol) followed by 0.4 ml boron trifluoride diethyl etherate. The mixture was stirred at RT for 4 hours. 1 g solid sodium bicarbonate was added and the mixture was stirred for 10 min. The mixture was chromatographed directly on silica gel eluting with 20:1 hexanes/ethyl acetate to give compound **247A** as an oil (5.33 g, 88%).

### B. N-(3,4-Dimethyl-5-isoxazolyl)-2'-[(1,1-dimethylethoxy)methyl]-4'-methoxycarbonyl-N-[(2-trimethylsilyl)ethoxymethyl] [1,1'-biphenyl]-2-sulfonamide

**247A** (5.3 g, 17.5 mmol) was subjected to Suzuki coupling according to General Method 1. Silica gel chromatography using hexanes/ethyl acetate as eluant provided **247B** (6.4 g, 88%) as an oil.

### C. N-(3,4-Dimethyl-5-isoxazolyl)-2'-[(1,1-dimethylethoxy)methyl]-4'-hydroxymethyl-N-(2-trimethylsilyl)ethoxymethyl] [1,1'-biphenyl]-2-sulfonamide

**247B** (6.4 g, 10.7 mmol) was reduced with DIBAL-H according to the procedure of Example 230, step E, to provide crude **247C** (5.5 g, 89%) as an orange oil.

### D. 4'-Bromomethyl-N-(3,4-dimethyl-5-isoxazolyl)-2'-[(1,1-dimethylethoxy)methyl]-N-[(2-trimethylsilyl)ethoxymethyl][1,1'-biphenyl]-2-sulfonamide

**247C** (5.5 g, 9.6 mmol) was converted to the corresponding bromide according to General Method 2, providing **247D** (5.6 g, 92%) as a yellow oil following silica gel chromatography using hexanes/ethyl acetate as eluant.

### E. 4'-[(2-Butyl-4-oxo-1,3-diazaspiro[4,4]non-1-en-3-yl)methyl]-N-(3,4-dimethyl-5-isoxazolyl)-2'-[(1,1-dimethylethoxy)methyl]-N-[(2-trimethylsilyl)ethoxymethyl][1,1'-biphenyl]-2-sulfonamide

**247D** (380 mg, 0.60 mmol) was used to alkylate 2-butyl-1,3-diazaspiro[4.4]non-1-en-4-one according to General Method 4. The crude residue was chromatographed on silica gel using hexanes/ethyl acetate as eluant to provide **247E** (410 mg, 92%) as a slightly yellow oil.

### E. 4'-[(2-Butyl-4-oxo-1,3-diazaspiro[4.4] non-1-en-3-yl)methyl] -N-(3,4-dimethyl-5-isoxazolyl)2'-[(1,1-dimethylethoxy)methyl] [1,1'-biphenyl]-2-sulfonamide

247E (410 mg, 0.55 mmol) was deprotected with TBAF according to General Method 10. The crude product was purified by reverse-phase preparative HPLC to provide the title compound (230 mg, 66%) as a white solid: MS m/e 621 (ESI+ mode); HPLC retention time 20.97 min (Method E ); HPLC purity 96%.

| **EXAMPLE** | **STRUCTURE** | **NAME** | **SARTING MATERIAL** | **General Methods Applied (yield, %)** | **M/z (MH)*** | **HPLC % Purity** | **HPLC ret time, min (meth -od)** |
|---|---|---|---|---|---|---|---|
| 248 * | 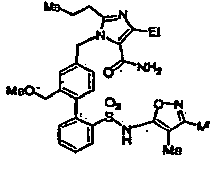 | 1-[[2'-[[(3,4-dimethyl-5-isoxazolyl)-amino]sulfonyl-2-methoxymethyl][ 1,1'-biphenyl]-4-yl]methyl]-4-ethyl-2-propyl-1H-imidazole-5-carboxamide | **P2** | 22 (39); | 566 | 95 | 2.79 |
| | | | | 8, EtOH, 15 (96); | | | (C) |
| | | | | 12 (38) | | | |
| 249 * | 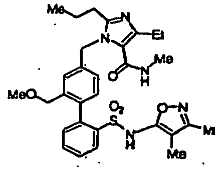 | 1-[[2'-[[(3,4-dimethyl-5-isoxazolyl)-amino]sulfonyl-2-methoxymethyl][1,1'-biphenyl]-4-yl]methyl]-4-ethyl-N-methyl-2-propyl-1H-imidazole-5-carboxamide | **P2** | 22 (39); | 580 | 96 | 2.90 |
| | | | | 8, EtOH, 15 (96); | | | (C) |
| | | | | 12 (34) | | | |
| 250 * | 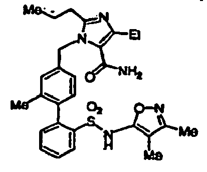 | 1-[[2'-[[(3,4-dimethyl-5-isoxazolyl)-amino]sulfonyl-2-methyl] [1,1'-biphenyl]-4-yl]methyl]-4-ethyl-2-propyl-1H-imidazole-5-carboxamide | **P13** | 22 (56); | 536 | 95 | 2.90 |
| | | | | 8, EtOH, 15 (94); | | | (C) |
| | | | | 12 (38) | | | |
| 251 * | 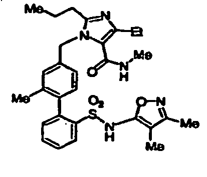 | 1-[[2'-[[(3,4-dimethyl-5-isoxazolyl)-amino]sulfonyl-2-methyl][1,1'-biphenyl]-4-yl]methyl]-4-ethyl-N-methyl-2-propyl-1H-imidazole-5-carboxamide | **P13** | 22 (56); | 550 | 97 | 2.99 |
| | | | | 8, EtOH, 15 (94); | | | (C) |
| | | | | 12 (37) | | | |

| | | | | | | | |
|---|---|---|---|---|---|---|---|
| * Reference Example | | | | | | | |

### Reference Example 252

### N-(4,5-Dimethyl-3-isoxazolyl)-2'-ethoxymethyl-4'-[[(3-methoxy-2,6-dimethyl-4-pyridinyl)oxy]methyl] [1,1'-biphenyl]-2-sulfonamide

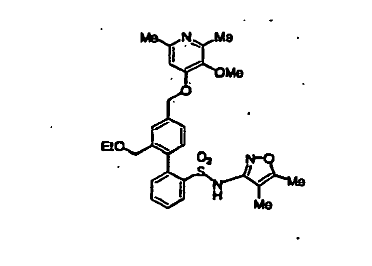

### A. 4-Bromo-3-(ethoxymethyl)benzonitrile

**P2A** (8.7 g, 32 mmol) was treated with sodium hydride and ethanol according to General Method 4, producing crude **252A** (5.8 g, 77%) as an oil.

### B. 4-Bromo-3-(ethoxymethyl)benzaldehyde

Crude **252A** (5.8 g, 24 mmol) was reduced with DIBAL-H according to General Method 14, yielding **252B** (4.7 g, 80%) as an oil following silica gel chromatography with hexanes/ethyl acetate as eluant.

### C. N-(4,5-Dimethyl-3-isoxazolyl)-2'-ethoxymethyl-4'-formyl-N-[(2-methoxyethoxy)methyl] [1.1'-biphenyl]-2-sulfonamide

**252B** (800 mg, 3.3 mmol) was subjected to Suzuki coupling with [2-[[(4,5-dimethyl-3-isoxazolyl)((2-methoxyethoxy)methyl]amino]-sulfonyl]phenyl]boronic acid according to General Method 1. **252C** (1.2 g, 70%) was obtained as an orange oil following silica gel chromatography using hexanes/ethyl acetate as eluant.

### D. N-(4,5-Dimethyl-3-isoxazolyl)-2'-ethoxymethyl-4'-hydroxymethyl-N-[(2-methoxyethoxy)methyl] [1,1'-biphenyl]-2-sulfonamide

**252C** (1.2 g, 2.3 mmol) was reduced with sodium borohydride in methanol according to General Method 11 to provide crude **252D**. This material was used without further purification.

### E. 4'-Bromomethyl-N-(4,5-dimethyl-3-isoxazolyl)-2'-ethoxymethyl-N-[(2-methoxyethoxy)methyl][1,1'-biphenyl]-2-sulfonamide

**252D** (entire sample) was converted to the corresponding bromide according to General Method 2. **252E** (1.1 g, 84% over two steps) was obtained as an oil following silica gel chromatography using hexanes/ethyl acetate as eluant.

### F. N-(4,5-Dimethyl-3-isoxazolyl)-2'-ethoxymethyl-4'-[[(3-methoxy-2,6-dimethyl-4-pyridinyl)oxy]methyl]-N-[(2-methoxymethoxy)methyl][1,1'-biphenyl]-2-sulfonamide

**252E** (360 mg, 0.63 mmol) was used to alkylate 3-methoxy-2,6-dimethyl-4-(1H)-pyridinone according to General Method 22. **252F** (310 mg, 76%) was produced as an oil following silica gel chromatography using hexanes/ethyl acetate as eluant.

### G. N-(4,5-Dimethyl-3-isoxazolyl)-2'-ethoxymethyl-4'-[[(3-methoxy-2,6-dimethyl-4-pyridinyl)oxy]methyl] [1,1'-biphenyl]-2-sulfonamide

**252F** (310 mg, 0.49 mmol) was deprotected according to General Method 7. The crude product was purified by silica gel column chromatography using methanol/dichloromethane as eluant to provide the title compound (240 mg, 91%) as a light yellow solid: mp 78-82 °C; MS m/e 552 (ESI+ mode); HPLC retention time 12.42 min (Method E); HPLC purity 97%.

### Reference Example 253

### 4'-[(2-Butyl-4-oxo-1,3-diazaspiro[4.4]non-1-en-3-yl)methyl]-N-(4,5-dimethyl-3-isoxazolyl)-2'-(2-fluoroethoxymethyl) [1,1'-biphenyl]-2-sulfonamide

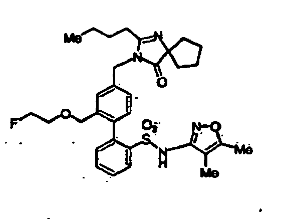

### A. 4-Bromo-3-[(2-fluoroethoxy)methyl]benzonitrile

**P2A** (4.1 g, 15 mmol) was treated with sodium hydride and 2-fluoroethanol according to General Method 4. Water was added to the reaction mixture to precipitate **253A** (2.9 g, 75%) as a brown solid.

### B. 4-Bromo-3-[(2-fluoroethoxy)methyl]benzaldehyde

**253A** (2.9 g, 11 mmol) was reduced with DIBAL-H according to General Method 14, yielding **253B** (2.5 g) as an oil, which was used without further purification.

### C. N-(4,5-Dimethyl-3-isoxazolyl)-2'-[(2-fluoroethoxy)methyl]-4'-formyl-N-[(2-methoxyethoxy)methyl] [1,1'-biphenyl]-2-sulfonamide

**253B** (2.5 g) was subjected to Suzuki coupling with [2-[[(4,5-dimethyl-3-isoxazolyl)[(2-methoxyethoxy)methyl] amino]-sulfonyl]phenyl]boronic acid according to General Method 1. **253C** (1.4 g, 23% over two steps) was obtained as an oil following silica gel chromatography using hexanes/ethyl acetate as eluant.

### D. N-(4,5-Dimethyl-3-isoxazolyl)-2'-[(2-fluoroethoxy)methyl]-4'-hydroxymethyl-N-[(2-methoxyethoxy)methyl][1,1'-biphenyl]-2-sulfonamide

**253C** (1.4 g, 2.6 mmol) was reduced with sodium borohydride in methanol according to General Method 11 to provide **253D** as an oil. This material was used without further purification.

### E. 4'-Bromomethyl-N-(4,5-dimethyl-3-isoxazolyl)-2'-[(2-fluoroethoxy)methyl]-N-[(2-methoxyethoxy)methyl][1,1'-biphenyl]-2-sulfonamide

**253D** (entire sample) was converted to the corresponding bromide according to General Method 2. **253E** (1.3 g, 80% over two steps) was obtained as an oil following silica gel chromatography using hexanes/ethyl acetate as eluant.

### F. 4'-[(2-Butyl-4-oxo-1,3-diazaspiro[4.4]non-1-en-3-yl)methyl]-N-(4,5-dimethyl-3-isoxazolyl)-2'-[(2-fluoroethoxy)methyl]-N-[(2-methoxyethoxy)methyl] [1,1'-biphenyl]-2-sulfonamide

**253E** (430 mg) was used to alkylate 2-butyl-1,3-diazaspiro[4.4]non-1-en-4-one according to General Method 4. **253F** (400 mg, 78%) was produced as an oil following silica gel chromatography using hexanes/ethyl acetate as eluant.

### G. 4'-[(2-Butyl-4-oxo-1,3-diazaspiro[4,4]non-1-en-3-yl)methyl]-N-(4,5-dimethyl-3-isoxazolyl)2'-[(2-fluoroethoxy)methyl] [1,1'-biphenyl]-2-sulfonamide

**253F** (400 mg, 0.57 mmol) was deprotected according to General Method 7. The crude product was purified by silica gel column chromatography using methanol/dichloromethane as eluant to provide the title compound (310 mg, 89%) as a white solid: mp 68-73 °C; MS m/e 611 (ESI+ mode); HPLC retention time 16.07 min (Method E); HPLC purity >97%.

### Reference Example 254

### N-(4,5-Dimethyl-3-isoxazolyl)-2'-[(2-fluoroethoxy)methyl]-4'-[[(3-methoxy-2,6-dimethyl-4-pyridinyl)oxy]methyl] [1,1'-biphenyl]-2-sulfonamide

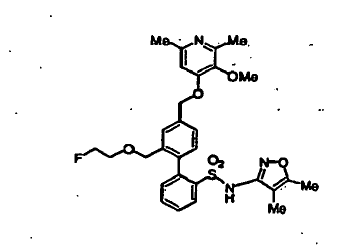

### B. N-(4,5-Dimethyl-3-isoxazolyl)-2'-[(2-fluoroethoxy)methyl]-4'-[[(3-methoxy-2,6-dimethyl-4-pyridinyl)oxy]methyl]-N-[(2-methoxyethoxy)methyl] [1,1'-biphenyl]-2-sulfonamide

**253E** (450 mg, 0.77 mmol) was used to alkylate 3-methoxy-2,6-dimethyl-4-(1H)-pyridinone according to General Method 22. The crude product was purified by silica gel chromatography using hexanes/ethyl acetate as eluant to provide **254A** (380 mg, 76%) as a slightly yellow oil.

### B. N-(4,5-Dimethyl-3-isoxazolyl)-2'-[(2-fluoroethoxy)methyl]-4'-[[(3-methoxy-2,6-dimethyl-4-pyridinyl)oxy]methyl] [1,1'-biphenyl]-2-sulfonamide

**254A** (380 mg, 0.58 mmol) was deprotected according to General Method 7. The crude product was purified by silica gel chromatography using methanol/chloroform as eluant to provide the title compound (310 mg, 93%) as a light yellow solid; mp 81-86 °C; MS m/e 570 (ESI+ mode); HPLC retention time 10.54 min (Method E); HPLC purity >97%.

### Reference Example 255

### N-(3,4-Dimethyl-5-isoxazolyl)-2'-[(2-fluoroethoxy)methyl]-4'-[[(3-methoxy-2,6-dimethyl-4-pyridinyl)oxy]methyl] [1,1'-biphenyl]-2-sulfonamide

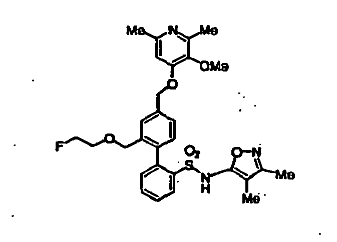

### A. N-(3,4-Dimethyl-5-isoxazolyl)-2'-[(2-fluoroethoxy)methyl]-4'-formyl-N-[[(2-trimethylsilyl)ethoxy]methyl] [1,1'-biphenyl]-2-sulfonamide

**253B** (1.3 g, 4.9 mmol) was subjected to Suzuki coupling with [2-[[(3,4-dimethyl-5-isoxazolyl)[[(2-trimethylsilyl)ethoxy]methyl]amino]-sulfonyl]phenyl]boronic acid according to General Method 1. **255A** (2.5 g, 90%) was obtained as an oil following silica gel chromatography using hexanes/ethyl acetate as eluant.

### B. N-(3,4-Dimethyl-5-isoxazolyl)-2'-[(2-fluoroethoxy)methyl]-4'-hydroxymethyl-N-[[(2-trimethylsily)ethoxy]methyl] [1,1'-biphenyl]-2-sulfonamide

**255A** (2.5 g, 4.4 mmol) was reduced with sodium borohydride in methanol according to General Method 11 to provide **255B** as an oil. This material was used without further purification.

### C. 4'-Bromomethyl-N-(3,4-dimethyl-5-isoxazolyl)-2'-[(2-fluoroethoxy)methyl]-N-[[(2-trimethylsilyl)ethoxy]methyl][1,1'-biphenyl]-2-sulfonamide

**255B** (entire sample) was converted to the corresponding bromide according to General Method 2, furnishing **255C** (2.1 g, 76% over two steps) as an oil following silica gel chromatography using hexanes/ethyl acetate as eluant.

### D. N-(3,4-dimethyl-5-isoxazolyl)-2'-[(2-fluoroethoxy)methyl]-4'-[[(3-methoxy-2,6-dimethyl-4-pyridinyl)oxy]methyl]-N-[[(2-trimethylsilyl)ethoxy]methyl] [1,1']-biphenyl]-2-sulfonamide

**255C** (590 mg, 0.93 mmol) was used to alkylate 3-methoxy-2,6-dimethyl-4-(1H)-pyridinone according to General Method 22. **255D** (290 mg, 44%) was produced as an oil following silica gel chromatography using hexanes/ethyl acetate as eluant.

### E. N-(3,4-Dimethyl-5-isoxazolyl)-2'-[(2-fluoroethoxy)methyl]-4'-[[(3-methoxy-2,6-dimethyl-4-pyridinyl)oxy]methyl] [1,1'-biphenyl]-2-sulfonamide

**255D** was deprotected according to General Method 7. The crude product was purified by silica gel column chromatography using methanol/dichloromethane as eluant to provide the title compound (180 mg, 75%) as a white solid: mp 90-95 °C; MS m/e 570 (ESI+ mode); HPLC retention time 10.71 min (Method E); HPLC purity >97%.

### Reference Example 256

### N-(3,4-Dimethyl-5-isoxazolyl)-2'-ethoxymethyl-4'-[[4-oxo-2-(3,3,3-trifluoropropyl)-1,3-diazaspiro[4.4]non-1-en-3-yl]methyl] [1,1'-biphenyl]-2-sulfonamide

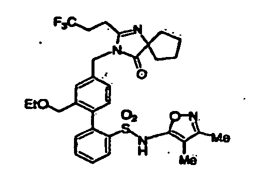

### A. Methyl 1-[(4,4,4-trifluorobutaonyl)amino]cyclopentane-1-carboxylate

A solution of 4,4,4-trifluorobutanoic acid (5.0 g, 35mmol) in dichloromethane (90 ml) was treated at 0 °C with oxalyl chloride (26 ml of a 2.0 M solution in dichloromethane, 52 mmol) and DMF (10 µl). After 20 min the mixture was concentrated to a volume of about 10 ml and 90 ml fresh dichloromethane was added. The mixture was cooled to 0 °C and methyl 1-aminocyclopentane-1-carboxylate hydrochloride (12.6 g, 70 mmol) was added, followed by triethylamine (30 ml, 210 mmol) and DMAP (10 mg). The mixture was stirred at RT for 60 hours. Aqueous sodium bicarbonate solution was added and the mixture was extracted three times with dichloromethane. The combined organic extracts were dried over sodium sulfate and concentrated. The residue was chromatographed on silica gel using 1:1 hexanes/ethyl acetate as eluant to give **256A** (3.1 g, 33%) as an orange oil.

### B. 2-(3,3,3-Trifluoropropyl)-1,3-diazaspiro[4,4]non-1-en-4-one

**256A** (3.1 g, 12 mmol) was treated according to the procedure of Example 22, step B. Evaporation of the crude extract provided **256B** (2.0 g, 71%) as a white solid, which was used without further purification.

### C. N-(3,4-Dimethyl-5-isoxazolyl)-2'-ethoxymethyl-4'-formyl-N-[[(2-trimethylsilyl)ethoxy]methyl] [1,1'-biphenyl]-2-sulfonamide

**252B** (7.6 g, 31 mmol) was subjected to Suzuki coupling with [2-[[(3,4-dimethyl-5-isoxazolyl)[[(2-trimethylsilyl)ethoxy]methyl]amino]-sulfonyl]phenyl]boronic acid according to General Method 1. **256C** (12.3 g, 72%) was obtained as an oil following silica gel chromatography using hexanes/ethyl acetate as eluant.

### D. N-(3,4-Dimethyl-5-isoxazolyl)-2'-ethoxymethyl-4'-hydroxymethyl-N-[[(2-trimethylsilyl)ethoxy]methyl] [1,1'-biphenyl]-2-sulfonamide

**256C** (1.0 g, 1.8 mmol) was reduced with sodium borohydride in ethanol according to General Method 11 to provide **256D** (0.97 g, 95%) as a crude brown oil, which was used without further purification.

### E. N-(3,4-Dimethyl-5-isoxazolyl)-2'-ethoxymethyl-4'-(methanesulfonyloxy)methyl-N-[[(2-trimethylsilyl)ethoxy]methyl] [1,1'-biphenyl]-2-sulfonamide

**256D** (490 mg) was converted to the corresponding mesylate according to General Method 3 to provide **256E** (560 mg) as crude orange oil.

### F. N-(3,4-dimethyl-5-isoxazolyl)-2'-ethoxymethyl-4'-[[4-oxo-2-(3,3,3-trifluoropropyl)-1,3-diazaspiro[4,4]non-1-en-3-yl]methyl]-N-[[(2-trimethylsilyl)ethoxy]methyl] [1,1'-biphenyl]-2-sulfonamide

**256E** (560 mg) was used to alkylate **256B** (270 mg) according to General Method 4. **256F** (570 mg) was produced as a crude brown oil, which was used without further purification.

### G. N-(3,4-Dimethyl-5-isoxazolyl)-2'-ethoxymethyl-4'-[[4-oxo-2-(3,3,3-trifluoropropyl)-1,3-diazaspiro[4,4]non-1-en-3-yl]methyl] [1,1'-biphenyl]-2-sulfonamide

**256F** (570 mg) was deprotected according to General Method 8 (EtOH). The crude product was purified by silica gel column chromatography using 2:1 hexanes/ethyl acetate as eluant to provide the title compound (92 mg, 8% from 256D) as a white solid: MS m/e 633 (ESI+ mode); HPLC retention time 3.96 min (Method C); HPLC purity 97%

### Reference Example 257

### 4'-[(2-Butyl-4-oxo-1,3-diazaspiro[4.4]non-1-en-3-yl)methyl]-N-(3,4-dimethyl-5-isoxazolyl)-2'-ethyl[1,1'-biphenyl]-2-sulfonamide

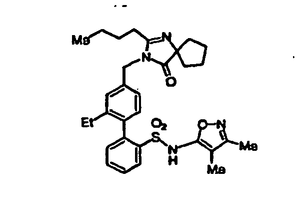

### A. Methyl 4-bromo-3-ethylbenzoate

Methyllithium (57 ml of a 1.4 M solution in ether, 80 mmol) was added dropwise to a suspension of copper(I) iodide (76 g, 40 mmol) in ether (20 ml) at 0 °C. The mixture was stirred for 20 min at 0 °C. Solid **232A** (12.3 g, 40 mmol) was added in portions over 45 min, after which THF (50 ml) was added. The heterogeneous mixture was stirred at 0 °C for 1 h, then aqueous ammonium chloride and aqueous ammonium hydroxide were added. The mixture was extracted with ethyl acetate, and the combined organic layers were concentrated. The residue was taken up in chloroform and partitioned against aqueous ammonium chloride. The chloroform layer was dried over sodium sulfate and concentrated, and the residue was chromatographed on silica gel, eluting with 9:1 hexanes/ethyl acetate, to provide **257A** (3.2 g, 32%) as a yellow oil.

### B. N-(3,4-Dimethyl-5-isoxazolyl)-2'-ethyl-4'-methoxycarbonyl-N-[[(2-trimethylsilyl)ethoxy]methyl] [1,1'-biphenyl]-2-sulfonamide

**257A** (1.4 g, 5.6 mmol) was subjected to Suzuki coupling with [2-[[(3,4-dimethyl-5-isoxazolyl)[[(2-trimethylsilyl)ethoxy]methyl] amino]-sulfonyl]phenyl]boronic acid according to General Method 1. Silica gel chromatography provided **257B** (3.2 g) as an oil, contaminated with byproducts deriving from the boronic acid.

### C. N-(3,4-Dimethyl-5-isoxazolyl)-2'-ethyl-4'-hydroxymethyl-N-[[(2-trimethylsilyl)ethoxy]methyl][1,1'-biphenyl]-2-sulfonamide

**257B** (3.2 g) was treated with DIBAL-H according to the procedure of Example 230, step E, to provide **257C** (4.0 g) as a crude oil.

### D. N-(3,4-Dimethyl-5-isoxazolyl)-2'-ethyl-4'-(methanesulfonyloxy)methyl-N-[[(2-trimethylsilyl)ethoxy]methyl] [1,1'-biphenyl]-2-sulfonamide

**257C** (4.0 g) was converted to the corresponding mesylate according to General Method 3 to provide **257D** (3.7 g) as crude orange oil.

### E. 4'-[[2-Butyl-4-oxo-1,3-diazaspiro[4,4]non-1-en-3-yl]methyl]-N-(3,4-dimethyl-5-isoxazolyl)-2'-ethyl-N-[[(2-trimethylsilyl)ethoxy]methyl] [1,1'-biphenyl]-2-sulfonamide

**257D** (0.92 g) was used to alkylate 2-butyl-1,3-diazaspiro[4.4]non-1-en-4-one according to General Method 4. **257E** (760 mg)' was produced as a colorless oil following silica gel chromatography using 2:1 hexanesi/ethyl acetate as eluant.

### F. 4'-[(2-Butyl-4-oxo-1,3-diazaspiro[4,4]non-1-en-3-yl)methy]-N-(3,4-dimethyl-5-isoxazolyl)-2'-ethyl [1,1'-biphenyl]-2-sulfonamide

**257D** (760 mg) was deprotected according to General Method 8 (EtOH). The crude product was purified by reverse-phase preparative HPLC to provide the title compound (200 mg) as a white solid following lyophilization: MS m/e 563 (ESI+ mode); HPLC retention time 3.69 min (Method C); HPLC purity >98%

### Reference Example 258

### (+/-)-4'-[(2-Butyl-4-oxo-1,3-diazaspiro[4.4]non-1-en-3-yl)methyl]-N-(3,4-dimethyl-5-isoxazolyl)-2'-(1-hydroxyethyl)[1,1'-biphenyl]-2-sulfonamide

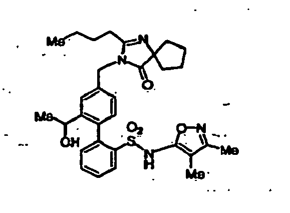

### A. 4'-[(2-Butyl-4-oxo-1-3-diazaspiro[4.4]non-1-en-3-yl)methyl]-N-(3,4-dimethyl-5-isoxazolyl)-2'-formyl-N-[(2-trimethylsilyl)ethoxymethyl] [1,1'-biphenyl]-2-sulfonamide

**23A** (600 mg, 1.0 mmol) was used to alkylate 2-butyl-1,3-diazaspiro[4.4]non-1-en-4-one according to General Method 4. **258A** (510 mg; 74%) was produced as a yellow oil following silica gel chromatography using 1:1 hexanes/ethyl acetate as eluant.

### B. (+/-)-4'-[(2-Butyl-4-oxo-1,3-diazaspiro[4.4]non-1-en-3-yl)methyl]-N-(3,4-dimethyl-5-isoxazolyl)-2'-(1-hydroxyethyl)-N-[(2-trimethylsilyl)ethoxymethyl][1,1'-biphenyl]-2-sulfonamide

A solution of **258A** (260 mg, 0.37 mmol) in dry ether (1 ml) was treated dropwise at -78 °C with methylmagnesium bromide (3.0 M solution in ether, 0.25 ml, 0.74 mmol). The mixture was allowed to warm to 0 °C and was then quenched with aqueous ammonium chloride. The mixture was extracted with ether. The combined organic extracts were dried over magnesium sulfate and concentrated to provide **258B** (210 mg) as a colorless oil, which was used without further purification.

### C. (+/-)-4'-[(2-Butyl-4-oxo-1,3-diazaspiro[4,4]non-1-en-3-yl)methyl]-N-(3,4-dimethyl-5-isoxazolyl)-2'-(1-hydroxyethyl) [1,1'-biphenyl]-2-sulfonamide

**258B** (210 mg) was deprotected according to General Method 8 (EtOH). The crude product was purified by preparative thin-layer silica gel chromatography using 9:1 chloroform/methanol as eluant to provide the title compound (6 mg, 3% from **258A)** as a white solid: MS m/e 579 (ESI+ mode); HPLC retention time 3.07 and 3.40 min (Method A; product appears as two peaks); HPLC purity 92%.

### Reference Example 259

### 4'-[(2-Butyl-4-oxo-1,3-diazaspiro[4.4]non-1-en-3-yl)methyl]-N-(4,5-dimethyl-3-isoxazolyl)-2'-propyl [1,1'-biphenyl]-2-sulfonamide

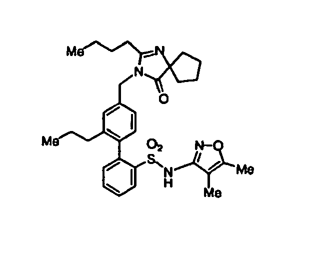

### A. N-(4,5-Dimethyl-3-isoxazolyl)-4'-formyl-N-methoxymethyl-2'-propyl [1,1'-biphenyl]-2-sulfonamide

**27C** (570 mg, 2.5 mmol) was subjected to Suzuki coupling with [2-[[(4,5-dimethyl-3-isoxazolyl)(methoxymethyl)amino]-sulfonyl]phenyl]boronic acid according to General Method 1. **259A** (1.1 g) was obtained as an impure yellow oil following silica gel chromatography using 4:1 hexanes/ethyl acetate.

### B. N-(4,5-Dimethyl-3-isoxazolyl)-4'-hydroxymethyl-N-methoxymethyl-2'-propyl[1,1'-biphenyl]-2-sulfonamide

**259A** (1.1 g) was reduced with sodium borohydride in methanol according to General Method 11. **259B** (520 mg, 47% over two steps) was obtained as an oil following silica gel chromatography with 1:1 hexanes/ethyl acetate as eluant.

### C. 4'-Bromomethyl-N-(4,5-dimethyl-3-isoxazolyl)-N-methoxymethyl-2'-propyl[1,1'-biphenyl]-2-sulfonamide

**259B** (520 mg, 1.2 mmol) was converted to the corresponding bromide according to General Method 2, providing **259C** (425 mg, 71%) as a yellow solid following silica gel chromatography using 9:1 hexanes/ethyl acetate as eluant.

### D. 4'-[(2-Butyl-4-oxo-1,3-diazaspiro[4.4]non-1-en-3-yl)methyl]-N-(4,5-dimethyl-3-isoxazolyl)-N-methoxymethyl-2'-propyl [1,1'-biphenyl]-2-sulfonamide

**259C** (800 mg, 1.6 mmol) was used to alkylate 2-butyl-1,3-diazaspiro[4.4]non-1-en-4-one according to General Method 4. **259D** (720 mg) was obtained as a crude oil, which was used without further purification.

### E. 4'-[(2-Butyl-4-oxo-1,3-diazaspiro[4.4]non-1-en-3-yl)methyl]-N-(4,5-dimethyl-3-isoxazolyl)-2'-propyl [1,1'-biphenyl]-2-sulfonamide

**259D** (720 mg) was deprotected according to General Method 7. The crude product was purified by silica gel column chromatography using 1:1 hexanes/ethyl acetate as eluant to provide the title compound (390 mg, 42% over two steps) as a white solid: mp 64-66 °C; MS m/e 577 (ESI+ mode); HPLC retention time 30.78 min (Method B); HPLC purity >98%.

### Reference Example 260

### N-(4,5-Dimethyl-3-isoxazolyl)-4'-[[(3-methoxy-2,6-dimethyl-4-pyridinyl)oxy]methyl]-2'-propyl [1,1'-biphenyl]-2-sulfonamide

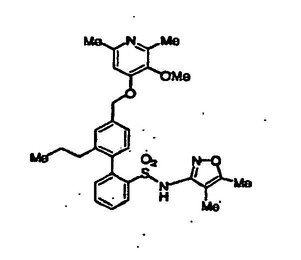

### A. N-(4,5-Dimethyl-3-isoxazolyl)-4'-[[(3-methoxy-2,6-dimethyl-4-pyridinyl)oxy]methyl]-N-methoxymethyl-2'-propyl [1,1'-biphenyl]-2-sulfonamide

**259C** (1.9 g, 3.7 mmol) was used to alkylate 3-methoxy-2,6-dimethyl-4-(1H)-pyridinone according to General Method 22. The crude product was chromatographed on silica gel using 1:1 hexanes/ethyl acetate as eluant to provide **260A** (1.3 g, 62%) as an oil.

### B. N-(4,5-Dimethyl-3-isoxazolyl)-4'-[[(3-methoxy-2,6-dimethyl-4-pyridinyl)oxy]methyl]-2'-propyl [1,1'-biphenyl]-2-sulfonamide

**260A** (1.3 g, 2.2 mmol) was deprotected according to General Method 7. The crude product was purified by silica gel column chromatography using 3% methanol in dichloromethane as eluant, providing the title compound (540 mg, 45%) as a white solid: mp 56-59 °C; MS m/e 536 (ESI+ mode); HPLC retention time 26.73 min (Method B); HPLC purity 97%.

### Reference Example 261

### 1-[[2'-[[(3,4-Dimethyl-5-isoxazolyl)amino]sulfonyl-2-(ethoxymethyl)][1,1'-biphenyl]-4-yl]methyl]-4-ethyl-2-propyl-1H-imidazole-5-carboxamide

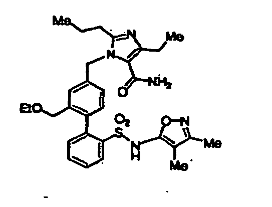

### A. N-(3,4-Dimethyl-5-isoxazolyl)-4'-[(5-ethoxycarbonyl-4-ethyl-2-propylimidazol-1-yl)methyl]-2'-ethoxymethyl-N-[(2-trimethylsilyl)ethoxymethyl] [1,1'-biphenyl]-2-sulfonamide

Ethyl 4-ethyl-2-propylimidazole-5-carboxylate (140 mg, 0.66 mmol) was alkylated with **256E** according to General Method 22. Silica gel chromatography using 1:3 hexanes/ethyl acetate as eluant provided **261A** (120 mg, 25%) as an orange oil.

### B. 4'-[(5-Carboxy-4-ethyl-2 propylimidazol-1-yl)methyl]-N-(3,4-dimethyl-5-isoxazolyl)-2'-ethoxymethyl[1,1'-biphenyl]-2-sulfonamide

**261A** (120 mg, 0.16 mmol) was subjected to sulfonamide deprotection according to General Method 8 (EtOH). The crude residue was dissolved in methanol (2 ml) and was then treated with 45% aqueous potassium hydroxide (2 ml) and heated at 65 °C for 3 h. The mixture was cooled, adjusted to pH 4 by the addition of concentrated hydrochloric acid and aqueous sodium dihydrogen phosphate, and was then extracted with ethyl acetate. The combined organic extracts were dried over sodium sulfate and concentrated to provide **261B** (64 mg) as a crude orange oil.

### C. 1-[[2'-[[(3,4-Dimethyl-5-isoxazolyl)amino]sulfonyl-2-(ethoxymethyl)][1,1'-biphenyl]-4-yl]methyl]-4-ethyl-2-propyl-1H-imidazole-5-carboxamide

**261B** (20 mg) was subjected to amide formation according to General Method 12 using aqueous ammonia as the amine component. The crude material was purified by preparative thin-layer silica gel chromatography using 1:1 hexanes/acetone as eluant to provide the title compound (13 mg) as a white solid: MS m/e 580 (ESI+ mode); HPLC retention time 2.97 min (Method C); HPLC purity 98%.

### Reference Example 262

### 1-[[2'-[[(3,4-Dimethyl-5-isoxazolyl)amino]sulfonyl-2-(ethoxymethyl)][1,1'-biphenyl]-4-yl]methyl]-4-ethyl-N-methyl-2-propyl-1H-imidazole-5-carboxamide

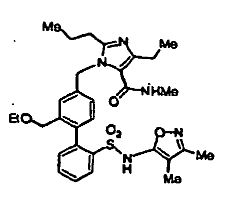

**261B** (44 mg) was subjected to amide formation according to General Method 12 using aqueous methylamine as the amine component. The crude material was purified by preparative thin-layer silica gel chromatography using 1:1 hexanes/acetone as eluant to provide the title compound (21 mg) as an off-white solid: MS m/e 594 (ESI+ mode); HPLC retention time 3.07 min (Method C); HPLC purity 97%.

### Reference Example 263

### 1-[[2'-[[(4,5-Dimethyl-3-isoxazolyl)amino]sulfonyl-2-methyl][1,1'-biphenyl]-4-yl]methyl]-4-ethyl-N-methyl-2-propyl-1H-imidazole-5-carboxamide

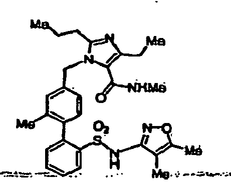

### A. N-(4,5-Dimethyl-3-isoxazolyl)-4'-methoxycarbonyl-N-methoxymethyl-2'-methyl[1,1'-biphenyl]-2-sulfonamide

Methyl 4-bromo-3-methylbenzoate (2.3 g, 10 mmol) was subjected to Suzuki coupling with [2-[[(4,5-dimethyl-3-isoxazolyl)(methoxymethyl)amino]-sulfonyl]phenyl]boronic acid (3.2 g, 6.5 mmol) according to General Method 1. **263A** (3.1 g) was obtained as an impure yellow oil following silica gel chromatography using 3:1 hexanes/ethyl acetate as eluant.

### B. N-(4,5-Dimethyl-3-isoxazolyl)-4'-hydroxymethyl-N-methoxymethyl-2'-methyl[1,1'-biphenyl]-2-sulfonamide

**263A** (3.1 g) was treated with DIBAL-H according to the procedure of Example 230, step E, to provide **263B** (2.7 g) as a crude yellow oil.

### C. 4'-Bromomethyl-N-(4,5-dimethyl-3-isoxazolyl)- N-methoxymethyl-2'-methyl[1,1'-biphenyl]-2-sulfonamide

263B (2.7 g) was converted to the corresponding bromide according to General Method 2, providing **263C** (1.7 g, 55% over 3 steps) as a colorless oil following silica gel choromatography using 5:1 hexanes/ethyl acetate as eluant.

### D. N-(4,5-Dimethyl-3-isoxazolyl)-4'-[(5-ethoxycarbonyl-4-ethyl-2-propylimidazol-1-yl)methyl]-N-methoxymethyl-2'-methyl [1,1'-biphenyl]-2-sulfonamide

**263C** (1.7 g) was used to alkylate ethyl 4-ethyl-2-propylimidazole-5-carboxylate according to General Method 22. Silica gel chromatography using 3:1 hexanes/ethyl acetate as eluant provided **263D** (880 mg, 41%) as an orange oil.

### E. 4'-[(5-Carboxy-4-ethyl-2-propylimidazol-1-yl)methyl]-N-(4,5-dimethyl-3-isoxazolyl)-2'-methyl[1,1'-biphenyl]-2-sulfonamide

**263D** (880 mg) was subjected to the procedure of Example 261, step B, to provide **263E** (870 mg) as a crude yellow solid.

### F. 1-[[2'-[[(4,5-Dimethyl-3-isoxazolyl)amino]sulfonyl-2-methyl][1,1'-biphenyl]-4-yl]methyl]-4-ethyl-N-methyl-2-propyl-1H-imidazole-5-carboxamide

263E (830 mg) was subjected to amide formation according to General Method 12 using aqueous methylamine as the amine component. The crude material was purified by silica gel chromatography using 1:1 hexanes/acetone as eluant to provide the title compound (400 mg) as a white solid following lyophilization: MS m/e 550 (ESI+ mode); HPLC retention time 2.98 min (Method C); HPLC purity >98%.

### Reference Example 264

### N,4-Diethyl-1-[[2'-[[(4,5-dimethyl-3-isoxazolyl)amino]sulfonyl-2-methyl][1,1'-biphenyl]-4-yl]methyl]-2-propyl-1H-imidazole-5-carboxamide

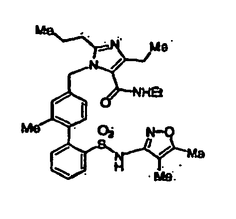

**263E** (20 mg) was subjected to amide formation according to General Method 12 using aqueous ethylamine as the amine component. The crude material was purified by preparative thin-layer silica gel chromatography using 9:1 methanol/chloroform as eluant to provide the title compound (10 mg) as a white solid following lyophilization: MS m/e 564 (ESI+ mode); HPLC retention time 3.10 min (Method C); HPLC purity 97%.

### Reference Example 265

### 1-[[2'-[[(3,4-Dimethyl-5-isoxazolyl)amino]sulfonyl-2-ethyl][1,1'-biphenyl]-4-yl]methyl]-4-ethyl-2-propyl-1H-imidazole-5-carboxamide

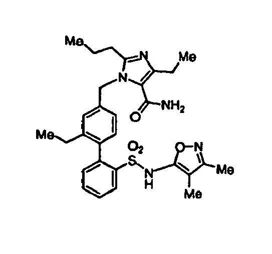

### A. N-(3,4-Dimethyl-5-isoxazolyl)-4'-[(5-ethoxycarbonyl-4-ethyl-2-propyl-imidazol-1-yl)methyl]-2'-ethyl-N-[(2-trimethylsilyl)ethoxymethyl] [1,1'-biphenyl]-2-sulfonamide

Ethyl 4-ethyl-2-propylimidazole-5-carboxylate (600 mg, 2.9 mmol) was alkylated with **257D** (1.76 g) according to General Method 22. Silica gel chromatography using 1:1 hexanes/ethyl acetate as eluant provided impure **265A** (785 mg) as an orange oil.

### B. 4'-[(5-Carboxy-4-ethyl-2-propylimidazol-1-yl)methyl]-N-(3,4-dimethyl-5-isoxazolyl)-2'-ethyl [1,1'-biphenyl]-2-sulfonamide

**265A** (785 mg) was subjected to the procedure of Example 261, step B. Purification of the crude material by preparative reverse-phase HPLC provided **265B** (240 mg) as yellow oil.

### C. 1-[[2'-[[(3,4-Dimethyl-5-isoxazolyl)amino]sulfonyl-2-ethyl][1,1'-biphenyl]-4-yl]methyl]-4-ethyl-2-prolyl-1H-imidazole-5-carboxamide

**265B** (240 mg) was subjected to amide formation according to General Method 12 using aqueous ammonia as the amine component. The crude material was purified by silica gel chromatography using 1:2 hexanes/acetone as eluant to provide the title compound (110 mg) as a white solid following lyophilization: MS m/e 550 (ESI+ mode); HPLC retention time 3.05 min (Method C); HPLC purity >98%.

### Reference Example 266

### 1-[[2'-[[(4,5-Dimethyl-3-isoxazolyl]amino]sulfonyl-2-methyl][1,1'-biphenyl]-4-yl]methyl]-4-ethyl-N-(1-methylethyl)-2-propyl-1H-imidazole-5-carboxamide

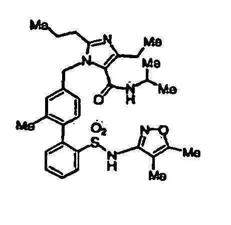

**263E** (150 mg) was subjected to amide formation according to General Method 12 using isopropylamine as the amine component. The crude material was chromatographed on silica gel using 1:1 hexanes/acetone as eluant to provide the title compound (27 mg) as a white solid: MS m/e 578 (ESI+ mode); HPLC retention time 3.21 min (Method C); HPLC purity 96%.

### Reference Example 267

### 1-[[2'-[[(3,4-Dimethyl-5-isoxazolyl)amino]sulfonyl-2-(2-fluoroethoxymethyl)][1,1'-biphenyl]-4-yl]methyl]-4-ethyl-2-propyl-1H-imidazole-5-carboxamide

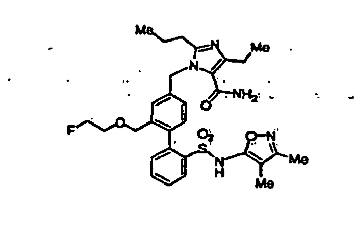

### A. N-(3,4-Dimethyl-5-isoxazolyl)-4'-[(5-ethoxycarbonyl-4-ethyl-2-propyl-imidazol-1-yl)methyl]-2'-(2-fluoroethoxymethyl)-N-[(2-trimethylsilyl)ethoxymethyl] [1,1'-biphenyl]-2-sulfonamide

**255C** (660 mg, 1.1 mmol) was used to alkylate ethyl 4-ethyl-2-propylimidazole-5-carboxylate according to General Method 22. Silica gel chromatography using 7:3 hexaneslethyl acetate as eluant provided **267A** (460 mg) as a yellow oil.

### B. 4'-[(5-Carboxy-4-ethyl-2-propylimidazol-1-yl)methyl]-N-(3,4-dimethyl-5-isoxazolyl)-2'-(2-fluoroethoxymethyl) [1,1'-biphenyl]-2-sulfonamide

**267A** (460 mg) was subjected to the procedure of Example 261, step B. **267B** (360 mg) was obtained as a crude yellow oil, which was used without further purification.

### C. 1-[[2'-[[(3,4-Dimethyl-5-isoxazolyl)amino]sulfonyl-2-(2-fluoroethoxymethyl)][1,1-biphenyl]-4-yl]methyl]-4-ethyl-2-propyl-1H-imidazole-5-carboxamide

267B (360 mg) was subjected to amide formation according to General Method 12 using aqueous ammonia as the amine component. The crude material was purified by silica gel chromatography using 1:2 hexanes/acetone as eluant to provide the title compound (180 mg) as a white solid following lyophilization: MS m/e 598 (ESI+ mode); HPLC retention time 2.83 min (Method C); HPLC purity >98%.

### Reference Example 268

### N-(4,5-Dimethyl-3-isoxazolyl)-2'-ethoxymethyl-4'-[[(6-ethyl-3-methoxy-2-methyl-4-pyridinyl)oxy]methyl] [1,1'-biphenyl]-2-sulfonamide

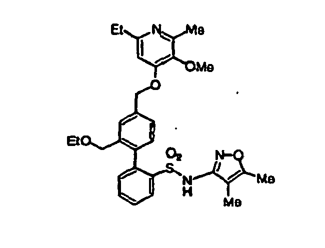

### A. N-(4,5-Dimethyl-3-isoxazolyl)-2'-ethoxymethyl-4'-[[(6-ethyl-3-methoxy-2-methyl-4-pyridinyl)oxy]methyl]-N-[(2-methoxyethoxy)methyl] [1,1'-biphenyl]-2-sulfonamide

**252E** (200 mg, 0.38 mmol) was used to alkylate 6-ethyl-3-methoxy-2-methyl-4-(1H)-pyridinone (prepared according to Katano, K; et. al. *Meiji Seika Kenkyu Nenpo,* **1996**, *35*, 62-65) following General Method 22. **268A** (155 mg, 70%) was produced as an oil following silica gel chromatography using 50:50:0.2 hexanes/ethyl acetate/triethylamine as eluant.

### B. N-(4,5-Dimethyl-3-isoxazolyl)-2'-ethoxymethyl-4'-[[(6-ethyl-3-methoxy-2-methyl-4-pyridinyl)oxy]methyl] [1,1'-biphenyl]-2-sulfonamide

**268A** was deprotected according to General Method 7. The crude product was purified by silica gel column chromatography using 1:30 methanol/dichloromethane as eluant, providing the title compound (108 mg, 74%) as a light yellow solid: mp 64-72 °C; MS m/e 566 (ESI+ mode); HPLC retention time 13.87 min (Method E); HPLC purity 95%.

### Reference Example 269

### N2-[[2'-[[(4,5-Dimethyl-3-isoxazolyl)amino]sulfonyl][1,1'-biphenyl]-4-yl]methyl]-N-methyl-N2-(1-oxobutyl)-L-valinamide

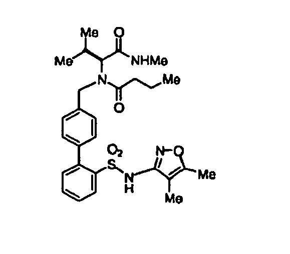

### A. N-(4,5-Dimethyl-3-isoxazolyl)-N-[[(2-trimethylsilyl)ethoxy]methyl]-2-bromobenzenesulfonamide

SEM-Cl (6.4 ml, 36 mmol) was added at 0 °C to a mixture of N-(4,5-dimethyl-3-isoxazolyl)-2-bromobenzenesulfonamide (11 g, 34 mmol), potassium carbonate (9.4 g, 68 mmol), and DMF (100 ml). The mixture was allowed to warm to rt and was stirred for 18 h. The solvent was evaporated, water (200 mL) was added, and the mixture was extracted with ethyl acetate. The crude extract was dried over sodium sulfate and concentrated, and the residue was chromatographed on silica gel using 4:1 hexanes/ethyl acetate as eluant to provide **269A** (11 g, 70%) as an oil.

### B. N-(4,5-Dimethyl-3-isoxazolyl)-4'-formyl-N-[[(2-trimethylsilyl)ethoxy]methyl] [1,1'-biphenyl]-2-sulfonamide

**269A** (2.3 g, 5.0 mmol) was subjected to Suzuki coupling with 4-formylphenylboronic acid according to General Method 1. **269B** (2.0 g, 83 %) was obtained as yellow crystalline solid following silica gel chromatography using 4:1 hexanes /ethyl acetate as eluant.

### C. N2-[[2'-[[N-(4,5-Dimethyl-3-isoxazolyl)-N-[[(2-trimethylsilyl)ethoxy]methyl]amino] sulfonyl] [1,1'-biphenyl]-4-yl]methyl]-N-methyl-L-valinamide

**269B** (490 mg, 1.0 mmol) and L-valine N-methylamide were subjected to reductive amination according to General Method 5. Crude **269C** ( 780 mg) was produced as an oil.

### D. N2-[[2'-[[N-(4,5-Dimethyl-3-isoxazolyl)-N-[[(2-trimethylsilyl)ethoxy]methyl]amino]sulfonyl][1,1-biphenyl]-4-yl]methyl]-N-methyl-N2-(1-oxobutyl)-L-valinamide

**269C** (750 mg) was acylated with butanoyl chloride according to General Method 6 -to provide crude **269D** (680 mg) as an oil.

### E. N2-[[2'-[[(4,5-Dimethyl-3-isoxazolyl)amino]sulfonyl][1.1'-biphenyl]-4-yl]methyl]-N-methyl-N2-(1-oxobutyl)-L-valinamide

**269D** (680 mg) was deprotected according to General Method 10. Silica gel chromatography of the crude residue using dichloromethane/ methanol as eluant provided the title compound (360 mg) as a white amorphous solid: MS m/e 541 (ESI+ mode); HPLC retention time 3.61 min (Method C); HPLC purity 95%.

### Reference Example 270

### N2-[[2'-[[(4,5-Dimethyl-3-isoxazolyl)amino]sulfonyl][1,1'-biphenyl]-4-yl]methyl]-N,N-dimethyl-N2-(1-oxopentyl)-L-valinamide

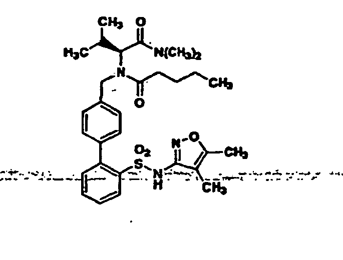

### A. N2-[[2'-[[N-(4,5-Dimethyl-3-isoxazolyl)-N-[[(2-trimethylsilyl)ethoxy]methyl]amino]sulfonyl][1,1'-biphenyl]-4-yl]methyl]-N,N-dimethyl-L-valinamide

**269B** (490 mg, 1.0 mmol) and L-valine N,N-dimethylamide were subjected to reductive animation according to General Method 5. Crude **270A** (780 mg) was produced as an oil.

### B. N2-[[2'-[[N-(4,5-Dimethyl-3-isoxazolyl)-N-[[(2-trimethylsilyl)ethoxy]methyl]amino]sulfonyl][1,1'-biphenyl-4-yl]methyl]-N,N-dimethyl-N2-(1-oxopentyl)-L-valinamide

**270A** (750 mg) was acylated with pentanoyl chloride according to General Method 6 to provide crude **270B** (710 mg) as an oil.

### C. N2-[[2'-[[(4,5-Dimethyl-3-isoxazolyl)amino]sulfonyl][1,1'-biphenyl]-4-yl]methyl]-N,N-dimethyl-N2-(1-oxopentyl)-L-valinamide

**270B** (710 mg) was deprotected according to General Method 10. Silica gel chromatography of the crude residue using dichloromethane/ methanol as eluant provided the title compound (400 mg) as a white amorphous solid: MS m/e 569 (ESI+ mode); HPLC retention time 3.82 min (Method C); HPLC purity 95%.

### Reference Example 271

### 4'-[(2-Butyl-4-oxo-1,3-diazaspiro[4.4]non-1-en-3-yl)methyl]-N-(4,5-dimethyl-3-isoxazolyl)-2'-ethyl[1,1'-biphenyl]-2-sulfonamide

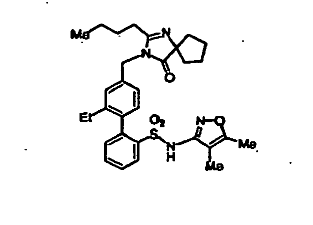

### A. N-(4,5-Dimethyl-3-isoxazolyl)-2'-ethyl-4'-methoxycarbonyl-N-(methoxymethyl) [1,1'-biphenyl]-2-sulfonamide

**257A** (5.9 g, 24 mmol) was subjected to Suzuki coupling with 2-[[N-(4,5-dimethyl-3-isoxazolyl)-N-(methoxymethyl)amino]sulfonyl]phenylboronic acid according to General Method 1. Silica gel chromatography provided **271A** (6.0 g) as an oil, contaminated with byproducts deriving from the boronic acid. **257A** (4.5 g) was also recovered. A single recycling of the recovered **257A** yielded 6.6 g of the **271A** product mix (12.6 g total combined yield).

### B. N-(4,5-Dimethyl-3-isoxazolyl)-2'-ethyl-4'-hydroxymethyl-N-(methoxymethyl) [1,1'-biphenyl]-2-sulfonamide

**271A** (11.1 g) was treated with DIBAL-H according to the procedure of Example 230, step E, to provide **271B** (6.5 g) as a crude oil.

### C. 4'-Bromomethyl-N-(4,5-dimethyl-3-isoxazolyl)-2'-ethyl-N-(methoxymethyl) [1,1'-biphenyl]-2-sulfonamide

**271B** (6.5 g) was converted to the corresponding bromide according to General Method 2 to provide **271C** (4.2 g) as an orange oil following silica gel chromatography using 3:1 hexanes/ethyl acetate as eluant.

### D. 4'-[[2-Butyl-4-oxo-1,3-diazaspiro[4.4]non-1-en-3-yl]methyl]-N-(4,5-dimethyl-3-isoxazolyl)-2'-ethyl-N-(methoxymethyl) [1,1'-biphenyl]-2-sulfonamide

**271C** (2.0 g) was used to alkylate 2-butyl-1,3-diazaspiro[4.4]non-1-en-4-one according to General Method 4. **271D** (1.6 g) was produced as a colorless oil following silica gel chromatography using 1:1 hexanes/ethyl acetate as eluant.

### E. 4'-[(2-Butyl-4-oxo-1,3-diazaspiro[4,4]non-1-en-3-yl)methyl]-N-(4,5-dimethyl-3-isoxazolyl)-2'-ethyl[1,1'-biphenyl]-2-sulfonamide

**271D** (1.6 g) was deprotected according to General Method 8 (EtOH). The crude product was purified by silica gel chromatography using 1:1 hexanes/ethyl acetate as eluant to provide the title compound (815 mg) as a white amorphous solid: MS m/e 563 (ESI+ mode); HPLC retention time 2.18 min (Method H); HPLC purity >98%.

### Reference Example 272

### 4'-[(5-Acetyl-4-ethyl-2-propylimidazol-1-yl)methyl]-N-(4,5-dimethyl-3-isoxazolyl)-2'-methyl[1,1'-biphenyl]-2-sulfonamide

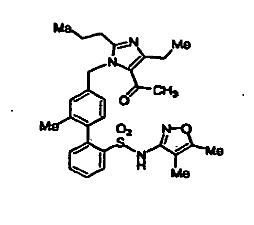

### A. 5-Acetyl-4-ethyl-2-propylimidazole

4-Ethyl-5-formyl-2-propylimidazole (4 g, 24 mmol) was subjected to the procedure of Example 38, steps A and B. Silica gel chromatography using 1:3 hexanes/ethyl acetate as eluant provided **272A** (2.4 g, 55% over two steps) as a brown oil.

### B. 4'-[(5-Acetvl-4-ethyl-2-propylimidazol-1-yl)methyl]-N-(4,5-dimethyl-3-isoxazolyl)-N-methoxymethyl-2'-methyl[1.1'-biphenyl]-2-sulfonamide

**272A** (800 mg, 4.4 mmol) was alkylated with 263C according to General Method 22. Silica gel chromatography using 1:3 hexanes/ethyl acetate as eluant provided **272B** (990 mg, 38%) as an orange oil.

### C. 4'-[(5-Acetyl-4-ethyl-2-propylimidazol-1-yl)methyl]-N-(4,5-dimethyl-3-isoxazolyl)-2'-methyl[1,1'-biphenyl]-2-sulfonamide

**272B** (940 mg; 1.6mmol) was subjected to sulfonamide deprotection according to General Method 8 (water). The crude material was purified by reverse-phase preparative HPLC followed by preparative thin-layer silica gel chromatography (chloroform/methanol eluant) to provide the title compound (9 mg) as a white solid following lyophilization: MS m/e 535 (ESI+ mode); HPLC retention time 1.98 min (Method H); HPLC purity 98%.

### Reference Example 273

### N-(4,5-Dimethyl-3-isoxazolyl)-2'-ethyl-4'-[[(3-methoxy-2,6-dimethyl-4-pyridinyl)oxy]methyl] [1,1'-biphenyl]-2-sulfonamide

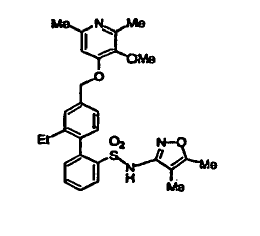

### A. N-(4,5-Dimethyl-isoxazolyl)-2'-ethyl-4'-[[(3-methoxy-2,6-dimethyl-4-pyridinyl)oxy]methyl]-N-(methoxymethyl) [1,1'-biphenyl]-2-sulfonamide

**271C** (2.0 g, 4.0 mmol) was used to alkylate 3-methoxy-2,6-dimethyl-4-(1H)-pyridinone according to General Method 22. The crude product was purified by silica gel chromatography using 1:1 hexanes/ethyl acetate as eluant to provide **273A** (1.2 g, 52%) as an orange oil.

### B. N-(4,5-Dimethyl-3-isoxazolyl)-2'-ethyl-4'-[[(3-methoxy-2,6-dimethyl-4-pyridinyl)oxy]methyl] [1,1-biphenyl]-2-sulfonamide

**273A** (1.2 g, 2.2 mmol) was deprotected according to General Method 8 (EtOH). The crude product was crystallized from methanol/dichloromethane to provide the title compound (172 mg) as a white solid: MS m/e 522 (ESI+ mode); HPLC retention time 1.53 min (Method H); HPLC purity 95%.

### Reference Example 274

### N-(4,5-Dimethyl-3-isoxazolyl)-4'-[[(6-ethyl-3-methoxy-2-methyl-4-pyridinyl)oxy]methyl]-2'-propyl[1,1'-biphenyl]-2-sulfonamide

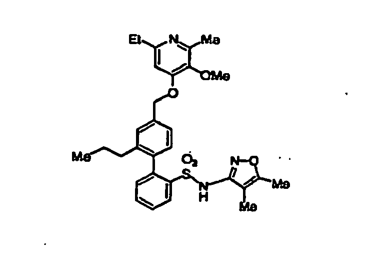

### A. N-(4,5-Dimethyl-3-isoxazolyl)-4'-[[(6-ethyl-3-methoxy-2-methyl-4-pyridinyl)oxy]methyl]-N-methoxymethyl-2'-propyl [1,1'-biphenyl]-2-sulfonamide

**259C** (1.97 g) was used to alkylate 6-ethyl-3-methoxy-2-methyl-4-(1H)-pyridinone (500 mg, prepared according to Katano, K.; et. al. *Meiji Seika Kenkyu Nenpo*, **1996**, *35,* 62-65) following General Method 22. **274A** (1.23 g, 76%) was produced as an oil following silica gel chromatography using hexanes/ethyl acetate/triethylamine as eluant.

### B. N-(4,5-Dimethyl-3-isoxazolyl)-4'-[[(6-ethyl-3-methoxy-2-methyl-4-pyridinyl)oxy]methyl]-2'-propyl [1,1'-biphenyl]-2-sulfonamide

**274A** (1.23 g) was deprotected according to General Method 7. The crude product was purified by silica gel column chromatography using methanol/dichloromethane as eluant providing the title compound (820 mg, 72%) as a white solid: mp 63-65 °C; MS m/e 550 (ESI+ mode); HPLC retention time 3.04 min (Method A); HPLC purity >98%.

### Reference Example 275

### 4'-[(2-Butyl-4-oxo-1,3-diazaspiro[4.4]non-1-en-3-yl)methyl]-N-(4,5-dimethyl-3-isoxazolyl)-2'-(ethoxymethyl)[1,1'-biphenyl]-2-sulfonamide [crystalline]

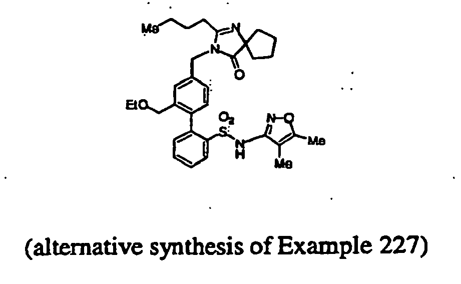

### A. Ethyl 4-bromo-3-(bromomethyl)benzoate

Ethyl 4-bromo-3-methylbenzoate (110 g, 450 mmol) was treated with NBS according to the procedure of **P2A**. Silica gel chromatography with hexanes/ethyl acetate as eluant provided **275A** (91 g, 62%) as a white solid.

### B. Ethyl 4-bromo-3-(ethoxymethyl)benzoate

A solution of **275A** (89 g, 280 mmol) in a mixture of ethanol (300 ml) and DMF (50 ml) was treated at 0 °C with sodium ethoxide (135 ml of a 21% solution in ethanol). The mixture was allowed to warm to rt and was stirred for 16 h. The ethanol was evaporated under reduced pressure. Ethyl acetate was added to the residue and the mixture was washed with water and brine. The organic layer was dried over sodium sulfate and concentrated, and the residue was chromatographed on silica gel using hexanes/ethyl acetate as eluant to provide **275B** (67 g, 84%) as a slightly yellow oil.

### C. N-(4,5-Dimethyl-3-isoxazolyl)-4'-(ethoxycarbonyl)-2'-(ethoxymethyl)-N-(methoxymethyl)[1,1'-biphenyl]-2-sulfonamide

275B (32 g, 100 mmol) was subjected to Suzuki coupling with 2-[[N-(4,5-dimethyl-3-isoxazolyl)-N-(methoxymethyl)amino]sulfonyl]-phenylboronic acid according to General Method 1. Silica gel chromatography using hexanes/ethyl acetate as eluant provided **275C** (52 g) as a yellow oil, contaminated with byproducts deriving from the boronic acid.

### D. N-(4,5-Dimethyl-3-isoxazolyl)-2'-(ethoxymethyl)-4'-(hydroxymethyl)-N-(methoxymethyl) [1,1'-biphenyl]-2-sulfonamide

**275C** (entire sample) was treated with DIBAL-H according to the procedure of Example 230, step E, with the following difference: Workup was by addition of saturated aqueous ammonium chloride to the cooled reaction mixture, followed by extraction with ethyl acetate. The combined organic layers were dried over sodium sulfate and concentrated to provide **275D** as a crude yellow oil.

### E. 4'-(Bromomethyl)-N-(4,5-dimethyl-3-isoxazolyl)-2'-(ethoxymethyl)-N-(methoxymethyl) [1.1'-biphenyl]-2-sulfonamide

**275D** (entire sample) was converted to the corresponding bromide according to General Method 2. Silica gel chromatography using hexanes/ethyl acetate as eluant provided **275E** (38 g, purity estimated to be 83% by ¹H NMR) as a light yellow oil.

### F. 4'-[(2-Butyl-4-oxo-1,3-diazaspiro [4.4]non-1-en-3-yl)methyl]-N-(4,5-dimethyl-3-isoxazolyl)-2'-(ethoxymethyl)-N-(methoxymethyl) [1,1-biphenyl]-2-sulfonamide

**275E** (entire sample) was used to alkylate 2-butyl-1,3-diazaspiro[4.4]non-1-en-4-one according to General Method 4. The crude residue was chromatographed on silica gel using hexanes/ethyl acetate/triethylamine as eluant to provide **275F** (32 g, 53% from **275B)** as a slightly yellow oil.

### G. 4'-[(2-Butyl-4-oxo-1,3-diazaspiro[4.4]non-1-en-3-yl)methyl]-N-(4,5-dimethyl-3-isoxazolyl)-2'-(ethoxymethyl) [1,1'-biphenyl]-2-sulfonamide

**275F** (32 g, 53 mmol) was deprotected according to General Method 7. The crude product was purified by silica gel chromatography using hexanes/ethyl acetate/acetic acid as eluant to provide the title compound (26 g, 88%) as an amorphous foam: MS m/e 593 (ESI+ mode); HPLC retention time 18.75 min (Method E); HPLC purity >96%.

### H. 4'-[(2-Butyl-4-oxo-1,3-diazaspiro[4.4]non-1-en-3-yl)methyl]-N-(4,5-dimethyl-3-isoxazolyl)-2'-(ethoxymethyl)[1,1'-biphenyl]-2-sulfonamide

### (crystallization)

The amorphous **275F** (1 g) was dissolved in 5 mL of isopropanol and 5 mL of water was added to the mixture dropwise and the mixture was warmed up to 40°C to provide a clear solution. The solution was let stand at room temperature and the white crystals thus obtained was filtered and washed with a small amount of 2:1 mixture of isopropanol / water and dried to give 0.87 g of a white crystalline solid. mp. 148°C.

### EXAMPLE 276

### 4'-[(2-Butyl-4-oxo-1,3-diazaspiro[4.4]non-1-en-3-yl)methyl]-3'-chloro-N-(4,5-dimethyl-3-isoxazolyl)-5-methoxy[1.1'-biphenyl]-2-sulfonamide

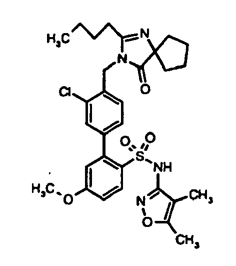

### A. (2-Butyl-4-oxo-1,3-diaspiro[4.4]non-1-en-3-yl)methyl-2-chloro-4-bromobenzene.

α-Bromomethyl-4-bromo-2-chlorobenzene (1.6gm) was used to alkylate 2-butyl-1,3-diaspiro[4.4]non-1-en-4-one (1.2gm) according to General Method 4 to yield **276A** (2.2gm, 98%) as a solid. MS m/e 399.13.

### B. 4'-[(2-Butyl-4-oxo-1,3-diazaspiro[4.4]non-1-en-3-yl)methyl]-3'-chloro-N-[(2-methoxyethoxy)methyl]-N-(4,5-dimethyl-3-isoxazolyl)[1,1'-biphenyl]-2-sulfonamide.

**276A** (218 mg) was subjected to Suzuki coupling with {2-[[4,5-dimethyl-3-isoxazolyl){2-methoxyethoxy)methyl]amino]-sulfonyl]-5-methoxyphenyl]boronic acid (270 mg)according to General Method 1 to provide **276B** (280 mg, 67%) as an oil.

### C. Title Compound

**276B** (280 mg) was deprotected according to General Method 8 to provide the title compound (178 mg, 98%) as a white amorphous solid: MS m/e 583.

### Examples 277-297

The compounds of Examples 277-297 were prepared in a manner similar to that described in Example 276. The reported HPLC retention times were obtained under the following conditions.
Column: YMC S5 ODS 4.6X50 mm (4min); Wavelength: 220nm;
Solvent: Gradient elution 10%MeOH--90% MeOH in water
(0.2% H3PO4).

| **EXAMPLE** | **SRUCTURE** | **NAME** | **M/z (MH)**^{**+**} | **HPLC ret time, min** |
|---|---|---|---|---|
| **277** | 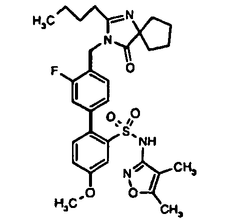 | 4'-[(2-Butyl-4-oxo-1,3-diazaspiro[4.4]non-1-en-3-yl)methyl]-N-(4,5-dimethyl-3-isoxazolyl)-3'-fluoro-4-methoxy[1,1'-biphenyl]-2-sulfonamide | 583.3 | 3.2min |
| **278** | 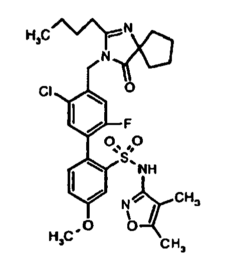 | 4'-[(2-Butyl-4-oxo-1,3-diazaspiro[4.4]non-1-en-3-yl)methyl]-5'-chloro-N-(4,5-dimethyl-3-isoxazolyl)-2'-fluoro-4-methoxy[1,1'-biphenyl]-2-sulfonamide | 617.3 | 3.3min |
| **279** | 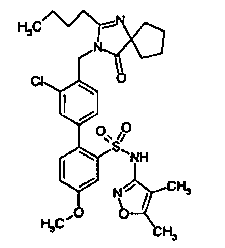 | 4'-[(2-Butyl-4-oxo-1,3-diazaspiro[4,4]non-1-en-3-yl)methyl]-3'-chloro-N-(4,5-dimethyl-3-isoxazolyl)-4-methoxy[1,1'-biphenyl]-2-sulfonamide | 599.2 | 3.4min |
| **280** | 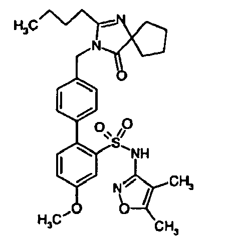 | 4'-[(2-Butyl-4-oxo-1,3-diazaspiro[4.4]non-1-en-3-yl)methyl]-N-(4,5-dimethyl-3-isoxazolyl)-4-methoxy[1,1'-biphenyl]-2-sulfonamide | 565.3 | 3.0min |
| **281** | 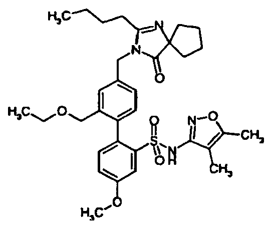 | 4'-[(2-Butyl-4-oxo-1,3-diazaspiro[4.4]non-1-en-3-yl)methyl]-N-(4,5-dimethyl-3-isoxazolyl)-2'-(ethoxymethyl)-4-methoxy[1,1'-biphenyl]-2-sulfonamide | 623.51 | 3.11min |
| **282** | 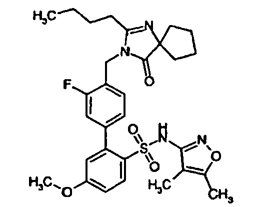 | 4'-[(2-Butyl-4-oxo-1,3-diazaspiro[4.4]non-1-en-3-yl)methyl]-N-(4,5-dimethyl-3-isoxazolyl)-3'-fluoro-5-methoxy[1,1'-biphenyl]-2-sulfonamide | 583 | 3.1min |
| **283** | 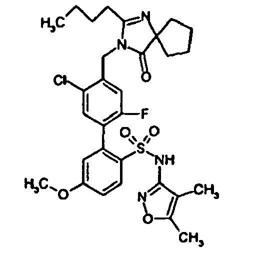 | 4'-[(2-Butyl-4-oxo-1,3-diazaspiro[4.4]non-1-en-3-yl)methyl]-5'-chloro-N-(4,5-dimethyl-3-isoxazolyl)-2'-fluoro-5-methoxy [1,1'-biphenyl]-2-sulfonamide | 617.5 | 3.3min |
| **284** | 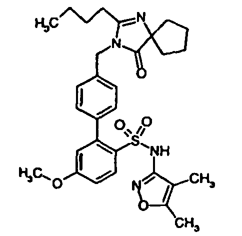 | 4'-[(2-Butyl-4-oxo-1,3-diazaspiro[4.4]non-1-en-3-yl)methyl]-N-(4,5-dimethyl-3-isoxazolyl)-5-methoxy[1,1'-biphenyl]-2-sulfonamide | 565.3 | 3.0min |
| **285** | 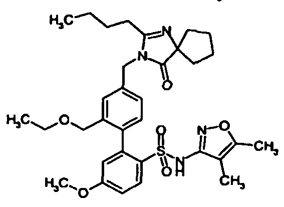 | 4'-[(2-Butyl-4-oxo-1,3-diazaspiro[4.4]non-1-en-3-yl)methyl]-N-(4,5-dimethyl-3-isoxazolyl)-2'-(ethoxymethyl)-5-methoxy[1,1'-biphenyl]-2-sulfonamide | 623.6 | 3.03min |
| **286** | 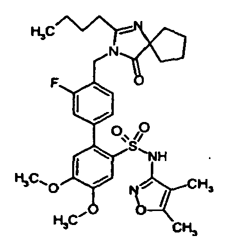 | 4'-[(2-Butyl-4-oxo-1,3-diazaspiro[4.4]non-1-en-3-yl)methyl]-N-(4,5-dimethyl-3-isoxazolyl)-3'-fluoro-4,5-dimethoxy[1,1'-biphenyl]-2-sulfonamide | 613.4 | 3.04min |
| **287** | 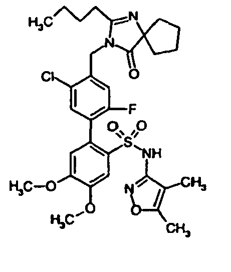 | 4'-[(2-Butyl-4-oxo-1,3-diazaspiro[4.4]non-1-en-3-yl)methyl]-5'-chloro-N-(4,5-dimethyl-3-isoxazolyl)-2'-fluoro-4,5-dimethoxy[1,1'-biphenyl]-2-sulfonamide | 647.2 | 3.2min |
| **288** | 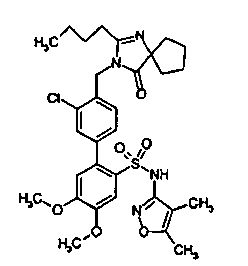 | 4'-[(2-Butyl-4-oxo-1,3-diazaspiro[4.4]non-1-en-3-yl)methyl]-3'-chloro-N-(4,5-dimethyl-3-isoxazolyl)-4,5-dimethoxy[1,1'-biphenyl]-2-sulfonamide | 629.2 | 3.2min |
| **289** | 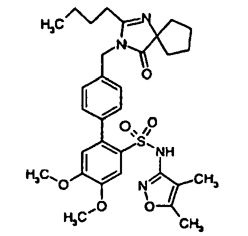 | 4'-[(2-Butyl-4-oxo-1,3-diazaspiro[4.4]non-1-en-3-yl)methyl]-N-(4,5-dimethyl-3-isoxazolyl)-4,5-dimethoxy [1,1'-biphenyl]-2-sulfonamide | 595.3 | 2.8min |
| **290** | 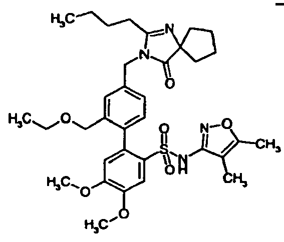 | 4'-[(2-Butyl-4-oxo-1,3-diazaspiro[4.4]non-1-en-3-yl)methyl]-N-(4,5-dimethyl-3-isoxazolyl)-2'-(ethoxymethyl)-4,5-dimethoxy[1,1'-biphenyl]-2-sulfonamide | 653.48 | 2.89min |
| **291** | 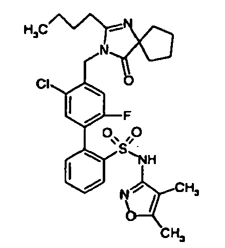 | 4'-[(2-Butyl-4-oxo-1,3-diazaspiro[4.4]non-1-en-3-yl)methyl]-5'-chloro-N-(4,5-dimethyl-3-isoxazolyl)-2'-fluoro[1,1'-biphenyl]-2-sulfonamide | 587.2 | 3.3min |
| **292** | 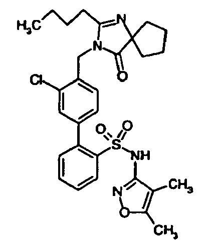 | 4'-[(2-Butyl-4-oxo-1,3-diazaspiro[4.4]non-1-en-3-yl)methyl]-3'-chloro-N-(4,5-dimethyl-3-isoxazolyl)[1,1'-biphenyl]-2-sulfonamide | 569.2 | 3.2min |
| **293** | 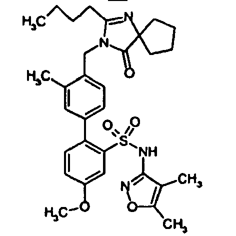 | 4'-[(2-Butyl-4-oxo-1,3-diazaspiro[4.4]non-1-en-3-yl)methyl]-N-(4,5-dimethyl-3-isoxazolyl)-4-methoxy-3'-methyl[1,1'-biphenyl]-2-sulfonamide | 579.4 | 3.2min |
| **294** | 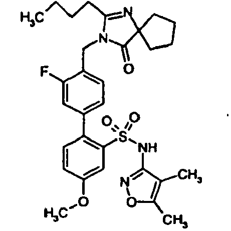 | 4'-[(2-Butyl-4-oxo-1,3-diazaspiro[4.4]non-1-en-3-yl)methyl]-N-(4,5-dimethyl-3-isoxazolyl)-3'-fluoro-4-methoxy[1,1'-biphenyl]-2-sulfonamide | 553.1 | 3.0min |
| **295** | 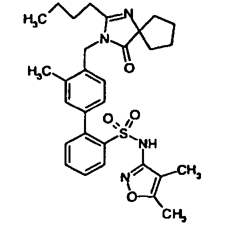 | 4'-[(2-Butyl-4-oxo-1,3-diazaspiro[4.4]non-1-en-3-yl)methyl]-N-(4,5-dimethyl-3-isoxazolyl)-3'-methyl[1,1'-biphenyl]-2-sulfonamide | 549.2 | 3.01min |
| **296** | 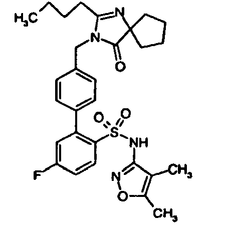 | 4'-[(2-Butyl-4-oxo-1,3-diazaspiro[4.4]non-1-en-3-yl)methyl]-N-(4,5-dimethyl-3-isoxazolyl)-5-fluoro[1,1'-biphenyl]-2-sulfonamide | 553.23 | 2.90min |

### Examples 297-309

The compounds of Examples 297-309 were prepared in a manner similar to that described in Example 269. The reported HPLC retention times were obtained under the following conditions.
Column: YMC S5 ODS 4.6X50 mm (4min); Wavelength: 220nm;
Solvent: Gradient elution 10%MeOH--90% MeOH in water
(0.2% H3PO4).

| **EXAMPLE** | **STRUCTURE** | **NAME** | M/z (MH)⁺ | **HPLC ret time, min** |
|---|---|---|---|---|
| **297 *** | 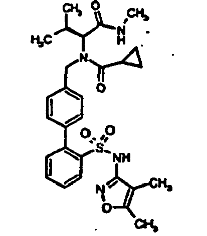 | N²-(Cyclopropylcarbonyl)-N²-[[2'-[[(4,5-dimethyl-3-isosazolyl)amino]sulfonyl] [1,1' -biphenyl]-4-yl]methyl]-N-methyl-L-valinamide | 539.17 | 3.08min |
| **298 *** | 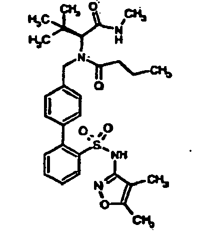 | N²-[[2'-[[(4,5-Dimethyl-3-isoxazolyl)amino]sulfonyl] [1,1' -biphenyl]-4-yl]methyl]-N,3-dimethyl-N²-(1-oxobutyl)-L-valinamide | 555.24 | 3.40min |
| **299 *** | 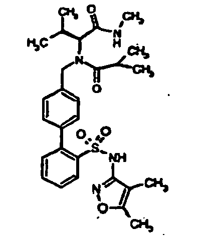 | N²-[[2'-[[(4,5-Dimethyl-3-isoxazolyl)amino]sulfonyl][1,1' -biphenyl]-4-yl]methyl]-N-methyl-N²-(2-methyl-1-oxopropyl)-L-valinamide | 541.29 | 3.17min |
| **300 *** | 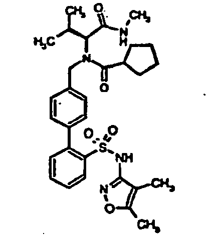 | N²-(Cyclopentylcarbonyl)-N²-[[2'-[[(4,5-dimethyl-3-isoxazolyl)amino]sulfonyl][1,1' -biphenyl]-4-yl]methyl]-N-methyl-L-valinamide | 567.28 | 3.40min |
| **301** * | 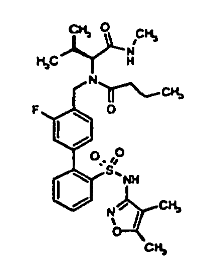 | N²-[[2'-[[(4,5-Dimethyl-3-isoxazolyl)amino]sulfonyl]-3-fluoro[1,1'-biphenyl]-4-yl]methyl]-N-methyl-N²-(1-oxobutyl)-L-valinamide | 559.2 | 3.40min |
| **302 *** | 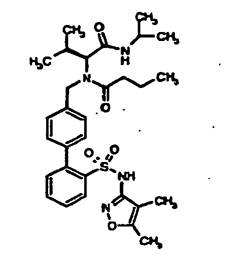 | N²-[[2'-[[(4,5-Dimethyl-3-isoxazolyl)amino]sulfonyl] [1,1' -biphenyl]-4-yl]methyl]-N-(1-methylethyl)-N²-(1-oxobutyl)-L-valinamide | 569.3 | 3.40min |
| **303** * | 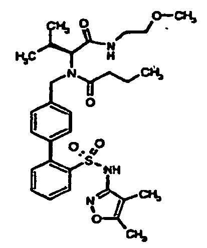 | N²-[[2'-[[(4,5-Dimethyl-3-isoxazolyl)amino]sulfonyl] [1,1' -biphenyl]-4-yl]methyl]-N-(2-methoxyethyl)-N²-(1-oxobutyl)-L-valinamide | 585.3 | 3.27min |
| **304** * | 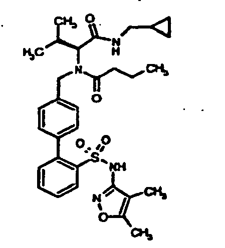 | N-(Cyclopropylmethyl)-N²-[[2'-[[(4,5-dimethyl-3-isoxazolyl)amino]sulfonyl][1,1' -biphenyl]-4-yl]methyl]-N²-(1-oxobutyl)-L-valinamide | 581.3 | 3.44min |
| **305 *** | 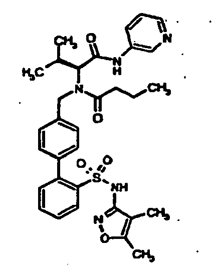 | N²-[[2'-[[(4,5-Dimethyl-3-isoxazolyl)amino]sulfonyl][1,1' -biphenyl]-4-yl]methyl]-N²-(1-oxobutyl)-N-(3-pyridinyl)-L-valinamide | 604.3 | 2.87min |
| **306** * | 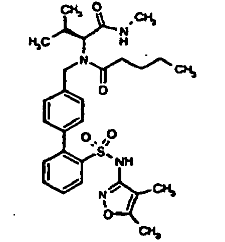 | N²-[[2'-[[(4,5-Dimethyl-3-isoxazolyl)amino]sulfonyl] [1,1' -biphenyl]-4-yl]methyl]-N-methyl-N²-(1-oxopentyl)-L-valinamide | 555.17 | 3.36min |
| **307** * | 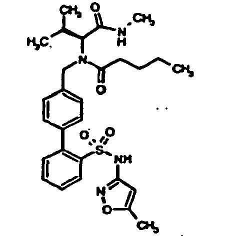 | N-Methyl-N²-[[2'-[[(5-methyl-3-isoxazolyl)amino]sulfonyl] [1,1' -biphenyl]-4-yl]methyl]-N²-(1-oxopentyl)-L-valinamide | 541.2 | 3.40min |
| **308 *** | 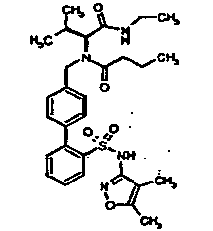 | N²-[[2'-[[(4,5-Dimethyl-3-isoxazolyl)amino]sulfonyl] [1,1' -biphenyl]-4-yl]methyl]-N-ethyl-N²-(1-oxobutyl)-L-valinamide | 555.3 | 3.17min |
| **309 *** | 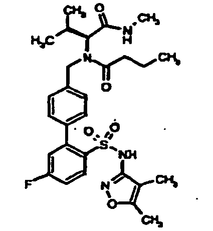 | N²-[[2'-[[(4,5-Dimethyl-3-isoxazolyl)amino]sulfonyl]-5'-fluoro[1,1'-biphenyl]-4-yl]methyl]-N-methyl-N²-(1-oxobutyl)-L-valinamide | 558.76 | 3.28min |

| | | | | |
|---|---|---|---|---|
| * Reference Example | | | | |

### Examples 310-311

The compounds of Examples 310-311 were prepared in a manner similar to that described in Example 252. The reported HPLC retention times were obtained under the following conditions. Column: YMC S5 ODS 4.6X50 mm (4min); Wavelength: 220nm; Solvent: Gradient elution 10%MeOH-90% MeOH in water

| **EXAMPLE** | **STRUCTURE** | **NAME** | **M/z (MH)**^{**+**} | **HPLCret time, min** |
|---|---|---|---|---|
| **310 *** | 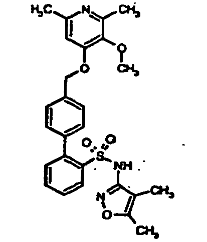 | N-(4,5-Dimethyl-3-isoxazolyl)-4'-[[(2,6-dimethyl-3-methoxy-4-pyridinyl)oxy]methyl] [1,1'-biphenyl]-2-sulfonamide | 494 | 2.28min |
| **311 *** | 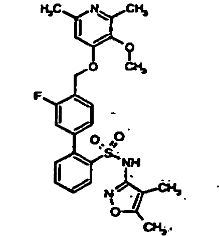 | N-(4,5-Dimethyl-3-isoxazolyl)-4'-[[(2,6-dimethyl-3-methoxy-4-pyridinyl)oxy]methyl]-3'-fluoro[1,1'-biphenyl]-2-sulfonamide | 512 | 2.41min |

| | | | | |
|---|---|---|---|---|
| * Reference Example | | | | |

### Examples 312-314

The compounds of Examples 312-314 were prepared in a manner similar to that described in Example 228. The reported HPLC retention times were obtained under the following conditions.
Column: YMC S5 ODS 4.6X50 mm (4min); Wavelength: 220nm;
Solvent: Gradient elution 10%MeOH-90% MeOH in water
(0.2% H3PO4).

| **EXAMPLE** | **STRUCTURE** | **NAME** | **M/z (MH)**^{**+**} | **HPLCret time, min** |
|---|---|---|---|---|
| **312 *** | 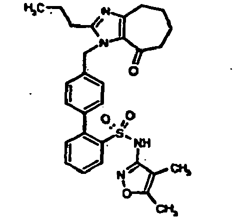 | N-(4,5-Dimethyl-3-isoxazolyl)-4'-[(1,4,5,6,7,8-hexahydro-8-oxo-2-propyl-1-cycloheptimidazolyl)methyl] [1, 1'-biphenyl]-2-sulfonamide, | 533.3 | 2.35min |
| **313** | 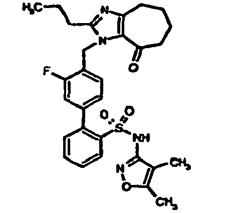 | N-(4,5-Dimethyl-3-isoxazolyl)-3'-fluoro-4'-[(1,4,5,6,7,8-hexahydro-8-oxo-2-propyl-1-cycloheptimidazolyl)methyl] [1, 1'-biphenyl]-2-sulfonamide | 551.3 | 2.35min |
| **314** | 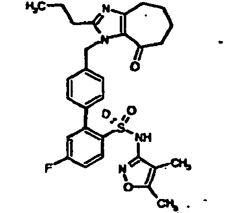 | N-(4,5-Dimethyl-3-isoxazolyl)-5-fluoro-4'-[(1,4,5,6,7,8-hexahydro-8-oxo-2-propyl-1-cycloheptimidazolyl)methyl] [1, 1'-biphenyl]-2-sulfonamide | 551.17 | 2.32min |

| | | | | |
|---|---|---|---|---|
| * Reference example | | | | |

### Reference Example 315

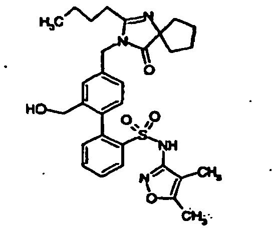

### 4'-[(2-Butyl-4-oxo-1,3-diazaspiro[4.4]non-1-en-3-yl)methyl]-N-(4,5-dimethyl-3-isoxazolyl)-2'-(hydrozymethyl)[1,1'-biphenyl]-2-sulfonamide

**A.** 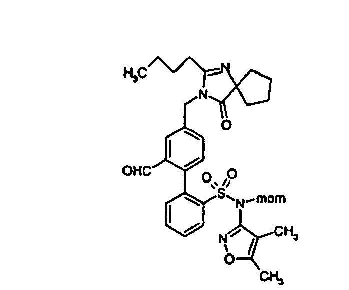 **5E** (4.49 g, 11.48 mmol) was subjected to Suzuki coupling with [2-[[(4,5-dimethyl-3-isoxazolyl)-N-methoxymethyl]amino]-sulfonyl]phenylboronic acid according to General Method 1. 316A (5.0 g, 72%) was obtained as an orange oil following silica gel chromatography using hexanes/ethyl acetate as eluant.
**B.** 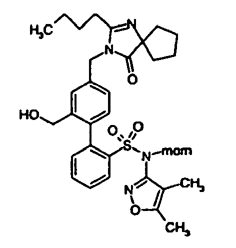 **315A** (0.7g) was dissolved in 10 mL of methylene chloride and 2 mL of triethylsilane and 2 mL of trifluoroacetic acid were added to the mixture. The solution was stirred at room tempertaure for 1h and concentrated. **315B** (0.6 g, 85%) was obtained as an orange oil following silica gel chromatography using hexanes/ethyl acetate as eluant.
**C**. **315B** (0.6 g) was deprotected according to General Method 8 (EtOH). The crude product was purified by silica gel chromatography using 1:1 hexanes/ethyl acetate as eluant to provide the title compound (0.44 g, 79%) as a white amorphous solid. mp. 90-98°C. **M+H**: 565.2

### Reference Example 316

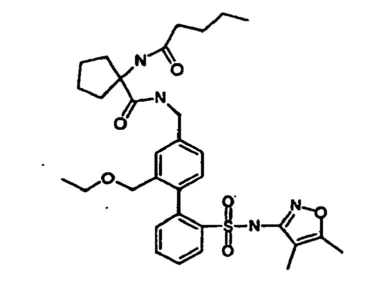

### N-[[2'-[[(4,5-Dimethyl-3-isoxazolyl)amino]sulfonyl]-2-(ethoxymethyl)[1,1'-biphenyl]-4-yl]methyl]-1-[(1-oxopentyl)amino]cyclopentanamide

### (A metabolite of Example 227)

**227** (30 mg) was dissolved in 1mL DMF and 0.6 mL of 20% aqueous NaOH was added and the mixture was stirred at 60°C for 24h. The mixture was purified by HPLC to provide the target compound as a white solid. HPLC RT: 3.21 min. M+H: 611.3

### Example 317

### (+)-4'-[(2-Butyl-4-oxo-1,3-diazaspiro[4.4]non-1-en-3-yl)methyl]-N-(4,5-dimethyl-3-isoxazolyl)-2'-(ethoxymethyl) [1,1'-biphenyl]-2-sulfonamide and (-)-4'-[(2-Butyl-4-oxo-1,3-diazaspiro[4,4]non-1-en-3-yl)methyl]-N-(4,5-dimethyl-3-isoxazolyl)-2'-(ethoxymethyl) [1,1'-biphenyl]-2-sulfonamide

10µl of a solution of **275F** (2mg/mL) in isopropanol was injected onto an HPLC column (Chiral AD, 250x4.6 (10µ)) eluting with a mobil phase of 80% hexane, 20% IPA, 0.1%TFA, 0.1%TEA at 0.75mL/min. The HPLC column temperature was maintained at 15°C. Detection was carried out at 210nm using a UV detector. The enantiomers of 275 were resolved into two peaks, with one enantiomer eluting at 11.6 min and the other at 15.1 min. yielding the two title compounds as pure enantiomers.

## Claims

1. A compound of the following formula I, or an enantiomer, diastereomer or salt thereof: 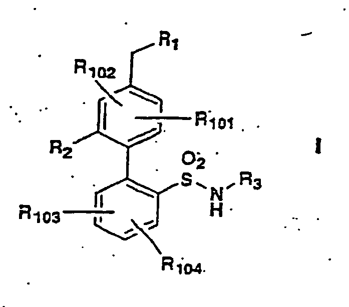 wherein:
R₁ is 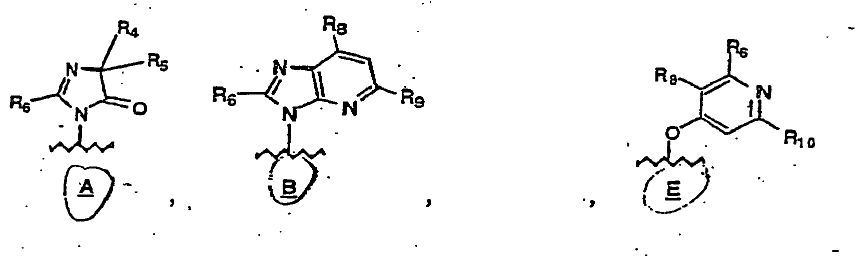 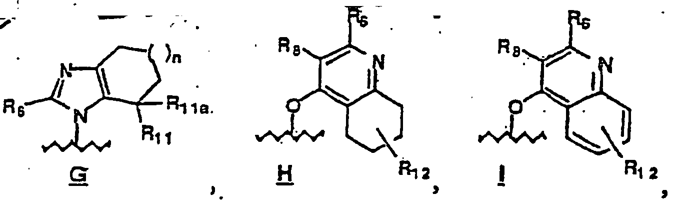 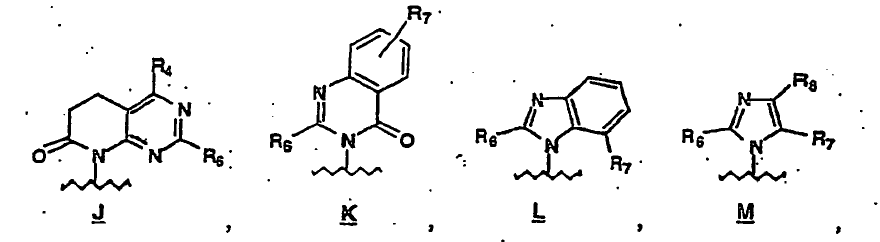 or 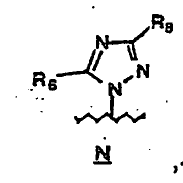
R₂ is hydrogen, halogen, -CHO, alkyl, haloalkyl, (cycloalkyl)alkyl, alkenyl, alkynyl, alkoxyalkyl, haloalkoxyalkyl, alkoxy, aryloxy, alkoxyalkoxy, cyano, hydroxy, hydroxyalkyl, nitro, -CH(OR₁₃)(OR₁₄), -(CH₂)_{w}Y; with the proviso that when R₁ is B, R₂ is not hydrogen, halogen, alkyl, haloalkyl, alkoxy, hydroxyalkyl, nitro, -(CH₂)_{w}NR₁₉R₂₀ or -NHSO₂R₂₂;
R₃ is heteroaryl;
R₄ and R₅ are each independently alkyl, hydroxyalkyl, cycloalkyl, hydroxy substituted cycloalkyl, alkoxyalkyl, or hydroxy substituted alkoxyalkyl, or R₄ and R₅ together form a cyclobutyl, cyclopentyl, cyclohexyl, tetrahydrofuranyl or tetrahydropyranyl ring which may be optionally substituted with one or more hydroxy groups;
R₆ is alkyl, hydroxyalkyl, haloalkyl, hydroxy substituted haloalkyl, cycloalkyl, hydroxy substituted cycloalkyl, (cycloalkyl)alkyl, hydroxy substituted (cycloalkyl)(alkyl), aralkyl, alkoxy, hydroxy substituted alkoxy, alkoxyalkyl, hydroxy substituted alkoxyalkyl, or -NR₁₆R₁₇;
R₇ is -(CH₂)_{w}-CO₂R₁₅, -(CH₂)_{w}-(C=O)NR₁₆R₁₇, -(CH₂)_{w}-NR₁₅(C=O)NR₁₆R₁₇, -(CH₂)_{w}-CH₂OH, -(CH₂)_{w}-(C=O)R₁₆, tetrazolyl, oxadiazolyl or triazolyl wherein said tetrazolyl, oxadiazolyl or triazolyl may optionally be substituted with hydrogen, alkyl, hydroxy or halogen;
R₈, R₉, R₉ₐ, R₁₀ and R₁₂ are each independently hydrogen, halogen, alkyl, hydroxyalkyl, cycloalkyl, (cycloalkyl)alkyl, aryl, heteroaryl, arylalkyl, alkylthioalkyl, alkoxy or alkoxyalkyl, or R₉ and R₉ₐ together with the carbon atom to which they are bonded form a cycloalkyl ring;
R₁₁ and R₁₁ₐ are each independently hydrogen, alkoxy, or together form a carbonyl;
R₁₃ and R₁₄ are alkyl or together form a five to six-membered ring;
R₁₅, R₁₆ and R₁₇ are independently hydrogen, alkyl, hydroxyalkyl, cycloalkyl, (cycloalkyl)alkyl, alkoxyalkyl, aralkyl, heterocycloalkyl, aryl, heteroaryl or -(CH₂)_{w} Q, or R₁₆ and R₁₇ may together form a four to six-membered heterocyclic ring;
n is 1 or 2;
w is 0, 1, or 2;
Y is heteroaryl, -COOH, -COOR₁₈, -CONR₁₉R₂₀, -NR₁₉R₂₀, -NR₁₉-OR₂₀, -NR₂₁(C=O)R₂₂, -NR₂₁(C=O) NR₁₉R₂₀, -N(R₁₉)-(alk)-NR₂₁(C=O)R₂₂, -NR₂₁(C=O)OR₁₈, -NR₂₁SO₂R₂₂, -SO₂R₂₂, Q, R or S;
Q is 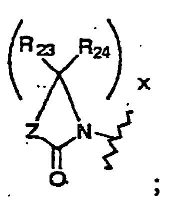
R is 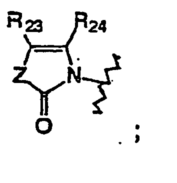
S is 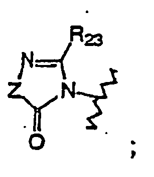
R₁₈, R₁₉, R₂₀, R₂₁ and R₂₂ are each independently hydrogen, alkyl, haloalkyl, alkoxyalkyl, cycloalkyl, alkenyl, alkynyl, aryl, aralkyl, heteroaryl, or R₁₉ and R₂₀ may together form a four to seven-membered heterocyclic ring;
R₂₃ and R₂₄ are each independently hydrogen, alkyl or cycloalkyl, or may together form a three to seven membered cycloalkyl ring;
Z is oxygen, 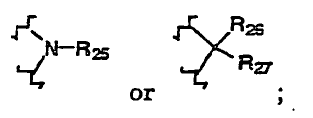
x is 2, 3 or 4;
R₂₅, R₂₆ and R₂₇ are each independently hydrogen, alkyl or cycloalkyl, or R₂₆ and R₂₇ may together form a three to seven-membered cycloalkyl ring;
R₁₀₁, R₁₀₂, R₁₀₃, and R₁₀₄ are each independently hydrogen, halogen, -CHO, alkyl, haloalkyl, (cycloalkyl)alkyl, alkenyl, alkynyl, alkoxyalkyl, haloalkoxyalkyl, alkoxy, alkoxyalkoxy, cyano, hydroxy, . hydroxyalkyl, nitro, -CH(OR₁₃)(OR₁₄), or -(CH₂)_{w} Y; provided that at least one of R₁₀₁, R₁₀₂ R₁₀₃, and R₁₀₄ is other than hydrogen
wherein said rings; aryl alone or as part of another group; or heteroaryl alone or as part of another group may each optionally be substituted by one or more hydrogen, halogen, cyano, alkyl, hydroxyalkyl, alkoxy, nitro or trifluoromethyl groups;
provided that when R₁ is A said compound is other than 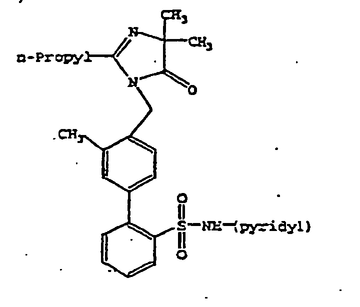 wherein the terms "alk" or "alkyl" refer to straight or branched chain hydrocarbon groups having 1 to 12' carbon atoms,
the term "alkenyl" refers to straight or branched chain hydrocarbon groups having 2 to 12 carbon atoms, and at least one double carbon to carbon double bond.
the term "alkynyl" refers to straight or branched chain hydrocarbon groups having 2 to 12 carbon atoms, and at least one triple carbon to carbon bond,
the term "alkoxy" refers to an alkyl group bonded through an oxygen (-O-),
the term "aryloxy" refers to an aryl group bonded through an oxygen (-O-),
the term "thioalkyl" refers to an alkyl group bonded through a sulfer (-S-),
the terms "ar" or "aryl" refer to phenyl, naphthyl and biphenyl, aryl groups, may be optionally substituted with one or more (such as one to three) of the following substituents: hydrogen, halogen, cyano, alkyl, alkoxy, nitro or trifluoromethyl groups,
the term "cycloalkyl" refers to fully saturated cyclic hydrocarbon groups having 3 to 8 ring carbon atoms,
haloalkyl refers to an alkyl chain substituted with from one to three halogens,
the term "heteroaryl" refers to furyl, thienyl, pyrrolyl, pyridyl, pyrimidyl, pyrazinyl, pyridazinyl, triazinyl, oxazolyl, isoxazolyl, thiazolyl, isothiazolyl, oxadiazolyl, thiadiazolyl, imidazolyl, triazolyl, and tetrazolyl, each of which may optionally be substituted where appropriate by one or more (such as one to three) of the following: hydrogen, halogen, cyano, alkyl, hydroxyalkyl, alkoxy, nitro or trifluoromethyl.
the terms "heterocyclic" or "heterocyclo" refer to optionally substituted, non-aromatic cyclic groups, 4 to 7 membered monocyclic, 7 to 11 membered bicyclic, or 10 to 15 membered tricyclic ring systems, which have at least one heteroatom in at least one carbon atom-containing ring
wherein monocyclic heterocyclic groups include azetidinyl, pyrrolidinyl, oxetanyl, imidazolinyl, oxazolidinyl, isoxazolinyl, thiazolidinyl, isothiazolidinyl, tetrahydrofuranyl, piperidinyl, piperazinyl, 2-oxopiperazinyl, 2-oxopiperidinyl, 2-oxopyrrolodinyl, 2-oxoazepinyl, azepinyl, 4-piperidonyl, tetrahydropyranyl, morpholinyl, thiamorpholinyl, thiamorpholinyl sulfoxide, thiamorpholinyl sulfone, 1,3-dioxolane and tetrahydro-1,1-dioxothiehyl;
the term "ring" encompasses homocyclic (i.e., as used herein, all the ring atoms are carbon) or "heterocyclic" (i.e., as used herein, the ring atoms include carbon and one to four heteroatoms selected from N, O and /or S, also referred to as heterocyclo), where, as used herein, each of which (homocyclic or heterocyclic) may be saturated or partially or completely unsaturated (such as heteroaryl), and each of which (homocyclic or heterocyclic) may optionally be substituted by one or more (such as one to three) hydrogen, halogen, cyano, alkyl, alkoxy, nitro or trifluoromethyl group.

2. A compound of claim 1, wherein R₁ is

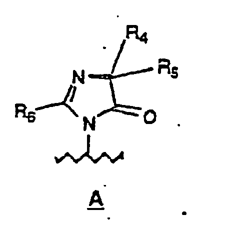

3. A compound of claim 1, wherein R₁ is

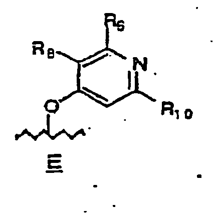

4. A compound of claim 1, wherein R₁ is

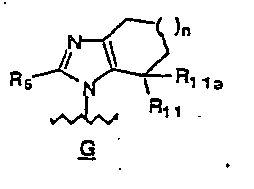

5. A compound of claim 1, wherein R₂ is alkoxyalkyl haloalkyl or haloalkoxyalkyl

6. A compound of anyone of claims 1-5, wherein R₃ is isoxazol-5-yl or isoxazol-3-yl independently substituted with two substituents selected from alkyl or halogen.

7. A compound of claim 1, wherein said compound is selected from 4'-[(2-Butyl-4-oxo-1,3-diazaspiro[4,4]non-1-en-3-yl)methyl]-3'-chloro-N-(4.5-dimethyl-3-isoxazolyl)-5-methoxy[1.1'-biphenyl]-2-sulfonamide
| | | | |
|---|---|---|---|
| 4'-[(2-Butyl-4-oxo-1,3-diazaspiro[4.4]non-1-en-3-yl)methyl]-N-(4,5-dimethyl-3-isoxazolyl)-3'-fluoro-4-methoxy[1,1'-biphenyl]-2-sulfonamide | 4'-[(2-Butyl-4-oxo-1,3-diazaspiro[4.4]non-1-en-3-yl)methyl]-5'-chloro-N-(4,5-dimethyl-3-isoxazolyl)-2'-fluoro-4-methoxy[1,1'-biphenyl]-2-sulfonamide | | 4'-[(2-Butyl-4-oxo-1,3-diazaspiro[4.4]non-1-en-3-yl)methyl]-3'-chloro-N-(4,5-dimethyl-3-isoxazolyl)-4-methoxy[1,1'-biphenyl]-2-sulfonamide |
| 4'-[(2-Butyl-4-oxo-1,3-diazaspiro[4.4]non-1-en-3-yl)methyl]-N-(4,5-dimethyl-3-isoxazolyl)-4-methoxy[1,1'-biphenyl]-2-sulfonamide | 4'-[(2-Butyl-4-ozo-1,3-diazaspiro[4.4]non-1-en-3-yl)methyl]-N-(4,5-dimethyl-3-isoxazolyl)-2'-(ethoxymethyl)-4-methoxy[1,1'-biphenyl]-2-sulfonamide | | 4'-[(2-Butyl-4-oxo-1,3-diazaspiro[4.4]non-1-en-3-yl)methyl]-N-(4,5-dimethyl-3-isoxazolyl)-3'-fluoro-5-methozy[1,1'-biphenyl]-2-sulfonamide |
| 4'-[(2-Butyl-4-oxo-1,3-diazaspiro[4.4]non-1-en-3-yl)methyl]-5'-chloro-N-(4,5-dimethyl-3-isoxazolyl)-2'-fluoro-5-methoxy[1,1'-biphenyl]-2-sulfonamide , | 4'-[(2-Butyl-4-oxo-1,3-diazaspiro[4.4]non-1-en-3-yl)methyl]-N-(4,5-dimethyl-3-isoxazolyl)-5-methoxy[1,1'-biphenyl]-2-sulfonamide, | | 4'-[(2-Butyl-4-oxo-1,3-diazaspiro[4.4]non-1-en-3-yl)methyl]-N-(4,5-dimethyl-3-isoxazolyl)-2'-(ethoxymethyl)-5-methoxy[1,1'-biphenyl]-2-sulfonamide |
| 4'-[(2-Butyl-4-oxo-1,3-diazaspiro[4.4]non-1-en-3-yl)methyl]-N-(4,5-dimethyl-3-isoxazolyl)-3'-fluoro-4,5-dimethoxy[1,1'-biphenyl]-2-sulfonamide | 4'-[(2-Butyl-4-oxo-1,3-diazaspiro[4.4]non-1-en-3-yl)methy]-5'-chloro-N-(4,5-dimethyl-3-isoxazolyl)-2'-fluoro-4,5-dimethozy[1,1'-biphenyl]-2-sulfonamide | | 4'-[(2-Butyl-4-oxo-1,3-diazaspiro[4.4]non-1-en-3-yl)methyl]-3'-chloro-N-(4,5-dimethyl-3-isoxazolyl)-4,5-dimethoxy[1,1'-biphenyl]-2-sulfonamide |
| 4'-[(2-Butyl-4-oxo-1,3-diazaspiro[4.4]non-1-en-3-yl)methyl]-N-(4,5-dimethyl-3-isoxazolyl)-4,5-dimethoxy[1,1'-biphenyl]-2-sulfonamide | 4'-[(2-Butyl-4-oxo-1,3-diazaspiro[4.4]non-1-en-3-yl)methyl]-N-(4,5-dimethyl-3-isoxazolyl)-2'-(ethoxymethyl)-4,5-dimethoxy[1,1'-biphenyl]-2-sulfonamide | | 4'-[2-Butyl-4-oxo-1,3-diazaspiro[4.4]non-1-en-3-yl)methyl]-5'-chloro-N-(4,5-dimethyl-3-isoxazolyl)-2'-fluoro[1,1'-biphenyl]-2-sulfonamide |
| 4'-[(2-Butyl-4-oxo-1,3-diazaspiro[4.4]non-1-en-3-yl)methyl]-3'-chloro-N-(4,5-dimethyl-3-isoxazolyl)[1,1'-biphenyl]-2-sulfonamide | 4'-[(2-Butyl-4-oxo-1,3-diazaspiro[4,4]non-1-en-3-yl)methyl]-N-(4,5-dimethyl-3-isoxazolyl)-4-methoxy-3'-methyl[1,1'-biphenyl]-2-sulfonamide , | | 4'-[(2-Butyl-4-oxo-1,3-diazaspiro[4.4]non-1-en-3-yl)methyl]-N-(4,5-dimethyl-3-isoxazolyl)-3'-fluoro-4-methoxy[1,1'-biphenyl]-2-sulfonamide |
| 4'-[2-Butyl-4-oxo-1,3-diazaspiro[4.4]non-1-en-3-yl)methyl]-N-(4,5-dimethyl-3-isoxazolyl)-3'-methyl[1,1'-biphenyl]-2-sulfonamide | and | 4'-[(2-Butyl-4-oxo-1,3-diazaspiro[4.4]non-1-en-3-yl)methyl]-N-(4,5-dimethyl-3-isoxazolyl)-5-fluoro[1,1'-biphenyl]-2-sulfonamide , | |

8. A compound of claim 1, wherein said compound is selected from
| | | |
|---|---|---|
| N-(4,5-Dimethyl-3-isoxazolyl)-4'-[[(2,6-dimethyl-3-methoxy-4-pyridinyl)oxy]methyl]-3'-fluoro[1,1'-biphenyl]-2-sulfonamide | N-(4,5-Dimethyl-3-isoxazolyl)-3'-fluoro-4'-[(1,4,5,6,7,8-hexahydro-8-oxo-2-propyl-1-cycloheptimidazolyl)methyl] [1, 1'-biphenyl]-2-sulfonamide, | and |
| N-(4,5-Dimethyl-3-isoxazolyl)-5-fluoro-4'-[(1,4,5,6,7,8-hexahydro-8-oxo-2-propyl-1-cycloheptimidazolyl)methyl] [1, 1'-biphenyl]-2-sulfonamide | | |

9. Use of a compound as defined in any one of claims 1-8 for the preparation of a pharmaceutical composition for the treatment of an endothelin-dependent or angiotensin II-dependent disorder (such as hypertension, pulmonary hypertension, primary pulmonary hypertension, low renin hypertension, male erectile dysfunction, male or female sexual dysfunction, heart failure atherosclerosis, restenosis, endotoxemia, inhibition of cell growth, cancer, migraine, asthma, ischemia, subarachnoid hemorrhage, benign prostatic hypertrophy, renal glomerular or mesangial cell disorders, acute or chronic renal failure, chronic obstructive pulmonary disease, pain associated with prostate cancer, organ damage associated with the cell poliferative effects of endothelin, general morbidity and mortality associated with endothelin-dependent or angiotensin II-depedendent disorders, diabetic nephropathy, and dementia).

10. A pharmaceutical composition comprising a pharmaceutically acceptable vehicle or diluent and at least one compound of any one of claim 1 - 8.

11. A compound of claim 1, wherein R₁ is

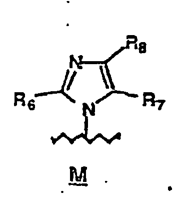

12. A compound of anyone of claims 2-4, wherein R₂ is hydrogen, alkyl, haloalkyl, alkoxyalkyl or haloalkoxyalkyl and R₁₀₁, R₁₀₂, R₁₀₃, R₁₀₄ are each independently halogen, or alkyl.

13. A compound of anyone of claims 1-5, wherein R₂ is -CH₂Y.

14. A compound of anyone of claims1-5, or 13 wherein Y is Q.

15. A compound of claim 1 wherein R₃ is other than pyridyl when R₁ is A.

16. The pharmaceutical composition of claim 10 further comprising at least one ACE inhibitor (such as captopril, zofenopril, fosinopril, ceranapril, alacepril, enalapril, delapril, pentopril, quinapril, ramipril, or lisinopril), vasopeptidase inhibitor (such as omapatrilat or gemopatrilat); HMG CoA reductase inhibitor ( such as pravastatin, lovastatin, atorvastatin, simvastatin, NK-104 or ZD-4522), anti-platelet agent (such as clopidigrel, ticlopidine, CS-747 or aspirin), anti-diabetic agent (such as biguanides or biguanide/glyburide combinations), beta-adrenergic agent (such as carvedilol or metoprolol), or mineralocorticoid receptor antagonist (such as spironolactone or eplerenone).

## Patentansprüche

1. Verbindung der folgenden Formel I, oder ein Enatiomer, Diastereomer oder Salz davon: 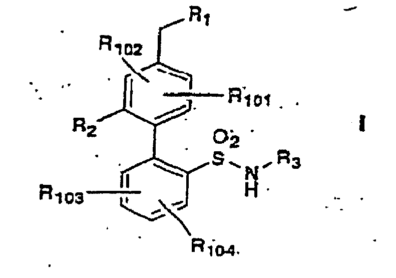 worin, R₁ 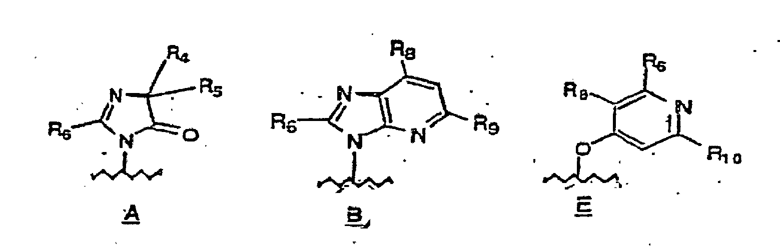 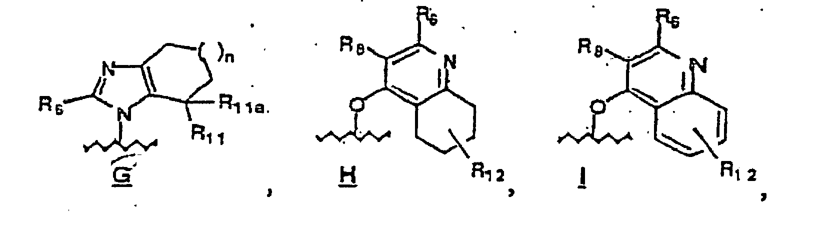 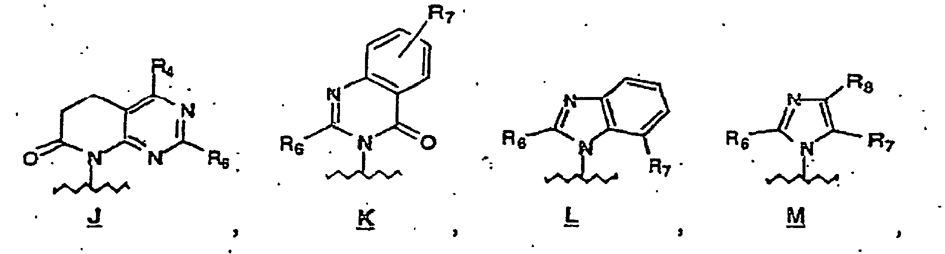 oder 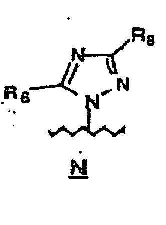 ist;
R₂ Wasserstoff, Halogen, -CHO, Alkyl, Haloalkyl, (Cycloalkyl)alkyl, Alkenyl, Alkinyl, Alkoxyalkyl, Haloalkoxyalkyl, Alkoxy, Aryloxy, Alkoxyalkoxy, Cyano, Hydroxy, Hydroxyalkyl, Nitro, -CH(OR₁₃)(OR₁₄), -(CH₂)_{w}Y ist; mit der Maßgabe, dass wenn R₁ B ist, R₂ nicht Wasserstoff, Halogen, Alkyl, Haloalkyl, Alkoxy, Hydroxyalkyl, Nitro, -(CH₂)_{w}NR₁₉R₂₀ oder -NHSO₂R₂₂ ist;
R₃ Heteroaryl ist;
R₄ und R₅ jeweils unabhängig voneinander Alkyl, Hydroxyalkyl, Cycloalkyl, Hydroxy-substituiertes Cycloalkyl, Alkoxyalkyl oder Hydroxy-substituiertes Alkoxyalkyl sind oder R₄ und R₅ zusammen einen Cyclobutyl-, Cyclopentyl-, Cyclohexyl-, Tetrahydrofuranyl- oder Tetrahydropyranylring bilden, der gegebenenfalls mit einer oder mehreren Hydroxygruppen substituiert sein kann;
R₆ Alkyl, Hydroxyalkyl, Haloalkyl, Hydroxy-substituiertes Haloalkyl, Cycloalkyl, Hydroxy-substituiertes Cycloalkyl, (Cycloalkyl)alkyl, Hydroxy-substituiertes (Cycloalkyl)alkyl, Aralkyl, Alkoxy, Hydroxy-substituiertes Alkoxy, Alkoxyalkyl, Hydroxy-substituiertes Alkoxyalkyl oder -NR₁₆R₁₇ ist;
R₇ -(CH₂)_{w}-CO₂R₁₅, -(CH₂)_{w}-(C=O)NR₁₆R₁₇, -(CH₂)_{w}-NR₁₅(C=O)NR₁₆R₁₇, -(CH₂)_{w-}CH₂OH, -(CH₂)_{w}-(C=O)R₁₅, Tetrazolyl, Oxadiazolyl oder Triazolyl ist, wobei Tetrazolyl, Oxadiazolyl oder Triazolyl gegebenenfalls mit Wasserstoff, Alkyl, Hydroxy oder Halogen substituiert sein kann;
R₈, R₉, R₉ₐ, R₁₀ und R₁₂ jeweils unabhängig voneinander Wasserstoff, Halogen, Alkyl, Hydroxyalkyl, Cycloalkyl, (Cycloalkyl)alkyl, Aryl, Heteroaryl, Arylalkyl, Alkylthioalkyl, Alkoxy oder Alkoxyalkyl sind, oder R₉ und R₉ₐ zusammen mit dem Kohlenstoffatom, an das sie gebunden sind, einen Cycloalkylring bilden;
R₁₁ und R₁₁ₐ unabhängig voneinander Wasserstoff, Alkoxy sind oder zusammen ein Carbonyl bilden;
R₁₈ und R₁₄ Alkyl sind oder zusammen einen fünf- bis sechsgliedrigen Ring bilden;
R₁₅, R₁₆ und R₁₇ unabhängig voneinander Wasserstoff, Alkyl, Hydroxyalkyl, Cycloalkyl, (Cycloalkyl)alkyl, Alkoxyalkyl, Aralkyl, Heterocycloalkyl, Aryl, Heteroaryl oder -(CH₂)_{w}Q sind, oder R₁₆ und R₁₇ zusammen einen vier- bis sechsgliedrigen heterocyclischen Ring bilden können;
n 1 oder 2 ist;
w 0, 1 oder 2 ist;
Y Heteroaryl, -COOH, -COOR₁₈, -CONR₁₉R₂₀, -NR₁₉R₂₀, -NR₁₉-OR₂₀, NR₂₁(C=O)R₂₂, -NR₂₁(C=O)NR₁₉R₂₀, -N(R₁₉)-(Alk)-NR₂₁(C=O)R₂₂, -NR₂₁(C=O)OR₁₈, -NR₂₁SO₂R₂₂, -SO₂R₂₂, Q, R, oder S ist;
Q 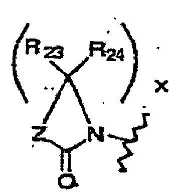 ist;
R 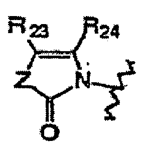 ist;
S 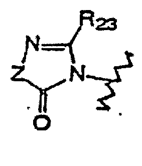 ist;
R₁₈, R₁₉, R₂₀, R₂₁ und R₂₂ jeweils unabhängig voneinander Wasserstoff, Alkyl, Haloalkyl, Alkoxyalkyl, Cycloalkyl, Alkenyl, Alkinyl, Aryl, Aralkyl, Heteroaryl sind, oder R₁₉ und R₂₀ zusammen einen vier- bis siebengliedrigen heterocyclischen Ring bilden können;
R₂₃ und R₂₄ unabhängig voneinander Wasserstoff, Alkyl oder Cycloalkyl sind, oder zusammen einen drei- bis siebengliedrigen Cycloalkylring bilden können;
Z Sauerstoff, 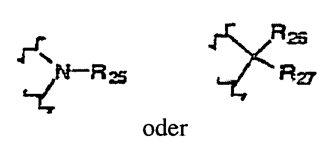 ist;
x 2, 3 oder 4 ist;
R₂₅, R₂₆ und R₂₇ jeweils unabhängig voneinander Wasserstoff, Alkyl oder Cycloalkyl sind, oder R₂₆ und R₂₇ zusammen einen drei- bis siebengliedrigen Cycloalkylring bilden können;
R₁₀₁, R₁₀₂, R₁₀₃ und R₁₀₄ jeweils unabhängig voneinander Wasserstoff, Halogen, -CHO, Alkyl, Haloalkyl, (Cycloalkyl)alkyl, Alkenyl, Alkinyl, Alkoxyalkyl, Haloalkoxyalkyl, Alkoxy, Alkoxyalkoxy, Cyano, Hydroxy, Hydroxyalkyl, Nitro, -CH(OR₁₃)(OR₁₄) oder -(CH₂)_{w}Y sind; mit der Maßgabe, dass mindestens einer der Reste R₁₀₁ , R₁₀₂, R₁₀₃ und R₁₀₄ von Wasserstoff verschieden ist;
wobei die oben genannten Ringe Aryl alleine oder als Teil einer anderen Gruppe oder Heteroaryl alleine oder als Teil einer anderen Gruppe jeweils gegebenenfalls mit einem oder mehreren Wasserstoff-, Halogen-, Cyano-, Alkyl-, Hydroxyalkyl-, Alkoxy-, Nitro-oder Trifluormethylresten substituiert sein können;
mit der Maßgabe, dass, wenn R₁ A ist, die Verbindung von 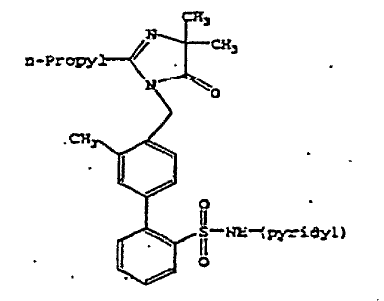 verschieden ist;
wobei die Begriffe "Alk" oder "Alkyl" sich auf geradkettige oder verzweigtkettige Kohlenwasserstoffreste mit 1 bis 12 Kohlenstoffatomen beziehen;
der Begriff "Alkenyl" sich auf geradkettige oder verzweigtkettige Kohlenwasserstoffreste mit 2 bis 12 Kohlenstoffatomen bezieht, welche mindestens eine Kohlenstoff-Kohlenstoff-Doppelbindung haben;
der Begriff "Alkinyl" sich auf geradkettige oder verzweigtkettige Kohlenwasserstoffreste mit 2 bis 12 Kohlenstoffatomen und mit mindestens einer Kohlenstoff-Kohlenstoff-Dreifachbindung bezieht;
der Begriff "Alkoxy" sich auf einen Alkylrest bezieht, welcher über einen Sauerstoff (-O-) gebunden ist;
der Begriff "Aryloxy" sich auf einen Arylrest bezieht, welcher über einen Sauerstoff (-O-) gebunden ist;
der Begriff "Thioalkyl" sich auf einen Alkylrest beziehen, welcher über einen Schwefel (-S-) gebunden ist;
die Begriffe "Ar" oder "Aryl" sich auf Phenyl, Naphthyl und Biphenyl beziehen, wobei Arylreste gegebenenfalls mit einem oder mehreren (wie 1 bis 3) der folgenden Substituenten substituiert sein können: Wasserstoff-, Halogen-, Cyano-, Alkyl-, Alkoxy-, Nitro- oder Trifluormethylreste;
der Begriff "Cycloalkyl" sich auf vollständig gesättigte, cyclische Kohlenwasserstoffreste bezieht, die 3 bis 8 Ringatome haben;
Haloalkyl sich auf eine Alkylkette bezieht, die mit einem bis drei Halogenatomen substituiert ist;
der Begriff "Heteroaryl" sich auf Furyl, Thienyl, Pyrrolyl, Pyridyl, Pyrimidyl, Pyrazinyl, Pyridazinyl, Triazinyl, Oxazolyl, Isoxazolyl, Thiazolyl, Isothiazolyl, Oxadiazolyl, Thiadiazolyl, Imidazolyl, Triazolyl, und Tetrazolyl bezieht, wobei jeder gegebenenfalls wo passend mit einem oder mehreren (z.B. 1 bis 3) der Folgenden substituiert sein kann: Wasserstoff, Halogen, Cyano, Alkyl, Hydroxyalkyl, Alkoxy, Nitro oder Trifluoromethyl;
die Begriffe "heterocyclisch" oder "Heterocyclo" sich auf gegebenenfalls substituierte, nicht-aromatische cyclische, 4- bis 7-gliedrige monocyclische, 7- bis 11-gliedrige bicyclische oder 10- bis 15-gliedrige tricyclische Ringsysteme beziehen, welche mindestens ein Heteroatom in mindestens einem der Kohlenstoff-enthaltenden Ringe haben;
wobei die monocyclischen heterocyclischen Gruppen Azetidinyl, Pyrrolidinyl, Oxetanyl, Imidazolinyl, Oxazolidinyl, Isoxazolinyl, Thiazolidinyl, Isothiazolidinyl, Tetrahydrofuranyl, Piperidinyl, Piperazinyl, 2-Oxopiperazinyl, 2-Oxopiperidinyl, 2-Oxopyrrolodinyl, 2-Oxoazepinyl, Azepinyl, 4-Piperidonyl, Tetrahydropyranyl, Morpholinyl, Thiamorpholinyl, Thiamorpholinylsulfoxid, Thiamorpholinylsulfon, 1,3-Dioxolan und Tetrahydro-1,1-dioxothienyl umfassen;
der Begriff "Ring" homocyclische (d.h., wie hierin verwendet, alle Ringatome sind Kohlenstoffatome) oder "heterocyclische" (d.h., wie hierin verwendet, die Ringatome umfassen Kohlenstoffatome und ein bis vier Heteroatome ausgewählt aus N, O und/oder S, auch als Heterocyclo bezeichnet) Ringe umfasst, wobei, wie hierin verwendet, jeder (homocyclisch oder hetercyclisch) gesättigt oder partiell oder komplett ungesättigt (wie z.B. Heteroaryl) sein kann und jeder davon (homocyclisch oder heterocyclisch) gegebenenfalls mit einem oder mehreren (wie ein bis drei) Wasserstoff-, Halogen-, Cyano-, Alkyl-, Alkoxy-, Nitro- oder Trifluormethylresten substituiert sein kann.

2. Verbindung nach Anspruch 1, wobei R₁ 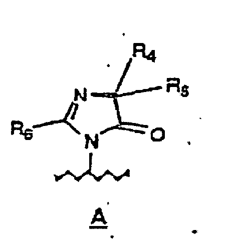 ist.

3. Verbindung nach Anspruch 1, wobei R₁ 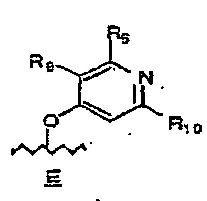 ist.

4. Verbindung nach Anspruch 1, wobei R₁ 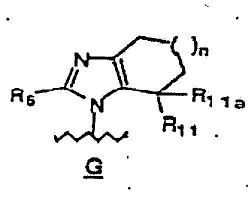 ist.

5. Verbindung nach Anspruch 1, wobei R₂ Alkoxyalkyl, Haloalkyl oder Haloalkoxyalkyl ist.

6. Verbindung nach einem der Ansprüche 1 bis 5, wobei R₃ Isoxazolyl-5-yl oder Isoxazol-3-yl ist, welche unabhängig mit zwei Substituenten ausgewählt aus Alkyl oder Halogen substituiert sind.

7. Verbindung nach Anspruch 1, wobei die Verbindung ausgewählt ist aus
4'-[(2-Butyl-4-oxo-1,3-diazaspiro[4.4]non-1-en-3-yl)methyl]-3'-chlor-N-(4,5-dimethyl-3-isoxazolyl)-5-methoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-3'-fluor-4-methoxy[1,1'-biphenyl]-2-sulfonamid,
4'-[(2-Butyl-4-oxo-1,3-diazaspiro[4.4]non-1-en-3-yl)methyl]-5'-chlor-N-(4,5-dimethyl-3-isoxazolyl)-2'-fluor-4-methoxy[1,1'-biphenyl]-2-sulfonamid,
4'-[(2-Butyl-4-oxo-1,3-diazaspiro[4.4]non-1-en-3-yl)methyl]-3'-chlor-N-(4,5-dimethyl-3-isoxazolyl)-4-methoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-4-methoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-2'-(ethoxymethyl)-4-methoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-3'-fluor-5-methoxy[1,1'-biphenyl]-2-sulfonamid,
4'-[(2-Butyl-4-oxo-1,3-diazaspiro[4.4]non-1-en-3-yl)methyl]-5'-chlor-N-(4,5-dimethyl-3-isoxazolyl)-2'-fluor-5-methoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-5-methoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-2'-(ethoxymethyl)-5-methoxy[1,1'-biphenyl] 2-sulfonamid,
4'-[(2-Butyl-4-oxo-1,3-diazaspiro[4.4]non-1-en-3-yl)methyl]-N-(4,5-dimethyl-3-isoxazolyl)-3'-fluor-4,5-dimethoxy[1,1'-biphenyl]-2-sulfonamid,
4'-[(2-Butyl-4-oxo-1,3-diazaspiro[4,4]non-1-en-3-yl)methyl]-5'-chlor-N-(4,5-dimethyl-3-isoxazolyl)-2'-fluor-4,5-dimethoxy[1,1'-biphenyl]-2-sulfonamid,
4'-[(2-Butyl-4-oxo-1,3-diazaspiro[4.4]non-1-en-3-yl)methyl]-3'-chlor-N-(4,5-dimethyl-3-isoxazolyl)-4,5-dimethoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-4,5-dimethoxy[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-2'-(ethoxymethyl)-4,5-dimethoxy[1,1'-biphenyl]-2-sulfonamid,
4'-[(2-Butyl-4-oxo-1,3-diazaspiro[4.4]non-1-en-3-yl)methyl]-5'-chlor-N-(4,5-dimethyl-3-isoxazolyl)-2'-fluor[1,1'-biphenyl]-2-sulfonamid,
4'-[(2-Butyl-4-oxo-1,3-diazaspiro[4.4]non-1-en-3-yl)methyl]-3'-chlor-N-(4,5-dimethyl-3-isoxazolyl)[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-4-methoxy-3'-methyl[1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-3'-fluor-4-methoxy[ 1,1'-biphenyl]-2-sulfonamid,
4'-[(2-Butyl-4-oxo-1,3-diazaspiro[4.4]non-1-en-3-yl)methyl]-N-(4,5-dimethyl-3-isoxazolyl)-3'-methyl[1,1'-biphenyl]-2-sulfonamid und
4'-[(2-Butyl-4-oxo-1,3-diazaspiro[4.4]non-1-en-3-yl)methyl]-N-(4,5-dimethyl-3-isoxazolyl)-5-fluor[1,1'-biphenyl]-2-sulfonamid.

8. Verbindung nach Anspruch 1, wobei die Verbindung ausgewählt ist aus
N-(4,5-Dimethyl-3-isoxazolyl)-4'-[[(2,6-dimethyl-3-methoxy-4-pyridinyl)oxy]methyl]-3'-fluor[1,1'-biphenyl]-2-sulfonamid,
N-(4,5-Dimethyl-3-isoxazolyl)-3'-fluor-4'-[(1, 4, 5, 6, 7, 8-hexahydro-8-oxo-2-propyl-1-cycloheptimidazolyl)methyl][1,1'-biphenyl]-2-sulfonamid und
N-(4,5-Dimethyl-3-isoxazolyl)-5-fluor-4'-[(1, 4, 5, 6, 7, 8-hexahydro-8-oxo-2-propyl-1-cyloheptimidazolyl)methyl] [1,1'-biphenyl]-2-sulfonamid.

9. Verwendung einer Verbindung wie in einem der Ansprüche 1 bis 8 definiert, für die Herstellung eines Arzneimittels zur Behandlung einer Endothelin-abhängigen oder Angiotensin-II-abhängigen Erkrankung (wie Bluthochdruck, Lungenhochdruck, primärer Lungenhochdruck, Low-Renin-Hypertension, Erektionstörung des Mannes, männliche oder weibliche sexuelle Funktionsstörung, Herzversagen, Atherosklerose, Restenose, Endotoxämie, Hemmung von Zellwachstum, Krebs, Migräne, Asthma, Ischämie, Subarachnoidalblutung, gutartige Prostatahypertrophie, renale glomeruläre oder mesangiole Zellerkrankungen, akutes oder chronisches Nierenversagen, chronische obstruktive Lungenerkrankung, mit Prostatakrebs verbundene Schmerzen, Organschäden, die mit zellproliferativen Effekten des Endothelins verbunden sind, allgemeine Morbidität und Mortalität, die mit Endothelin-abhängigen oder Angiotensin-II-abhängigen Erkrankungen in Zusammenhang stehen, diabetische Nephropathie und Demenz).

10. Arzneimittel, umfassend ein pharmazeutisch verträgliches Vehikel oder Verdünnungsmittel und mindestens eine Verbindung nach einem der Ansprüche 1 bis 8.

11. Verbindung nach Anspruch 1, wobei R₁ 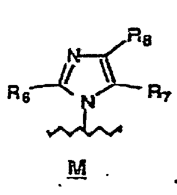 ist.

12. Verbindung nach einem der Ansprüche 2 bis 4, wobei R₂ Wasserstoff, Alkyl, Haloalkyl, Alkoxyalkyl oder Haloalkoxyalkyl ist und R₁₀₁, R₁₀₂, R₁₀₃, R₁₀₄ jeweils unabhängig voneinander Halogen oder Alkyl sind.

13. Verbindung nach einem der Ansprüche 1 bis 5, wobei R₂ -CH₂Y ist.

14. Verbindung nach einem der Ansprüche 1 bis 5 oder 13, wobei Y Q ist.

15. Verbindung nach Anspruch 1, wobei R₃ von Pyridyl verschieden ist, wenn R₁ A ist.

16. Arzneimittel nach Anspruch 10, zusätzlich umfassend mindestens einen ACE-Inhibitor (wie Captopril, Zofenopril, Fosinopril, Ceranapril, Alacepril, Enalapril, Delapril, Pentopril, Quinapril, Ramipril oder Lisinopril) einen Vasopeptidase-Inhibitor (wie Omapatrilat oder Gemopatrilat); einen HMG-CoA-Reduktaseinhibitor (wie Pravastatin, Lovastatin, Atorvastatin, Simvastatin, NK-104 oder ZD-4522), einen Thrombozytenaggregationshemmer (wie Clopidigrel, Ticlopidin, CS-747 oder Aspirin), ein anti-diabetisches Mittel (wie Biguanide oder Biguanid/Glyburidkombinationen), beta-adrenerge Mittel (wie Carvedilol oder Metoprolol) oder Mineralocorticoidrezeptor-Antagonisten (wie Spironolacton oder Eplerenon).

## Revendications

1. Composé de formule I suivante, ou un énantiomère, diastéréomère ou sel de celui-ci : 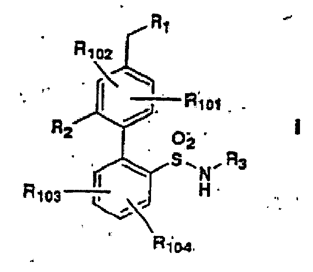 dans laquelle :
R₁ est 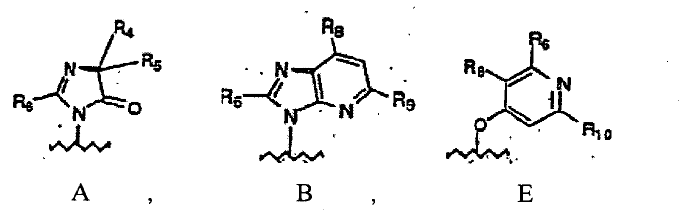 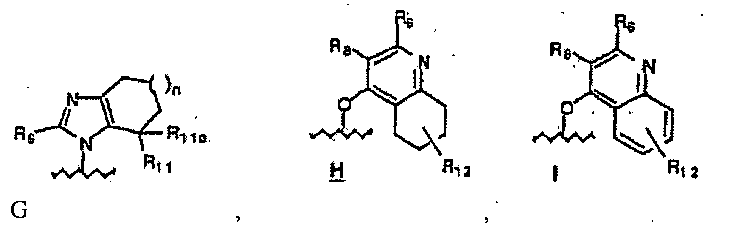 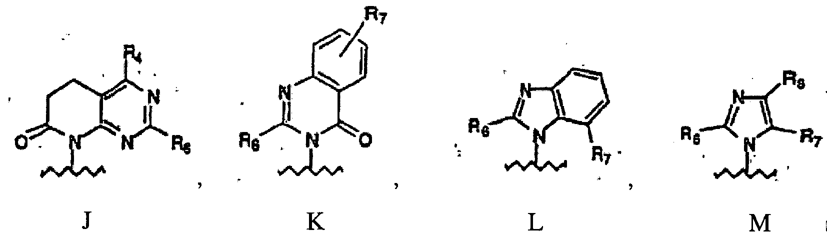 ou 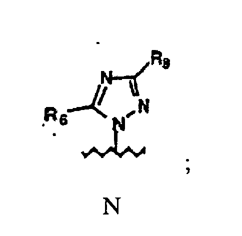
R₂ est hydrogène, halogène, -CHO, alkyle, haloalkyle, (cycloalkyl)alkyle, alkényle, alkynyle, alkoxyalkyle, haloalkoxyalkyle, alkoxy, aryloxy alkoxyalkoxy, cyano, hydroxy, hydroxyalkyle, nitro, -CH(OR₁₃)(OR₁₄), -(CH₂)_{w}Y ;
à condition que lorsque R₁ est B, R₂ n'est pas l'hydrogène, halogène, alkyle, haloalkyle, alkoxy, hydroxyalkyle, nitro, -(CH₂)_{w}NR₁₉R₂₀ ou -NHSO₂R₂₂ ;
R₃ est hétéroaryle ;
R₄ et R₅ sont chacun indépendamment alkyle, hydroxyalkyle, cycloalkyle, cycloalkyle substitué par hydroxy, alkoxyalkyle ou alkoxyalkyle substitué par hydroxy, ou R₄ et R₅ forment ensemble un cycle cyclobutyle, cyclopentyle, cyclohexyle, tétrahydrofuranyle ou tétrahydropyranyle qui peut être éventuellement substitué par un ou plusieurs groupes hydroxy ;
R₆ est alkyle, hydroxyalkyle, haloalkyle, haloalkyle substitué par hydroxy, cycloalkyle, cycloalkyle substitué par hydroxy, (cycloalkyl)alkyle, (cycloalkyl)alkyle substitué par hydroxy, aralkyle, alkoxy, alkoxy substitué par hydroxy, alkoxyalkyle, alkoxyalkyle substitué par hydroxy ou -NR₁₆R₁₇ ;
R₇ est -(CH₂)_{w}-CO₂R₁₅, -(CH₂)_{w}-(C=O)NR₁₆R₁₇, -(CH₂)_{w}-NR₁₅(C=O)NR₁₆R₁₇, -(CH₂)_{w-}CH₂OH, -(CH₂)_{w}-(C=O)R₁₅, tétrazolyle, oxadiazolyle ou triazolyle, ledit tétrazolyle, oxadiazolyle ou triazolyle pouvant être éventuellement substitué par hydrogène, alkyle, hydroxy ou halogène ;
R₈, R₉, R₉ₐ, R₁₀ et R₁₂ sont chacun indépendamment hydrogène, halogène, alkyle, hydroxyalkyle, cycloalkyle, (cycloalkyl)alkyle, aryle, hétéroaryle, arylalkyle, alkylthioalkyle, alkoxy ou alkoxyalkyle, ou R₉ et R₉ₐ forment ensemble avec le carbone auquel ils sont attachés un cycle cycloalkyle ;
R₁₁ et R₁₁ₐ sont chacun indépendamment hydrogène, alkoxy ou forment ensemble un carbonyle ;
R₁₈ et R₁₄ sont alkyle ou forment ensemble un cycle comprenant 5 à 6 chaînons ;
R₁₅, R₁₆ et R₁₇ sont indépendamment hydrogène, alkyle, hydroxyalkyle, cycloalkyle, (cycloalkyl)alkyle, alkoxyalkyle, aralkyle, hétérocycloalkyle, aryle, hétéroaryle ou -(CH₂)_{w}Q, ou R₁₆ et R₁₇ peuvent former ensemble un cycle hétérocyclique comprenant 4 à 6 chaînons;
n est 1 ou 2 ;
w est 0, 1 ou 2 ;
Y est hétéroaryle, -COOH, -COOR₁₈, CONR₁₉R₂₀, -NR₁₉R₂₀, -NR₁₉-OR₂₀, -NR₂₁(C=O)R₂₂, -NR₂₁(C=O)NR₁₉R₂₀, -N(R₁₉)-(alk)-NR₂₁(C=O)R₂₂, -NR₂₁(C=O)OR₁₈, -NR₂₁SO₂R₂₂, -SO₂R₂₂, Q, R ou S;
Q est 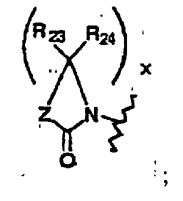
R est 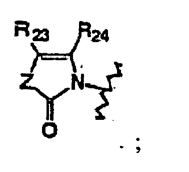
S est 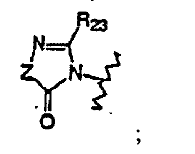
R₁₈, R₁₉, R₂₀, R₂₁ et R₂₂ sont chacun indépendamment hydrogène, alkyle, haloalkyle, alkoxyalkyle, cycloalkyle, alkényle, alkynyle, aryle, aralkyle, hétéroaryle ou R₁₉ et R₂₀ peuvent former ensemble un cycle, hétérocyclique comprenant 4 à 7 chaînons ;
R₂₃ et R₂₄ sont chacun indépendamment hydrogène, alkyle ou cycloalkyle ou peuvent former ensemble un cycle cycloalkyle comprenant 3 à 7 chaînons ;
Z est oxygène, 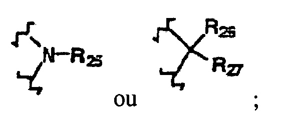
x est 2, 3 ou 4 ;
R₂₅, R₂₆ et R₂₇ sont chacun indépendamment hydrogène, alkyle ou cycloalkyle, ou R₂₆ et R₂₇ peuvent former ensemble un cycle cycloalkyle comprenant 3 à 7 chaînons ;
R₁₀₁, R₁₀₂, R₁₀₃ et R₁₀₄ sont chacun indépendamment hydrogène, halogène, -CHO, alkyle, haloalkyle, (cycloalkyl)alkyle, alkényle, alkynyle, alkoxyalkyle, haloalkoxyalkyle, alkoxy, alkoxyalkoxy, cyano, hydroxy, hydroxyalkyle, nitro, -CH(OR₁₃)(OR₁₄), ou -(CH₂)_{w}Y, à condition qu'au moins un de R₁₀₁, R₁₀₂, R₁₀₃ et R₁₀₄ soit différent de hydrogène ;
dans laquelle lesdits cycles ; aryle seul ou en tant que partie d'un autre groupe ; ou hétéroaryle seul ou en tant que partie d'un autre groupe peuvent être chacun éventuellement substitués par un ou plusieurs groupes hydrogène, halogène, cyano, alkyle, hydroxyalkyle, alkoxy, nitro ou trifluorométhyle ;
à condition que lorsque R₁ est A, ledit composé est différent de 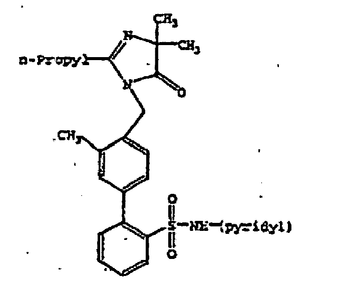 dans laquelle
les termes « alk » ou « alkyle » désignent des groupes hydrocarbonés à chaînes linéaires ou ramifiées ayant de 1 à 12 atomes de carbone,
le terme « alkényl » désigne des groupes hydrocarbonés à chaînes linéaires ou ramifiées ayant de 2 à 12 atomes de carbone et au moins une double liaison carbone-carbone,
le terme « alkynyl » désigne des groupes hydrocarbonés à chaînes linéaires ou ramifiées ayant de 2 à 12 atomes de carbone et au moins une triple liaison carbone-carbone,
le terme « alkoxy » désigne un groupe alkyle lié par un oxygène (-O-),
le terme « aryloxy » désigne un groupe aryle lié par un oxygène (-O-),
le terme « thioalkyle » désigne un groupe alkyle lié par un soufre (-S-),
les termes « ar » ou « aryle » désignent phényle, naphtyle et biphényle, les groupes aryle peuvent être éventuellement substitués par un ou plusieurs (tel que 1 à 3) des substituants suivants : groupes hydrogène, halogène, cyano, alkyle, alkoxy, nitro ou trifluorométhyle,
le terme « cycloalkyle » désigne des groupes hydrocarbonés cycliques totalement saturés ayant 3 à 8 atomes de carbone dans le cycle,
haloalkyle désigne une chaîne alkyle substituée par 1 à 3 halogènes,
le terme « hétéroaryle » désigne furyle, thiényle, pyrrolyle, pyridyle, pyrimidyle, pyrazinyle, pyridazinyle, triazinyle, oxazolyle, isoxazolyle, thiazolyle, isothiazolyle, oxadiazolyle, thiadiazolyle, imidazolyle, triazolyle et tétrazolyle, chacun d'eux pouvant éventuellement être substitué, quand cela est approprié, par un ou plusieurs (tel que 1 à 3) des suivants : hydrogène, halogène, cyano, alkyle, hydroxyalkyle, alkoxy, nitro ou trifluorométhyle,
les terme « hétérocyclique » ou «hétérocyclo» désignent des groupes cycliques non-aromatiques, éventuellement substitués, systèmes cycliques monocycliques comprenant 4 à 7 chaînons, bicycliques comprenant 7 à 11 chaînons ou tricycliques comprenant 10 à 15 chaînons, qui ont au moins un hétéroatome dans au moins un cycle contenant des atomes de carbone,
dans laquelle les groupes monocycliques, hétérocycliques comprennent azétidinyle, pyrrolidinyle, oxétanyle, imidazolinyle, oxazolidinyle, isoxazolinyle, thiazolidinyle, isothiazolidinyle, tétrahydrofuranyle, pipéridinyle, pipérazinyle, 2-oxopipérazinyle, 2-oxopipéridinyle, 2-oxopyrrolodinyle, 2-oxoazépinyle, azépinyle, 4-pipéridonyle, tétrahydropyranyle, morpholinyle, thiamorpholinyle, thiamorpholinyle sulfoxyde, thiamorpholinyle sulfone, 1,3-dioxolane et tétrahydro-1,1-dioxothiényle,
le terme « cycle » englobe homocyclique (c'est à dire, tel qu'utilisé ici, tous les atomes du cycle sont des carbones) ou « hétérocyclique (c'est à dire, tel qu'utilisé ici, les atomes du cycle comprennent le carbone et 1 à 4 hétéroatomes choisis parmi N, O et/ou S, également désigné par hétérocyclo), où, tel qu'utilisé ici, chacun d'eux (homocyclique ou hétérocyclique) peut être saturé ou partiellement ou complètement insaturé (comme hétéroaryle), et chacun d'eux (homocyclique ou hétérocyclique) peut être éventuellement substitué par un ou plusieurs (tel que 1 à 3) groupes hydrogène, halogène, cyano, alkyle, alkoxy, nitro ou trifluorométhyle.

2. Composé selon la revendication 1, dans lequel R₁ est

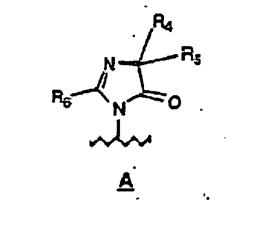

3. Composé selon la revendication 1, dans lequel R₁ est

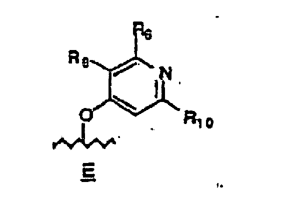

4. Composé selon la revendication 1, dans lequel R₁ est

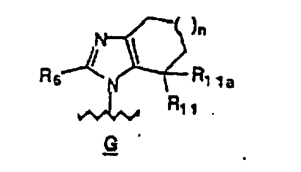

5. Composé selon la revendication 1, dans lequel R₂ est alkoxyalkyle, haloalkyle ou haloalkoxyalkyle.

6. Composé selon l'une quelconque des revendications 1 à 5, dans lequel R₃ est isoxazol-5-yle ou isoxazol-3-yle indépendamment substitué par deux substituants choisis parmi alkyle ou halogène.

7. Composé selon la revendication 1, ledit composé étant choisi parmi :
4'-[(2-butyl-4-oxo-1,3-diazaspiro[4,4]non-1-èn-3-yl)méthyl]-3'-chloro-N-(4,5-diméthyl-3-isoxazolyl)-5-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-3'-fluoro-4-méthoxy[1,1'-biphényl]-2-sulfonamide,
4'-[(2-butyl-4-oxo-1,3-diazaspiro[4,4]non-1-èn-3-yl)méthyl]-5'-chloro-N-(4,5-diméthyl-3-isoxazolyl)-2'-fluoro-4-méthoxy[1,1'-biphényl]-2-sulfonamide,
4'-[(2-butyl-4-oxo-1,3-diazaspiro[4,4]non-1-èn-3-yl)méthyl]-3'-chloro-N-(4,5-diméthyl-3-isoxazolyl)-4-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-4-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-2'-(éthoxyméthyl)-4-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-3'-fluoro-5-méthoxy[1,1'-biphényl]-2-sulfonamide,
4'-[(2-butyl-4-oxo-1,3-diazaspiro[4,4]non-1-èn-3-yl)méthyl]-5'-chloro-N-(4,5-diméthyl-3-isoxazolyl)-2'-fluoro-5-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-5-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-2'-(éthoxyméthyl)-5-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-3'-fluoro-4,5-diméthoxy[1,1'-biphényl]-2-sulfonamide,
4'-[(2-butyl-4-oxo-1,3-diazaspiro[4,4]non-1-èn-3-yl)méthyl]-5'-chloro-N-(4,5-diméthyl-3-isoxazolyl)-2'-fluoro-4,5-diméthoxy[1,1'-biphényl]-2-sulfonamide,
4'-[(2-butyl-4-oxo-1,3-diazaspiro[4,4]non-1-èn-3-yl)méthyl]-3'-chloro-N-(4,5-diméthyl-3-isoxazolyl)-4,5-diméthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-4,5-diméthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-2' -(éthoxyméthyl)-4,5-diméthoxy[1,1' -biphényl]-2-sulfonamide,
4'-[(2-butyl-4-oxo-1,3-diazaspiro[4,4]non-1-èn-3-yl)méthyl]-5'-chloro-N-(4,5-diméthyl-3-isoxazolyl)-2'-fluoro[1,1'-biphényl]-2-sulfonamide,
4'-[(2-butyl-4-oxo-1,3-diazaspiro[4,4]non-1-èn-3-yl)méthyl]-3'-chloro-N-(4,5-diméthyl-3-isoxazolyl)[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-4-méthoxy-3'-méthyl[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-3'-fluoro-4-méthoxy[1,1'-biphényl]-2-sulfonamide,
4'-[(2-butyl-4-oxo-1,3-diazaspiro[4,4]non-1-èn-3-yl)méthyl]-N-(4,5-diméthyl-3-isoxazolyl)-3'-méthyl[1,1'-biphényl]-2-sulfonamide, et
4'-[(2-butyl-4-oxo-1,3-diazaspiro[4,4]non-1-èn-3-yl)méthyl]-N-(4,5-diméthyl-3-isoxazolyl)-5-fluoro[1,1'-biphényl]-2-sulfonamide.

8. Composé selon la revendication 1, ledit composé étant choisi parmi :
N-(4,5-diméthyl-3-isoxazolyl)-4'-[[(2,6-diméthyl-3-méthoxy-4-pyridinyl)oxy]méthyl]-3'-fluoro[1,1'-biphényl]-2-sulfonamide,
N-(4,5-diméthyl-3-isoxazolyl)-3'-fluoro-4'-[(1,4,5,6,7,8-hexahydro-8-oxo-2-propyl-1-cycloheptimidazolyl)méthyl][1,1'-biphényl]-2-sulfonamide, et
N-(4,5-diméthyl-3-isoxazolyl)-5-fluoro-4'-[(1,4,5,6,7,8-hexahydro-8-oxo-2-propyl-1-cycloheptimidazolyl)méthyl] [1,1'-biphényl]-2-sulfonamide.

9. Utilisation d'un composé tel que défini dans l'une quelconque des revendications 1 à 8 pour la préparation d'une composition pharmaceutique destinée au traitement d'un désordre dépendant de l'endothéline ou dépendant de l'angiotensine II (tel que hypertension, hypertension pulmonaire, hypertension pulmonaire primaire, hypertension à rénine basse, dysfonctionnement érectile, dysfonctionnement sexuel mâle ou femelle, insuffisance cardiaque, athérosclérose, resténose, endotoxémie, inhibition de la croissance cellulaire, cancer, migraine, asthme, ischémie, hémorragie méningée, hypertrophie prostatique bénigne, désordres de cellules mésangiales ou glomérulaires rénales, insuffisance rénale aiguë ou chronique, maladie pulmonaire obstructive chronique, douleur associée au cancer de la prostate, dommage aux organes associés aux effets de l'endothéline sur la prolifération cellulaire, morbidité et mortalité associées à des désordres dépendants de l'endothéline ou dépendants de l'angiotensine II, néphropathie diabétique et démence).

10. Composition pharmaceutique, comprenant un véhicule acceptable sur le plan pharmaceutique ou diluant et au moins un composé de l'une quelconque des revendications 1 à 8.

11. Composé selon la revendication 1, dans lequel R₁ est

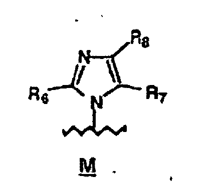

12. Composé selon l'une quelconque des revendications 2 à 4, dans lequel R₂ est hydrogène, alkyle, haloalkyle, alkoxyalkyle ou haloalkoxyalkyle et R₁₀₁, R₁₀₂, R₁₀₃, R₁₀₄ sont chacun indépendamment halogène ou alkyle.

13. Composé selon l'une quelconque des revendications 1 à 5, dans lequel R₂ est -CH₂Y.

14. Composé selon l'une quelconque des revendications 1 à 5 ou 13, dans lequel Y est Q.

15. Composé selon la revendication 1, dans lequel R₃ est différent de pyridyl lorsque R₁ est A.

16. Composition pharmaceutique selon la revendication 10, comprenant en outre au moins un inhibiteur d'ACE (tel que captopril, zofénopril, fosinopril, céranapril, alacépril, énalapril, délapril, pentopril, quinapril, ramipril ou lisinopril), inhibiteur de vasopeptidase (tel que omapatrilat ou gémopatrilat), inhibiteur de HMG CoA réductase (tel que pravastatine, lovastatine, atorvastatine, simvastatine, NK-104 ou ZD-4522), agent antiplaquettaire (tel que clopidigrel, ticlopidine, CS-747 ou aspirine), agent antidiabétique (tel que biguanides ou combinaisons biguanide/glyburide), agent bêta-adrénergique (tel que carvedilol ou métoprolol) ou antagoniste de récepteur minéralocorticoïde (tel que spironolactone ou éplérénone).
